# EUROPÄISCHE PATENTSCHRIFT

(11) **EP 2 697 191 B1**
(45) Veröffentlichungstag und Bekanntmachung des Hinweises auf die Patenterteilung: **18.11.2015**
(21) Anmeldenummer: 12711861.0
(22) Anmeldetag: 28.03.2012
(51) Int. Cl.: C07C 233/55, A61P 9/00, A61K 31/195, A61K 45/06, A61K 31/196, C07C 235/38

(54) **VERZWEIGTE 3-PHENYLPROPIONSÄURE-DERIVATE UND IHRE VERWENDUNG**
BRANCHED 3-PHENYLPROPIONIC ACID DERIVATIVES AND THE USE THEREOF
DÉRIVÉS RAMIFIÉS DE L'ACIDE 3-PHÉNYLPROPIONIQUE ET LEUR UTILISATION

(30) Priorität: 13.04.2011 DE 102011007272
(43) Veröffentlichungstag der Anmeldung: 19.02.2014
(73) Patentinhaber: Bayer Intellectual Property GmbH, 40789 Monheim (DE)
(72) Erfinder: HAHN, Michael, 40764 Langenfeld (DE); LAMPE, Thomas, 40545 Düsseldorf (DE); STASCH, Johannes-Peter, 42651 Solingen (DE); SCHLEMMER, Karl-Heinz, 42113 Wuppertal (DE); WUNDER, Frank, 42117 Wuppertal (DE); LI, Volkhart, Min-Jian, 42553 Velbert (DE); BECKER-PELSTER, Eva-Maria, 42327 Wuppertal (DE); STOLL, Friedericke, 40215 Düsseldorf (DE); KNORR, Andreas, 40699 Erkrath (DE); WOLTERING, Elisabeth, 40723 Hilden (DE)
(74) Vertreter: BIP Patents
(86) Internationale Anmeldenummer: PCT/EP2012/055474
(87) Internationale Veröffentlichungsnummer: WO 2012/139888

(56) Entgegenhaltungen:
- WO-A1-2009/127338
- WO-A1-2011/051165

## Beschreibung

Die vorliegende Anmeldung betrifft neue, in 3-Position einen verzweigten oder cyclischen Alkylsubstituenten tragende 3-Phenylpropionsäure-Derivate, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu.erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise [O.V. Evgenov et al., Nature Rev. Drug Disc. 5 (2006), 755].

In den letzten Jahren wurden Substanzen identifiziert, die die lösliche Guanylatcyclase direkt, d.h. ohne vorherige Freisetzung von NO stimulieren. Mit dem Indazolderivat DC-1 wurde erstmals ein NO-unabhängiger, jedoch Häm-abhängiger Stimulator der sGC beschrieben [Evgenov et al., *ibid*.]. Ausgehend von YC-1 wurden weitere Substanzen gefunden, welche eine höhere Potenz als YC-1 besitzen und keine relevante Hemmung von Phosphodiesterasen (PDE) aufweisen. Dies führte zur Identifizierung der Pyrazolopyridin-Derivate BAY 41-2272, BAY 41-8543 und BAY 63-2521. Diese Verbindungen bilden gemeinsam mit den kürzlich publizierten, strukturell diversen Substanzen CMF-1571 und A-350619 die neue Klasse der sGC-Stimulatoren [Evgenov et al., *ibid*.]*.* Gemeinsames Charakteristikum dieser Substanzklasse ist eine NO-unabhängige und selektive Aktivierung der hämhaltigen sGC. Darüber hinaus zeigen die sGC-Stimulatoren in Kombination mit NO einen synergistischen Effekt auf die sGC-Aktivierung, welcher auf einer Stabilisierung des Nitrosyl-Häm-Komplexes basiert. Die genaue Bindungsstelle der sGC-Stimulatoren an der sGC ist bis heute Gegenstand der Diskussion. Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten Stimulatoren stimulierbar [Evgenov et al., *ibid.].*

Darüber hinaus wurden NO- und Häm-unabhängige sGC-Aktivatoren, mit BAY 58-2667 als Prototyp dieser Klasse, identifiziert. Gemeinsame Charakteristika dieser Substanzen sind, dass sie in Kombination mit NO nur einen additiven Effekt auf die Enzymaktivierung ausüben, und dass die Aktivierung des oxidierten oder hämfreien Enzyms im Vergleich zum hämhaltigen Enzym deutlich stärker ist [Evgenov et al., *ibid*.; J.P. Stasch et al., Br. J. Pharmacol. 136 (2002), 773; J.P. Stasch et al., J. Clin. Invest. 116 (2006), 2552]. Spektroskopische Untersuchungen lassen erkennen, dass BAY 58-2667 die oxidierte Hämgruppe verdrängt, die durch Schwächung der Eisen-Histidin-Bindung nur schwach an der sGC gebunden ist. Auch wurde gezeigt, dass das charakteristische sGC-Hämbindungsmotiv Tyr-x-Ser-x-Arg sowohl für die Interaktion der negativ geladenen Propionsäuren der Hämgruppe als auch für die Wirkung von BAY 58-2667 zwingend erforderlich ist. Vor diesem Hintergrund wird angenommen, dass die Bindungsstelle von BAY 58-2667 an der sGC identisch zur Bindungsstelle der Hämgruppe ist [J.P. Stasch et al., J. Clin. Invest. 116 (2006), 2552].

Die in der vorliegenden Erfindung beschriebenen Verbindungen sind nun ebenfalls in der Lage, die Häm-freie Form der löslichen Guanylatcyclase zu aktivieren. Dies wird auch dadurch belegt, dass diese neuartigen Aktivatoren einerseits am Häm-haltigen Enzym keine synergistische Wirkung mit NO zeigen und andererseits sich ihre Wirkung nicht durch den Häm-abhängigen Inhibitor der löslichen Guanylatcyclase, 1*H*-1,2,4-Oxadiazolo[4,3-a]chinoxalin-1-on (ODQ), blockieren lässt, sondern durch diesen sogar potenziert wird [vgl. O.V. Evgenov et al., Nature Rev. Drug Disc. 5 (2006), 755; J.P. Stasch et al., J. Clin. Invest. 116 (2006), 2552].

Aufgabe der vorliegenden Erfindung war somit die Bereitstellung neuer Verbindungen, welche in der oben beschriebenen Weise als Aktivatoren der löslichen Guanylatcyclase wirken und als solche insbesondere zur Behandlung und zur Prävention kardiovaskulärer Erkrankungen eingesetzt werden können.

In WO 00/64888-A1, EP 1 216 980-A1, EP 1 285 908-A1, EP 1 348 698-A1, EP 1 375 472-A1, EP 1 452 521-A1, US 2005/0187266-A1 und US 2005/0234066-A1 werden verschiedene Arylalkancarbonsäure-Derivate als PPAR-Agonisten zur Behandlung von Diabetes, Dyslipidämie, Arteriosklerose, Obesitas und anderen Erkrankungen beschrieben. In EP 1 312 601-A1 und EP 1 431 267-A1 werden substituierte Arylalkancarbonsäuren als PGE₂-Rezeptorantagonisten zur Behandlung beispielsweise von Schmerzzuständen, urologischen Erkrankungen, der Alzheimer'schen Krankheit und Krebs offenbart. Weiterhin werden Arylalkancarbonsäuren in WO 2005/ 086661-A2 als GPR40-Modulatoren für die Behandlung von Diabetes und Dyslipidämien beansprucht, und in WO 2004/099170-A2, WO 2006/050097-A1 sowie WO 2006/055625-A2 werden Phenyl-substituierte Carbonsäuren als PTP-1B-Inhibitoren für die Behandlung von Diabetes, Krebs und neurodegenerativen Erkrankungen beschrieben. Ferner sind aus WO 96/12473-A1 und WO 96/30036-A1 einzelne Phenylacetamido-substituierte Phenylalkancarbonsäuren bekannt, die in Form nicht-kovalenter Mischungen die Zuführung von Peptid-Wirkstoffen innerhalb des Körpers verbessern. In WO 2009/067493-A2 werden 3,5-disubstituierte Phenylessigsäure-Derivate zur Behandlung der Alzheimer'schen Krankheit beansprucht. In WO 2009/127338-A1 und WO 2010/ 102717-A1 werden oxo-heterocyclisch substituierte Carbonsäure-Derivate offenbart, die als Aktivatoren der löslichen Guanylatcyclase wirken.

Gegenstand der vorliegenden Erfindung sind Verbindungen der allgemeinen Formel (I) 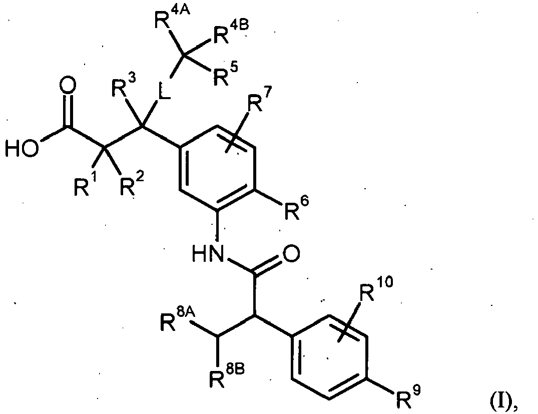 in welcher
- R¹, R² und R³: unabhängig voneinander für Wasserstoff oder Methyl stehen,
- L: für eine Bindung oder für -CH₂- steht,
- R^{4A} und R^{4B}: unabhängig voneinander für Methyl, Trifluormethyl oder Ethyl stehen
oder
- R^{4A} und R^{4B}: miteinander verknüpft sind und gemeinsam mit dem Kohlenstoffatom, an das sie gebunden sind, einen Cyclopropyl- oder Cyclobutyl-Ring bilden, der bis zu zweifach mit Fluor substituiert sein kann,
- R⁵: für Wasserstoff, Fluor, Methyl oder Methoxy steht,
- R⁶: für Wasserstoff, Fluor, Chlor, Brom, Cyano, Methyl, Trifluormethyl, Ethyl, Methoxy oder Trifluormethoxy steht,
- R⁷: für Wasserstoff, Fluor, Chlor oder Methyl steht,
- R^{8A}: für Methyl oder Ethyl steht,
- R^{8B}: für Trifluormethyl steht,
oder
- R^{8A} und R^{8B}: miteinander verknüpft sind und gemeinsam mit dem Kohlenstoffatom, an das sie gebunden sind, einen optional difluor-substituierten Cyclopentyl-Ring der Formel 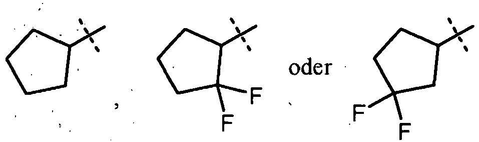 bilden,
- R⁹: für Fluor, Chlor, Brom, Cyano, (C₁-C₄)-Alkyl, (C₂-C₄)-Alkenyl, Cyclopropyl oder Cyclobutyl steht, wobei
(C₁-C₄)-Alkyl und (C₂-C₄)-Alkenyl bis zu dreifach mit Fluor
und
Cyclopropyl und Cyclobutyl bis zu zweifach mit Fluor substituiert sein können,
und
- R¹⁰: für Wasserstoff, Fluor, Chlor, Methyl, Trifluormethyl, Ethyl oder Methoxy steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen insbesondere die Salze üblicher Basen, wie beispielhaft und vorzugsweise Alkalimetall-Salze (z.B. Natrium- und Kaliumsalze), Erdalkali-Salze (z.B. Calcium- und Magnesiumsalze) und Ammonium-Salze, abgeleitet von Ammoniak oder organischen Aminen mit 1 bis 16 C-Atomen, wie beispielhaft und vorzugsweise Ethylamin, Diethylamin, Triethylamin, *N,N*-Diisopropylethylamin, Monoethanolamin, Diethanolamin, Triethanolamin, Dimethylaminoethanol, Diethylaminoethanol, Procain, Dicyclohexylamin, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Außerdem offenbart sind auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper auf beispielsweise metabolischem oder hydrolytischem Wege zu erfindungsgemäßen Verbindungen umgesetzt werden.

Insbesondere offenbart sind als Prodrugs hydrolysierbare Ester-Derivate der erfindungsgemäßen Carbonsäuren der Formel (I). Hierunter werden Ester verstanden, die in physiologischen Medien, unter den Bedingungen der im weiteren beschriebenen biologischen Tests und insbesondere *in vivo* auf enzymatischem oder chemischem Wege zu den freien Carbonsäuren, als den biologisch hauptsächlich aktiven Verbindungen, hydrolysiert werden können. Als solche Ester werden (C₁-C₄)-Alkylester, in welchen die Alkylgruppe geradkettig oder verzweigt sein kann, bevorzugt. Besonders bevorzugt sind Methyl-, Ethyl- oder *tert*.-Butylester.

Im Rahmen der vorliegenden Erfindung haben die Substituenten, soweit nicht anders spezifiziert, die folgende Bedeutung:
(C₁-C₄)-Alkyl steht im Rahmen der Erfindung für einen geradkettigen oder verzweigten Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl und *tert*.-Butyl*.*
(C₂-C₄)-Alkenyl und (C₂-C₃)-Alkenyl stehen im Rahmen der Erfindung für einen geradkettigen oder verzweigten Alkenylrest mit einer Doppelbindung und 2 bis 4 bzw. 2 oder 3 Kohlenstoffatomen. Bevorzugt ist ein geradkettiger oder verzweigter Alkenylrest mit 2 oder 3 Kohlenstoffatomen. Beispielhaft und vorzugsweise seien genannt: Vinyl, Allyl, *n*-Prop-1-en-1-yl, Isopropenyl, *n*-But-1-en-1-yl, *n*-But-2-en-1-yl, *n*-But-3-en-1-yl, 2-Methylprop-1-en-1-yl und 2-Methylprop-2-en-1-yl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder zwei oder drei gleichen oder verschiedenen Substituenten ist bevorzugt. Besonders bevorzugt ist die Substitution mit einem oder zwei gleichen oder verschiedenen Substituenten.

Bevorzugt im Rahmen der vorliegenden Erfindung sind Verbindungen der Formel (I), in welcher
- R¹: für Wasserstoff oder Methyl steht,
- R²: für Wasserstoff steht,
- R³: für Wasserstoff oder Methyl steht,
- L: für eine Bindung oder für -CH₂- steht,
- R^{4A} und R^{4B}: beide für Methyl stehen oder miteinander verknüpft sind und gemeinsam mit dem Kohlenstoffatom, an das sie gebunden sind, einen Cyclopropyl- oder Cyclobutyl-Ring bilden, der bis zu zweifach mit Fluor substituiert sein kann,
- R⁵: für Wasserstoff, Fluor, Methyl oder Methoxy steht,
- R⁶: für Fluor, Chlor, Methyl oder Ethyl steht,
- R⁷: für Wasserstoff oder Fluor steht,
- R^{8A}: für Methyl steht,
- R^{8B}: für Trifluormethyl steht,
oder
- R^{8A} und R^{8B}: miteinander verknüpft sind und gemeinsam mit dem Kohlenstoffatom, an das sie gebunden sind, einen difluor-substituierten Cyclopentyl-Ring der Formel 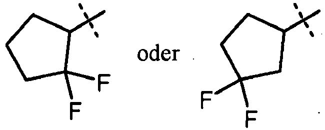 bilden,
- R⁹: für Fluor, Chlor, (C₁-C₄)-Alkyl, (C₂-C₃)-Alkenyl, Cyclopropyl oder Cyclobutyl steht, wobei
(C₁-C₄)-Alkyl und (C₂-C₃)-Alkenyl bis zu dreifach mit Fluor
und
Cyclopropyl und Cyclobutyl bis zu zweifach mit Fluor substituiert sein können,
und
- R¹⁰: für Wasserstoff, Fluor, Chlor, Methyl oder Methoxy steht,
sowie ihre Salze, Solvate und Solvate der Salze.

Eine besondere Ausführungsform der vorliegenden Erfindung umfasst Verbindungen der Formel (I), in welcher
- R¹ und R²: beide für Wasserstoff stehen,
sowie ihre Salze, Solvate und Solvate der Salze.

Eine weitere besondere Ausführungsform der vorliegenden Erfindung umfasst Verbindungen der Formel (I), in welcher
- R³: für Wasserstoff oder Methyl steht
und
- L: für eine Bindung steht,
sowie ihre Salze, Solvate und Solvate der Salze.

Eine weitere besondere Ausführungsform der vorliegenden Erfindung umfasst Verbindungen der Formel (I), in welcher
- R³: für Wasserstoff steht
und
- L: für -CH₂- steht,
sowie ihre Salze, Solvate und Solvate der Salze.

Eine weitere besondere Ausuhrungsform der vorliegenden Erfindung umfasst Verbindungen der Formel (I), in welcher
- R^{4A} und R^{4B}: beide für Methyl stehen
und
- R⁵: für Wasserstoff steht,
sowie ihre Salze, Solvate und Solvate der Salze.

Eine weitere besondere Ausführungsform der vorliegenden Erfindung umfasst Verbindungen der Formel (I), in welcher
- R^{4A} und R^{4B}: miteinander verknüpft sind und gemeinsam mit dem Kohlenstoffatom, an das sie gebunden sind, einen Cyclopropyl- oder Cyclobutyl-Ring bilden, der bis zu zweifach mit Fluor substituiert sein kann,
und
- R⁵: für Wasserstoff, Fluor oder Methyl steht,
sowie ihre Salze, Solvate und Solvate der Salze.

Eine weitere besondere Ausführungsform der vorliegenden Erfindung umfasst Verbindungen der Formel (I), in welcher
- R⁶: für Chlor steht
und
- R⁷: für Wasserstoff steht,
sowie ihre Salze, Solvate und Solvate der Salze.

Eine weitere besondere Ausuhrungsform der vorliegenden Erfindung umfasst Verbindungen der Formel (I), in welcher
- R^{8A}: für Methyl steht
und
- R^{8B}: für Trifluormethyl steht,
sowie ihre Salze, Solvate und Solvate der Salze.

Eine weitere besondere Ausführungsform der vorliegenden Erfindung umfasst Verbindungen der Formel (I), in welcher
- R^{8A} und R^{8B}: miteinander verknüpft sind und gemeinsam mit dem Kohlenstoffatom, an das sie gebunden sind, einen difluor-substituierten Cyclopentyl-Ring der Formel 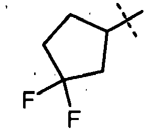 bilden,
sowie ihre Salze, Solvate und Solvate der Salze.

Eine weitere besondere Ausführungsform der vorliegenden Erfindung umfasst Verbindungen der Formel (I), in welcher
- R⁹: für Fluor, Chlor, (C₁-C₄)-Alkyl oder Cyclopropyl steht, wobei (C₁-C₄)-Alkyl bis zu dreifach mit Fluor und Cyclopropyl bis zu zweifach mit Fluor substituiert sein können,
sowie ihre Salze, Solvate und Solvate der Salze.

Eine weitere besondere Ausführungsform der vorliegenden Erfindung umfasst Verbindungen der Formel (I), in welcher
- R¹⁰: für Wasserstoff, Fluor, Chlor, Methyl oder Methoxy steht,
sowie ihre Salze, Solvate und Solvate der Salze.

Besonders bevorzugt im Rahmen der vorliegenden Erfindung sind Verbindungen der Formel (I), in welcher
- R¹ und R²: beide für Wasserstoff stehen,
- R³: für Wasserstoff oder Methyl steht,
- L: für eine Bindung oder für -CH₂- steht,
- R^{4A} und R^{4B}: beide für Methyl stehen oder miteinander verknüpft sind und gemeinsam mit dem Kohlenstoffatom, an das sie gebunden sind, einen Cyclopropyl- oder Cyclobutyl-Ring bilden, der bis zu zweifach mit Fluor substituiert sein kann,
- R⁵: für Wasserstoff, Fluor oder Methyl steht,
- R⁶: für Chlor steht,
- R⁷: für Wasserstoff steht,
- R^{8A}: für Methyl steht,
- R^{8B}: für Trifluormethyl steht,
- R⁹: für Fluor, Chlor, Methyl, Trifluormethyl, Ethyl, 2,2,2-Trifluorethyl, Isopropyl, *tert*.-Butyl, Cyclopropyl oder 2,2-Difluorcyclopropyl steht,
und
- R¹⁰: für Wasserstoff, Fluor, Methyl oder Methoxy steht,
sowie ihre Salze, Solvate und Solvate der Salze.

Von besonderer Bedeutung im Rahmen der vorliegenden Erfindung sind Verbindungen der Formel (I-A) 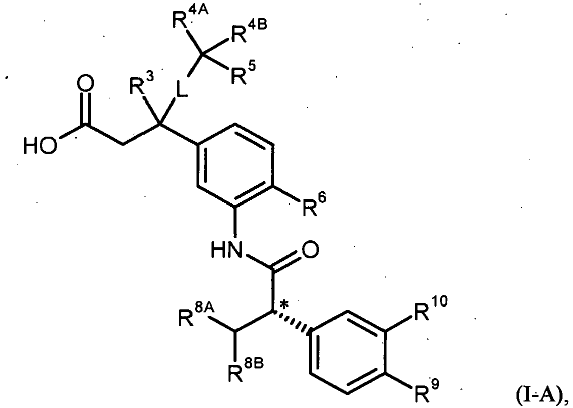 in welcher das mit * gekennzeichnete C-Atom der Phenylacetamid-Gruppierung die abgebildete *S*-Konfiguration aufweist
und
die Reste R³, R^{4A}, R^{4B}, R⁵, R⁶, R^{8A}, R^{8B}, R⁹ und R¹⁰ sowie L jeweils die oben angegebenen Bedeutungen haben,
sowie ihre Salze, Solvate und Solvate der Salze.

Die in den jeweiligen Kombinationen bzw. bevorzugten Kombinationen von Resten im einzelnen angegebenen Reste-Defintionen werden unabhängig von den jeweiligen angegebenen Kombinationen der Reste beliebig auch durch Reste-Definitionen anderer Kombinationen ersetzt. Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Carbonsäure der Formel (II) 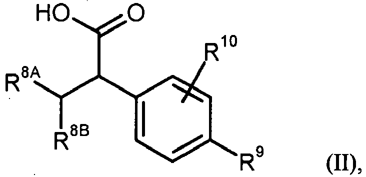 in welcher R^{8A}, R^{8B}, R⁹ und R¹⁰ die oben angegebenen Bedeutungen haben,
in einem inerten Lösungsmittel mit Hilfe eines Kondensationsmittels oder über die Zwischenstufe des entsprechenden Carbonsäurechlorids in Gegenwart einer Base mit einem Amin der Formel (III) 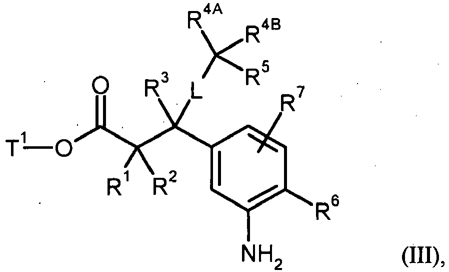 in welcher L, R¹, R², R³, R^{4A}, R^{4B}, R⁵, R⁶ und R⁷ die oben angegebenen Bedeutungen haben
und
- T¹: für (C₁-C₄)-Alkyl oder Benzyl steht,
zu einem Carbonsäureamid der Formel (IV) 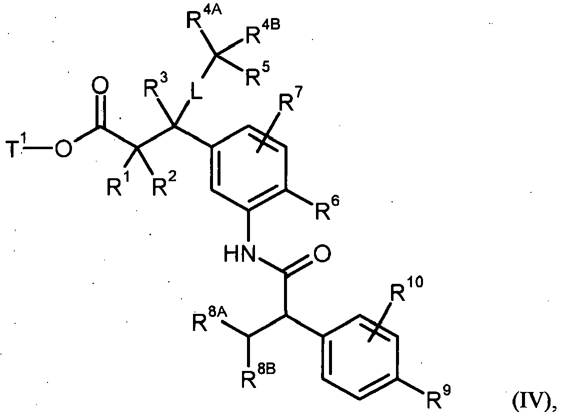 in welcher L, R¹, R², R³, R^{4A}, R^{4B}, R⁵, R⁶, R⁷, R^{8A}, R^{8B}, R⁹ R¹⁰ und T¹ die oben angegebenen Bedeutungen haben,
kuppelt und anschließend den Ester-Rest T¹ durch basische oder saure Solvolyse oder im Fall, dass T¹ für Benzyl steht, auch durch Hydrogenolyse unter Erhalt der Carbonsäure der Formel (I) abspaltet und gegebenenfalls die Verbindungen der Formel (I) nach dem Fachmann bekannten Methoden in ihre Enantiomere und/öder Diastereomere trennt und/oder mit den entsprechenden (i) Lösungsmitteln und/oder (ii) Basen zu ihren Solvaten, Salzen und/oder Solvaten der Salze umsetzt.

Inerte Lösungsmittel für den Verfahrensschritt (II) + (III) → (IV) [Amid-Kupplung] sind beispielsweise Ether wie Diethylether, tert.-Butyl-methylether, Tetrahydrofuran, 1,4-Dioxan, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethän, Trichlorethylen oder Chlorbenzol, oder andere Lösungsmittel wie Aceton, Acetonitril, Ethylacetat, Pyridin, Dimethylsulfoxid (DMSO), *N,N*-Dimethylformamid (DMF), *N,N*'-Dimethylpropylenharnstoff (DMPU) oder *N*-Methylpyrrolidinon (NMP). Ebenso ist es möglich, Gemische der genannten Lösungsmittel einzusetzen. Bevorzugt werden Dichlormethan, Tetrahydrofuran, Dimethylformamid oder Gemische dieser Lösungsmittel verwendet.

Als Kondensationsmittel für diese Kupplungsreaktion eignen sich beispielsweise Carbodiimide wie *N,N*'-Diethyl-, *N*,*N*-Dipropyl-, *N,N*'-Diisopropyl-, *N,N*'-Dicyclohexylcarbodiimid (DCC) oder N-(3-Dimethylaminopropyl)-*N*-ethylcarbodiimid-Hydrochlorid (EDC), Phosgen-Derivate wie *N,N*'-Carbonyldiimidazol (CDI) oder Isobutylchlorformiat, 1,2-Oxazolium-Verbindungen wie 2-Ethyl-5-phenyl-1,2-oxazolium-3-sulfat oder 2-tert.-Butyl-5-methylisoxazolium-perchlorat, Acyl-amino-Verbindungen wie 2-Ethoxy-1-ethoxycarbonyl-1,2-dihydrochinolin, α-Chlorenamine wie 1-Chlor-2-methyl-1-dimethylamino-1-propen, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris-(pyrrolidino)phosphonium-hexafluorophosphat (PyBOP), oder Uronium-Verbindungen wie *O*-(Benzotriazol-1-yl)-*N,N,N*',*N'*-tetramethyluronium-tetrafluoroborat (TBTU), *O*-(Benzotriazol-1-yl)-*N,N,N',N*'-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N*,*N*,*N*',*N'*-tetramethyluronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N-*Hydroxysuccinimid (HOSu), sowie als Basen Alkalicarbonate, z.B. Natrium- oder Kaliumcarbonat, oder tertiäre Aminbasen wie Triethylamin, *N-*Methylmorpholin, *N-*Methylpiperidin, *N,N-*Diisopropylethylamin, Pyridin oder *4 N,N-*Dimethylaminopyridin. Bevorzugt eingesetzt werden *O*-(7-Azabenzotriazol-1-yl)-*N,N,N',N*'-tetramethyluronium-hexafluorophosphat (HATU) in Kombination mit Pyridin oder *N,N*-Diisopropylethylamin, oder *N-*(3-Dimethylaminopropyl)-*N*'-ethylcarbodiimid-Hydrochlorid (EDC) in Verbindung mit 1-Hydroxybenzotriazol (HOBt) und Triethylamin, oder 1-Chlor-2-methyl-1-dimethylamino-1-propen zusammen mit Pyridin.

Die Reaktion (II) + (III) → (IV) wird in der Regel in einem Temperaturbereich von 0°C bis +60°C, bevorzugt bei +10°C bis +40°C durchgeführt.

Bei Einsatz eines der Verbindung (II) entsprechenden Carbonsäurechlorids wird die Kupplung mit der Amin-Komponente (III) in Gegenwart, einer üblichen organischen Hilfsbase wie Triethylamin, *N-*Methylmorpholin, *N*-Methylpiperidin, *N,N*-Diisopropylethylamin, Pyridin, 4-*N,N*-Dimethylaminopyridin, 1,8-Diazabicyclo[5.4.0]undec-7-en (DBU) oder 1,5-Diazabicyclo[4.3.0]non-5-en (DBN) durchgeführt. Bevorzugt wird Triethylamin oder *N*,*N-*Diisopropylethylamin verwendet.

Die Umsetzung des Amins (III) mit dem Carbonsäurechlorid erfolgt im Allgemeinen in einem Temperaturbereich von -20°C bis +60°C, bevorzugt im Bereich von -10°C bis +30°C.

Die Herstellung der Carbonsäurechloride selbst geschieht auf übliche Weise durch Behandlung der Carbonsäure (II) mit Thionylchlorid oder Oxalylchlorid.

Die Abspaltung der Ester-Gruppe T¹ im Verfahrensschritt (IV) → (I) wird nach üblichen Methoden durchgeführt, indem man den Ester in inerten Lösungsmitteln mit Säuren oder Basen behandelt, wobei bei letzterer Variante das zunächst entstehende Salz durch Behandeln mit Säure in die freie Carbonsäure überführt wird. Im Falle der tert.-Butylester erfolgt die Esterspaltung vorzugsweise mit Säuren. Benzylester werden bevorzugt durch Hydrogenolyse (Hydrierung) in Gegenwart eines geeigneten Katalysators, wie beispielsweise Palladium auf Aktivkohle, abgespalten.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, *n-*Propanol, Isopropanol, *n*-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium- oder Kaliumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von -20°C bis +100°C, bevorzugt bei 0°C bis +605°C.

Die Intermediate der Formel (II) können beispielsweise dadurch hergestellt werden, dass man
[A] einen Carbonsäureester der Formel (V) 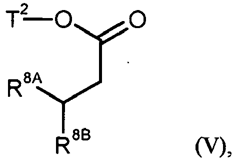 in welcher R^{8A} und R^{8B} die oben angegebenen Bedeutungen haben
   und
   - T²: für (C₁-C₄)-Alkyl oder Benzyl steht,
   in einem inerten Lösungsmittel zunächst mit Hilfe einer Base deprotoniert und dann in Gegenwart eines geeigneten Palladium-Katalysators mit einem Phenylbromid der Formel (VI) 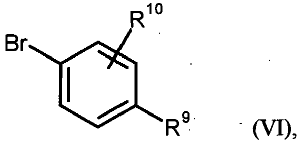 in welcher R⁹ und R¹⁰ die oben angegebenen Bedeutungen haben,
   zu einer Verbindung der Formel (VII) 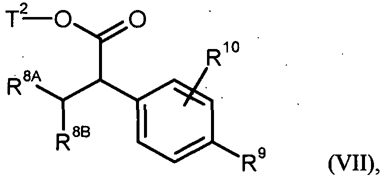 in welcher R^{8A}, R^{8B}, R⁹, R¹⁰ und T² die oben angegebenen Bedeutungen haben,
   aryliert
   oder
[B] einen Phenylessigsäureester der Formel (VIII) 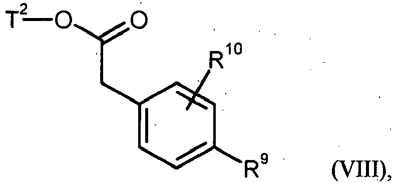 in welcher R⁹ und R¹⁰ die oben angegebenen Bedeutungen haben
   und
   - T²: für (C₁-C₄)-Alkyl oder Benzyl steht,
   in einem inerten Lösungsmittel in Gegenwart einer Base mit einer Verbindung der Formel (IX) 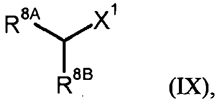 in welcher R^{8A} und R^{8B} die oben angegebenen Bedeutungen haben
   und
   - X¹: für eine geeignete Abgangsgruppe wie beispielsweise Brom oder Iod steht,
   zu der Verbindung der Formel (VII) 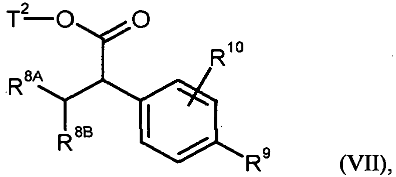 in welcher R^{8A}, R^{8B}, R⁹, R¹⁰ und T² die oben angegebenen Bedeutungen haben,
   alkyliert
und jeweils nachfolgend den Ester-Rest T² durch basische oder saure Solvolyse oder im Fall, dass T² für Benzyl steht, auch durch Hydrogenolyse unter Erhalt der Carbonsäure (II) abspaltet.

Die Arylierungsreaktion im Verfahrensschritt (V) + (VI) → (VII) wird vorzugsweise in Toluol oder Toluol/Tetrahydrofuran-Gemischen in einem Temperaturbereich von +20°C bis +100°C durchgeführt. Als Base zur Deprotonierung des Esters (V) wird hierbei bevorzugt Lithium-bis(trimethylsilyl)amid eingesetzt. Geeignete Palladium-Katalysatoren sind beispielsweise Palladium(II)acetat oder Tris(dibenzylidenaceton)-dipalladium, jeweils in Kombination mit einem elektronenreichen, sterisch anspruchsvollen Phosphin-Liganden wie 2-Dicyclohexylphosphino-2'-(*N,N*-dimethylamino)-biphenyl oder 2-Di-*tert*.-butylphosphino-2'-(*N,N*-dimethylamino)biphenyl [vgl. z.B. W.A. Moradi, S.L. Buchwald, J. Am. Chem. Soc. 123, 7996-8002 (2001)].

Inerte Lösungsmittel für die Alkylierungsreaktion (VIII) + (IX) → (VII) sind beispielsweise Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder dipolar-aprotische Lösungsmittel wie *N,N-*Dimethylformamid (DMF), Dimethylsulfoxid (DMSO), *N,N*'-Dimethylpropylenharnstoff (DMPU) oder *N-*Methylpyrrolidinon (NMP). Ebenso ist es möglich, Gemische der genannten Lösungsmittel einzusetzen. Bevorzugt werden Tetrahydrofuran, Dimethylformamid oder Gemische hiervon verwendet.

Als Base für den Verfahrensschritt (VIII) + (IX) → (VII) eignen sich übliche starke anorganische oder organische Basen. Hierzu gehören insbesondere Alkalialkoholate wie Natrium- oder Kaliummethanolat, Natrium- oder Kaliumethanolat oder Natrium- oder Kalium-*tert*.-butylat, Alkalihydride wie Natrium- oder Kaliumhydrid, oder Amide wie Lithium-, Natrium- oder Kalium-bis(trimethylsilyl)amid oder Lithiumdiisopropylamid. Bevorzugt wird Kalium-*tert*.-butylat, Natriumhydrid oder Lithiumdiisopropylamid verwendet.

Die Umsetzung (VIII) + (IX) → (VII) wird im Allgemeinen in einem Temperaturbereich von -80°C bis +40°C, bevorzugt bei -20°C bis +20°C durchgeführt.

Die Abspaltung der Ester-Gruppe T² im Verfahrensschritt (VII) → (II) erfolgt auf analoge Weise wie zuvor für den Ester-Rest T¹ beschrieben.

### Intermediate der Formel (II-A)

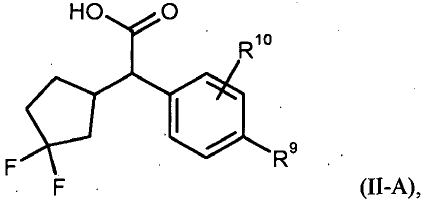

in welcher R⁹ und R¹⁰ die oben angegebenen Bedeutungen haben,
können alternativ auch dadurch hergestellt werden, dass man den Phenylessigsäureester der Formel (VIII) 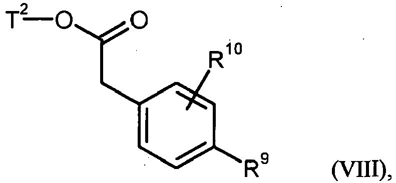 in welcher R⁹, R¹⁰ und T² die oben angegebenen Bedeutungen haben,
zunächst durch baseninduzierte Addition an 2-Cyclopenten-1-on zu einer Verbindung der Formel (X) 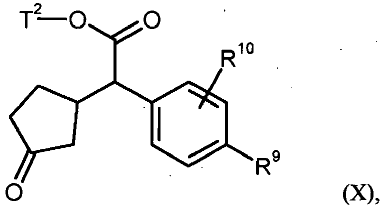 in welcher R⁹, R¹⁰ und T² die oben angegebenen Bedeutungen haben,
umsetzt, diese anschließend mit 1,1'-[(Trifluor-λ⁴-sulfanyl)imino]bis(2-methoxyethan) unter Bortrifluorid-Katalyse zu einer Verbindung der Formel (VII-A) 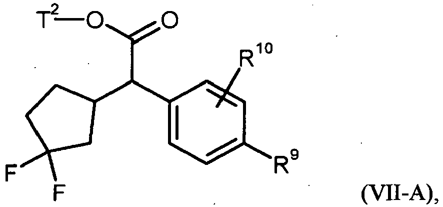 in welcher R⁹, R¹⁰ und T² die oben angegebenen Bedeutungen haben,
fluoriert und nachfolgend wiederum die Ester-Gruppe T² unter Erhalt der Carbonsäure (II-A) abspaltet.

Im Verfahrensschritt (VIII) → (X) wird zur Deprotonierung des Esters (VIII) bevorzugt eine Amid-Base wie Lithiumdiisopropylamid oder Lithium-bis(trimethylsilyl)amid verwendet. Zur Deoxy-Fluorierung in der Transformation (X) → (VII-A) können an Stelle des oben genannten 1,1'-[(Trifluor-λ⁴-sulfanyl)imino]bis(2-methoxyethan) ("Desoxofluor") gegebenenfalls auch andere bekannte Fluorierungsagentien, wie Diethylaminoschwefeltrifluorid (DAST) oder Morpholinoschwefeltrifluorid (Morpho-DAST), eingesetzt werden [zur Reaktionssequenz (VIII) → (X) → (VII-A) vgl. z.B. T. Mase et al., J Org. Chem. 66 (20), 6775-6786 (2001)].

Die Intermediate der Formel (III) können in Abhängigkeit von ihrem Substitutionsmuster beispielsweise dadurch hergestellt werden, dass man entweder

### [C-1] einen Phosphonoessigsäureester der Formel (XI)

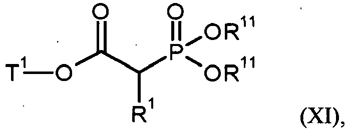

in welcher R¹ und T¹ die oben angegebenen Bedeutungen haben
und
- R¹¹: für (C₁-C₄)-Alkyl steht,
in einem inerten Lösungsmittel in einer Basen-induzierten Olefinierungsreaktion mit einer 3-Nitrobenzoyl-Verbindung der Formel (XII) 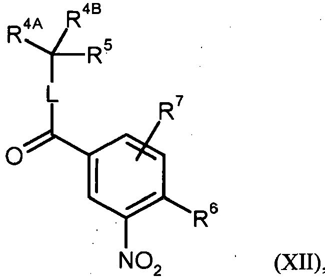 in welcher L, R^{4A}, R^{4B}, R⁵, R⁶ und R⁷die oben angegebenen Bedeutungen haben,
zu einer Verbindung der Formel (XIII) 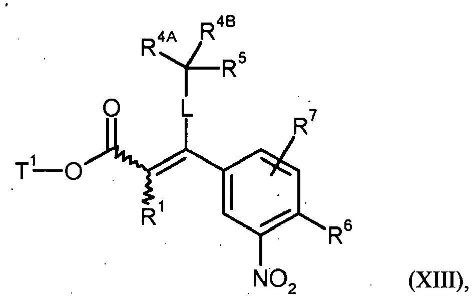 in welcher L, R¹, R^{4A}, R^{4B}, R⁵, R⁶, R⁷ und T¹ die oben angegebenen Bedeutungen haben,
umsetzt und diese dann in Gegenwart eines geeigneten Palladium- oder Platin-Katalysators zu einem 3-(3-Aminophenyl)propionsäureester der Formel (III-A) 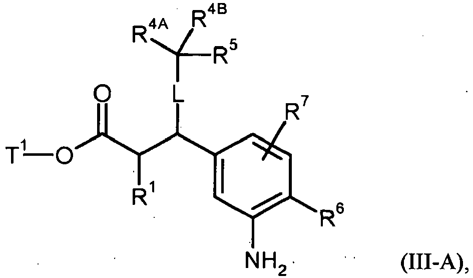 in welcher L, R¹, R^{4A}, R^{4B}, R⁵, R⁶, R⁷ und T¹ die oben angegebenen Bedeutungen haben,
hydriert,
oder

### [C-2] einen Phosphonoessigsäureester der Formel (XI)

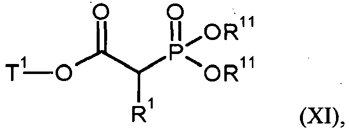

in welcher R¹ und T¹ die oben angegebenen Bedeutungen haben
und
- R¹¹: für (C₁-C₄)-Alkylsteht,
in einem inerten Lösungsmittel in einer Basen-induzierten Olefinierungsreaktion mit einer geschützten 3-Aminobenzoyl-Verbindung der Formel (XIV) 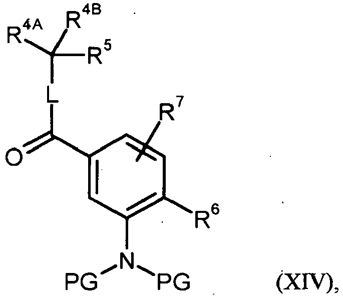 in welcher L, R^{4A}, R^{4B}, R⁵, R⁶ und R⁷ die oben angegebenen Bedeutungen haben
und
- PG: für Benzyl oder 4-Methoxybenzyl als inerte Amino-Schutzgruppe steht,
zu einer Verbindung der Formel (XV) 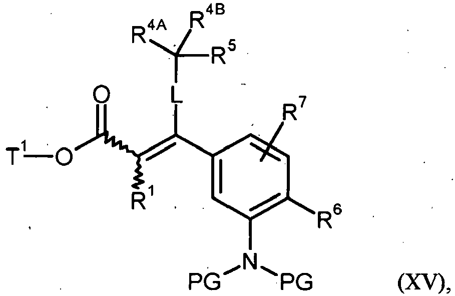 in welcher L, PG, R¹, R^{4A}, R^{4B}, R⁵, R⁶, R⁷ und T¹ die oben angegebenen Bedeutungen haben,
umsetzt, diese dann entweder (i) mit Magnesium in Methanol zu einer Verbindung der Formel (XVI) 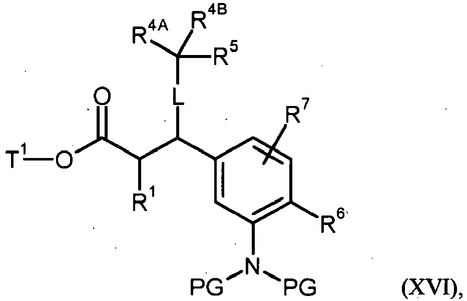 in welcher L, PG, R¹, R^{4A}, R^{4B}, R⁵, R⁶, R⁷ und T¹ die oben angegebenen Bedeutungen haben,
reduziert und anschließend die Amino-Schutzgruppen PG nach üblichen Methoden durch Hydrogenolyse oder auf oxidativem Wege unter Erhalt des 3-(3-Aminophenyl)propionsäure-esters der Formel (III-A) 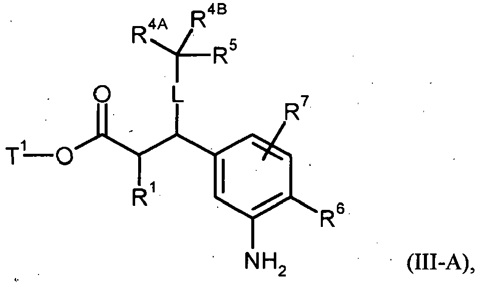 in welcher L, R¹, R^{4A}, R^{4B}, R⁵, R⁶, R⁷ und T¹ die oben angegebenen Bedeutungen haben,
entfernt oder (*ii*) die Verbindung der Formel (XV) in einem einstufigen Verfahren durch Hydrierung in Gegenwart eines geeigneten Palladium- oder Platin-Katalysators in den 3-(3-Aminophenyl)propionsäureester der Formel (III-A) überführt,
oder

### [D] ein Acrylsäureester-Derivat der Formel (XVII)

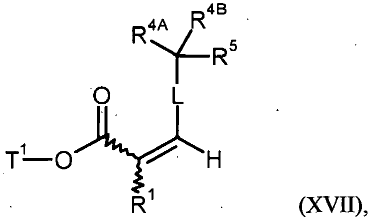

in welcher L, R¹, R^{4A}, R^{4B}, R⁵ und T¹ die oben angegebenen Bedeutungen haben,
in einem inerten Lösungsmittel unter Palladium-Katalyse mit einem 3-Amino- oder 3-Nitro-phenylbromid der Formel (XVIII) 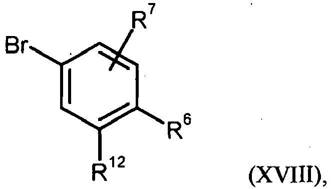 in welcher R⁶ und R⁷ die oben angegebenen Bedeutungen haben
und
- R¹²: für Amino oder Nitro steht,
zu einer Verbindung der Formel (XIX) 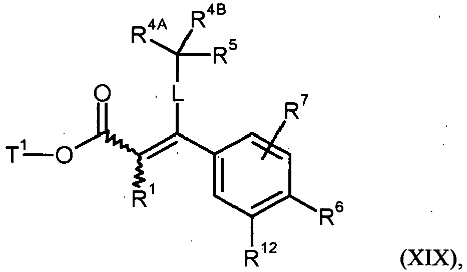 in welcher L, R¹, R^{4A}, R^{4B}, R⁵, R⁶, R⁷, R¹² und T¹ die oben angegebenen Bedeutungen haben,
kuppelt und diese dann mit Wasserstoff in Gegenwart eines geeigneten Palladium- oder Platin-Katalysators oder im Fall, dass R¹² für Amino steht, alternativ mit Magnesium in Methanol zum 3-(3-Aminophenyl)propionsäureester der Formel (III-A) 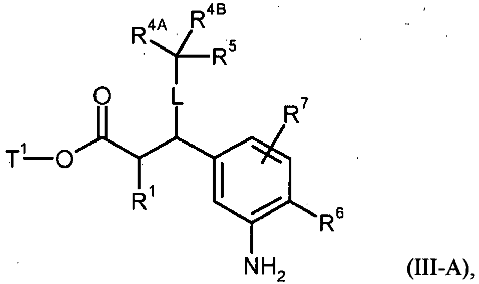 in welcher L, R¹, R^{4A}, R^{4B}, R⁵, R⁶, R⁷ und T¹ die oben angegebenen Bedeutungen haben,
reduziert,
oder

### [E-1] ein Phenyliodid der Formel (XX)

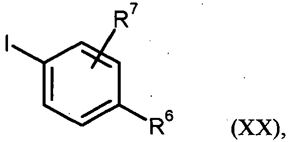

in welcher R⁶ und R⁷ die oben angegebenen Bedeutungen haben,
in einem inerten Lösungsmittel mit Isopropylmagnesiumchlorid in Gegenwart von Lithium-chlorid in die entsprechende Phenylmagnesium-Verbindung überführt, diese dann *in situ* unter Kupfer(I)-Katalyse mit einem Alkylidenmalonsäureester der Formel (XXI) 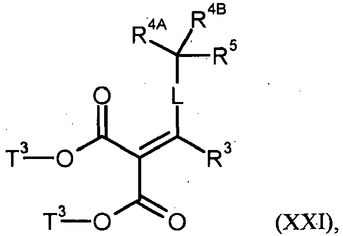 in welcher L, R³, R^{4A}, R^{4B} und R⁵ die oben angegebenen Bedeutungen haben
und
- T³: für Methyl oder Ethyl steht,
zu einer Verbindung der Formel (XXII) 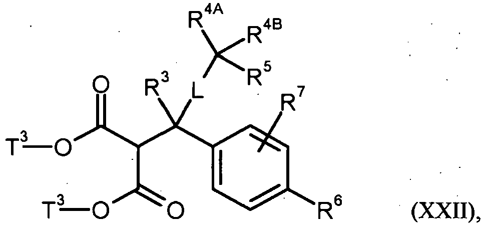 in welcher L, R³, R^{4A}, R^{4B}, R⁵, R⁶, R⁷ und T³ die oben angegebenen Bedeutungen haben,
kuppelt, anschließend eine der beiden Ester-Gruppierungen durch Erhitzen mit Lithiumchlorid in einem DMSO/Wasser-Gemisch abspaltet, den resultierenden 3-Phenylpropionsäureester der Formel (XXIII) 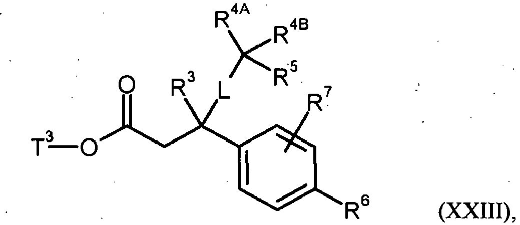 in welcher L, R³, R^{4A}, R^{4B}, R⁵, R⁶, R⁷ und T³ die oben angegebenen Bedeutungen haben,
dann durch Umsetzung mit Nitroniumtetrafluoroborat in das 3-Nitrophenyl-Derivat der Formel (XXIV) 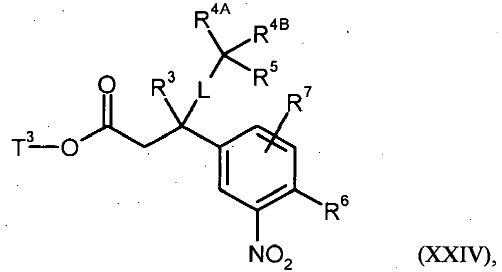 in welcher L, R³, R^{4A}, R^{4B}, R⁵, R⁶, R⁷ und T³ die oben angegebenen Bedeutungen haben,
überführt und schließlich in Gegenwart eines geeigneten Palladium- oder Platin-Katalysators zu einem 3-(3-Aminophenyl)propionsäureester der Formel (III-B) 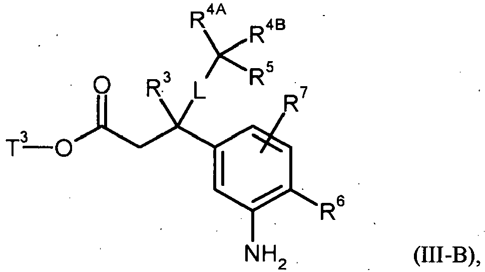 in welcher L, R³, R^{4A}, R^{4B}, R⁵, R⁶, R⁷ und T³ die oben angegebenen Bedeutungen haben,
hydriert,
oder

### [E-2] ein geschütztes 3-Aminophenyliodid der Formel (XXV)

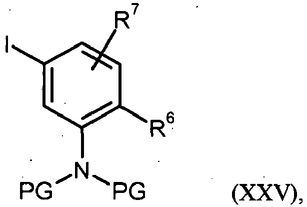

in welcher R⁶ und R⁷ die oben angegebenen Bedeutungen haben
und
- PG: für Benzyl oder 4-Methoxybenzyl als inerte Amino-Schutzgruppe steht,
in einem inerten Lösungsmittel mit Isopropylmagnesiumchlorid in Gegenwart von Lithiumchlorid in die entsprechende Phenylmagnesium-Verbindung überführt, diese dann *in situ* unter Kupfer(I)-Katalyse mit einem Alkylidenmalonsäureester der Formel (XXI) 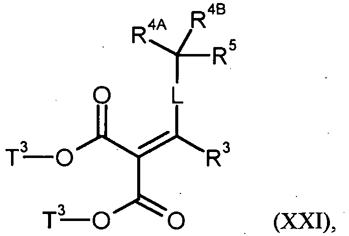 in welcher L, R³, R^{4A}, R^{4B} und R⁵ die oben angegebenen Bedeutungen haben und
- T³: für Methyl oder Ethyl steht,
zu einer Verbindung der Formel (XXVI) 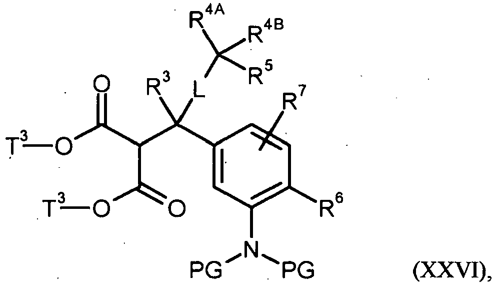 in welcher L, PG, R³, R^{4A}, R^{4B}, R⁵, R⁶, R⁷ und T³ die oben angegebenen Bedeutungen haben,
kuppelt, diese anschließend durch Hydrogenolyse oder durch Behandlung mit einem geeigneten Oxidationsmittel wie beispielsweise 2,3-Dichlor-5,6-dicyano-1,4-benzochinon (DDQ) zu einer Verbindung der Formel (XXVII) 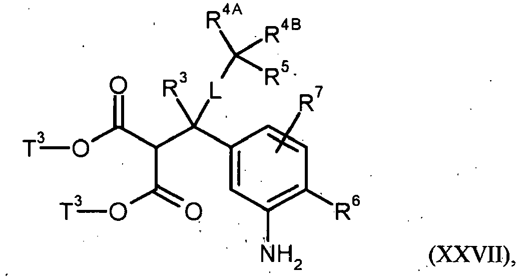 in welcher L, R³, R^{4A}, R^{4B}, R⁵, R⁶, R⁷ und T³ die oben angegebenen Bedeutungen haben,
entschützt und dann eine der beiden Ester-Gruppierungen durch Erhitzen mit Lithiumchlorid in einem DMSO/Wasser-Gemisch unter Erhalt des 3-(3-Aminophenyl)propionsäureesters der Formel (III-B) 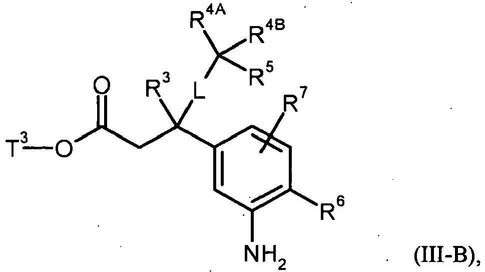 in welcher L, R³, R^{4A}, R^{4B}, R⁵, R⁶, R⁷ und T³ die oben angegebenen Bedeutungen haben,
abspaltet,
oder

### [F] einen Carbonsäureester der Formel (XXVIII)

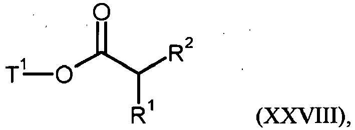

in welcher R¹, R² und T¹ die oben angegebenen Bedeutungen haben,
in einem inerten Lösungsmittel nach α-Deprotonierung mit einer 3-Brombenzyl-Verbindung der Formel (XXIX) 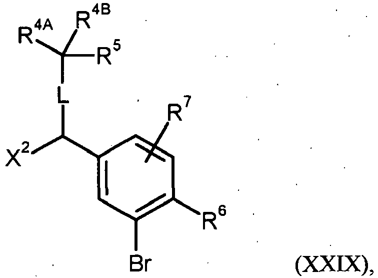 in welcher L, R^{4A}, R^{4B}, R⁵, R⁶ und R⁷ die oben angegebenen Bedeutungen haben
und
- X²: für eine geeignete Abgangsgruppe wie Chlor, Brom, Iod, Mesylat, Triflat oder Tosylat steht,
zu einer Verbindung der Formel (XXX) 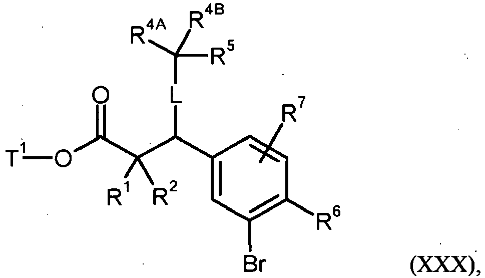 in welcher L, R¹, R², R^{4A}, R^{4B}, R⁵, R⁶, R⁷ und T¹ die oben angegebenen Bedeutungen haben,
alkyliert, anschließend mit Benzylamin in Gegenwart einer Base und eines PalladiumKatalysators zu einer Verbindung der Formel (XXXI) 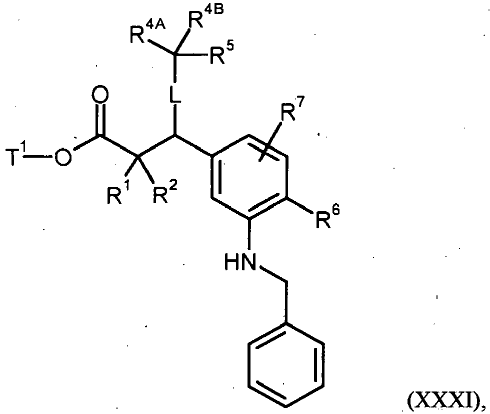 in welcher L, R¹, R², R^{4A}, R^{4B}, R⁵, R⁶, R⁷ und T¹ die oben angegebenen Bedeutungen haben,
umsetzt und die *N*-Benzylgruppe dann durch Hydrogenolyse unter Erhalt eines 3-(3-Amino-phenyl)propionsäureesters der Formel (III-C) 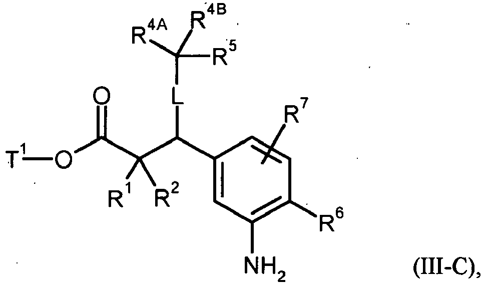 in welcher L, R¹, R², R^{4A}, R^{4B}, R⁵, R⁶, R⁷ und T¹ die oben angegebenen Bedeutungen haben,
entfernt.

Zur Deprotonierung des Phosphonoesters (XI) bei den Olefinierungsreaktionen (XI) + (XII) → (XIII) und (XI) + (XIV) → (XV) eignen sich insbesondere nicht-nukleophile, starke Basen wie beispielsweise Natrium- oder Kaliumhydrid, Lithium-, Natrium- oder Kalium-bis(trimethylsilyl)amid oder Lithiumdiisopropylamid; bevorzugt wird Natriumhydrid verwendet.

Die Hydrierung in den Verfahrensschritten (XIII) → (III-A), (XV) → (III-A), (XIX) → (III-A) und (XXIV) → (III-B) wird in der Regel unter einer stationären Wasserstoffatmosphäre bei normalem oder erhöhtem Druck durchgeführt. Als Katalysator wird hierbei bevorzugt Palladium oder Platin auf Aktivkohle (als Trägermaterial) eingesetzt. Die Entfernung der Amino-Schutzgruppe(n) in den Transformationen (XVI) → (III-A), (XXVI) → (XXVII) und (XXXI) → (III-C) erfolgt üblicherweise durch Hydrogenolyse nach der gleichen Prozedur; im Falle, dass PG in (XVI) bzw. (XXVI) für *p*-Methoxybenzyl steht, kann dies alternativ auch auf oxidativem Wege geschehen, beispielsweise mit Hilfe von 2,3-Dichlor-5,6-dicyano-1,4-benzochinon (DDQ) oder Ammoniumcer(IV)nitrat.

Als Palladium-Katalysator für die Umsetzung (XVII) + (XVIII) → (XIX) [Heck-Reaktion] wird vorzugsweise Palladium(II)acetat oder Tris(dibenzylidenaceton)dipalladium(0), jeweils in Kombination mit einem Phosphin-Liganden wie beispielsweise Tri-*tert*.-butylphosphin, Triphenylphosphin oder Tri-2-tolylphosphin, eingesetzt.

Die Umwandlung des Phenyliodids (XX) in die korrespondierende Phenylmagnesium-Verbindung und deren Kupfer(I)-vermittelte 1,4-Addition an das Alkylidenmalonat (XXI) zum Produkt der Formel (XXII) werden nach einer literaturbekannten allgemeinen Methode durchgeführt [siehe z.B. P. Knochel et al., Tetrahedron 56, 2727-2731 (2000) und dort zitierte Literatur]; gleiches gilt für die analoge Umsetzung (XXV) + (XXI) → (XXVI).

Zur α-Deprotonierung des Carbonsäureesters (XXVIII) bei der Alkylierungsreaktion (XXVIII) + (XXIX) → (XXX) eignen sich insbesondere nicht-nukleophile, starke Basen wie beispielsweise Natrium- oder Kalium-*tert*.-butylat, Natrium- oder Kaliumhydrid, Lithiumdiisopropylamid oder Lithium-, Natrium- oder Kalium-bis(trimethylsilyl)amid; bevorzugt wird Lithiumdiisopropylamid verwendet. Als inerte Lösungsmittel werden bei dieser Reaktion vorzugsweise Ether wie Diethylether, Diisopropylether, Methyl-*tert*.-butylether, Tetrahydrofuran, Glykoldimethylether oder Di-ethylenglykoldimethylether eingesetzt. Die Umsetzung erfolgt üblicherweise in einem Temperaturbereich von -80°C bis +25°C.

Für die Transformation (XXX) → (XXXI) [Buchwald-Hartwig-Kupplung mit Benzylamin] wird bevorzugt Tris(dibenzylidenaceton)dipalladium(0) als Katalysator in Verbindung mit (±)-2,2'-Bis-(diphenylphosphino)-1,1'-binaphthyl als Phosphin-Liganden und Natrium- oder Kalium-*tert*.-butylat als Base verwendet [vgl. z.B. J. P. Wolfe und S. L. Buchwald, Organic Syntheses, Coll. Vol. 10, 423 (2004), Vol. 78, 23 (2002)].

Die zuvor beschriebenen Reaktionen können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (II), (III), (IV), (VII), (XVI), (XXII), (XXIII), (XXIV), (XXVI), (XXVII), (XXX) oder (XXXI) erfolgen, welche dann in separierter Form entsprechend den zuvor beschriebenen Verfahrenssequenzen weiter umgesetzt werden. Eine solche Auftrennung der Stereoisomeren läßt sich nach üblichen, dem Fachmann bekannten Methoden durchführen. Im Rahmen der vorliegenden Erfindung werden vorzugsweise chromatographische Verfahren an achiralen bzw. chiralen Trennphasen angewandt; im Falle von Carbonsäuren als Zwischen- oder Endprodukten kann alternativ auch eine Trennung über diastereomere Salze erfolgen.

Die Verbindungen der Formeln (V), (VI), (VIII), (IX), (XI), (XII), (XIV), (XVII), (XVIII), (XX), (XXI), (XXV), (XXVIII) und (XXIX) sind entweder kommerziell erhältlich oder als solche in der Literatur beschrieben, oder sie können auf für den Fachmann offenkundigem Wege in Analogie zu in der Literatur publizierten Methoden hergestellt werden. Zahlreiche detaillierte Vorschriften sowie Literaturangaben zur Herstellung der Ausgangsmaterialien befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden:

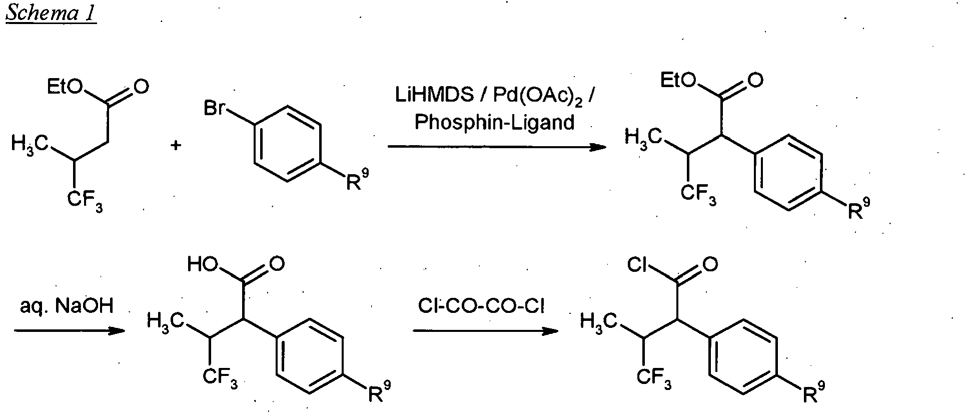

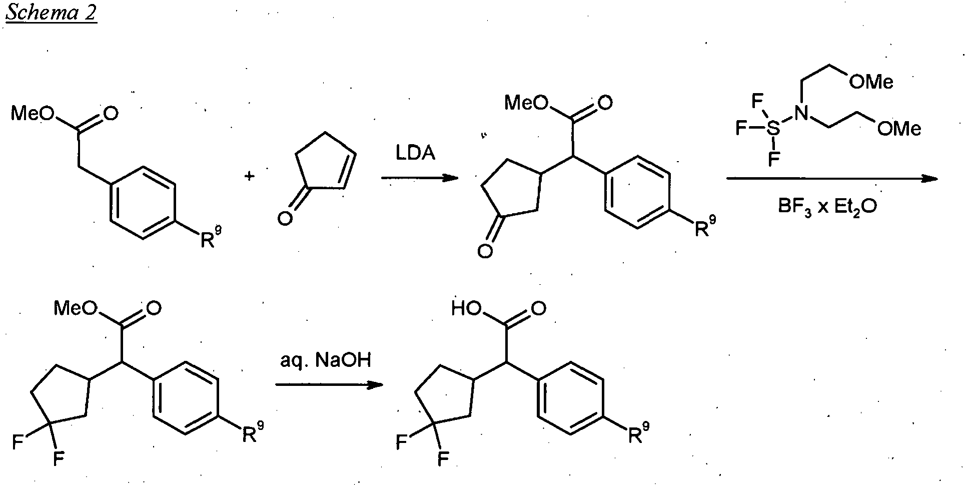

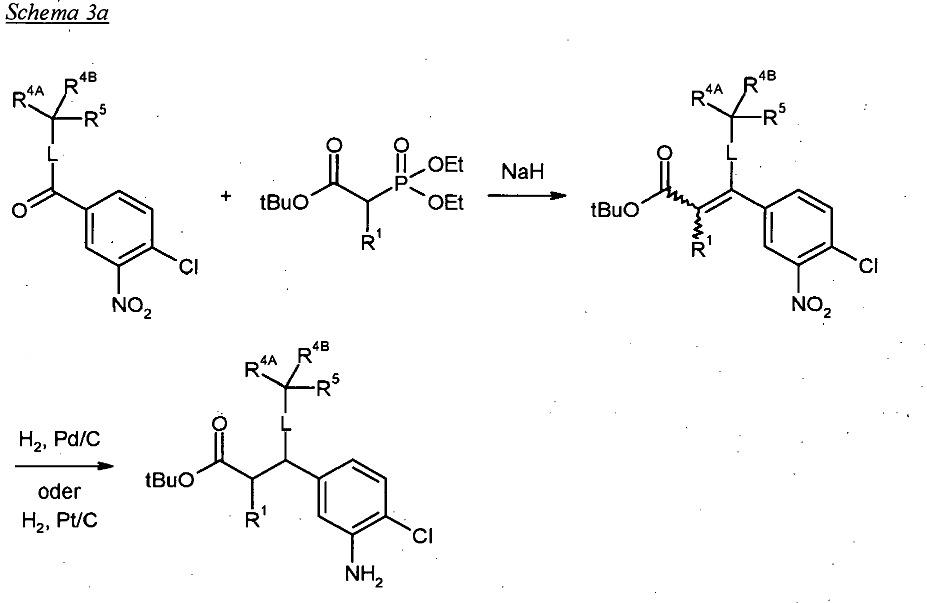

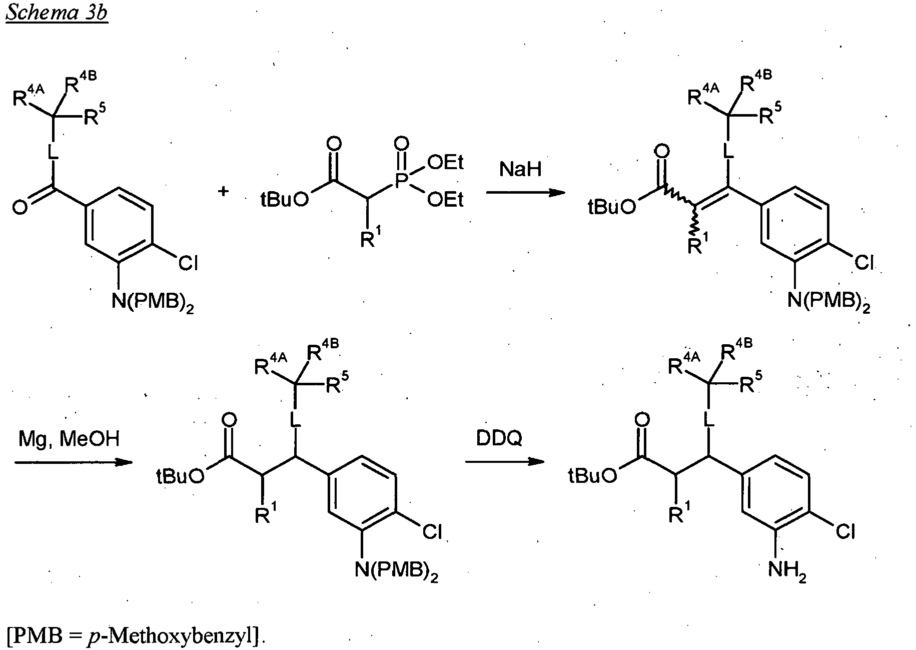

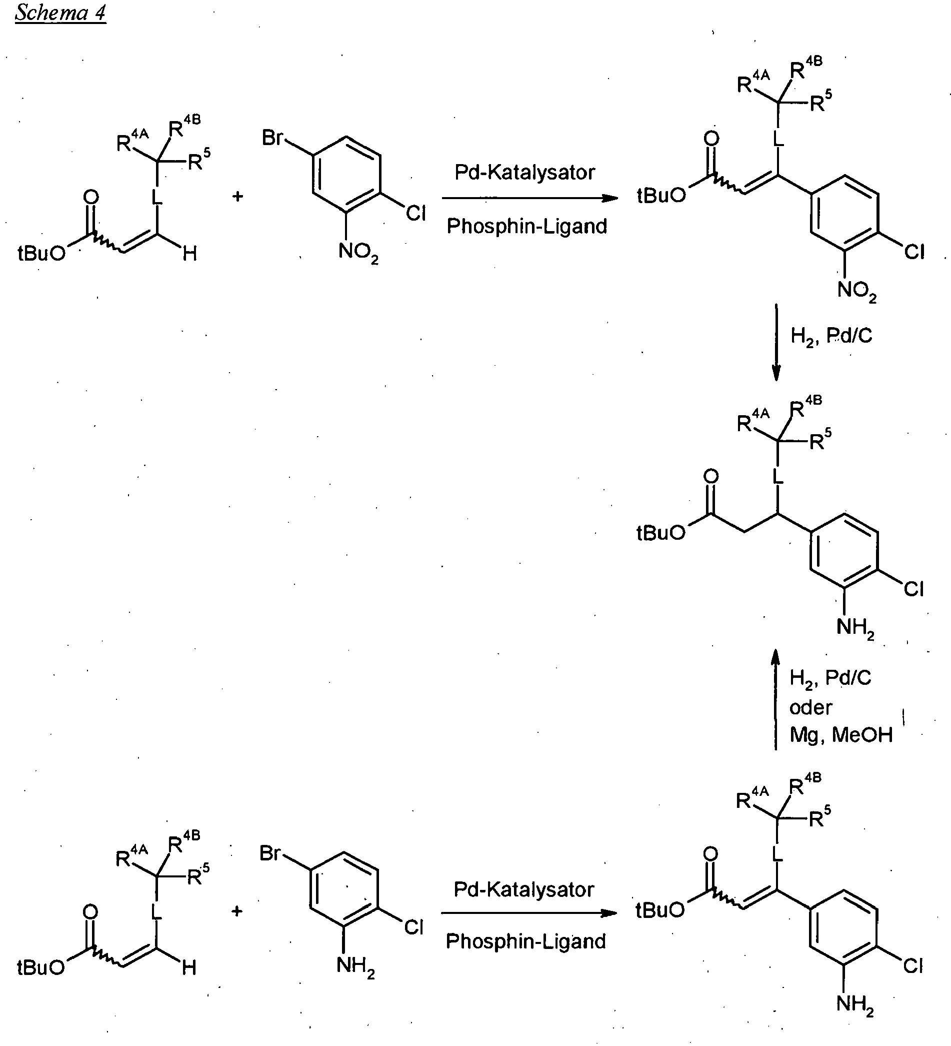

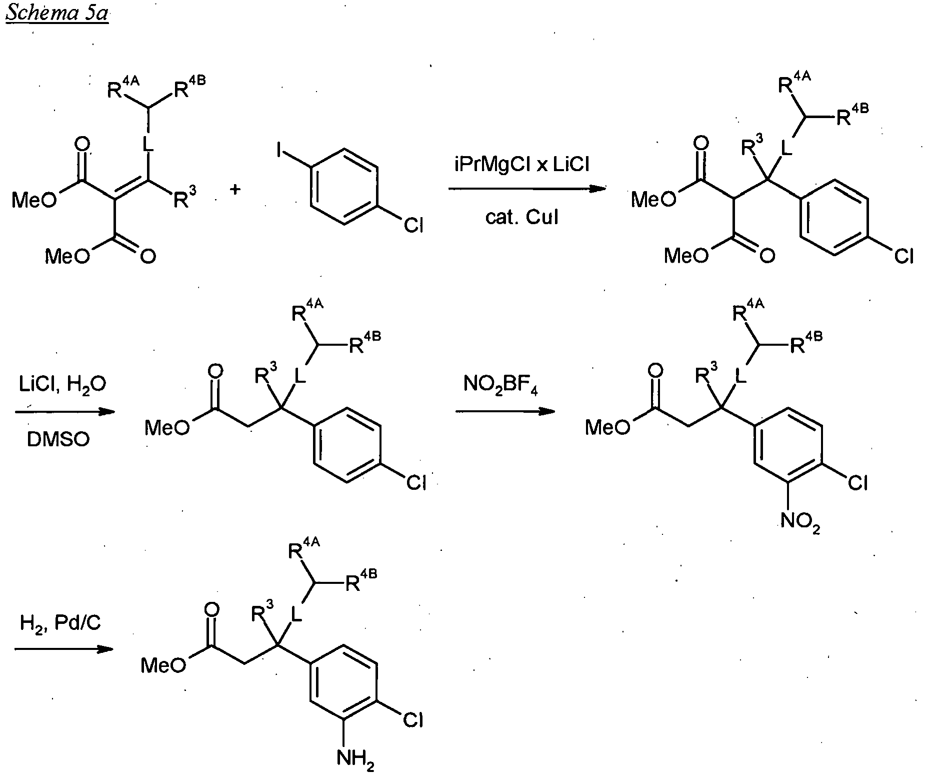

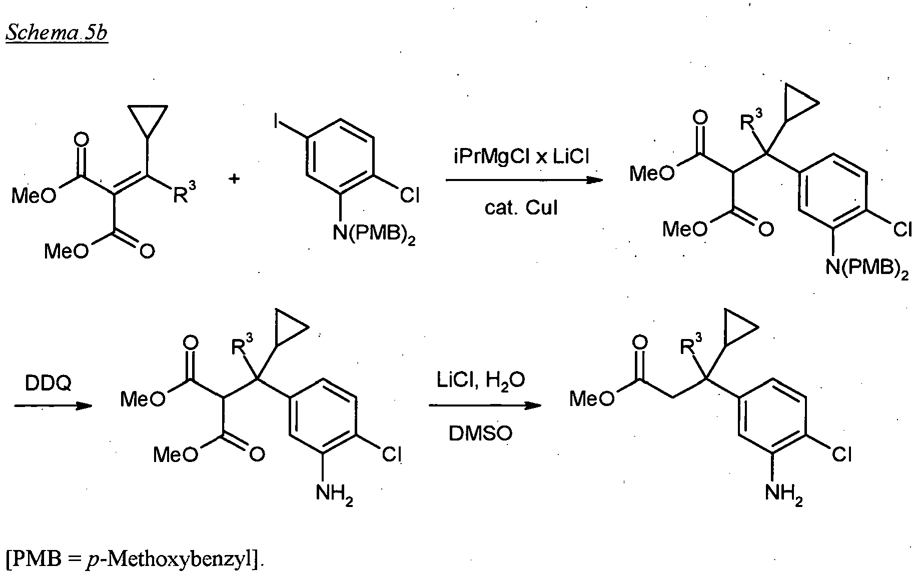

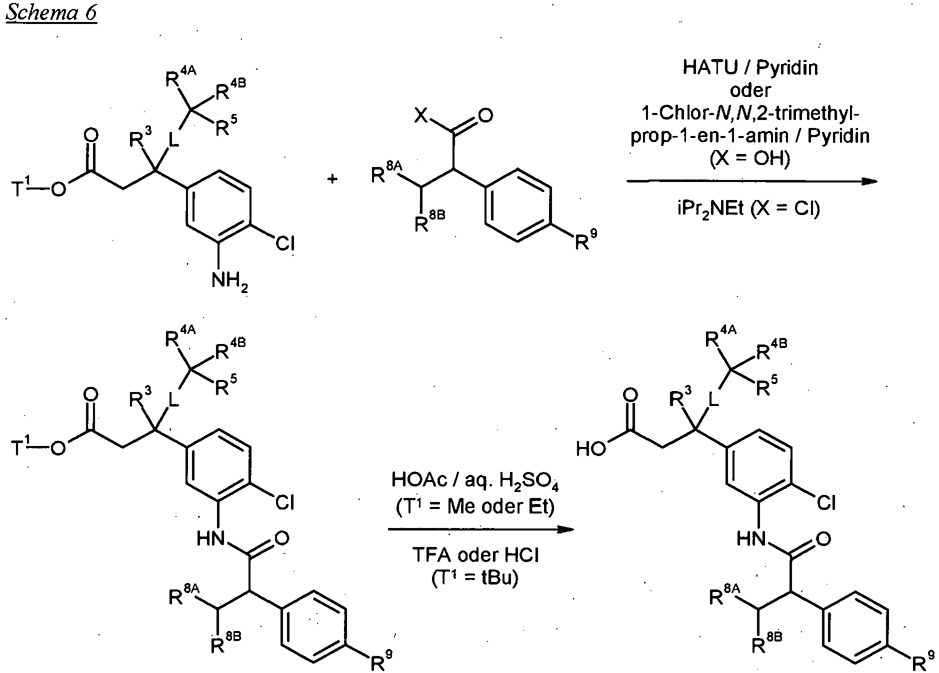

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente Aktivatoren der löslichen Guanylatcyclase dar. Sie führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte, Häm-unabhängige Aktivierung der löslichen Guanylatcyclase und einen intrazellulären cGMP-Anstieg vermittelt.

Darüber hinaus verfügen die erfindungsgemäßen Verbindungen über vorteilhafte pharmakokinetische Eigenschaften, insbesondere bezüglich ihrer Bioverfügbarkeit und/oder Wirkdauer nach intravenöser oder oraler Gabe.

Die erfindungsgemäßen Verbindungen eignen sich in besonderem Maße zur Behandlung und/oder Prävention von kardiovaskulären, pulmonalen, thromboembolischen und fibrotischen Erkrankungen.

Die erfindungsgemäßen Verbindungen können daher in Arzneimitteln eingesetzt werden zur Behandlung und/oder Prävention von kardiovaskulären Erkrankungen, wie beispielsweise Bluthochdruck (Hypertonie), Herzinsuffizienz, koronare Herzerkrankung, stabile und instabile Angina pectoris, pulmonale arterielle Hypertonie (PAH) und andere Formen der pulmonalen Hypertonie (PH), renale Hypertonie, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Darüber hinaus können die erfindungsgemäßen Verbindungen auch zur Behandlung und/oder Prävention von Arteriosklerose, Lipidstoffwechselstörungen, Hypolipoproteinämien, Dyslipidämien, Hypertriglyceridämien, Hyperlipidämien, kombinierten Hyperlipidämien, Hypercholesterolämien, Abetalipoproteinämie, Sitosterolämie, Xanthomatose, Tangier-Krankheit, Fettsucht (Adipositas), Fettleibigkeit (Obesitas) sowie des Metabolischen Syndroms eingesetzt werden.

Weiterhin können die erfindungsgemäßen Verbindungen zur Behandlung und/oder Prävention von primärem und sekundärem Raynaud-Phänomen, Mikrozirkulationsstörungen, Claudicatio, Tinnitus, peripheren und autonomen Neuropathien, diabetischen Mikroangiopathien, diabetischer Retinopathie, diabetischen Geschwüren an den Extremitäten, Gangrän, CREST-Syndrom, Erythematose, Onychomykose sowie von rheumatischen Erkrankungen verwendet werden.

Die erfindungsgemäßen Verbindungen können darüber hinaus zur Verhinderung von ischämie- und/oder reperfusionsbedingten Schädigungen von Organen oder Geweben sowie als Zusatzstoffe für Perfusions- und Konservierungslösungen von Organen, Organteilen, Geweben oder Gewebeteilen menschlichen oder tierischen Ursprungs, insbesondere bei chirurgischen Eingriffen oder im Bereich der Transplantationsmedizin, Verwendung finden.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Die erfindungsgemäßen Verbindungen eignen sich auch zur Behandlung und/oder Prävention von asthmatischen Erkrankungen, chronisch-obstruktiven Atemwegserkrankungen (COPD), des akuten Atemwegssyndroms (ARDS) und der akuten Lungenschädigung (ALI), alpha-1-Antitrypsin-Defizienz (AATD), Lungenfibrose, Lungenemphysem (z.B. durch Zigarettenrauch induziertes Lungenemphysem) und zystischer Fibrose (CF) sowie von pulmonaler arterieller Hypertonie (PAH) und anderen Formen der pulmonalen Hypertonie (PH), einschließlich der mit Linksherzerkrankung, HIV, Sichelzellanämie, Thromboembolien, Sarkoidose, COPD oder Lungenfibrose assoziierten pulmonalen Hypertonie.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierte Lern- und Gedächtnisstörungen, altersassoziierte Gedächtnisverluste, vaskuläre Demenz, Schädel-Hirn-Trauma, Schlaganfall, Demenz, die nach Schlaganfällen auftritt ("post stroke dementia"), post-traumatisches Schädel-Hirn-Trauma, allgemeine Konzentrationsstörungen, Konzentrationsstörungen bei Kindern mit Lern- und Gedächtnisproblemen, Alzheimer'sche Krankheit, Demenz mit Lewy-Körperchen, Demenz mit Degeneration der Frontallappen einschließlich des Pick's-Syndroms, Parkinson'sche Krankheit, progressiver nuclear palsy, Demenz mit corticobasaler Degeneration, Amyolateralsklerose (ALS), Huntington'sche Krankheit, Demyelinisation, Multiple Sklerose, thalamische Degeneration, Creutzfeld-Jacob-Demenz, HIV-Demenz, Schizophrenie mit Demenz oder Korsakoff-Psychose. Sie eignen sich auch zur Behandlung und/oder Prävention von Erkrankungen des Zentralnervensystems wie Angst-, Spannungs- und Depressionszuständen, zentralnervös bedingten Sexualdysfiznktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, rheumatoiden Erkrankungen, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Aufgrund ihres Wirkprofils eignen sich die erfindungsgemäßen Verbindungen insbesondere zur Behandlung und/oder Prävention kardiovaskulärer Erkrankungen wie Herzinsuffizienz, Angina pectoris, Hypertonie und pulmonale Hypertonie, sowie von thromboembolischen Erkrankungen und Ischämien, Gefäßerkrankungen, Mikrozirkulationsstörungen, Niereninsuffizienz, fibrotischen Erkrankungen und Arteriosklerose.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin All-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausfuhrungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspeilsionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

| | |
|---|---|
| abs. | absolut |
| Ac | Acetyl |
| AIBN | 2,2'-Azobis-(2-methylpropionitril) |
| aq. | wässrig, wässrige Lösung |
| ATP | Adenosin-5'-triphosphat |
| Bn | Benzyl |
| Brij^{®} | Polyethylenglycoldodecylether |
| BSA | bovines Serumalbumin |
| Bsp. | Beispiel |
| Bu | Butyl |
| c | Konzentration |
| ca. | *circa,* ungefähr |
| cat. | katalytisch |
| CI | chemische Ionisation (bei MS) |
| d | Tag(e) |
| DAST | Diethylaminoschwefeltrifluorid |
| DC | Dünnschichtchromatographie |
| DCI | direkte chemische Ionisation (bei MS) |
| DDQ | 2,3-Dichlor-5,6-dicyano-1,4-benzochinon |
| de | Diastereomerenüberschuss |
| DMF | Dimethylformamid |
| DMSO | Dimethylsulfoxid |
| d. Th. | der Theorie (bei Ausbeute) |
| DTT | Dithiothreitol |
| EDC | *N*'-(3-Dimethylaminopropyl)-*N*-ethylcarbodiimid-Hydrochlorid |
| ee | Enantioriterenüberschuss |
| EI | Elektronenstoß-Iönisation (bei MS) |
| ent | enantiomerenrein, Enantiomer |
| eq. | Äquivalent(e) |
| ESI | Elektrospray-Ionisation (bei MS) |
| Et | Ethyl |
| GC | Gaschromatographie |
| ges. | gesättigt |
| GTP | Guanosin-5'-triphosphat |
| h | Stunde(n) |
| HATU | *O*-(7-Azabenzotriazol-1-yl)-*N,N,N',N'*-tetramethyluronium-Hexafluorophosphat |
| HOBt | 1-Hydroxy-*1H*-benzotriazol-Hydrat |
| HPLC | Hochdruck-, Hochleistungsflüssigchromatographie |
| iPr | Isopropyl |
| konz. | konzentriert |
| LC-MS | Flüssigchromatographie-gekoppelte Massenspektroskopie |
| LDA | Lithiumdiisopropylamid |
| LiHMDS | Lithiumhexamethyldisilazid [Lithium-bis(trimethylsilyl)amid] |
| Me | Methyl |
| min | Minute(n) |
| MS | Massenspektroskopie |
| NBS | *N*-Bromsuccinimid |
| NMP | *N*-Methylpyrrolidin-2-on |
| NMR | Kernresonanzspektroskopie |
| *p* | para |
| Pd/C | Palladium auf Aktivkohle |
| Ph | Phenyl |
| PMB | *p*-Methoxybenzyl |
| Pr | Propyl |
| Pt/C | Platin auf Aktivkohle |
| rac | racemisch, Racemat |
| R_{f} | Retentionsindex (bei DC) |
| RP | reverse phase (Umkehrphase, bei HPLC) |
| RT | Raumtemperatur |
| Rₜ | Retentionszeit (bei HPLC oder GC) |
| s.o. | siehe oben |
| tBu | tert.-Butyl |
| TEA | Triethanolamin |
| TFA | Trifluoressigsäure |
| THF | Tetrahydrofuran |
| UV | Ultraviolett-Spektroskopie |
| v/v | Volumen zu Volumen-Verhältnis (einer Lösung) |
| zus. | zusammen |

### GC-MS- und LC-MS-Methoden:

### Methode 1 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 2 (LC-MS):

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 3 (LC-MS):

Gerätetyp MS: Micromass ZQ; Gerätetyp HPLC: HP 1100 Series; UV DAD; Säule: Phenomenex Gemini 3 µ 30 mm x 3.00 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS):

Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 5 (LC-MS):

Instrument: Waters Acquity SQD UPLC System; Säule: Waters Acquity UPLC HSS T3 1.8 µ, 50 mm x 1 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 6 (GC-MS):

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Methode 7 (LC-MS):

Instrument: Waters Acquity SQD UPLC System; Säule: Waters Acquity UPLC HSS T3 1.8 µ, 30 mm x 2 mm; Eluent A: 11 Wasser + 0.25 ml 99%-ige Ameisensäure, Eluent B: 11 Acetonitril + 0.25 ml 99%-ige Ameisensäure; Gradient: 0.0 min 90% A → 1.2 min 5% A → 2.0 min 5% A; Fluss: 0.60 ml/min; Ofen: 50°C; UV-Detektion: 208-400 nm.

### Methode 8 (LC-MS):

Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 97% A → 0.5 min 97% A → 3.2 min 5% A → 4.0 min 5% A; Fluss: 0.3 ml/min; Ofen: 50°C; UV-Detektion: 210 nm.

### Ausgangsverbindungen und Intermediate:

### Beispiel 1A

### tert.-Butyl-(2E/Z)-4-methoxy-4-methylpent-2-enoat

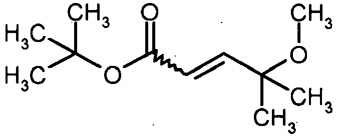

Zu einem Gemisch von 24 ml (48 mmol) einer 2 M Lösung von Oxalsäuredichlorid in Dichlormethan und weiteren 100 ml Dichlormethan wurden bei -70°C unter Argon 6.8 ml (96 mmol) DMSO in 10 ml Dichlormethan getropft und der Ansatz 15 min nachgerührt. Anschließend wurden 5.2 ml (48 mmol) 2-Methoxy-2-methylpropan-1-ol [H. Garcia et al., Chem. Eur. J. 16 (28), 8530-8536 (2010)], gelöst in 15 ml Dichlormethan, zugetropft und erneut 15 min bei -70°C nachgerührt. Nach langsamer Zugabe von 22.1 ml (158 mmol) Triethylamin wurde das Reaktionsgemisch nochmals 15 min nachgerührt und dann langsam auf Raumtemperatur erwärmt. Danach wurde die Reaktionsmischung mit 22 g (58 mmol) *tert*.-Butyl(triphenyl-λ⁵-phosphanyliden)acetat versetzt und über Nacht bei Raumtemperatur gerührt. Die Reaktionslösung wurde dann langsam auf 100 ml Eiswasser gegeben und die erhaltenen Phasen getrennt. Die organische Phase wurde zweimal mit jeweils 100 ml Wasser gewaschen, über Magnesiumsulfat getrocknet und am Rotationsverdampfer im Vakuum eingeengt (Wasserbad-Temperatur 40°C, Druck nicht unter 150 mbar): Der erhaltene Rückstand wurde in ca. 100 ml Diethylether aufgenommen und 2 Tage bei +3°C im Kühlschrank stehengelassen. Das ausgefallene Triphenylphosphinoxid wurde abfiltriert und das Filtrat im Vakuum eingeengt. Der erhaltene Rückstand wurde durch Chromatographie an Silicagel gereinigt (Laufmittel Cyclohexan/Ethylacetat 100:1 → 50:1). Es wurden 7.06 g (73% d. Th.) der Titelverbindung als farblose Flüssigkeit erhalten.

GC-MS (Methode 6): Rₜ = 3.32 min, m/z = 218 (M+NH₄)⁺.

Analog zu Synthesebeispiel 1A wurden die beiden folgenden Verbindungen erhalten:

| **Beispiel** | **Name / Struktur / Edukte** | **Analytische Daten** |
|---|---|---|
| **2A** | *tert.* -Butyl-(2*E*)-3-(3,3 -difluorcyclobutyl)acrylat | ¹H-NMR (400 MHz, DMSO-*d*₆): |
| | 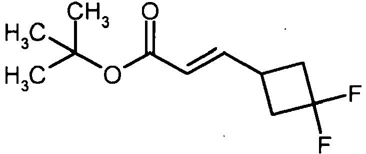 | δ [ppm] = 1.42 (s, 9H), 2.48-2.64 (m, 2H, teilweise verdeckt durch DMSO-Signal), 2.70-2.84 (m, 2H), 2.90-3.04 (m, 1H), 5.84 (d, 1H, *³J*= 16.38, Hz), 6.88 (dd, 1H). |
| | aus *tert*.-Butyl(triphenyl-λ⁵-phosphanyliden)acetat und (3,3-Difluorcyclobutyl)methanol [CAS Reg.-Nr. 681128-39-2] | |
| **3A** | *tert*.-Butyl-(2*E*)-4-cyclopropylbut-2-enoat | GC-MS (Methode 6): |
| | 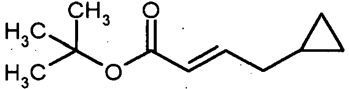 | Rₜ = 3.42 min, m/z = 200 (M+NH₄)⁺. |
| | aus *tert*.-Butyl(triphenyl-λ⁵-phosphanyliden)acetat und 2-Cyclopropylethanol | ¹H-NMR (400 MHz, DMSO-*d*₆):δ [ppm] = -0.04-0.02 (m, 2H), 0.33-0.40 (m, 2H), 0.63-0.75 (m, 1H), 1.34 (s, 9H), 1.94-2.00 (m, 2H), 5.69-5.76 (m, 1H), 6.69-6.79 (m, 1H). |

### Beispiel 4A und Beispiel 5A

### Methyl-(2E/Z)-3-(3-amino-4-chlorphenyl)-4-methylpent-2-enoat und Methyl-3-(3-amino-4-chlorphenyl)-4-methylpent-3-enoat

Unter Argon wurde eine Mischung aus 3.22 g (15.6 mmol) 5-Brom-2-chloranilin, 3.0 g (23.4 mmol) Methyl-(2*E*)-4-methylpent-2-enoat, 143 mg (0.16 mmol) Tris(dibenzylidenaceton)dipalladium, 63 mg (0.31 mmol) Tri-*tert*.-butylphosphin sowie 3.64 ml (17.2 mmol) *N*,*N-*Dicyclohexylmethylamin in 30 ml Dioxan auf 120°C erhitzt und drei Tage bei dieser Temperatur gerührt. Jeweils nach dem ersten und dem zweiten Reaktionstag wurde nochmals die gleiche Menge an Palladium-Katalysator und Phosphin-Ligand dem Reaktionsgemisch zugegeben. Anschließend wurde das Reaktionsgemisch über Celite filtriert und das Filtrat im Vakuum eingeengt. Der Rückstand wurde durch Chromatographie an Silicagel (Laufmittel Cyclohexan/Ethylacetat 50:1) in seine Komponenten aufgetrennt. Es wurden 1.52 g Methyl-(2*E*/*Z*)-3-(3-amino-4-chlorphenyl)-4-methylpent-2-enoat (38% d. Th.) sowie 906 mg Methyl-3-(3-amino-4-chlorphenyl)-4-methylpent-3-enoat (22% d. Th.) erhalten.

### Beispiel 4A

### Methyl-(2E/Z)-3-(3-amino-4-chlorphenyl)-4-methylpent-2-enoat

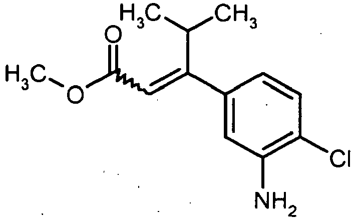

LC-MS (Methode 2): Rₜ = 2.46 min, m/z = 254 (M+H)⁺.

¹H-NMR (400 MHz, DMSO-*d*₆): δ [ppm] = 1.03 (d, 6H), 3.65 (s, 3H), 3.90-4.03 (m, 1H), 5.42 (br. s, 2H), 5.63 (s, 1H), 6.40 (dd, 1H), 6.69 (d, 1H), 7.16 (d, 1H).

### Beispiel 5A

### Methyl-3-(3-amino-4-chlorphenyl)-4-methylpent-3-enoat

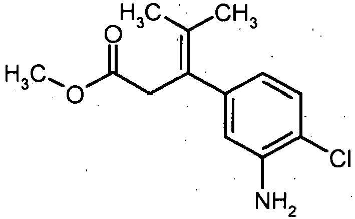

LC-MS (Methode 2): Rₜ = 2.28 min, m/z = 254 (M+H)⁺.

Analog zu Synthesebeispiel 4A / 5A wurde die folgende Verbindung erhalten:

| **Beispiel** | **Name / Struktur / Edukte** | **Analytische Daten** |
|---|---|---|
| **6A** | *tert*.-Butyl-(2*E*/*Z*)-3-(3-amino-4-chlorphenyl)-4-methoxy-4-methylpent-2-enoat | LC-MS (Methode 5): |
| | 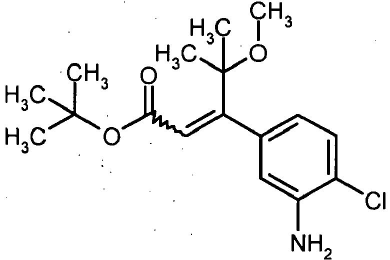 | Rₜ = 1.25 min, m/z = 326/328 (M+H)⁺. |
| | aus *tert.* -Butyl-(2*E*/*Z*)-4-methoxy-4-methylpent-2-enoat und 5-Brom-2-chloranilin | |

### Beispiel 7A

### tert.-Butyl-(2E)-3-cyclobutylacrylat

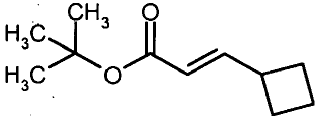

### Stufe 1:

Eine Lösung von 11.1 ml (116.1 mmol) Oxalylchlorid in 50 ml abs. Dichlormethan wurde auf-78°C abgekühlt und tropfenweise mit einer Lösung von 16.5 ml (232.2 mmol) DMSO in 50 ml abs. Dichlormethan versetzt, wobei die Temperatur unterhalb von -50°C gehalten wurde. Nach 5 min wurde eine Lösung von 10.0 g (116.1 mmol) Cyclobutanmethanol in 20 ml abs. Dichlormethan zugetropft. Nach weiteren 15 min Rühren bei -78°C wurden 80.9 ml (580.5 mmol) Triethylamin hinzugefügt. Nach 5 min wurde die Kühlung entfernt und die Mischung langsam auf RT erwärmt, bevor die Reaktionsmischung auf Wasser gegeben wurde. Es wurde mit Natriumchlorid gesättigt, und die abgetrennte organische Phase wurde zweimal mit gesättigter Natriumchlorid-Lösung, dreimal mit 1 N Salzsäure und dreimal mit pH 7-Pufferlösung gewaschen, über Natriumsulfat getrocknet und im Vakuum (500 mbar) eingeengt. Es wurden 6.28 g Cyclobutancarbaldehyd als Rohprodukt erhalten, das direkt weiter umgesetzt wurde.

### Stufe 2:

Zu einer auf 0°C gekühlten Suspension von 1.05 g (60%-ig in Mineralöl, 26.2 mmol) Natriumhydrid in einer Mischung aus 22 ml THF und 22 ml DMF wurden 6.4 ml (27.3 mmol) *tert.*-Butyl-(diethoxyphosphoryl)acetat getropft. Nach 30 min wurde die Mischung auf -10°C abgekühlt, und in mehreren Portionen wurden 2.0 g (roh, ca. 23.8 mmol) Cyclobutancarbaldehyd hinzugefügt. Die Reaktionsmischung wurde 5 h bei 0°C gerührt und dann langsam über Nacht auf RT erwärmt, bevor auf Wasser gegeben und dreimal mit Ethylacetat extrahiert wurde. Die organischen Phasen wurden vereinigt und im Vakuum eingeengt. Der Rückstand wurde durch Chromatographie an Silicagel gereinigt (Laufmittel Cyclohexan/Ethylacetat 50:1). Es wurden 1.21 g des Zielprodukts (ca. 28% d. Th.) erhalten.

GC-MS (Methode 1): Rₜ = 3.26 min; m/z = 126 (M-C₄H₈)⁺.

¹H-NMR (400 MHz, DMSO-*d*₆): δ [ppm] = 1.42 (s, 9H), 1.74-1.96 (m, 4H), 2.05-2.17 (m, 2H), 3.03-3.16 (m, 1H), 5.66 (dd, 1H), 6.86 (dd, 1H).

### Beispiel 8A und Beispiel 9A

### tert.-Butyl-3-(3-amino-4-chlorphenyl)-3-cyclobutylacrylat und tert.-Butyl-3-(3-amino-4-chlorphenyl)-3-cyclobutylidenpropanoat

Zu einer Mischung von 385.2 mg (1.87 mmol) 5-Brom-2-chloranilin und 510 mg (2.80 mmol) *tert.-*Butyl-(2*E*)-3-cyclobutylacrylat in 2.8 ml DMF wurden 0.78 ml (5.60 mmol) Triethylamin gegeben. Die Mischung wurde dreimal evakuiert und jeweils mit Argon wieder belüftet. Nach Zugabe von 41.9 mg (0.187 mmol) Palladium(II)acetat und 113.6 mg (0.373 mmol) Tri-2-tolylphosphin wurde erneut zweimal evakuiert und jeweils mit Argon belüftet und die Reaktionsmischung dann 3 h lang bei 150°C gerührt. Danach wurden weitere 193 mg 5-Brom-2-chloranilin hinzugefügt und die Reaktionsmischung nochmals 1 h bei 150°C gerührt. Nach dem Abkühlen wurde die Reaktionsmischung über Celite filtriert und der Filter-Rückstand zweimal mit DMF nachgewaschen. Das vereinigte Filtrat wurde im Hochvakuum eingeengt, und aus dem Rückstand wurden durch Chromatographie an Silicagel (Laufmittel Cyclohexan/Ethylacetat 60:1) die beiden isomeren Zielprodukte isoliert. Es wurden 203 mg *tert*.-Butyl-3-(3-amino-4-chlorphenyl)-3-cyclobutylacrylat (35.4% d. Th.) sowie 137 mg *tert*.-Butyl-3-(3-amino-4-chlorphenyl)-3-cyclobutylidenpropanoat (23.8% d. Th.) erhalten.

### Beispiel 8A

### tert.-Butyl-3-(3-amino-4-chlorphenyl)-3-cyclobutylacrylat

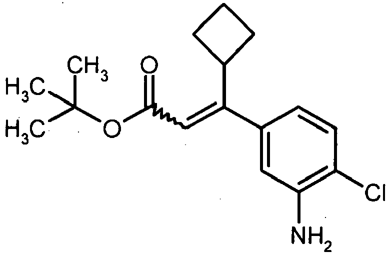

LC-MS (Methode 5): Rₜ = 1.36 min, m/z = 308 (M+H)⁺.

¹H-NMR (400 MHz, DMSO-*d*₆): δ [ppm] = 1.45 (s, 9H), 1.52-1.63 (m, 1H), 1.74-1.85 (m, 3H), 2.09-2.18 (m, 2H), 4.10 (quin, 1H), 5.35-5.41 (m, 2H), 5.55 (d, 1H), 6.38 (dd, 1H), 6.66 (d, 1H), 7.16 (d, 1H).

### Beispiel 9A

### tert.-Butyl-3-(3-amino-4-chlorphenyl)-3-cyclobutylidenpropanoat

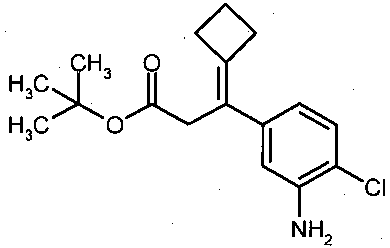

LC-MS (Methode 5): Rₜ = 1.27 min, m/z = 252 (M+H-C₄H₈)⁺.

¹H-NMR (400 MHz, DMSO-*d*₆): δ [ppm] = 1.31 (s, 9H), 1.93 (quin, 2H), 2.72-2.86 (m, 4H), 3.12 (s, 2H), 5.18-5.24 (m, 2H), 6.42 (dd, 1H), 6.69 (d, 1H), 7.06-7.11 (m, 1H).

Analog zu Synthesebeispiel 8A / 9A wurden die folgenden Verbindungen erhalten:

| **Beispiel** | **Name / Struktur / Edukte** | **Analytische Daten** |
|---|---|---|
| **10A** | *tert*.-Butyl-(2*E*/*Z*)-3-(3-amino-4-chlorphenyl)-3-(3,3-difluorcyclobutyl)acrylat | ¹H-NMR (400 MHz, DMSO-*d*₆): δ [ppm] = 1.46 (s, 9H), 2.23-2.41 (m, 2H), 2.77-2.90 (m, 2H), 3.88-4.01 (m, 1H), 5.45 (br. s, 2H), 5.73 (d, 1H), 6.41 (dd, 1H), 6.66 (d, 1H), 7.19 (d, 1H). |
| | 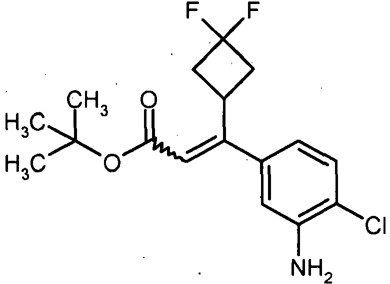 | |
| | | LC-MS (Methode 7): Rₜ = 1.37 min, m/z = 344 (M+H)⁺. |
| | aus *tert*.-Butyl-(2*E*)-3-(3,3-difluorcyclobutyl)-acrylat und 5-Brom-2-chloranilin | |
| **11A** | *tert.* -Butyl-3-(3-amino-4-chlorphenyl)-3-(3,3-difluorcyclobutyliden)propanoat | ¹H-NMR (400 MHz, DMSO-*d*₆): δ [ppm] = 1.31 (s, 9H), 3.24 (s, 2H), 3.32-3.47 (m, 4H, teilweise verdeckt durch H₂O-Signal), 5.30 (br. s, 2H), 6.46 (dd, 1H), 6.74 (d, 1H), 7.13 (d, 1H). |
| | 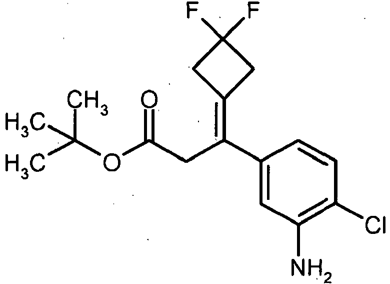 | |
| | | LC-MS (Methode 7): Rₜ = 1.28 min, m/z = 344 (M+H)⁺. |
| | aus *tert*.-Butyl-(2*E*)-3-(3,3-difluorcyclobutyl)-acrylat und 5-Brom-2-chloranilin | |
| **12A** | *tert.*-Butyl-(2*E*/*Z*)-3-(3-amino-4-chlorphenyl)-4-cyclopropylbut-2-enoat | ¹H-NMR (400 MHz, DMSO-*d*₆): δ [ppm] = 0.05-0.11 (m, 2H), 0.27-0.34 (m, 2H), 0.64-0.75 (m, 1H), 1.45 (s, 9H), 2.91 (d, 2H), 5.42 (br. s, 2H), 5.84 (s, 1H), 6.70 (dd, 1H), 6.96 (d, 1H), 7.19 (d, 1H). |
| | 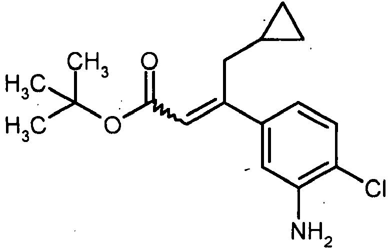 | |
| | | LC-MS (Methode 5): Rₜ = 1.35 min, m/z = 308 (M+H)⁺. |
| | aus *tert.* -Butyl-(2*E*)-4-cyclopropylbut-2-enoat und 5-Brom-2-chloranilin | |

### Beispiel 13A

### Methyl-3-(3-amino-4-chlorphenyl)-4-methylpentanoat

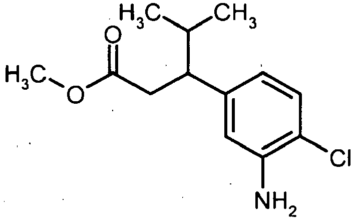

Zu 2.2 g (90.7 mmol) Magnesiumspänen und einigen Körnchen Iod wurde bei RT eine Lösung von 6.77 g (26.7 mmol) Methyl-(2*E*/*Z*)-3-(3-amino-4-chlorphenyl)-4-methylpent-2-enoat in 130 ml Methanol gegeben. Nach ca. 30 min stieg die Innentemperatur auf ca. 60°C an. Nachdem die Reaktionslösung auf Raumtemperatur abgekühlt war, wurde noch 2 h bei Raumtemperatur nachgerührt. Die dunkle Reaktionsmischung wurde dann langsam mit 50 ml gesättigter wässriger Ammoniumchlorid-Lösung versetzt und mehrfach mit Diethylether extrahiert. Die vereinigten organischen Phasen wurden mit gesättigter Natriumhydrogencarbonat-Lösung und gesättigter Natriumchlorid-Lösung gewaschen, über Magnesiumsulfat getrocknet und im Vakuum eingeengt. Der erhaltene Rückstand wurde durch Chromatographie an Silicagel gereinigt (Laufmittel Cyclohexan/Ethylacetat 10:1). Es wurden 2.95 g (40% d. Th.) der Titelverbindung als Öl erhalten.

LC-MS (Methode 5): Rₜ = 1.06 min; m/z = 256 (M+H)⁺.

¹H-NMR (400 MHz, DMSO-*d*₆): δ [ppm] = 0.69 (d, 3H), 0.87 (d, 3H), 1.67-1.80 (m, 1H), 2.44-2.56 (m, 1H, verdeckt durch DMSO-Signal), 2.57-2.66 (m, 1H), 2.69-2.77 (m, 1H), 3.46 (s, 3H), 5.15-5.26 (br. s, 2H), 6.35 (dd, 1H), 6.58 (d, 1H), 7.05 (d, 1H).

Analog zu Synthesebeispiel 13A wurden die folgenden Verbindungen erhalten:

| **Beispiel** | **Name / Struktur / Edukt** | **Analytische Daten** |
|---|---|---|
| **14A** | *tert.*-Butyl-3-(3-amino-4-chlorphenyl)-4-methoxy-4-methylpentanoat | LC-MS (Methode 7): |
| | | Rₜ = 1.26 min, m/z = 328/330 (M+H)⁺. |
| | 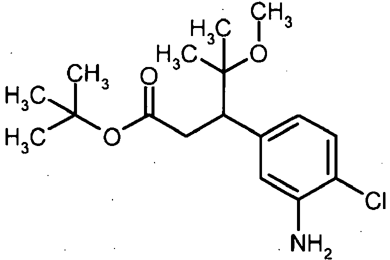 | |
| | aus *tert*.-Bütyl-(2*E*/*Z*)-3-(3-amino-4-chlorphenyl)-4-methoxy-4-methylpent-2-enoat | |
| **15A** | *tert.* -Butyl-3-(3-amino-4-chlorphenyl)-3-(3,3-difluorcyclobutyl)propanoat | ¹H-NMR (400 MHz, DMSO-*d*₆): δ [ppm] = 1.24 (s, 9H), 1.99-2.17 (m, 1H), 2.19-2.40 (m, 4H), 2.46-2.57 (m, 1H, teilweise verdeckt durch DMSO-Signal), 2.59-2.72 (m, 1H), 2.72-2.83 (m, 1H), 5.24 (br. s, 2H), 6.42 (dd, 1H), 6.63 (d, 1H), 7.07 (d, 1H). |
| | 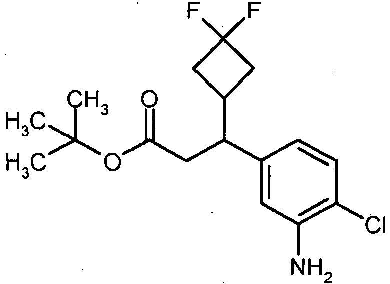 | |
| | | LC-MS (Methode 7): |
| | aus *tert*.-Butyl-(2*E*/*Z*)-3-(3-amino-4-chlorphenyl)-3-(3,3-difluorcyclobutyl)acrylat | Rₜ = 1.28 min, m/z = 346 (M+H)⁺. |
| **16A** | *tert.*-Butyl-3-(3-amino-4-chlorphenyl)-4-cyclopropylbutanoat | LC-MS (Methode 5): |
| | | Rₜ = 1.32 min, m/z = 310 (M+H)⁺. |
| | 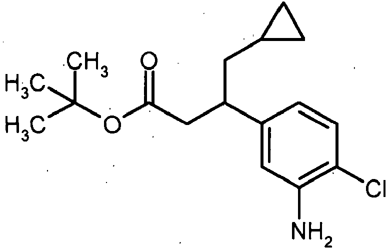 | |
| | aus *tert*.-Butyl-(2*E*/*Z*)-3-(3-amino-4-chlorphenyl)-4-cyclopropylbut-2-enoat | |

### Beispiel 17A und Beispiel 18A

### Methyl-3-(3-amino-4-chlorphenyl)-4-methylpentanoat (Enantiomer 1 und 2)

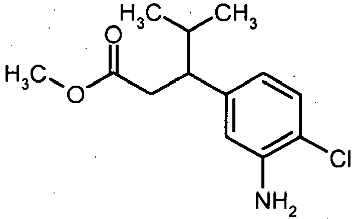

960 mg (3.75 mmol) des Racemats von Methyl-3-(3-amino-4-chlorphenyl)-4-methylpentanoat (Beispiel 13A) wurden mittels präparativer HPLC an chiraler Phase in die Enantiomere aufgetrennt [Säule: Daicel Chiralpak AD-H, 5 µm, 250 mm x 20 mm; Elutionsmittel: Isohexan/Isopropanol 90:10 (v/v); Fluss: 20 ml/min; UV-Detektion: 230 nm; Temperatur: 25°C]:

### Beispiel 17A (Enantiomer 1):

Ausbeute: 315 mg

Rₜ = 6.90 min; chemische Reinheit >99%; >99% ee
[Säule: Daicel AD-H, 5 µm, 250 mm x 4 mm; Elutionsmittel: Isohexan/(Isopropanol + 0.2% Diethylamin) 90:10 (v/v); Fluss: 1 ml/min; UV-Detektion: 220 nm; Temperatur: 25°C].

LC-MS (Methode 8): Rₜ = 2.34 min; m/z = 256 (M+H)⁺.

¹H-NMR (400 MHz, DMSO-*d*₆): δ [ppm] = 0.69 (d, 3H), 0.87 (d, 3H), 1.67-1.80 (m, 1H), 2.44-2.56 (m, 1H, verdeckt durch DMSO-Signal), 2.57-2.66 (m, 1H), 2.69-2.77 (m, 1H), 3.46 (s, 3H), 5.15-5.26 (br. s, 2H), 6.35 (dd, 1H), 6.58 (d, 1H), 7.05 (d, 1H).

### Beispiel 18A (Enantiomer 2):

Ausbeute: 247 mg

Rₜ = 7.76 min; chemische Reinheit >99%; >99% ee
[Säule: Daicel AD-H, 5 µm, 250 mm x 4 mm; Elutionsmittel: Isohexan/(Isopropanol + 0.2% Diethylamin) 90:10 (v/v); Fluss: 1 ml/min; UV-Detektion: 220 nm; Temperatur: 25°C].

LC-MS (Methode 8): Rₜ = 2.34 min; m/z = 256 (M+H)⁺.

¹H-NMR (400 MHz, DMSO-*d*₆): δ [ppm] = 0.69 (d, 3H), 0.87 (d, 3H), 1.67-1.80 (m, 1H), 2.44-2.56 (m, 1H, verdeckt durch DMSO-Signal), 2.57-2.66 (m, 1H), 2.69-2.77 (m, 1H), 3.46 (s, 3H), 5.15-5.26 (br. s, 2H), 6.35 (dd, 1H), 6.58 (d, 1H), 7.05 (d, 1H).

### Beispiel 19A

### 2-Chlor-5-iod-N,N-bis(4-methoxybenzyl)anilin

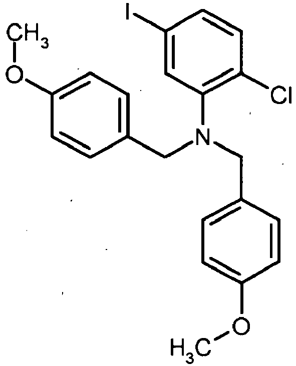

Unter Argon wurden 12.62 g (316.16 mmol, 60% in Mineralöl) Natriumhydrid in 250 ml abs. DMF suspendiert und auf 0°C abgekühlt. Anschließend wurden 32 g (126.3 mmol) 2-Chlor-5-iodanilin, gelöst in 80 ml abs. DMF, langsam zugetropft und das Gemisch 30 min bei 0°C gerührt. Danach wurden langsam 41 ml (303 mmol) 1-(Chlormethyl)-4-methoxybenzol zur Reaktionsmischung gegeben und der Ansatz dann auf Raumtemperatur erwärmt. Das Gemisch wurde über Nacht bei RT gerührt und danach vorsichtig auf 150 ml Eiswasser gegossen. Nach Abtrennung der organischen Phase wurde die wässrige Phase noch dreimal mit Diethylether extrahiert. Die vereinigten organischen Phasen wurden über Magnesiumsulfat getrocknet. Nach Filtration wurde das Lösungsmittel im Vakuum entfernt. Das erhaltene Rohprodukt wurde durch Chromatographie an Silicagel gereinigt (Laufmittel Cyclohexan/Ethylacetat 40:1). Es wurden 59 g der Titelverbindung erhalten (94% d. Th.).

LC-MS (Methode 4): Rₜ = 1.77 min; m/z = 494/496 (M+H)⁺.

¹H-NMR (400 MHz, DMSO-*d*₆): δ [ppm] = 3.71 (s, 6H), 4.08 (s, 4H), 6.86 (d, 4H), 7.22 (d, 5H), 7.29-7.35 (m, 2H).

### Beispiel 20A

### {3-[Bis(4-methoxybenzyl)amino]-4-chlorphenyl}(1-methylcyclopropyl)methanon

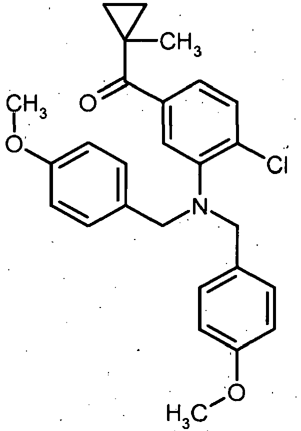

7.587 g (15.37 mmol) 2-Chlor-5-iod-*N*,*N-*bis(4-methoxybenzyl)anilin wurden unter Argon in 100 ml THF gelöst und auf -78°C abgekühlt. Anschließend wurden 7.65 ml (15.27 mmol) einer 2 M Lösung von Isopropylmagnesiumchlorid in Diethylether langsam zugetropft. Die Reaktionslösung wurde danach langsam auf -40°C erwärmt und 30 min bei dieser Temperatur nachgerührt. Anschließend wurden zur Reaktionslösung langsam 2 g (13.97 mmol) *N*-Methoxy-*N*,1-dimiethyl-cyclopropancarboxamid [R. Shintani et al., Chem. Eur. J., 15 (35), 8692-8694 (2009)], gelöst in 20 ml THF, getropft. Das erhaltene Reaktionsgemisch wurde danach langsam auf Raumtemperatur erwärmt und über Nacht bei dieser Temperatur nachgerührt. Dann wurden 50 ml einer eiskalten, gesättigten wässrigen Ammoniumchlorid-Lösung zur Reaktionsmischung gegeben. Nach Trennung der Phasen wurde die wässrige Phase noch dreimal mit Essigsäureethylester extrahiert, und die vereinigten organischen Phasen wurden über Magnesiumsulfat getrocknet, filtriert und bis zur Trockene eingeengt. Das erhaltene Rohprodukt wurde über Kieselgel chromatographisch gereinigt (Laufmittel Cyclohexan/Essigsäureethylester 10:1). Es wurden 3.977 g (63% d. Th.) der Titelverbindung erhalten.

LC-MS (Methode 5): Rₜ = 1.50 min; m/z = 450/452 (M+H)⁺.

¹H-NMR (400 MHz, DMSO-*d*₆): δ [ppm] = 0.72-0.76 (m, 2H), 0.93-0.98 (m, 2H), 1.09 (s, 3H), 3.69 (s, 6H), 4.15 (s, 4H), 6.85 (d, 4H), 7.23 (d, 4H), 7.25-7.29 (m, 2H), 7.52-7.57 (m, 1H).

### Beispiel 21A

### tert. -Butyl-(2E/Z)-3-{3-[bis(4-methoxybenzyl)amino]-4-chlorphenyl}-3-(1-methylcyclopropyl)-acrylat

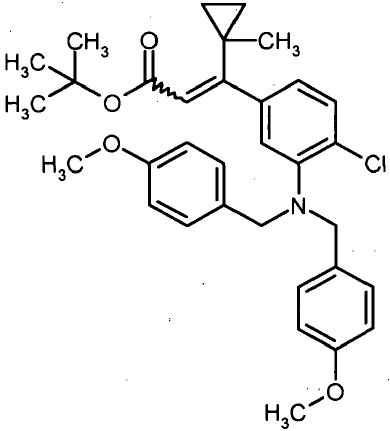

Zu einer auf 0°C gekühlten Suspension von 143 mg (60% in Mineralöl, 3.57 mmol) Natriumhydrid in 15 ml THF wurden 0.84 ml (3.57 mmol) *tert*.-Butyl-(diethoxyphosphoryl)acetat getropft. Nach 30 min wurden 1070 mg (2.38 mmol) {3-[Bis(4-methoxybenzyl)amino]-4-chlorphenyl}(1-methylcyclopropyl)methanon, gelöst in 10 ml THF, hinzugefügt. Das Kältebad wurde entfernt und die Reaktionsmischung über Nacht bei RT gerührt. Dann wurden 50 ml einer eiskalten, gesättigten wässrigen Ammoniumchlorid-Lösung zur Reaktionsmischung gegeben. Nach Trennung der Phasen wurde die wässrige Phase noch dreimal mit Essigsäureethylester extrahiert, und die vereinigten organischen Phasen wurden über Magnesiumsulfat getrocknet, filtriert und bis zur Trockene eingeengt. Der Rückstand wurde durch Chromatographie an Silicagel gereinigt (Laufmittel Cyclohexan/Ethylacetat 50:1). Es wurden 960 mg des Zielprodukts als *E*/*Z*-Isomerengemisch erhalten (74% d. Th.). LC-MS (Methode 7): Rₜ = 1.67 min (*Isomer 1*), m/z = 548/550 (M+H)⁺; Rₜ = 1.70 min (*Isomer 2*), m/z = 548/550 (M+H)⁺.

### Beispiel 22A

### tert.-Butyl-3-{3-[bis(4-methoxybenzyl)amino]-4-chlorphenyl}-3-(1-methylcyclopropyl)propanoat

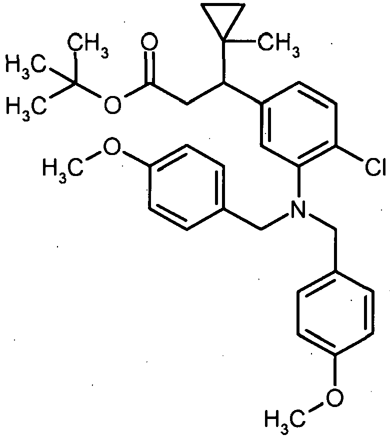

130 mg (1.58 mmol) Magnesiumspäne und einige Körnchen Iod wurden vorgelegt, mit 865 mg (1.58 mmol) *tert*. -Butyl-(2*E*/*Z*)-3-{3-[bis(4-methoxybenzyl)amino]-4-chlorphenyl}-3-(1-methylcyclopropyl)acrylat in 10 ml Methanol versetzt und bei Raumtemperatur gerührt. Nach ca. 10 min begann eine schwache Gasentwicklung in Verbindung mit einem Temperaturanstieg. Mit Hilfe eines Eisbades wurde die Temperatur bei 35°-40°C gehalten. Nach Beendigung der Reaktion wurden dem Reaktionsgemisch 10 ml einer gesättigten wässrigen Ammoniumchlorid-Lösung und 20 ml Dichlormethan zugesetzt. Anschließend wurde die organische Phase abgetrennt und die wässrige Phase noch dreimal mit jeweils ca. 10 ml Dichlormethan extrahiert. Die vereinigten organischen Phasen wurden über Magnesiumsulfat getrocknet und im Vakuum eingeengt. Aus dem Rückstand wurde das Produkt durch präparative RP-HPLC isoliert (Eluent Methanol/Wasser 9:1 isokratisch). Es wurden 159 mg des Zielprodukts erhalten (18% d. Th.).

LC-MS (Methode 4): Rₜ = 1.91 min; m/z = 550/552 (M+H)⁺.

### Beispiel 23A

### tert.-Butyl-3-(3-amino-4-chlorphenyl)-3-(1-methylcyclopropyl)propanoat

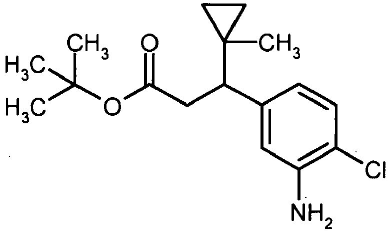

159 mg (0.29 mmol) *tert*.-Butyl-3-{3-[bis(4-methoxybenzyl)amino]-4-chlorphenyl}-3-(1-methylcyclopropyl)propanoat wurden in 7 ml Dichlormethan und 1.2 ml Wasser aufgenommen. Anschließend wurden 145 mg (0.64 mmol) 2,3-Dichlor-5,6-dicyano-1,4-benzochinon (DDQ) hinzugefügt und die Reaktionslösung 2 h bei Raumtemperatur gerührt. Danach wurde das Reaktionsgemisch auf 10 ml gesättigte wässrige Natriumhydrogencarbonat-Lösung gegeben. Nach Trennung der Phasen wurde die wässrige Phase noch dreimal mit jeweils ca. 10 ml Dichlormethan extrahiert. Die vereinigten organischen Phasen wurden über Magnesiumsulfat getrocknet und im Vakuum eingeengt. Aus dem Rückstand wurde das Produkt durch präparative RP-HPLC isoliert (Eluent Methanol/Wasser). Es wurden 31 mg des Zielprodukts erhalten (34% d. Th.).

LC-MS (Methode 7): Rₜ = 1.35 min; m/z = 310 (M+H)⁺.

### Beispiel 24A

### (4-Chlor-3-nitrophenyl)(cyclopropyl)methanon

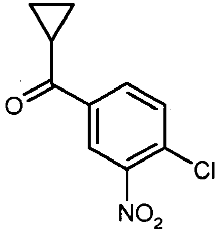

Unter Argon wurden zu 60 ml konzentrierter Salpetersäure bei -10°C langsam 20 g (110.7 mmol) (4-Chlorphenyl)(cyclopropyl)methanon gegeben. Anschließend wurde das Reaktionsgemisch langsam auf 5°C erwärmt und 6 h bei dieser Temperatur gerührt. Danach wurde die Reaktionslösung vorsichtig unter Rühren auf ca. 100 ml Eiswasser gegeben. Dabei fiel ein weißer Feststoff aus, der abgesaugt und mehrfach mit Wasser gewaschen wurde. Der so erhaltene Feststoff wurde dann im Hochvakuum getrocknet. Es wurden 24.3 g (97% d. Th.) des gewünschten Produkts erhalten.

LC-MS (Methode 7): Rₜ = 1.06 min; m/z = 224/226 (M-H)⁻.

¹H-NMR (400 MHz, DMSO-*d*₆): δ [ppm] = 1.05-1.18 (m, 4H), 2.92-3.02 (m, 1H), 7.97 (d, 1H), 8.32 (dd, 1H), 8.66 (d, 1H).

Analog zu Synthesebeispiel 24A wurde die folgende Verbindung erhalten:

| **Beispiel** | **Name / Struktur / Edukt** | **Analytische Daten** |
|---|---|---|
| **25A** | (4-Chlor-3-nitrophenyl)(1-fluorcyclopropyl)-methanon | LC-MS (Methode 7): |
| | | Rₜ = 1.11 min, m/z = 242 (M-H)⁻. |
| | 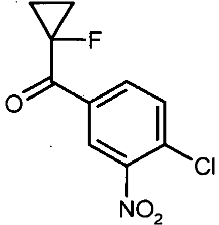 | |
| | aus (4-Chlorphenyl)(1-fluorcyclopropyl)methanon [Herstellung gemäß DE 3704262-A1, Beispiel (II-1)] | |

### Beispiel 26A

### tert.-Butyl-(2E/Z)-3-(4-chlor-3-nitrophenyl)-3-cyclopropylacrylat

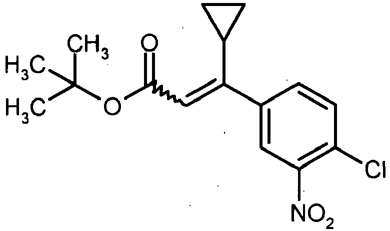

Zu einer auf 0°C gekühlten Suspension von 2.3 g (60% in Mineralöl, 57.6 mmol) Natriumhydrid in 50 ml THF und 50 ml DMF wurden 13.5 ml (57.6 mmol) *tert*.-Butyl-(diethoxyphosphoryl)acetat getropft. Nach 30 min wurden 10 g (44.3 mmol) (4-Chlor-3-nitrophenyl)(cyclopropyl)methanon portionsweise hinzugefügt, das Kältebad entfernt und die Reaktionsmischung über Nacht bei RT gerührt. Dann wurden 50 ml einer eiskalten, gesättigten wässrigen Ammoniumchlorid-Lösung zur Reaktionsmischung gegeben. Nach Trennung der Phasen wurde die wässrige Phase noch dreimal mit Essigsäureethylester extrahiert, und die vereinigten organischen Phasen wurden über Magnesiumsulfat getrocknet, filtriert und bis zur Trockene eingeengt. Der Rückstand wurde durch Chromatographie an Silicagel gereinigt (Laufmittel Cyclohexan → Cyclohexan/Ethylacetat 40:1). Erhalten wurden 13.4 g des Zielprodukts als *E*/*Z*-Isomerengemisch (93% d. Th.).

MS (DCI): m/z = 324 (M+H)⁺, 341 (M+NH₄)⁺.

¹H-NMR (400 MHz, DMSO-*d*₆): δ [ppm] = 0.32-0.39 (m, 0.5H), 0.51-0.58 (m, 1.5H), 0.79-0.87 (m, 1.5H), 0.88-0.96 (m, 0.5H), 1.17 (s, 6.75H), 1.47 (s, 2.25H), 1.73-1.82 (m, 0.75H), 2.81-2.90 (m, 0.25H), 5.84 (s, 0.25H), 5.88 (s, 0.75H), 7.43 (dd, 0.75H), 7.59 (dd, 0.25H), 7.72-7.78 (m, 1H), 7.81 (d, 0.75H), 7.95 (d, 0.25H).

Analog zu Synthesebeispiel 26A wurden die folgenden Verbindungen erhalten:

| **Beispiel** | **Name / Struktur / Edukte** | **Analytische Daten** |
|---|---|---|
| **27A** | *tert.-*Butyl-(2*E*/*Z*)-3-(4-chlor-3-nitrophenyl)-3-(1-fluorcyclopropyl)acrylat | MS (DCI): m/z = 359 (M+NH₄)⁺. |
| | 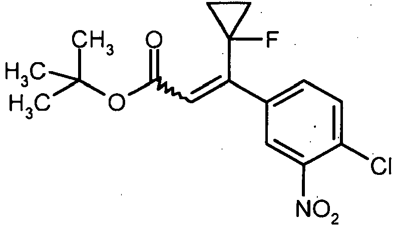 | ¹H-NMR (400 MHz, DMSO-*d*₆): δ [ppm] = 1.01-1.10 (m, 2H), 1.19 (s, 7.74H), 1.31-1.41 (m, 2H), 1.51 (s, 1.26H), 6.13 (s, 0.86H), 6.77 (s, 0.14H), 7.55 (dd, 1H), 7.81 (d, 0.86H), 7.84 (d, 0.14H), 7.95 (d, 0.86H), 8.29 (d, 0.14H). |
| | aus (4-Chlor-3-nitrophenyl)(1-fluorcyclopropyl)-methanon und *tert.* -Butyl-(diethoxyphosphoryl)acetat | |
| **28A** | Ethyl-(2*E*/*Z*)-3-(4-chlor-3-nitrophenyl)-3-cyclopropyl-2-methylacrylat | MS (DCI): m/z = 327 (M+NH₄)⁺. |
| | 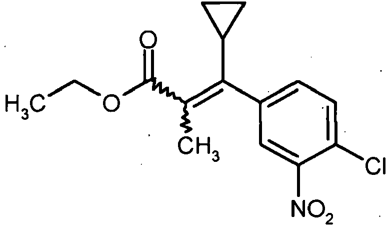 | LC-MS (Methode 7): |
| | | Rₜ = 1.26 min; m/z = 310 (M+H)⁺. |
| | aus (4-Chlor-3-nitrophenyl)(cyclopropyl)methanon und Ethyl-2-(diethoxyphosphoryl)propanoat | |

### Beispiel 29A

### tert.-Butyl-3-(3-amino-4-chlorphenyl)-3-cyclopropylpropanoat

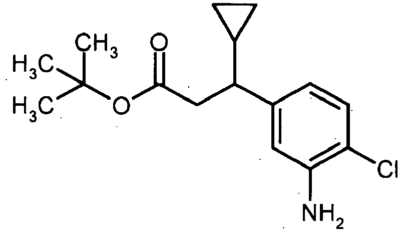

200 mg (0.62 mmol) *tert*.-Butyl-(2*E*/*Z*)-3-(4-chlor-3-nitrophenyl)-3-cyclopropylacrylat wurden in 12 ml Ethylacetat gelöst und mit 20 mg (0.06 mmol) Platin (10% auf Kohle) versetzt. Die Reaktionsmischung wurde bei RT 12 Stunden unter einer Wasserstoffatmosphäre bei Normaldruck gerührt. Das Reaktionsgemisch wurde danach über Kieselgur abgesaugt und das Filtrat eingeengt. Das Rohprodukt wurde durch Chromatographie an Silicagel gereinigt (Laufmittel Cyclohexan/ Ethylacetat 40:1). Es wurden 96 mg (52.1% d. Th.) der Zielverbindung erhalten.

LC-MS (Methode 5): Rₜ = 1.24 min; m/z = 296 (M+H)⁺.

### Beispiel 30A und Beispiel 31A

### tert.-Butyl-3-(3-amino-4-chlorphenyl)-3-cyclopropylpropanoat (Enantiomer 1 und 2)

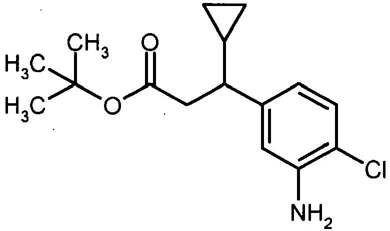

500 mg (1.69 mmol) des Racemats von *tert*.-Butyl-3-(3-amino-4-chlorphenyl)-3-cyclopropylpropanoat (Beispiel 29A) wurden mittels präparativer HPLC an chiraler Phase in die Enantiomere aufgetrennt [Säule: Daicel Chiralpak AZ-H, 5 µm, 250 mm x 20 mm; Elutionsmittel: Isohexan/ Ethanol 90:10 (v/v); Fluss: 15 ml/min; UV-Detektion: 220 nm; Temperatur: 30°C]:

### Beispiel 30A (Enantiomer 1):

Ausbeute: 237 mg

Rₜ = 4.91 min; chemische Reinheit >99%; >99% ee
[Säule: Daicel AZ-H, 5 µm, 250 mm x 4.6 mm; Elutionsmittel: Isohexan/Ethanol 90:10 (v/v); Fluss: 1 ml/min; UV-Detektion: 220 nm; Temperatur: 30°C].

LC-MS (Methode 5): Rₜ = 1.23 min; m/z = 296 (M+H)⁺.

¹H-NMR (400 MHz, DMSO-*d*₆): δ [ppm] = 0.02-0.10 (m, 1H), 0.16-0.25 (m, 1H), 0.27-0.36 (m, 1H), 0.45-0.54 (m, 1H), 0.85-0.98 (m, 1H), 1.28 (s, 9H), 2.02-2.11 (m, 1H), 2.43-2.62 (m, 2H, teilweise verdeckt durch DMSO-Signal), 5.21 (br. s, 2H), 6.43 (dd, 1H), 6.64 (d, 1H), 7.06 (d, 1H).

[α]_{D}²⁰ = -22.3°, c = 0.465, Methanol.

### Beispiel 31A (Enantiomer 2):

Ausbeute: 207 mg

Rₜ = 5.25 min; chemische Reinheit >99%; >99% ee
[Säule: Daicel AZ-H, 5 µm, 250 mm x 4.6 mm; Elutionsmittel: Isohexan/Ethanol 90:10 (v/v); Fluss: 1 ml/min; UV-Detektion: 220 nm; Temperatur: 30°C].

LC-MS (Methode 5): Rₜ = 1.23 min; m/z = 296 (M+H)⁺.

¹H-NMR (400 MHz, DMSO-*d*₆): δ [ppm] = 0.02-0.10 (m, 1H), 0.16-0.25 (m, 1H), 0.27-0.36 (m, 1H), 0.45-0.54 (m, 1H), 0.85-0.98 (m, 1H), 1.28 (s, 9H), 2.02-2.11 (m, 1H), 2.43-2.62 (m, 2H, teilweise verdeckt durch DMSO-Signal), 5.21 (br. s, 2H), 6.43 (dd, 1H), 6.64 (d, 1H), 7.06 (d, 1H).

[α]_{D}²⁰ = +24.1°, c = 0.330, Methanol.

### Beispiel 32A

### tert.-Butyl-3-(3-amino-4-chlorphenyl)-3-(1-fluorcyclopropyl)propanoat

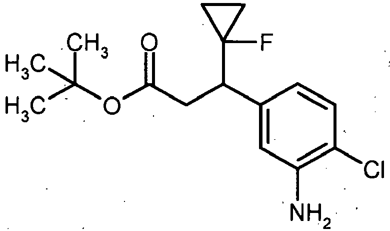

384 mg (1.12 mmol) *tert*.-Butyl-(2*E*/*Z*)-3-(4-chlor-3-nitrophenyl)-3-(1-fluorcyclopropyl)acelat wurden in 12 ml Ethylacetat gelöst und mit 38 mg (0.17 mmol) Platin(IV)oxid versetzt. Die Reaktionsmischung wurde bei RT über Nacht unter einer Wasserstoffatmosphäre bei Normaldruck gerührt. Das Reaktionsgemisch wurde danach über Kieselgur abgesaugt und das Filtrat eingeengt. Aus dem Rückstand wurde das Produkt durch präparative RP-HPLC isoliert (Eluent Methanol/ Wasser). Es wurden 68 mg (19% d. Th.) der Zielverbindung erhalten.

LC-MS (Methode 7): Rₜ = 1.24 min; m/z = 314 (M+H)⁺.

### Beispiel 33A

### (+/-)-tert.-Butyl-3-(3-amino-4-chlorphenyl)-3-cyclobutylpropanoat

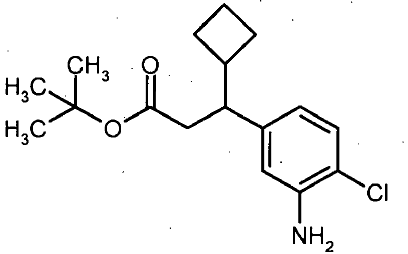

### Verfahren A:

133 mg (9.432 mmol) *tert*.-Butyl-3-(3-amino-4-chlorphenyl)-3-cyclobutylidenpropanoat wurden in 20 ml Ethylacetat gelöst. Die Lösung wurde mit Argon deoxygeniert und mit 30 mg 10%-igem Palladium auf Kohle versetzt. Die Reaktionsmischung wurde über Nacht bei RT unter einer Wasserstoffatmosphäre bei Normaldruck gerührt. Danach wurde über Celite abfiltriert und das Filtrat im Vakuum eingeengt. Aus dem Rückstand wurde das Produkt durch präparative RP-HPLC isoliert (Eluent Acetonitril/Wasser). Es wurden 67 mg der Zielverbindung erhalten (50% d. Th.).

LC-MS (Methode 5): Rₜ = 1.31 min; m/z = 310 (M+H)⁺.

¹H-NMR (400 MHz, DMSO-*d*₆): δ [ppm] = 1.24 (s, 9H), 1.47-1.57 (m, 1H), 1.57-1.77 (m, 4H), 1.94-2.05 (m, 1H), 2.19 (dd, 1H), 2.31-2.40 (m, 1H), 2.43 (dd, 1H), 2.71 (td, 1H), 5.13-5.22 (m, 2H), 6.36 (dd, 1H), 6.59 (d, 1H), 7.04 (d, 1H).

### Verfahren B:

Zu 39 mg (1.60 mmol) Magnesiumspänen und einigen Körnchen Iod wurde bei RT eine Lösung von 189 mg (0.614 mmol) *tert*.-Butyl-3-(3-amino-4-chlorphenyl)-3-cyclobutylacrylat in 0.9 ml Methanol gegeben. Die dunkle Reaktionsmischung wurde über Nacht bei RT gerührt, dann auf Wasser gegeben und mit Ethylacetat extrahiert. Die organische Phase wurde mit gesättigter Natriumhydrogencarbonat-Lösung und gesättigter Natriumchlorid-Lösung gewaschen, über Magnesiumsulfat getrocknet und im Vakuum eingeengt. Aus dem Rückstand wurde das Produkt durch präparative RP-HPLC isoliert. Es wurden 57.7 mg der Zielverbindung erhalten (30.3% d. Th.).

### Beispiel 34A

### Ethyl-(2E/Z)-3-(3-amino-4-chlorphenyl)-3-cyclopropyl-2-methylacrylat

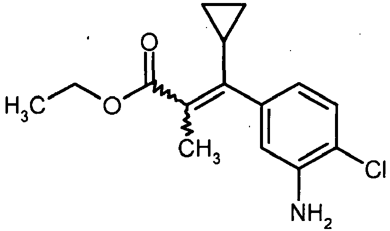

Unter Argon wurden 2.53 g (8.17 mmol) Ethyl-(2*E*/*Z*)-3-(4-chlor-3-nitrophenyl)-3-cyclopropyl-2-methylacrylat in 10 ml Dioxan gelöst und mit 9.22 g (40,84 mmol) Zinn(II)chlorid-Dihydrat versetzt. Anschließend wurde die Reaktionsmischung auf 70°C erwärmt und über Nacht bei dieser Temperatur gerührt. Nach Abkühlen auf Raumtemperatur wurde das Reaktionsgemisch mit ca. 20 ml Essigsäureethylester versetzt und dann auf ca. 20 ml einer 10%-igen wässrigen Kaliumfluorid-Lösung gegeben. Das resultierende Gemisch wurde 10 min kräftig gerührt. Nach Abtrennung der Phasen wurde die wässrige Phase noch zweimal mit je 10 ml Essigsäureethylester extrahiert. Die vereinigten organischen Phasen wurden mit ca. 50 ml einer gesättigten Natriumchlorid-Lösung gewaschen, über Magnesiumsulfat getrocknet und im Vakuum eingeengt. Es wurden 2.2 g (96% d. Th.) der Zielverbindung erhalten, welche ohne weitere Aufreinigung in der nächsten Stufe eingesetzt wurde.

LC-MS (Methode 7): Rₜ = 1.19 min; m/z = 280/282 (M+H)⁺.

### Beispiel 35A

### Ethyl-3-(3-amino-4-chlorphenyl)-3-cyclopropyl-2-methylpropanoat (Diastereomerengemisch)

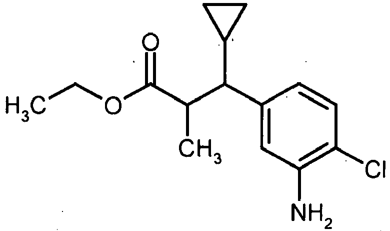

Zu 497 mg (20.45 mmol) Magnesiumspänen und einigen Körnchen Iod wurde unter Argon bei RT eine Lösung von 2.2 g (7.86 mmol) Ethyl-(2*E*/*Z*-)-3-(3-amino-4-chlorphenyl)-3-cyclopropyl-2-methylacrylat in 20 ml Methanol gegeben. Die dunkle Reaktionsmischung wurde über Nacht bei RT gerührt und anschließend unter Argon zwei Tage stehengelassen. Danach wurde die Reaktionslösung mit Ethylacetat verdünnt und mit 1 M Salzsäure versetzt. Das Gemisch wurde 5 min gerührt und anschließend mit gesättigter Natriumhydrogencarbonat-Lösung auf pH 8-9 eingestellt. Die organische Phase wurde abgetrennt, und die wässrige Phase wurde noch zweimal mit Essigsäureethylester extrahiert. Die vereinigten organischen Phasen wurden mit gesättigter Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Ethylacetat 100:1 → 50:1 → 20:1). Es wurden 1.38 g (62% d. Th.) der Zielverbindung erhalten.

LC-MS (Methode 5): Rₜ = 1.13 min; m/z = 282/284 (M+H)⁺.

### Beispiel 36A

### Dimethyl-(3-methylbutan-2-yliden)malonat

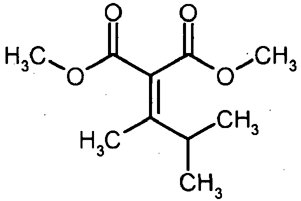

Unter Argon wurden bei 0°C zu einer Lösung von 16.6 ml (151.4 mmol) Titantetrachlorid in 60 ml Chloroform 10 g (75.7 mmol) Malonsäuredimethylester in 20 ml Chloroform langsam zugetropft. Nach Beendigung der Zugabe wurde die Reaktionslösung noch 30 min bei 0°C nachgerührt. Anschließend wurden bei 0°C 6.52 g (75.7 mmol) 3-Methyl-2-butanon in 20 ml Chloroform zugetropft. Das Reaktionsgemisch wurde langsam auf Raumtemperatur erwärmt und 4 h bei dieser Temperatur nachgerührt. Danach wurde die Reaktionslösung wieder auf 0°C abgekühlt und mit 30.6 ml (378.5 mmol) Pyridin in 20 ml Chloroform versetzt. Nach Beendigung der Zugabe wurde die Lösung auf Raumtemperatur erwärmt und über Nacht bei dieser Temperatur nachgerührt. Anschließend wurde die Reaktionslösung wieder auf 0°C abgekühlt und langsam mit 50 ml Wasser versetzt. Die erhaltenen Phasen wurden getrennt und die wässrige Phase noch zweimal mit je ca. 50 ml Dichlormethan extrahiert. Die vereinigten organischen Phasen wurden mit gesättigter Natriumhydrogencarbonat-Lösung und mit gesättigter Natriumchlorid-Lösung gewaschen, über Magnesiumsulfat getrocknet und im Vakuum eingeengt. Das Rohprodukt wurde durch Chromatographie an Silicagel gereinigt (Laufmittel Cyclohexan/Ethylacetat 20:1). Es wurden 9.4 g (62% d. Th.) der Zielverbindung erhalten.

GC-MS (Methode 1): Rₜ = 3.57 min; m/z = 185 (M-CH₃)⁺.

¹H-NMR (400 MHz, DMSO-*d*₆): δ [ppm] = 1.00 (d, 6H), 1.92 (s, 3H), 2.86-2.98 (m, 1H), 3.67 (s, 3H), 3.69 (s, 3H).

Analog zu Synthesebeispiel 36A wurden die folgenden Verbindungen erhalten:

| **Beispiel** | **Name / Struktur / Edukte** | **Analytische Daten** |
|---|---|---|
| **37A** | Dimethyl-(1-cyclobutylethyliden)malonat | MS (DCI): m/z = 213 (M+H)⁺. |
| | 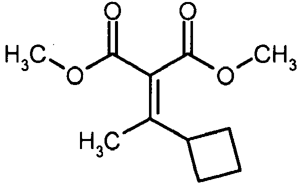 | ¹H-NMR (400 MHz, DMSO-*d*₆): δ [ppm] = 1.64-1.77 (m, 1H), 1.79-1.93 (m, 1H), 1.94-2.09 (m, 4H), 2.03 (s, 3H), 3.43-3.55 (m, 1H), 3.66 (s, 3H), 3.68 (s, 3H). |
| | aus Malonsäuredimethylester und 1-Cyclobutylethanon | |
| **38A** | Dimethyl-(1-cyclopropylethyliden)malonat | GC-MS (Methode 1): |
| | 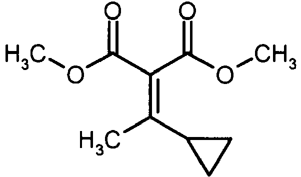 | Rₜ = 4.36 min; m/z = 198 (M)⁺. |
| | | ¹H-NMR (400 MHz, DMSO-*d*₆): δ [ppm] = 0.83-0.89 (m, 4H), 1.62 (s, 3H), 2.10-2.20 (m, 1H), 3.67 (s, 3H), 3.70 (s, 3H). |
| | aus Malonsäuredimethylester und 1-Cyclopropylethanon | |

### Beispiel 39A

### Dimethyl-[2-(4-chlorphenyl)-3-methylbutan-2-yl]malonat

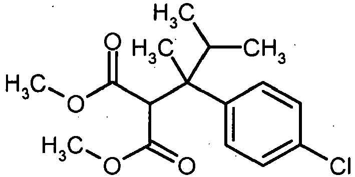

6.2 g (26 mmol) 1-Chlor-4-iodbenzol wurden unter Argon in 50 ml THF gelöst und auf -78°C abgekühlt. Anschließend wurden 24 ml (31.2 mmol) einer 1.3 M Lösung von Isopropylmagnesiumchlorid x Lithiumchlorid in THF langsam zugetropft. Die Reaktionslösung wurde danach langsam auf -40°C erwärmt und 2 h bei dieser Temperatur nachgerührt. Dann wurde die Reaktionslösung auf -10°C erwärmt und mit 495 mg (2.6 mmol) Kupfer(I)iodid versetzt. Anschließend wurden zu der Reaktionslösung langsam 5 g (24.97 mmol) Dimethyl-(3-methylbutan-2-yliden)malonat, gelöst in 30 ml THF, zugetropft. Das erhaltene Reaktionsgemisch wurde langsam auf Raumtemperatur erwärmt und 1 h bei dieser Temperatur nachgerührt. Danach wurde das Gemisch auf 0°C abgekühlt und vorsichtig mit eiskalter 1 M Salzsäure versetzt (pH ∼2). Nach Trennung der Phasen wurde die wässrige Phase noch dreimal mit Essigsäureethylester extrahiert, und die vereinigten organischen Phasen wurden über Magnesiumsulfat getrocknet, filtriert und bis zur Trockene eingeengt. Das erhaltene Rohprodukt wurde zunächst über Kieselgel chromatographisch vorgereinigt (Laufmittel Cyclohexan/Essigsäureethylester 20:1). Anschließend wurde das Produkt durch präparative RP-HPLC nachgereinigt (Eluent Methanol/Wasser). Es wurden 3.38 g (42% d. Th.) der Zielverbindung erhalten.

¹H-NMR (400 MHz, DMSO-*d*₆): δ [ppm] = 0.63-0.71 (m, 6H), 1.52 (s, 3H), 2.11-2.24 (m, 1H), 3.43 (s, 3H), 3.63 (s, 3H), 4.31 (s, 1H), 7.29-7.38 (m, 4H).

Analog zu Synthesebeispiel 39A wurden die folgenden Verbindungen erhalten:

| **Beispiel** | **Name / Struktur / Edukte** | **Analytische Daten** |
|---|---|---|
| **40A** | Dimethyl-[1-(4-chlorphenyl)-1-cyclobutyl-ethyl]malonat | ¹H-NMR (400 MHz, DMSO-*d*₆): δ [ppm] = 1.34-1.49 (m, 3H), 1.53 (s, 3H), 1.55-1.65 (m, 2H), 1.66-1.76 (m, 1H), 2.79-2.91 (m, 1H), 3.38 (s, 3H), 3.66 (s, 3H), 4.07 (s, 1H), 7.35 (q, 4H). |
| | 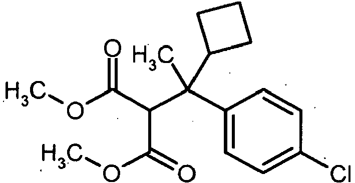 | |
| | aus 1-Chlor-4-iodbenzol und Dimethyl-(1-cyclobutylethyliden)malonat | |
| **41A** | Dimethyl-(1-{3-[bis(4-methoxybenzyl)amino]-4-chlorphenyl}-1-cyclopropylethyl)malonat | LC-MS (Methode 5): |
| | | Rₜ = 1.53 min; m/z = 566/568 (M+H)⁺. |
| | 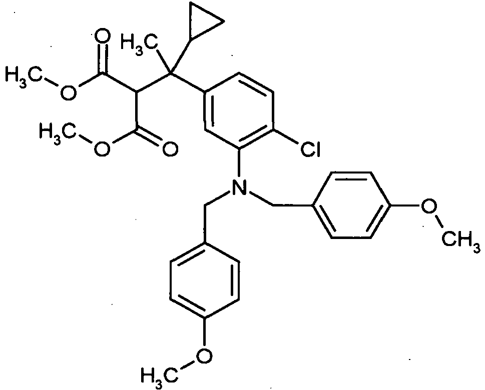 | ¹H-NMR (400 MHz, DMSO-*d*₆): δ [ppm] = -0.16--0.07 (m, 1H), 0.01-0.09 (m, 1H), 0.16-0.24 (m, 1H), 0.24-0.32 (m, 1H), 1.04 (s, 3H), 1.35-1.44 (m, 1H), 3.46 (s, 3H), 3.50 (s, 3H), 3.69 (s, 6H), 4.06 (s, 4H), 4.15 (s, 1H), 6.83 (d, 4H), 7.05 (dd, 1H), 7.10 (d, 1H), 7.21 (d, 4H), 7.28 (d, 1H). |
| | aus 2-Chlor-5-iod-*N,N-*bis(4-methoxybenzyl)anilin und Dimethyl-(1-cyclopropylethyliden)malonat | |

### Beispiel 42A

### Dimethyl-[1-(3-amino-4-chlorphenyl)-1-cyclopropylethyl]malonat

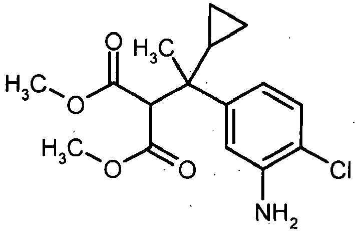

627 mg (1.11 mmol) Dimethyl-(1-{3-[bis(4-methoxybenzyl)amino]-4-chlorphenyl}-1-cyclopropylethyl)malonat wurden in 60 ml Dichlormethan und 15 ml Wasser aufgenommen. Anschließend wurden 553 mg (2.44 mmol) 2,3-Dichlor-5,6-dicyano-1,4-benzochinon (DDQ) hinzugefügt und das Reaktionsgemisch 2 Stunden bei Raumtemperatur gerührt. Danach wurde die Reaktionsmischung auf ca. 50 ml gesättigte wässrige Natriumhydrogencarbonat-Lösung gegeben. Nach Trennung der Phasen wurde die wässrige Phase noch dreimal mit jeweils ca. 10 ml Dichlormethan extrahiert. Die vereinigten organischen Phasen wurden über Magnesiumsulfat getrocknet und im Vakuum eingeengt. Aus dem Rückstand wurde das Produkt durch präparative RP-HPLC isoliert (Eluent Methanol/Wasser). Es wurden 283 mg des Zielprodukts erhalten (78% d. Th.).

LC-MS (Methode 5): Rₜ = 1.03 min; m/z = 326/328 (M+H)⁺.

¹H-NMR (400 MHz, DMSO-*d*₆): δ [ppm] = 0.11-0.18 (m, 2H), 0.31-0.39 (m, 2H), 1.12 (s, 3H), 1.40-1.49 (m, 1H), 3.53 (s, 3H), 3.57 (s, 3H), 4.10 (s, 1H), 5.20 (s, 2H), 6.61 (dd, 1H), 6.91 (d, 1H), 7.05 (d, 1H).

### Beispiel 43A

### Methyl-3-(4-chlorphenyl)-3,4-dimethylpentanoat

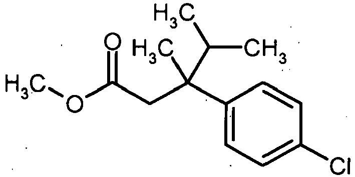

3.38 g (10.81 mmol) Dimethyl-[2-(4-chlorphenyl)-3-methylbutan-2-yl]malonat, 0.92 g (21.61 mmol) Lithiumchlorid und 0.2 ml Wasser wurden in 10 ml DMSO 4 h unter Rückfluss erhitzt. Nach dem Abkühlen wurde die Reaktionslösung mit ca. 50 ml Diethylether versetzt und die Phasen getrennt. Die organische Phase wurde zweimal mit Wasser gewaschen, über Magnesiumsulfat getrocknet und im Vakuum eingeengt. Das Rohprodukt wurde durch Chromatographie an Silicagel gereinigt (Laufmittel Cyclohexan/Ethylacetat 10:1). Es wurden 2.3 g (84% d. Th.) der Zielverbindung erhalten.

GC-MS (Methode 1): Rₜ = 5.43 min; m/z = 254 (M)⁺.

¹H-NMR (400 MHz, DMSO-*d*₆): δ [ppm] = 0.54 (d, 3H), 0.83 (d, 3H), 1.33 (s, 3H), 1.86-1.98 (m, 1H), 2.62 (d, 1H), 2.87 (d, 1H), 3.35 (s, 3H), 7.32 (s, 4H).

Analog zu Synthesebeispiel 43A wurden die folgenden Verbindungen erhalten: n

| **Beispiel** | **Name / Struktur / Edukt** | **Analytische Daten** |
|---|---|---|
| **44A** | Methyl-3-(4-chlorphenyl)-3-cyclobutylbutanoat | MS (DCI): m/z = 284 (M+NH₄)⁺. |
| | 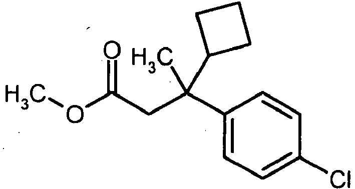 | ¹H-NMR (400 MHz, DMSO-*d*₆): δ [ppm] = 1.36 (s, 3H), 1.46-1.58 (m, 2H), 1.58-1.67 (m, 2H), 1.67-1.78 (m, 2H), 2.45-2.55 (m, 1H, teilweise verdeckt durch DMSO-Signal), 2.55-2.64 (m, 1H), 2.81 (d, 1H), 3.41 (s, 3H), 7.28-7.35 (m, 4H). |
| | aus Dimethyl-[1-(4-chlorphenyl)-1-cyclobutylethyl]-malonat | |
| **45A** | Methyl-3-(3-amino-4-chlorphenyl)-3-cyclopropyl-butanoat | LC-MS (Methode 7): |
| | | Rₜ = 1.12 min; m/z = 268/270 (M+H)⁺. |
| | 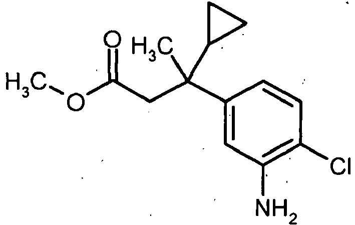 | |
| | aus Dimethyl-[1-(3-amino-4-chlorphenyl)-1-cyclopropylethyl]malonat | |

### Beispiel 46A

### Methyl-3-(4-chlor-3-nitrophenyl)-3,4-dimethylpentanoat

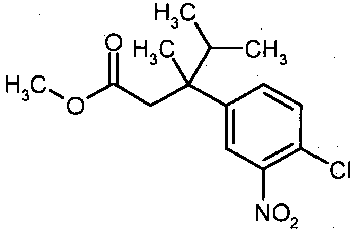

2.3 g (9.03 mmol) Methyl-3-(4-chlorphenyl)-3,4-dimethylpentanoat wurden in 50 ml Dichlormethan gelöst und auf 0°C abgekühlt. Anschließend wurden 1.44 g (10.8 mmol) Nitroniumtetrafluoroborat portionsweise zugegeben. Nach Beendigung der Zugabe wurde die Reaktionslösung zunächst 1 h bei 0°-10°C gerührt. Danach wurde der Ansatz langsam auf Raumtemperatur erwärmt und noch 2 h bei dieser Temperatur nachgerührt. Dann wurde das Reaktionsgemisch auf ca. 50 ml Wasser gegeben, die Phasen getrennt und die organische Phase über Magnesiumsulfat getrocknet. Nach Eindampfen der Lösung wurde der erhaltene Rückstand durch Chromatographie an Silicagel gereinigt (Laufmittel Cyclohexan/Ethylacetat 20:1). Es wurden 2.3 g (85% d. Th.) der Zielverbindung erhalten.

MS (DCI): m/z = 317 (M+NH₄)⁺.

¹H-NMR (400 MHz, DMSO-*d*₆): δ [ppm] = 0.56 (d, 3H), 0.84 (d, 3H), 1.35 (s, 3H), 1.89-2.02 (m, 1H), 2.66 (d, 1H), 3.02 (d, 1H), 3.39 (s, 3H), 7.63-7.71 (m, 2H), 7.96 (s, 1H).

Analog zu Synthesebeispiel 46A wurde die folgende Verbindung erhalten:

| **Beispiel** | **Name / Struktur / Edukt** | **Analytische Daten** |
|---|---|---|
| **47A** | Methyl-3-(4-chlor-3-nitrophenyl)-3-cyclobutyl-butanoat | GC-MS (Methode 6): |
| | | Rₜ = 7.62 min; m/z = 329 (M+NH₄)⁺. |
| | 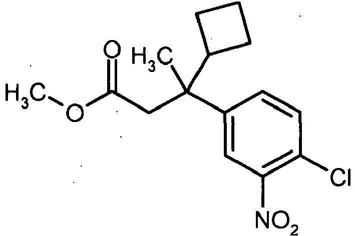 | ¹H-NMR (400 MHz, DMSO-*d*₆): δ [ppm] = 1.38 (s, 3H), 1.50-1.58 (m, 2H), 1.58-1.70 (m, 2H), 1.70-1.81 (m, 2H), 2.54 (d, 1H, teilweise verdeckt durch DMSO-Signal), 2.57-2.66 (m, 1H), 2.95 (d, 1H), 3.44 (s, 3H), 7.62-7.70 (m, 2H), 7.94 (d, 1H). |
| | aus Methyl-3-(4-chlorphenyl)-3-cyclobutylbutanoat | |

### Beispiel 48A

### Methyl-3-(3-amino-4-chlorphenyl)-3-cyclobutylbutanoat

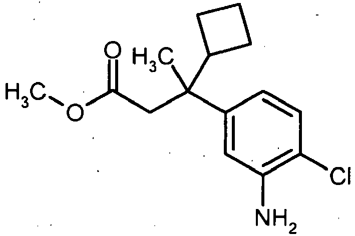

1.79 g (5.74 mmol) Methyl-3-(4-chlor-3-nitrophenyl)-3-cyclobutylbutanoat wurden in 50 ml Ethylacetat gelöst und mit ca. 150 mg 10%-igem Palladium auf Kohle versetzt. Die Reaktionsmischung wurde über Nacht bei RT unter einer Wasserstoffatmosphäre bei Normaldruck kräftig gerührt. Es wurde danach über Celite filtriert und das erhaltene Filtrat bis zur Trockene eingedampft. Das Rohprodukt wurde durch Chromatographie an Silicagel gereinigt (Laufmittel Cyclohexan/Ethyl-acetat 20:1). Erhalten wurden 1.36 g des Zielprodukts (84% d. Th.).

LC-MS (Methode 7): Rₜ = 1.22 min; m/z = 282 (M+H)⁺.

¹H-NMR (400 MHz, DMSO-*d*₆): δ [ppm] = 1.31 (s, 3H), 1.45-1.67 (m, 4H), 1.68-1.77 (m, 2H), 2.43 (d, 1H), 2.48-2.60 (m, 1H, teilweise verdeckt durch DMSO-Signal), 2.66 (d, 1H), 3.43 (s, 3H), 5.16 (br. s, 2H), 6.47 (dd, 1H), 6.73 (d, 1H), 7.04 (d, 1H).

Analog zu Synthesebeispiel 48A wurde die folgende Verbindung erhalten:

| **Beispiel** | **Name / Struktur / Edukt** | **Analytische Daten** |
|---|---|---|
| **49A** | Methyl-3-(3-amino-4-chlorphenyl)-3,4-dimethyl-pentanoat | LC-MS (Methode 5): |
| | | Rₜ = 1.11 min; m/z = 270/272 (M+H)⁺. |
| | 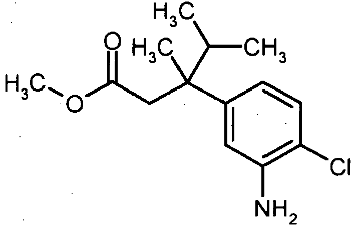 | ¹H-NMR (400 MHz, DMSO-*d*₆): δ [ppm] = 0.56 (d, 3H), 0.83 (d, 3H), 1.28 (s, 3H), 1.80-1.92 (m, 1 H), 2.57 (d, 1H), 2.72 (d, 1H), 3.38 (s, 3H), 5.15 (br. s, 2H), 6.48 (dd, 1H), 6.73 (d, 1H), 7.04 (d, 1H). |
| | aus Methyl-3-(4-chlor-3-nitrophenyl)-3,4-dimethylpentanoat | |

### Beispiel 50A und Beispiel 51A

### Methyl-3-(3-amino-4-chlorphenyl)-3,4-dimethylpentanoat (Enantiomer 1 und 2)

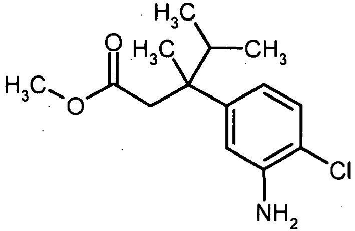

1700 mg (6.30 mmol) des Racemats von Methyl-3-(3-amino-4-chlorphenyl)-3,4-dimethylpentanoat (Beispiel 49A) wurden mittels präparativer HPLC an chiraler Phase in die Enantiomere getrennt [Säule: Daicel Chiralpak AY-H, 5 µm, 250 mm x 20 mm; Elutionsmittel: Isohexan/Isopropanol 95:5 (v/v); Fluss: 20 ml/min; UV-Detektion: 230 nm; Temperatur: 25°C]. Das jeweils erhaltene Material wurde durch Chromatographie an Silicagel nochmals nachgereinigt (Laufmittel Cyclohexan/Ethylacetat 10:1).

### Beispiel 50A (Enantiomer 1):

Ausbeute: 588 mg

Rₜ = 7.21 min; chemische Reinheit >99%; >99% ee
[Säule: Daicel AY-H, 5 µm, 250 mm x 4.6 mm; Elutionsmittel: Isohexan/(Isopropanol + 0.2% Diethylamin) 95:5 (v/v); Fluss: 1 ml/min; UV-Detektion: 230 nm; Temperatur: 30°C].

LC-MS (Methode 5): Rₜ = 1.15 min; m/z = 270 (M+H)⁺.

¹H-NMR (400 MHz, DMSO-*d*₆): δ [ppm] = 0.56 (d, 3H), 0.83 (d, 3H), 1.28 (s, 3H), 1.80-1.92 (m, 1H), 2.57 (d, 1H), 2.72 (d, 1H), 3.38 (s, 3H), 5.15 (br. s, 2H), 6.48 (dd, 1H), 6.73 (d, 1H), 7.04 (d, 1H).

[α]_{D}²⁰ = -30°, c = 0.275, Methanol.

### Beispiel 51A (Enantiomer 2):

Ausbeute: 499 mg

Rₜ = 8.59 min; chemische Reinheit >99%; >96.7% ee
[Säule: Daicel AY-H, 5 µm, 250 mm x 4.6 mm; Elutionsmittel: Isohexan/(Isopropanol + 0.2% Diethylamin) 95:5 (v/v); Fluss: 1 ml/min; UV-Detektion: 230 nm; Temperatur: 30°C].

LC-MS (Methode 5): Rₜ = 1.15 min; m/z = 270 (M+H)⁺.

¹H-NMR (400 MHz, DMSO-*d*₆): δ [ppm] = 0.56 (d, 3H), 0.83 (d, 3H), 1.28 (s, 3H), 1.80-1.92 (m, 1H), 2.57 (d, 1H), 2.72 (d, 1H), 3.38 (s, 3H), 5.15 (br. s, 2H), 6.48 (dd, 1H), 6.73 (d, 1H), 7.04 (d, 1H).

[α]_{D}²⁰ = +29°, c = 0.270, Methanol.

### Beispiel 52A und Beispiel 53A

### Methyl-3-(3-amino-4-chlorphenyl)-3-cyclobutylbutanoat (Enantiomer 1 und 2)

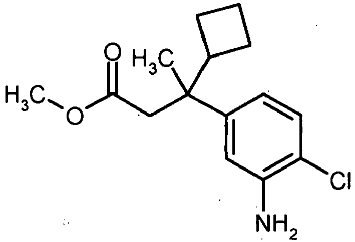

1075 mg (3.82 mmol) des Racemats von Methyl-3-(3-amino-4-chlorphenyl)-3-cyclobutylbutanoat (Beispiel 48A) wurden mittels präparativer HPLC an chiraler Phase in die Enantiomere getrennt [Säule: Daicel Chiralpak AY-H, 5 µm, 250 mm x 20 mm; Elutionsmittel: Isohexan/Ethanol 95:5 (v/v); Fluss: 15 ml/min; UV-Detektion: 220 nm; Temperatur: 25°C]:

### Beispiel 52A (Enantiomer 1):

Ausbeute: 472 mg

Rₜ = 6.40 min; chemische Reinheit >99%; >99% ee
[Säule: Daicel AY-H, 5 µm, 250 mm x 4.6 mm; Elutionsmittel: Isohexan/(Ethanol 0.2% Diethylamin) 95:5 (v/v); Fluss: 1 ml/min; UV-Detektion: 220 nm; Temperatur: 40°C].

LC-MS (Methode 5): Rₜ = 1.15 min; m/z = 282/284 (M+H)⁺.

¹H-NMR (400 MHz, DMSO-*d*₆): δ [ppm] = 1.31 (s, 3H), 1.45-1.67 (m, 4H), 1.68-1.78 (m, 2H), 2.43 (d, 1H), 2.48-2.60 (m, 1H, teilweise verdeckt durch DMSO-Signal), 2.66 (d, 1H), 3.43 (s, 3H), 5.16 (br. s, 2H), 6.47 (dd, 1H), 6.73 (d, 1H), 7.04 (d, 1H).

[α]_{D}²⁰ = -2.3°, c = 0.450, Methanol.

### Beispiel 53A (Enantiomer 2):

Ausbeute: 489 mg

Rₜ = 7.85 min; chemische Reinheit >99%; >99% ee
[Säule: Daicel AY-H, 5 µm, 250 mm x 4.6 mm; Elutionsmittel: Isohexan/(Ethanol + 0.2% Diethylamin) 95:5 (v/v); Fluss: 1 ml/min; UV-Detektion: 220 nm; Temperatur: 40°C].

[α]_{D}²⁰ = +2.5°, c = 0.330, Methanol.

### Beispiel 54A

### 1-(4-Chlorphenyl)prop-2-en-1-on

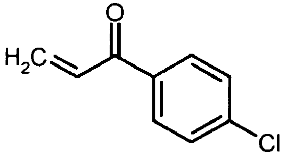

60 g (295.5 mmol) 3-Chlor-1-(4-chlorphenyl)propan-1-on wurden in 900 ml Acetonitril gelöst. Anschließend wurden zu der Lösung unter Eisbad-Kühlung 41.2 ml (295.5 mmol) Triethylamin langsam zugetropft (exotherme Reaktion). Nach Beendigung der Zugabe wurde die Reaktionslösung 4 h bei Raumtemperatur gerührt. Danach wurde das Reaktionsgemisch mit ca. einem Liter Wasser, einem Liter Essigsäureethylester und ca. 250 ml gesättigter Natriumchlorid-Lösung versetzt. Nach Trennung der Phasen wurde die organische Phase über Magnesiumsulfat getrocknet, filtriert und das Filtrat bis zur Trockene eingeengt. Das erhaltene Rohprodukt wurde durch Chromatographie an Silicagel (ca. 1.3 kg) gereinigt (Laufmittel Cyclohexan/Ethylacetat 6:1). Erhalten wurden 45 g des Zielprodukts (91% d. Th.).

¹H-NMR (400 MHz, DMSO-*d₆*): δ [ppm] = 6.02 (d, 1H), 6.36 (dd, 1H), 7.34-7.44 (m, 1H), 7.63 (d, 1H), 8.03 (d, 2H).

### Beispiel 55A

### (4-Chlorphenyl)(2,2-difluorcyclopropyl)methanon

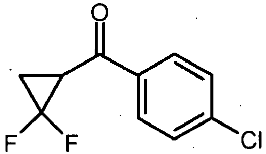

Unter Argon wurden in einem 3 Liter-Dreihalskolben 91 g (546 mmol) 1-(4-Chlorphenyl)prop-2-en-1-on, 2.293 g (54.6 mmol) Natriumfluorid sowie 2.41 g (10.92 mmol) 2,6-Di-*tert*.-butyl-4-methyl-phenol auf 110°C erhitzt und 5 min bei dieser Temperatur gerührt. Anschließend wurden zu der Lösung bei 110°-125°C Innentemperatur innerhalb von 30-35 min 183 ml (928.5 mmol) Trimethylsilyl-2,2-difluor-2-(fluorsulfonyl)acetat langsam zugetropft (Vorsicht: Gasentwicklung). Nach Beendigung der Zugabe und der Gasentwicklung wurde die Reaktionslösung noch 20 min weitergerührt. Nach Abkühlen wurde das Reaktionsgemisch in mehreren Litern Essigsäureethylester aufgenommen und mit gesättigter wässriger Natriumhydrogencarbonat-Lösung ausgeschüttelt. Nach Trennung der Phasen wurde die organische Phase über Magnesiumsulfat getrocknet, filtriert und das Filtrat bis zur Trockene eingeengt. Das erhaltene Rohprodukt wurde durch Chromatographie an Silicagel (ca. 2 kg) gereinigt (Laufmittel Cyclohexan/Ethylacetat 10:1). Erhalten wurden 63 g des Zielprodukts (53% d. Th.).

¹H-NMR (400 MHz, DMSO-*d₆*): δ [ppm] = 2.04-2.14 (m, 1H), 2.21-2.31 (m, 1H), 3.98-4.09 (m, 1H), 7.65-7.70 (m, 2H), 8.06-8.11 (m, 2H).

### Beispiel 56A

### Methyl-(2Z)-3-(4-chlorphenyl)-3-(2,2-difluorcyclopropyl)acrylat und Methyl-(2E)-3-(4-chlorphenyl)-3-(2,2-difluorcyclopropyl)acrylat

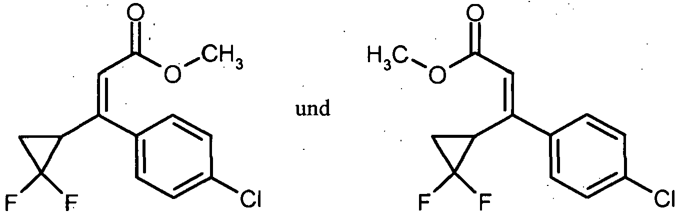

2.2 g (60% in Mineralöl, 55 mmol) Natriumhydrid wurden mit 20 ml THF verrührt, dann abgesaugt und mit 20 ml THF nachgewaschen. Das so gereinigte Natriumhydrid wurde unter Argon in 200 ml THF eingetragen. Das Gemisch wurde anschließend auf 0°C gekühlt und mit 10.1 g (55 mmol) Methyl-(diethoxyphosphoryl)acetat, gelöst in 10 ml THF, versetzt. Nach Erwärmung auf Raumtemperatur wurde die Lösung 1 h nachgerührt. Anschließend wurden 5.15 g (19.73 mmol) (4-Chlorphenyl)(2,2-difluorcyclopropyl)methanon in 50 ml THF zugetropft. Nach vollständiger Zugabe wurde die Lösung auf Rückfluss erhitzt und 2 h gerührt. Danach wurde auf 5°C abgekühlt und der Ansatz auf 400 ml Eiswasser gegossen. Nach Trennung der Phasen wurde die wässrige Phase noch dreimal mit *tert.* -Butylmethylether extrahiert. Die vereinigten organischen Phasen wurden nacheinander mit 1 M Salzsäure und gesättigter Natriumchlorid-Lösung gewaschen, über Natriumsulfat getrocknet, filtriert und bis zur Trockene eingeengt. Das erhaltene Rohprodukt wurde durch Chromatographie an Silicagel gereinigt (Laufmittel Cyclohexan/Ethylacetat 20:1 → 8:1). Hierbei wurden die *E*/*Z*-Isomere in getrennter Form isoliert. Es wurden so 2.23 g (37% d. Th.) Methyl-(2E)-3-(4-chlorphenyl)-3-(2,2-difluorcyclopropyl)acrylat und 1.6 g (24.4% d. Th.) Methyl-(2*Z*)-3-(4-chlorphenyl)-3-(2,2-difluorcyclopropyl)acrylat erhalten.

Methyl-(2*E*)-3-(4-chlorphenyl)-3-(2,2-difluorcyclopropyl)acrylat:
¹H-NMR (400 MHz, DMSO-*d*₆): δ [ppm] = 1.00-1.12 (m, 1H), 1.92-2.06 (m, 1H), 3.21-3.37 (m, 1H, teilweise verdeckt durch H₂O-Signal), 3.71 (s, 3H), 6.42 (d, 1H), 7.49 (d, 2H), 7.55 (d, 2H).

Methyl-(2*Z*)-3-(4-chlorphenyl)-3-(2,2-difluorcyclopropyl)acrylat:
¹H-NMR (400 MHz, DMSO-*d*₆): δ [ppm] = 1.83-1.96 (m, 1H), 1.97-2.09 (m, 1H), 2.76-2.88 (m, 1H), 3.51 (s, 3H), 6.10 (s, 1H), 7.23 (d, 2H), 7.46 (d, 2H).

### Beispiel 57A

### Methyl-3-(4-chlorphenyl)-3-(2,2-difluorcyclopropyl)propanoat und Methyl-3-(4-chlorphenyl)-5,5-difluorhexanoat

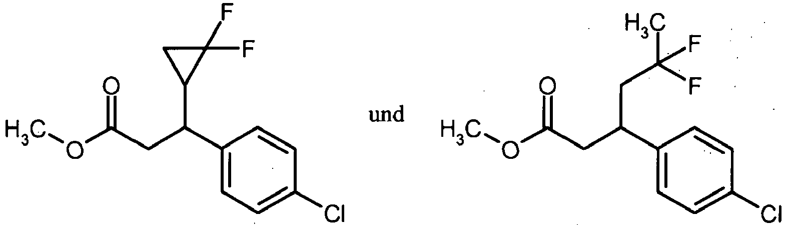

1000 mg (3.67 mmol) Methyl-(2*Z*)-3-(4-chlorphenyl)-3-(2,2-difluorcyclopropyl)acrylat wurden in 75 ml Ethylacetat gelöst und in einer mit einer Katalysator-Kartusche (10% Palladium auf Kohle) ausgestatteten Durchfluss-Hydrierapparatur (H-Cube, Firma Thales Nano, Budapest) bei einem Durchfluss von 1 ml/min und bei Raumtemperatur und Normaldruck mit Wasserstoff hydriert. Nach vollständiger Umsetzung wurde das Reaktionsgemisch im Vakuum eingeengt. Es wurden 980 mg eines Produktgemisches bestehend aus Methyl-3-(4-chlorphenyl)-3-(2,2-difluorcyclopropyl)-propanoat und Methyl-3-(4-chlorphenyl)-5,5-difluorhexanoat als farbloses Öl erhalten.

GC-MS (Methode 6): Rₜ = 5.38 min; m/z = 292/294/296 (M+NH₄)⁺.

### Beispiel 58A

### Methyl-3-(4-chlor-3-nitrophenyl)-3-(2,2-difluorcyclopropyl)propanoat und Methyl-3-(4-chlor-3-nitrophenyl)-5,5-difluorhexanoat

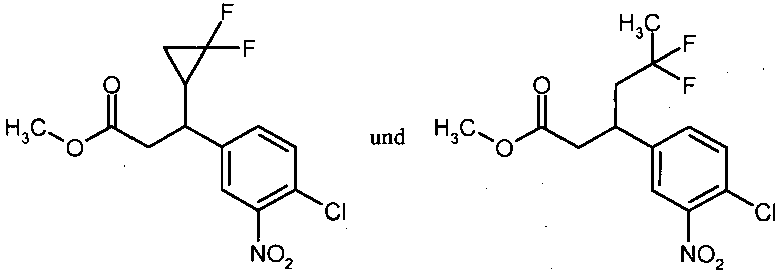

610 mg des Gemisches bestehend aus Methyl-3-(4-chlorphenyl)-3-(2,2-difluorcyclopropyl)propanoat und Methyl-3-(4-chlorphenyl)-5,5-difluorhexanoat (Beispiel 57A) wurden in 12 ml Dichlormethan gelöst und auf 0°C gekühlt. Anschließend wurden 351 mg (2.65 mmol) Nitroniumtetrafluoroborat portionsweise zugegeben. Nach Beendigung der Zugabe wurde die Reaktionslösung 1 h bei 0°-10°C gerührt. Danach wurde der Ansatz langsam auf Raumtemperatur erwärmt und weitere 2 h bei dieser Temperatur nachgerührt. Anschließend wurde das Reaktionsgemisch auf ca. 20 ml Wasser gegeben, die Phasen getrennt und die organische Phase über Magnesiumsulfat getrocknet. Nach Eindampfen der Lösung wurde der erhaltene Rückstand durch Chromatographie an Silicagel gereinigt (Laufmittel Cyclohexan/Ethylacetat 20:1). Es wurden 637 mg des Gemisches der beiden Zielverbindungen erhalten.

GC-MS (Methode 6): Rₜ = 6.74 min; m/z = 337/339/341 (M+NH₄)⁺.

### Beispiel 59A

### Methyl-3-(3-amino-4-chlorphenyl)-3-(2,2-difluorcyclopropyl)propanoat und Methyl-3-(3-amino-4-chlorphenyl)-5,5-difluorhexanoat

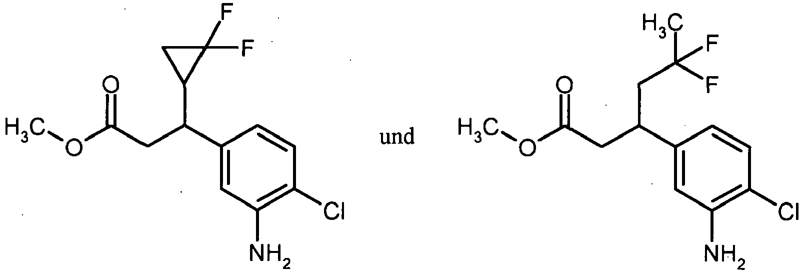

640 mg des Gemisches bestehend aus Methyl-3-(4-chlor-3-nitrophenyl)-3-(2,2-difluorcyclopropyl)-propanoat und Methyl-3-(4-chlor-3-nitrophenyl)-5,5-difluorhexanoat (Beispiel 58A) wurden in 40 ml Ethylacetat gelöst und mit 106 mg Palladium auf Kohle (10%) versetzt. Die Reaktionsmischung wurde über Nacht unter einer Wasserstoffatmosphäre bei Normaldruck kräftig gerührt. Es wurde dann über Celite filtriert und das erhaltene Filtrat bis zur Trockene eingedampft. Das Rohprodukt wurde durch Chromatographie an Silicagel gereinigt (Laufmittel Cyclohexan/ Ethylacetat 4:1). Es wurden 361 mg des Gemisches der beiden Zielverbindungen erhalten.

LC-MS (Methode 5): Rₜ = 0.98 min; m/z = 290/292 (M+H)⁺.

### Beispiel 60A

### (+)-Ethyl-(3R)-4,4,4-trifluor-3-methylbutanoat

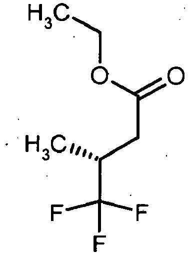

287 g (1.65 mol) (3*R*)-4,4,4-Trifluor-3-methylbutansäure [A. Gerlach und U. Schulz, Speciality Chemicals Magazine 24 (4), 37-38 (2004); CAS Acc.-Nr. 142:179196] in 580 ml Ethanol wurden bei Raumtemperatur langsam mit 133 ml (1.82 mol) Thionylchlorid versetzt. Die Reaktionslösung wurde anschließend auf 80°C erwärmt und 2 h bei dieser Temperatur gerührt. Danach wurde auf Raumtemperatur abgekühlt, langsam mit 250 ml Wasser versetzt und dreimal mit je 150 ml *tert.-*Butylmethylether extrahiert. Die vereinigten organischen Phasen wurden über Natriumsulfat getrocknet. Nach Filtration wurde das Lösungsmittel im Vakuum bei 30°C und einem Druck von 300 mbar entfernt. Das Rohprodukt wurde anschließend bei 100 bar und einer Kopftemperatur von 65°C destilliert. Es wurden 225.8 g (113 mol, 74% d. Th.) der Titelverbindung als farblose Flüssigkeit isoliert.

¹H-NMR (400 MHz, DMSO-d₆, δ/ppm): 4.10 (2H, q), 2.88-2.72 (1H, m), 2.66-2.57 (1H, m), 2.46-2.36 (1H, m), 1.19 (3H, t), 1.11 (3H, d).

GC-MS (Methode 1): Rₜ = 1.19 min; m/z = 184 (M)⁺.

[α]_{D}²⁰ = +16.1°, c = 0.41, Methanol.

### Beispiel 61A

### Ethyl-4,4,4-trifluor-3-methyl-2-(4-methylphenyl)butanoat (Diastereomerengemisch)

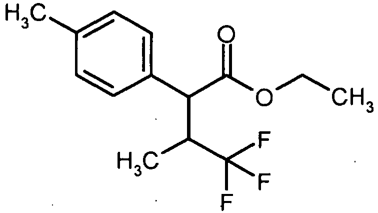

Unter Argon wurden 196.9 mg (0.88 mmol) Palladium(II)acetat und 724.8 mg (1.84 mmol) 2-Di-cyclohexylphosphino-2'-(*N,N*-dimethylamino)biphenyl in 50 ml wasserfreiem Toluol vorgelegt. Die Reaktionslösung wurde anschließend langsam mit 43.8 ml (43.8 mmol) einer 1 M Lösung von Lithiumhexamethyldisilazid in THF versetzt und 10 min bei RT gerührt. Nachfolgend wurde die Reaktionslösung auf -10°C abgekühlt, langsam mit 7 g (38.0 mmol) (+/-)-Ethyl-4,4,4-trifluor-3-methylbutanoat versetzt und 10 min bei -10°C nachgerührt. Danach wurden 5 g (29.2 mmol) 4-Bromtoluol, gelöst in 50 ml Toluol, zugetropft und die Reaktionslösung erst auf RT und dann auf 80°C erwärmt. Das Gemisch wurde 2 h bei dieser Temperatur gerührt, dann auf RT abgekühlt und über Nacht nachgerührt. Nach erfolgter Umsetzung (DC-Kontrolle; Laufmittel Cyclohexan/ Dichlormethan 2:1) wurde die Reaktionsmischung über Kieselgur filtriert, der Rückstand mehrfach mit Essigsäureethylester und Dichlormethan gewaschen und die vereinigten Filtrate im Vakuum eingeengt. Das erhaltene Rohprodukt wurde über Kieselgel chromatographisch gereinigt (Laufmittel Petrolether/Dichlormethan 4:1 → 3:1). Es wurden 3.91 g (14.3 mmol, 48.8% d. Th.) der Titelverbindung als farblose Flüssigkeit isoliert.

¹H-NMR (400 MHz, DMSO-d₆, δ/ppm): 7.26 (2H, d), 7.20-7.12 (2H, m), 4.17-3.95 (2H, m), 3.74 (0.25H, d), 3.66 (0.75H, d), 3.35-3.07 (1H, m), 2.29 (2.25H, s), 2.28 (0.75H, s), 1.17 (0.75H, d), 1.11 (3H, t), 0.76 (2.25H, d).

GC-MS (Methode 1): Rₜ = 4.20 min, m/z = 275 (M+H)⁺ (*Diastereomer 1*); Rₜ = 4.23 min, m/z = 275 (M+H)⁺ *(Diastereomer 2).*

### Beispiel 62A

### Ethyl-(3R)-4,4,4-trifluor-3-methyl-2-(4-methylphenyl)butanoat (Diastereomerengemisch)

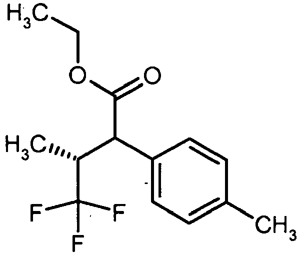

Herstellung von Lösung A: 16.3 ml einer 1 M Lösung von Lithiumhexamethyldisilazid in Toluol wurden unter Argon auf -10°C bis -20°C gekühlt (Kühlung mittels Aceton/Trockeneis) und langsam mit 2 g (10.86 mmol) (+)-Ethyl-(3*R*)-4,4,4-trifluor-3-methylbutanoat, gelöst in 10 ml Toluol, versetzt, wobei darauf geachtet wurde, dass eine Temperatur von -10°C nicht überschritten wurde. Die Lösung wurde anschließend 10 min bei maximal -10°C nachgerührt.

Herstellung von Lösung B: 2.415 g (14.12 mmol) 4-Bromtoluol wurden unter Argon bei RT in 10 ml Toluol gelöst und mit 73 mg (0.33 mmol) Palladium(II)acetat und 269 mg (0.68 mmol) 2-Di-cyclohexylphosphino-2'-(*N,N*-dimethylamino)biphenyl versetzt. Die Lösung wurde 10 min bei RT nachgerührt.

Zunächst wurde das Kühlbad von Lösung A entfernt. Anschließend wurde zu der noch kalten Lösung A langsam die Lösung B hinzugetropft. Die nun vereinigten Lösungen wurden langsam auf RT erwärmt und 1 h bei dieser Temperatur gerührt. Anschließend wurde die Reaktionslösung auf 80°C (Innentemperatur) erwärmt und 3 h bei dieser Temperatur gerührt. Danach wurde die Reaktionslösung langsam auf RT abgekühlt und noch 12 h nachgerührt. Das Reaktionsgemisch wurde schließlich über Kieselgur filtriert, der Rückstand mehrfach mit Toluol nachgewaschen und die vereinigten Filtrate im Vakuum eingeengt. Das erhaltene Rohprodukt wurde über Kieselgel chromatographisch gereinigt (Laufmittel Cyclohexan/Dichlormethan 10:1→ 4:1). Es wurden 2.35 g (79% d. Th.) der Titelverbindung isoliert.

¹H-NMR (400 MHz, DMSO-d₆, δ/ppm): 0.76 (d, 2.13H), 1.11 (t, 3H), 1.17 (d, 0.87H), 3.07-3.30 (m, 1H), 3.66 (d, 0.7H), 3.75 (d, 0.3H), 3.94-4.15 (m, 2H), 7.12-7.20 (m, 2H), 7.23-7.29 (m, 2H).

GC-MS (Methode 1): Rₜ = 3.88 min, m/z = 275 (M+H)⁺ *(Diastereomer 1*); Rₜ = 3.90 min, m/z = 275 (M+H)⁺ (*Diastereomer 2).*

### Beispiel 63A

### Ethyl-(3R)-2-(4-chlorphenyl)-4,4,4-trifluor-3-methylbutanoat (Diastereomerengemisch)

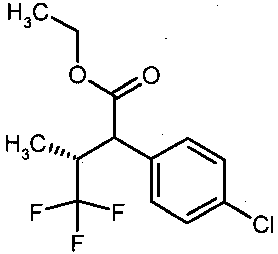

Herstellung von Lösung A: 163.9 ml einer 1 M Lösung von Lithiumhexamethyldisilazid in Toluol wurden unter Argon auf -10°C bis -20°C gekühlt (Kühlung mittels Aceton/Trockeneis) und langsam mit 20 g (108.6 mmol) (+)-Ethyl-(3*R*)-4,4,4-trifluor-3-methylbutanoat, gelöst in 150 ml Toluol, versetzt, wobei darauf geachtet wurde, dass eine Temperatur von -10°C nicht überschritten wurde. Die Lösung wurde anschließend 10 min bei maximal -10°C nachgerührt.

Herstellung von Lösung B: 27.03 g (141.2 mmol) 1-Brom-4-chlorbenzol wurden unter Argon bei RT in 100 ml Toluol gelöst und mit 731 mg (3.26 mmol) Palladium(II)acetat und 2.693 g (6.84 mmol) 2-Dlcyclohexylphosphino-2'-(*N,N-*dimethylamino)biphenyl versetzt. Die Lösung wurde 10 min bei RT nachgerührt.

Zunächst wurde das Kühlbad von Lösung A entfernt. Anschließend wurde zu der noch kalten Lösung A langsam die Lösung B hinzugetropft. Die nun vereinigten Lösungen wurden langsam auf RT erwärmt und 1 h bei dieser Temperatur gerührt. Anschließend wurde die Reaktionslösung auf 80°C (Innentemperatur) erwärmt und 3 h bei dieser Temperatur gerührt. Danach wurde die Reaktionslösung langsam auf RT abgekühlt und noch 12 h nachgerührt. Das Reaktionsgemisch wurde schließlich über Kieselgur filtriert, der Rückstand mehrfach mit Toluol nachgewaschen und die vereinigten Filtrate im Vakuum eingeengt. Das erhaltene Rohprodukt wurde über Kieselgel chromatographisch gereinigt (Laufmittel Cyclohexan/Dichlormethan 4:1). Es wurden 27.4 g (92.98 mmol, 86% d. Th.) der Titelverbindung als gelbes Öl in einem Diastereomerenverhältnis von 3:1 isoliert.

GC-MS (Methode 1): Rₜ = 4.45 min, m/z = 294 (M)⁺ *(Diastereomer 1*); Rₜ = 4.48 min, m/z = 294 (M)⁺ (*Diastereomer 2).*

Analog zu Synthesebeispiel 61A und 63A wurden die folgenden Verbindungen erhalten:

| **Beispiel** | **Name / Struktur / Edukte** | **Analytische Daten** |
|---|---|---|
| **64A** | Ethyl-(3*R*)-4,4,4-trifluor-2-(4-isopropylphenyl)-3-methylbutanoat | GC-MS (Methode 1): |
| | | Rₜ = 4.61 min, m/z = 302 (M)⁺ (*Diastereomer 1*); |
| | 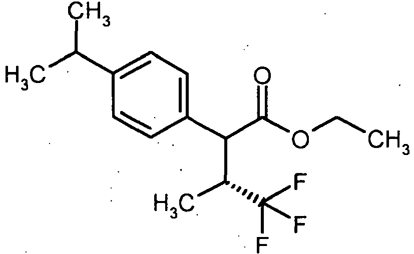 | Rₜ = 4.64 min, m/z = 302 (M)⁺ (*Diastereomer 2*)*.* |
| | aus 1-Brom-4-Isopropylbenzol und Ethyl-(3*R*)-4,4,4-trifluor-3-methylbutanoat | |
| **65A** | Ethyl-(3*R*)-2-(4-*tert*.-butylphenyl)-4,4,4-trifluor-3-methylbutanoat | GC-MS (Methode 1): |
| | | Rₜ = 4.83 min, m/z = 317 (M+H)⁺ (*Diastereomer 1*); |
| | 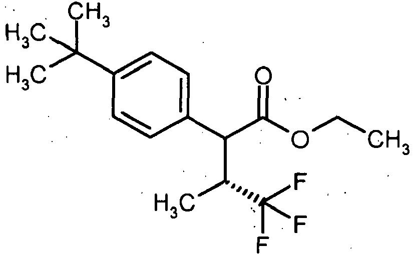 | Rₜ = 4.85 min, m/z = 317 (M+H)⁺ (*Diastereomer 2*). |
| | | MS (DCI): m/z = 334 (M+NH₄)⁺. |
| | aus 1-Brom-4-*tert.*-butylbenzol und Ethyl-(3*R*)-4,4,4-trifluor-3-methylbutanoat | |
| **66A** | Ethyl-(3*R*)-2-(4-chlor-3-methoxyphenyl)-4,4,4-trifluor-3 -methylbutanoat | GC-MS (Methode 1): |
| | | Rₜ = 5.34 min; m/z = 324/326 (M)⁺. |
| | 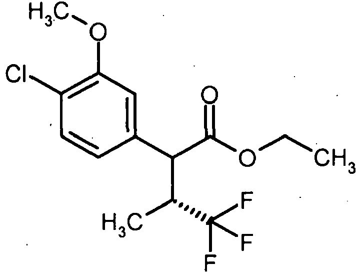 | |
| | aus 4-Brom-1-chlor-2-methoxybenzol und Ethyl-(3*R*)-4,4,4-trifluor-3-methylbutanoat | |
| **67A** | Ethyl-2-(4-chlor-3-methylphenyl)-4,4,4-trifluor-3-methylbutanoat | GC-MS (Methode 1): |
| | | Rₜ = 4.81 min, m/z = 308/310 (M)⁺ (D*iastereomer 1*); |
| | 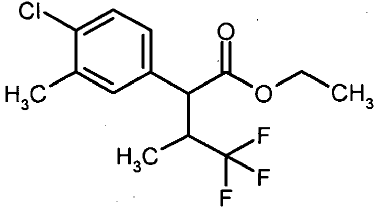 | Rₜ = 4.84 min, m/z = 308/310 (M)⁺ (*Diastereomer 2*). |
| | aus 4-Brom-1-chlor-2-methylbenzol und Ethyl-4,4,4-trifluor-3-methylbutanoat | |

### Beispiel 68A

### (3R)-2-(4-Ethylphenyl)-4,4,4-trifluor-3-methylbutansäureethylester (Diastereomerengemisch)

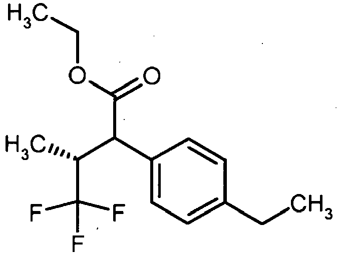

24.4 ml (24.4 mmol) einer 1 M Lösung von Lithiumhexamethyldisilazid in Toluol wurden auf -10°C abgekühlt und tropfenweise mit einer Lösung von 3.0 g (16.29 mmol) (+)-Ethyl-(3*R*)-4,4,4-trifluor-3-methylbutanoat in 15 ml abs. Toluol versetzt. Die Mischung wurde 10 min nachgerührt. Anschließend wurde bei -10°C eine zuvor hergestellte Lösung von 3.92 g (21.18 mmol) 1-Brom-4-ethylbenzol, 110 mg (0.49 mmol) Palladium(II)acetat und 404 mg (1.03 mmol) 2'-Dicyclohexylphosphino-2-(*N,N-*dimethylamino)biphenyl in 20 ml abs. Toluol zugetropft. Die resultierende Reaktionsmischung wurde zunächst 1 h bei RT, dann 3 h bei 80°C gerührt. Danach wurde die Mischung im Vakuum eingeengt und der Rückstand in Ethylacetat aufgenommen und auf Wasser gegeben. Die wässrige Phase wurde mit Ethylacetat rückextrahiert, und die vereinigten organischen Phasen wurden mit gesättigter Ammoniumchlorid-Lösung und gesättigter Natriumchlorid-Lösung gewaschen, über Magnesiumsulfat getrocknet und im Vakuum eingeengt. Aus dem Rückstand wurden nach Chromatographie an Silicagel (Laufmittel zunächst Cyclohexan, dann Gradient Cyclohexan/Ethylacetat 200:1 → 50:1) 3.051 g der Titelverbindung erhalten (64.9% d. Th., Diastereomerenverhältnis ca. 3:1).

LC-MS (Methode 4): Rₜ = 1.52 min, m/z = 289 (M+H)⁺ (*Neben-Diastereomer*); Rₜ = 1.54 min, m/z = 289 (M+H)⁺ (*Haupt-Diastereomer*).

¹H-NMR (400 MHz, DMSO-*d*₆): *Haupt-Diastereomer*: δ [ppm] = 0.76 (d, 3H), 1.13 (t, 3H), 1.17 (t, 3H), 2.55-2.63 (m, 2H), 3.21-3.31 (m, 1H), 3.67 (d, 1H), 3.95-4.16 (m, 2H), 7.15-7.23 (m, 2H), 7.25-7.31 (m, 2H).

Auf vergleichbare Weise wurden ausgehend von (+)-Ethyl-(3*R*)-4,4,4-trifluor-3-methylbutanoat und den entsprechenden Phenylbromiden die folgenden Verbindungen hergestellt:

### Beispiel 69A

### (3R)-4,4,4-Trifluor-3-methyl-2-(4-vinylphenyl)butansäureethylester (Diastereomerengemisch)

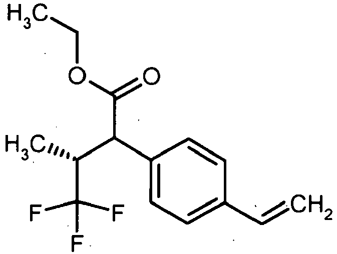

GC-MS (Methode 1): Rₜ = 4.64 min und 4.66 min; jeweils m/z = 286 (M)⁺.

¹H-NMR (400 MHz, DMSO-*d*₆): *Haupt-Diastereomer:* δ [ppm] = 0.79 (d, 3H), 1.12 (t, 3H), 3.22-3.32 (m, 1H), 3.73 (d, 1H), 3.99-4.17 (m, 2H), 5.28 (d, 1H), 5.84 (d, 1H), 6.72 (dd, 1H), 7.34-7.40 (m, 2H), 7.45-7.51 (m, 2H).

### Beispiel 70A

### (3R)-4,4,4-Trifluor-2-(4-fluorphenyl)-3-methylbutansäureethylester (Diastereomerengemisch)

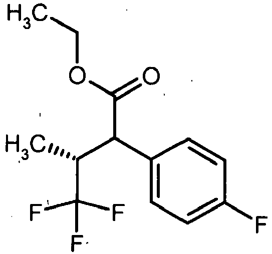

GC-MS (Methode 1): Rₜ = 3.63 min, m/z = 278 (M)⁺ (*Neben*-*Diastereomer*); Rₜ = 3.66 min, m/z = 278 (M)⁺ (*Haupt-Diastereomer*).

¹H-NMR (400 MHz, DMSO-*d*₆): *Haupt-Diastereomer:* δ [ppm] = 0.77 (d, 3H), 1.12 (t, 3H), 3.23-3.30 (m, 1H), 3.79 (d, 1H), 4.01-4.14 (m, 2H), 7.19-7.24 (m, 2H), 7.43-7.47 (m, 2H).

### Beispiel 71A

### (3R)-2-(4-Chlor-3-fluorphenyl)-4,4,4-trifluor-3-methylbutansäureethylester (Diastereomerengemisch)

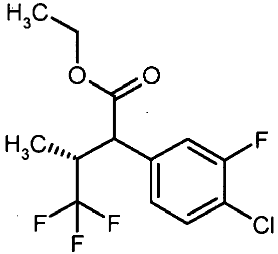

GC-MS (Methode 1): Rₜ = 4.33 min und 4.36 min; jeweils m/z = 312 (M)⁺.

¹H-NMR (400 MHz, DMSO-*d₆*): *Haupt-Diastereomer:* δ [ppm] = 0.80 (d, 3H), 1.08-1.19 (m, 3H), 3.34-3.41 (m, 1H), 3.88 (d, 1H), 4.01-4.18 (m, 2H), 7.28-7.34 (m, 1H), 7.51-7.64 (m, 2H).

### Beispiel 72A

### Ethyl-(3R)-2-[4-(2,2-difluorcyclopropyl)phenyl]-4,4,4-trifluor-3-methylbutanoat

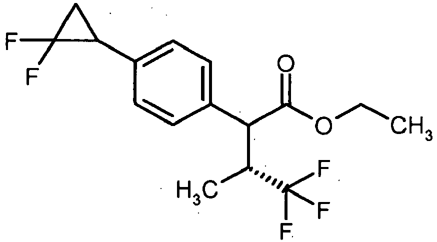

1.58 g (5.52 mmol) Ethyl-(3*R*)-4,4,4-trifluor-3-methyl-2-(4-vinylphenyl)butanoat, 23 mg (0.55 mmol) Natriumfluorid und 24 mg (0.11 mmol) 2,6-Di-*tert*.-butyl-4-methylphenol wurden auf 110°C erhitzt und 5 min gerührt. Anschließend wurden langsam 1.9 ml (9.38 mmol) Trimethylsilyl-2,2-difluor-2-(fluorsulfonyl)acetat zugetropft und das Gemisch 60 min bei 110°C nachgerührt (Vorsicht: Gasentwicklung nach ca. 30 min). Nach Abkühlen auf Raumtemperatur und Zugabe von Essigsäureethylester und gesättigter wässriger Natriumhydrogencarbonat-Lösung wurde die organische Phase abgetrennt, über Magnesiumsulfat getrocknet, filtriert und bis zur Trockene eingeengt. Das Rohprodukt wurde über Kieselgel chromatographisch gereinigt (Laufmittel Cyclohexan/ Dichlormethan 4:1). Es wurden 1.5 g der Titelverbindung erhalten (81% d. Th.).

GC-MS (Methode 1): Rₜ = 4.99 min, m/z = 336 (M)⁺ (*Diastereomer 1*); Rₜ = 5.01 min, m/z = 336 (M)⁺ *(Diastereomer 2).*

MS (DCI): m/z = 354 (M+NH₄)⁺.

### Beispiel 73A

### Ethyl-2-[4-(brommethyl)phenyl]-4,4,4-trifluor-3-methylbutanoat

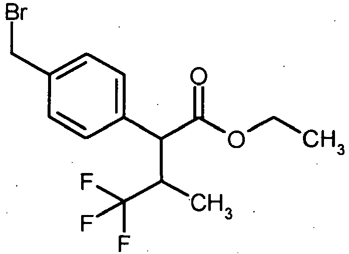

2.25 g (8.2 mmol) Ethyl-4,4,4-trifluor-3-methyl-2-(4-methylphenyl)butanoat, 1.53 g (8.6 mmol) *N-*Bromsuccinimid und 67 mg (0.41 mmol) 2,2'-Azobis-(2-methylpropannitril) in 36 ml Trichlormethan wurden über Nacht unter Rückfluss gerührt. Nach vollständiger Umsetzung wurde das Succinimid abfiltriert, der Filterrückstand mit Dichlormethan nachgewaschen und das Filtrat im Vakuum eingeengt. Das Rohprodukt wurde über Kieselgel chromatographisch gereinigt (Laufmittel Cyclohexan/Essigsäureethylester 40:1). Es wurden 2.667 g (7.5 mmol, 92% d. Th.) eines gelblichen Öls isoliert.

GC-MS (Methode 1): Rₜ = 5.72 min, m/z = 373 (M-Br)⁺ (*Diastereomer 1*); Rₜ = 5.74 min, m/z = 373 (M-Br)⁺ *(Diastereomer 2).*

### Beispiel 74A

### Ethyl-4,4,4-trifluor-3-methyl-2-[4-(2,2,2-trifluorethyl)phenyl]butanoat

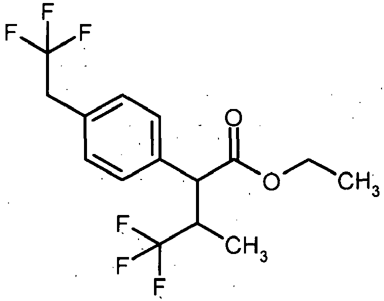

3.77 g (10.67 mmol) Ethyl-2-[4-(brommethyl)phenyl]-4,4,4-trifluor-3-methylbutanoat in 40 ml 1-Methylpyrrolidin-2-on wurden mit 529 mg (2.78 mmol) Kupfer(I)iodid und 4 g (20.82 mmol) Methyl-2,2-difluor-2-(fluorsulfonyl)acetat versetzt und über Nacht bei 80°C gerührt. Nach erfolgter Umsetzung wurde die Reaktionslösung langsam auf 100 ml Eiswasser gegossen. Das erhaltene Gemisch wurde anschließend dreimal mit Diethylether extrahiert. Die vereinigten organischen Phasen wurden über Magnesiumsulfat getrocknet. Nach Filtration wurde das Lösungsmittel im Vakuum entfernt. Das erhaltene Rohprodukt wurde über Kieselgel chromatographisch gereinigt (Laufmittel Cyclohexan/Dichlormethan 4:1). Es wurden 1.48 g (4.32 mmol, 41% d. Th.) der Titelverbindung als gelbliches Öl isoliert.

GC-MS (Methode 1): Rₜ = 4.06 min, m/z = 342 (M)⁺ (*Diastereomer 1*); Pₜ = 4.09 min, m/z = 342 (M)⁺ *(Diastereomer 2).*

MS (DCI): m/z = 360 (M+NH₄)⁺.

### Beispiel 75A

### Methyl-(4-chlorphenyl)(3-oxocyclopentyl)acetat

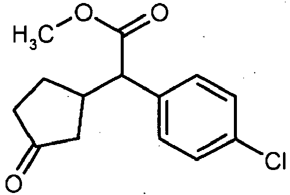

Unter Argon wurden 14.8 ml (105.6 mmol) Diisopropylamin in 150 ml THF vorgelegt, auf -30°C abgekühlt und langsam mit 42.3 ml (105.75 mmol) einer 2.5 M Lösung von *n*-Butyllithium in Hexan versetzt. Anschließend wurde die Reaktionslösung auf -20°C erwärmt, langsam mit 15 g (81.25 mmol) Methyl-(4-chlorphenyl)acetat, gelöst in 90 ml THF, versetzt und 2 h bei dieser Temperatur nachgerührt. Die Reaktionslösung wurde dann auf -78°C abgekühlt und langsam mit 7.2 ml (86.1 mmol) 2-Cyclopenten-1-on, gelöst in 60 ml THF, versetzt. Nach beendeter Zugabe wurde die Lösung 1 h bei -78°C nachgerührt. Nach DC-Kontrolle (Laufmittel Cyclohexan/Essigsäureethylester 9:1) wurde der Ansatz mit gesättigter wässriger Ammoniumchlorid-Lösung versetzt und in Essigsäureethylester aufgenommen. Die wässrige Phase wurde zweimal mit Essigsäureethylester extrahiert. Die vereinigten organischen Phasen wurden über Magnesiumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 4:1). Es wurden 15.65 g (58.67 mmol, 72% d. Th.) der Titelverbindung als gelbliches Öl isoliert.

GC-MS (Methode 1): Rₜ = 7.02 min, m/z = 266 (M)⁺ (*Diastereomer 1*); Rₜ = 7.04 min, m/z = 266 (M)⁺ *(Diastereomer 2).*

MS (DCI): m/z = 284 (M+NH₄)⁺.

### Beispiel 76A

### Methyl-(4-chlorphenyl)(3,3-difluorcyclopentyl)acetat

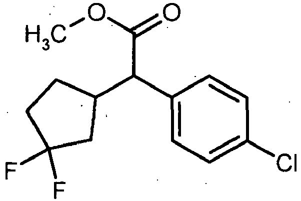

Unter Argon wurden 82.5 ml (82.14 mmol) einer 50%-igen Lösung von 1,1'-[(Trifluor-λ⁴-sulfanyl)-imino]bis(2-methoxyethan) (Desoxofluor) in THF, verdünnt mit 200 ml Toluol, vorgelegt, auf 5°C abgekühlt und langsam mit 744 µl (5.87 mmol) einer 1 M Bortrifluorid-Diethylether-Komplex-Lösung versetzt. Die Mischung wurde 2 h bei 5°C nachgerührt. Anschließend wurde die Reaktionslösung langsam mit 15.65 g (58.67 mmol) Methyl-(4-chlorphenyl)(3-oxocyclopentyl)-acetat, gelöst in 200 ml Toluol, versetzt, dann auf 55°C erwärmt und 60 h bei dieser Temperatur nachgerührt. Die Reaktionsmischung wurde danach in ein auf 0°C gekühltes Gemisch bestehend aus 100 ml Toluol und 100 ml 2 M Natronlauge gegeben. Die organische Phase wurde abgetrennt, und die wässrige Phase wurde noch dreimal mit Essigsäureethylester extrahiert. Die vereinigten organischen Phasen wurden über Natriumsulfat getrocknet. Nach Filtration wurde das Lösungsmittel im Vakuum entfernt. Das Rohprodukt wurde über Kieselgel chromatographisch gereinigt (Laufmittel Cyclohexan/Essigsäureethylester 7:1). Es wurden 13.24 g (45.86 mmol, 78% d. Th.) der Titelverbindung als farbloses Öl isoliert.

MS (DCI): m/z = 306 (M+NH₄)⁺.

GC-MS (Methode 1): Rₜ = 5.83 min, m/z = 288 (M)⁺ *(Diastereomer 1*); Rₜ = 5.86 min, m/z = 288 (M)⁺ *(Diastereomer 2).*

### Beispiel 77A

### (+)-(2S,3R)-2-(4-Chlorphenyl)-4,4,4-trifluor-3-methylbutansäure

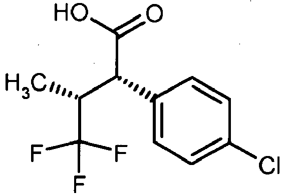

### Methode A:

5.086 g (17.26 mmol) Ethyl-(3*R*)-2-(4-chlorphenyl)-4,4,4-trifluor-3-methylbutanoat wurden in 68 ml Dioxan gelöst und mit 34 ml 1 N Natronlauge versetzt. Der Ansatz wurde 2 h bei 50°C gerührt. Das Reaktionsgemisch wurde dann mit 1 N Salzsäure auf pH 1 angesäuert und mehrmals mit Dichlormethan extrahiert. Die vereinigten organischen Phasen wurden mit gesättigter Natriumchlorid-Lösung gewaschen, über Natriumsulfat getrocknet und im Vakuum eingeengt. Es wurden 3.9 g (14.63 mmol, 85% d. Th., 83% de) der Zielverbindung erhalten.

¹H-NMR (400 MHz, DMSO-*d₆*, δ/ppm): 12.95-12.73 (1H, br. s), 7.49-7.34 (4H, m), 3.68 (1H, d), 3.31-3.18 (1H, m), 1.20 (0.25H, d), 0.78 (2.75H, d).

GC-MS (Methode 1): Rₜ = 4.85 min; m/z = 266 (M)⁺.

[α]_{D}²⁰ = +57.2°, c = 0.41, Methanol.

### Methode B:

16.28 g (55.24 mmol) Ethyl-(3*R*)-2-(4-chlorphenyl)-4,4,4-trifluor-3-methylbutanoat wurden in 220 ml Dioxan gelöst und mit 110.5 ml 1 N Natronlauge versetzt. Der Ansatz wurde 3 h bei 50°C gerührt. Anschließend wurde das Dioxan am Rotationsverdampfer entfernt und die verbliebene wässrige Lösung unter Eiskühlung mit 1 N Salzsäure neutralisiert (∼ pH 7). Der ausgefallene Feststoff wurde abgesaugt und über Nacht im Hochvakuum bei 40°C getrocknet. Es wurden 9.2 g der Zielverbindung als leicht beiger Feststoff erhalten (Fraktion 1; 62.5% d. Th., 94% de). Das Filtrat wurde durch weitere Zugabe von 1 N Salzsäure angesäuert (∼ pH 1) und über Nacht gerührt. Der ausgefallene Feststoff wurde wiederum abgesaugt und im Hochvakuum über Nacht bei 40°C getrocknet. Es wurden weitere 3.46 g der Zielverbindung als weißer Feststoff erhalten (Fraktion 2; verunreinigt mit 10% des zweiten Diastereomers). Das verbliebene wässrige Filtrat wurde mehrmals mit Dichlormethan extrahiert, und die vereinigten organischen Phasen wurden über Magnesiumsulfat getrocknet und im Vakuum eingeengt. Es wurden so nochmals 2.44 g der Zielverbindung als farbloses Öl erhalten (Fraktion 3; verunreinigt mit 15% des zweiten Diastereomers). Die Fraktionen 2 und 3 wurden schließlich vereinigt und über Kieselgel chromatographisch nachgereinigt (Laufmittel Cyclohexan/Essigsäureethylester 10:1). Hieraus wurden 3.7 g der Zielverbindung als weißer Feststoff isoliert (Fraktion 4; 25% d. Th., >95% de).

Fraktion 1 (= Natriumsalz der Titelverbindung):
¹H-NMR (400 MHz, DMSO-*d₆*, δ/ppm): 7.44-7.33 (4H, m), 3.61 (1H, d), 3.30-3.15 (1H, m), 1.17 (0.09H, d, Nebendiastereomer), 0.76 (2.91H, d, Hauptdiastereomer).

Fraktion 4:
¹H-NMR (400 MHz, DMSO-*d₆*, δ/ppm): 13.03-12.69 (br. s, 1H), 7.47-7.39 (4H, m), 3.68 (1H, d), 3.39-3.17 (1H, m, teilweise verdeckt durch H₂O-Signal), 0.77 (3H, d).

Auf analoge Weise wurden die in der folgenden Tabelle aufgeführten Verbindungen hergestellt:

| **Beispiel** | **Name / Struktur / Edukt** | **Analytische Daten** |
|---|---|---|
| **78A** | (2*S*,3*R*)-4,4,4-Trifluor-3-methyl-2-(4-methyl-phenyl)butansäure | GC-MS (Methode 1): |
| | | Rₜ = 4.17 min; m/z = 246 (M)⁺. |
| | 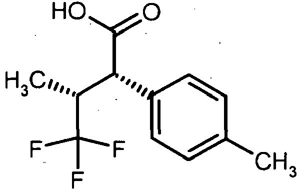 | ¹H-NMR (400 MHz, DMSO-d₆, δ/ppm): 0.75 (d, 2.75H, Hauptdiastereomer), 1.19 (d, 0.25H, Nebendiastereomer), 2.29 (s, 3H), 3.15-3.28 (m, 1H), 3.55 (d, 0.915H, Hauptdiastereomer), 3.60 (d, 0.085H, Nebendiastereomer), 7.17 (d, 2H), 7.24 (d, 2H), 12.68 (br. s. 1H) (83% de). |
| | aus Ethyl-(3*R*)-4,4,4-trifluor-3-methyl-2-(4-methylphenyl)butanoat | |
| **79A** | (2*S*,3*R*)-2-(4-Ethylphenyl)-4,4,4-trifluor-3-methylbutansäure | LC-MS (Methode 5): |
| | | Rₜ = 1.06 min; m/z = 259 (M-H)⁻. |
| | 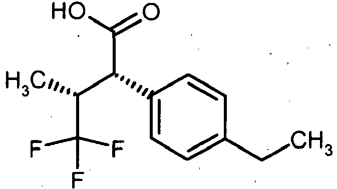 | ¹H-NMR (400 MHz, DMSO-d₆, δ/ppm): 0.75 (d, 3H), 1.17 (t, 3H), 2.59 (q, 2H), 3.14-3.29 (m, 1H), 3.56 (d, 1H), 7.20 (d, 2H), 7.27 (d, 2H), 12.53-12.86 (br. s, 1H). |
| | aus Ethyl-(3*R*)-2-(4-ethylphenyl)-4,4,4-trifluor-3-methylbutanoat | |
| **80A** | (2*S*,3*R*)-2-(4-Chlor-3-fluorphenyl)-4,4,4-trifluor-3-methylbutansäure | LC-MS (Methode 5): |
| | | Rₜ= 1.06 min; m/z = 259 (M-H)⁻. |
| | 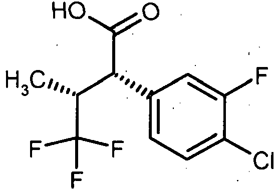 | ¹H-NMR (400 MHz, DMSO-d₆, δ/ppm): 0.80 (d, 2.75H, Hauptdiastereomer), 1.19 (d, 0.25H, Nebendiastereomer), 3.21-3.37 (m, 1H, teilweise verdeckt durch H₂O-Signal), 3.75 (d, 1H), 7.29 (dd, 1H), 7.51 (dd, 1H), 7.60 (t, 1H), 12.97 (br. s, 1H) (83% de). |
| | aus Ethyl-(3*R*)-2-(4-chlor-3-fluorphenyl)-4,4,4-trifluor-3-methylbutanoat | |
| **81A** | (2*S*,3*R*)-4,4,4-Trifluor-2-(4-fluorphenyl)-3-methylbutansäure | LC-MS (Methode 5): |
| | | Rₜ = 0.97 min; m/z = 249 (M-H)⁻. |
| | 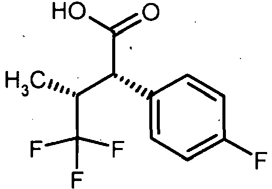 | ¹H-NMR (vom Natriumsalz; 400 MHz, DMSO-d₆, δ/ppm): 0.76 (d, 2.73H, Hauptdiastereomer), 1.19 (d, 0.27H, Nebendiastereomer), 3.16-3.31 (m, 1H), 3.66 (d, 1H), 7.15-7.23 (m, 2H), 7.37-7.46 (m, 2H) (82% de). |
| | aus Ethyl-(3R)-4,4,4-trifluor-2-(4-fluorphenyl)-3-methylbutanoat | |
| **82A** | (2*S*,3*R*)-4,4,4-Trifluor-2-(4-isopropylphenyl)-3-methylbutansäure | ¹H-NMR (400 MHz, DMSO-d₆, δ/ppm): 12.56 (1H, br. s), 7.25 (4H, q), 3.56 (1H, d), 3.28-3.16 (1H, m), 2.94-2.81 (1H, m), 1.19 (6H, d), 0.75 (3H, d). |
| | 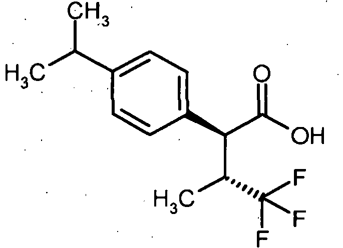 | |
| | | GC-MS (Methode 1): |
| | | Rₜ = 4.93 min; m/z = 274 (M)⁺. |
| | aus Ethyl-(3*R*)-4,4,4-trifluor-2-(4-isopropylphenyl)-3-methylbutanoat | |
| **83A** | (2*S*,3*R*)-2-(4-*tert*.-Butylphenyl)-4,4,4-trifluor-3-methylbutansäure | GC-MS (Methode 1): |
| | | Rₜ = 5.15 min; m/z = 288 (M)⁺. |
| | 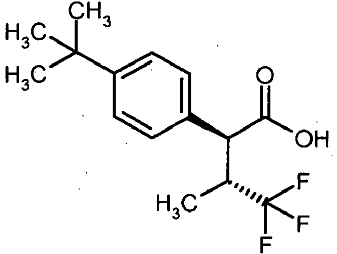 | |
| | aus Ethyl-(2S,3R)-2-(4-tert.-butylphenyl)-4,4,4-trifluor-3-methylbutanoat | |
| **84A** | 4,4,4-Trifluor-3-methyl-2-[4-(2,2,2-trifluorethyl)-phenyl]butansäure | ¹H-NMR (400 MHz, DMSO-d₆, δ/ppm): 12.95-12.59 (1H, br. s), 7.37 (4H, q), 3.70-3.57 (3H, m), 3.30-3.18 (1H, m), 0.76 (3H, d). |
| | 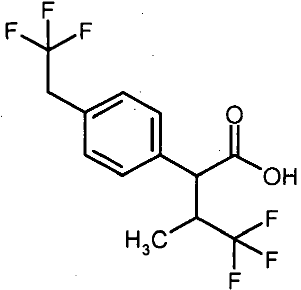 | |
| | | GC-MS (Methode 1): |
| | | Rₜ = 4.45 min; m/z = 315 (M+H)⁺. |
| | aus Ethyl-4,4,4-trifluor-3-methyl-2-[4-(2,2,2-trifluorethyl)phenyl]butanoat | |
| **85A** | (4-Chlorphenyl)(3,3-difluorcyclopentyl)essigsäure | ¹H-NMR (400 MHz, DMSO-d₆, δ/ppm): 12.59 (1H, br. s), 7.38 (4H, q), 3.51 (0.5H, d), 3.48 (0.5H, d), 2.77-2.60 (1H, m), 2.42-2.27 (0.5H, m), 2.26-1.20 (5.5H, m). |
| | 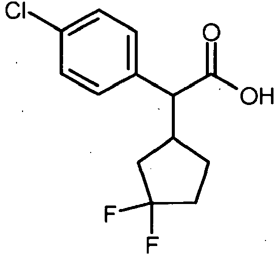 | |
| | | GC-MS (Methode 1): |
| | aus Methyl-(4-chlorphenyl)(3,3-difluorcyclopentyl)acetat | Rₜ = 6.33 min, m/z = 274 (M)⁺ *(Diastereomer 1*); |
| | | Rₜ = 6.38 min, m/z = 274 (M)⁺ *(Diastereomer 2).* |
| **86A** | (2*S*,3*R*)-2-(4-Chlor-3-methoxyphenyl)-4,4,4-trifluor-3-methylbutansäure | ¹H-NMR (400 MHz, DMSO-d₆, δ/ppm): 12.91-12.71 (1H, br. s), 7.41 (1H, d), 7.18 (1H, d), 6.98 (1H, dd), 3.86 (3H, s), 3.66 (1H, d), 3.40-3.19 (1H, m), 0.79 (3H, d). |
| | 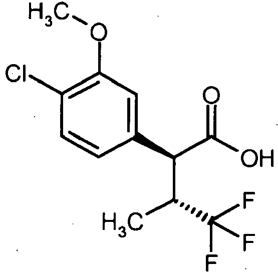 | |
| | | LC-MS (Methode 2): Rₜ = 2.20 min; m/z = 295/297 (M-H)⁻. |
| | aus Ethyl-(3*R*)-2-(4-chlor-3-methoxyphenyl)-4,4,4-trifluor-3-methylbutanoat | |
| **87A** | 2-(4-Chlor-3-methylphenyl)-4,4,4-trifluor-3-methylbutansäure | GC-MS (Methode 1): |
| | | Rₜ = 5.20 min; m/z = 280/282 (M)⁺ |
| | 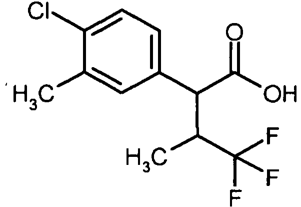 | *(Diastereomer 1*); |
| | | Rₜ = 5.23 min; m/z = 280/282 (M)⁺ |
| | | *(Diastereomer 2).* |
| | aus Ethyl-2-(4-chlor-3-methylphenyl)-4,4,4-trifluor-3-methylbutanoat | |
| **88A** | (2*S*,3*R*)-2-[4-(2,2-Difluorcyclopropyl)phenyl]-4,4,4-trifluor-3-methylbutansäure (*Diastereomerengemisch*) | LC-MS (Methode 5): |
| | | Rₜ = 1.09 min; m/z = 307 (M-H)⁻. |
| | | ¹H-NMR (400 MHz, DMSO-*d*₆): δ [ppm] = 0.76 (d, 3H), 1.86-2.04 (m, 2H), 2.92-3.06 (m, 1H), 3.18-3.29 (m, 1H), 3.61 (d, 1H), 7.27 (d, 2H), 7.34 (d, 2H), 12.72 (br. s, 1H). |
| | 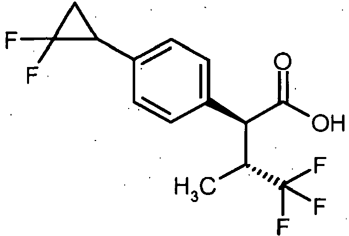 | |
| | (aus Ethyl-(2*S*,3*R*)-2-[4-(2,2-difluorcyclopropyl)-phenyl]-4,4,4-trifluor-3-methylbutanoat) | |

### Beispiel 89A

### (3R)-2-(4-Ethylphenyl)-4,4,4-trifluor-3-methylbutansäure (Diastereomerengemisch)

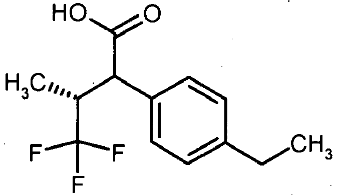

3.0 g (3*R*)-2-(4-Ethylphenyl)-4,4,4-trifluor-3-methylbutansäureethylester (ca. 88% Reinheit, ca. 9.16 mmol; Diastereomerengemisch) wurden in einem Gemisch aus je 12.4 ml Methanol, THF und Wasser gelöst und portionsweise mit 5.49 g (137.35 mmol) Natriumhydroxid versetzt. Die Reaktionsmischung wurde 9 h bei 40°C gerührt. Nach Abkühlen wurden die flüchtigen Lösungsmittel im Vakuum weitgehend entfernt und der Rückstand mit Wasser verdünnt. Durch Zugabe von Salzsäure wurde angesäuert, und die wässrige Phase wurde dreimal mit Ethylacetat extrahiert. Die vereinigten organischen Phasen wurden über Natriumsulfat getrocknet, im Vakuum eingeengt und der Rückstand im Hochvakuum getrocknet. Erhalten wurden 2.61 g der Titelverbindung als Rohprodukt, welches nicht weiter aufgereinigt wurde (Diastereomerenverhältnis ca. 9:1).

LC-MS (Methode 5): Rₜ = 1.08 min, m/z = 259 (M-H)⁻ (*Neben*-*Diastereomer*); Rₜ = 1.11 min, m/z = 259 (M-H)- (*Haupt-Diastereomer*).

¹H-NMR (400 MHz, DMSO-*d*₆): *Haupt-Diastereomer:* δ [ppm] = 0.76 (d, 3H), 1.17 (t, 3H), 2.54-2.66 (m, 4H), 3.10-3.29 (m, 1H), 3.56 (d, 1H), 7.14-7.22 (m, 2H), 7.22-7.32 (m, 2H), 12.58 (br. s, 1H).

Auf vergleichbare Weise (Reaktionstemperatur: RT bis +40°C; Reaktionszeit: 9-12 h) wurden aus den entsprechenden Estern die folgenden Carbonsäuren hergestellt:

### Beispiel 90A

### (3R)-4,4,4-Trifluor-2-(4-fluorphenyl)-3-methylbutansäure (Diastereomerengemisch)

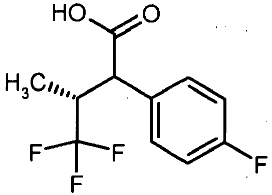

Diastereomerenverhältnis ca. 9:1.

¹H-NMR (400 MHz, DMSO-*d*₆): *Haupt-Diastereomer:* δ [ppm] = 0.77 (d, 3H), 3.18-3.30 (m, 1H), 3.67 (d, 1H), 7.17-7.24 (m, 2H), 7.39-7.47 (m, 2H), 12.78 (br. s, 1H).

### Beispiel 91A

### (3R)-2-(4-Chlor-3-fluorphenyl)-4,4,4-trifluor-3-methylbutansäure (Diastereomerengemisch)

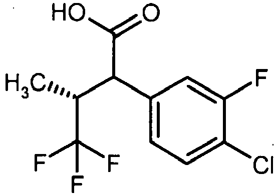

Diastereömerenverhältnis ca. 1:1.

GC-MS (Methode 1): Rₜ = 4.79 min; m/z = 284 (M)⁺.

¹H-NMR (400 MHz, DMSO-*d₆*): *beide Diastereomere:* δ [ppm] = 0.80/1.19 (je d, 3H), 3.18-3.29 (m; 1H), 3.74/3.77 (je dd, 1H), 7.28 (d, 1H), 7.43-7.65 (m, 2H), 12.91/13.24 (je br. s, 1H).

### Beispiele 92A - 95A

### (4-Chlorphenyl)(3,3-difluorcyclopentyl)essigsäure (Isomer 1 - 4)

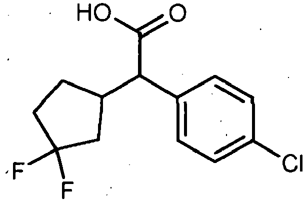

4 g (14.56 mmol) des Diastereomerengemisches von (4-Chlorphenyl)(3,3-difluorcyclopentyl)essigsäure (Beispiel 85A) wurden mittels präparativer HPLC an chiraler Phase in die vier enantiomerenreinen Diastereomere aufgetrennt [Säule: Daicel Chiralpak AD-H, 5 µm, 250 mm x 20 mm; Elutionsmittel: Isohexan/(Ethanol + 0.2% Trifluoressigsäure + 1% Wasser) 95:5 (v/v); Fluss: 20 ml/min; UV-Detektion: 230 nm; Temperatur: 25°C]:

### Beispiel 92A (Isomer 1):

Ausbeute: 682 mg

Rₜ = 8.12 min; chemische Reinheit >94%
[Säule: Daicel AD-H, 5 µm, 250 mm x 4.6 mm; Elutionsmittel: Isohexan/(Ethanol + 0.2% Trifluoressigsäure + 1% Wasser) 95:5 (v/v); Fluss: 1.25 ml/min; UV-Detektion: 230 nm; Temperatur: 30°C].

LC-MS (Methode 5): Rₜ = 1.03 min; m/z = 273 (M-H)⁻.

¹H-NMR (400 MHz, DMSO-*d*₆): δ [ppm] = 1.46-1.82 (m, 3H), 1.96-2.27 (m, 3H), 2.62-2.77 (m, 1H), 3.50 (d, 1H), 7.35 (d, 2H), 7.41 (d, 2H), 12.60 (br. s, 1H).

[α]_{D}²⁰ = -54.2°, c = 0.490, Methanol.

### Beispiel 93A (Isomer 2):

Ausbeute: 543 mg

Rₜ = 9.53 min; chemische Reinheit >97%
[Säule: Daicel AD-H, 5 µm, 250 mm x 4.6 mm; Elutionsmittel: Isohexan/(Ethanol + 0.2% Trifluoressigsäure + 1% Wasser) 95:5 (v/v); Fluss: 1.25 ml/min; UV-Detektion: 230 nm; Temperatur: 30°C].

LC-MS (Methode 5): Rₜ = 1.03 min; m/z = 273 (M-H)⁻.

¹H-NMR (400 MHz, DMSO-*d*₆): δ [ppm] = 1.46-1.82 (m, 3H), 1.96-2.27 (m, 3H), 2.63-2.77 (m, 1H), 3.50 (d, 1H), 7.35 (d, 2H), 7.41 (d, 2H), 12.61 (br. s, 1H).

[α]_{D}²⁰ = +53.0°, c = 0.375, Methanol.

### Beispiel 94A (Isomer 3):

Ausbeute: 530 mg

Rₜ = 10.36 min; chemische Reinheit >92%
[Säule: Daicel AD-H, 5 µm, 250 mm x 4.6 mm; Elutionsmittel: Isohexan/(Ethanol + 0.2% Trifluoressigsäure + 1% Wasser) 95:5 (v/v); Fluss: 1.25 ml/min; UV-Detektion: 230 nm; Temperatur: 30°C].

LC-MS (Methode 5): Rₜ = 1.04 min; m/z = 273 (M-H)⁻.

¹H-NMR (400 MHz, DMSO-*d*₆): δ [ppm] = 1.21-1.34 (m, 1H), 1.34-1.45 (m, 1H), 1.76-2.17 (m, 3H), 2.27-2.42 (m, 1H), 2.60-2.75 (m, 1H), 3.49 (d, 1H), 7.35 (d, 2H), 7.41 (d, 2H), 12.60 (br. s, 1H).

[α]_{D}²⁰ = -61.0°, c = 0.340, Methanol.

### Beispiel 95A (Isomer 4):

Ausbeute: 560 mg

Rₜ = 11.35 min; chemische Reinheit >91%
[Säule: Daicel AD-H, 5 µm, 250 mm x 4.6 mm; Elutionsmittel: Isohexan/(Ethanol + 0.2% Trifluoressigsäure + 1% Wasser) 95:5 (v/v); Fluss: 1.25 ml/min; UV-Detektion: 230 nm; Temperatur: 30°C].

LC-MS (Methode 5): Rₜ = 1.04 min; m/z = 273 (M-H)⁻.

¹H-NMR (400 MHz, DMSO-*d*₆): δ [ppm] = 1.21-1.34 (m, 1H), 1.34-1.45 (m, 1H), 1.77-2.17 (m, 3H), 2.27-2.42 (m, 1H), 2.60-2.75 (m, 1H), 3.49 (d, 1H), 7.35 (d, 2H), 7.41 (d, 2H), 12.59 (br. s, 1H).

[α]_{D}²⁰ = +56.4°, c = 0.485, Methanol.

### Beispiel 96A

### Methyl-3-(4-chlor-3-{[(2S,3R)-2-(4-chlorphenyl)-4,4,4-trifluor-3-methylbutanoyl]amino}phenyl)-4-methylpentanoat (Diastereomer 1)

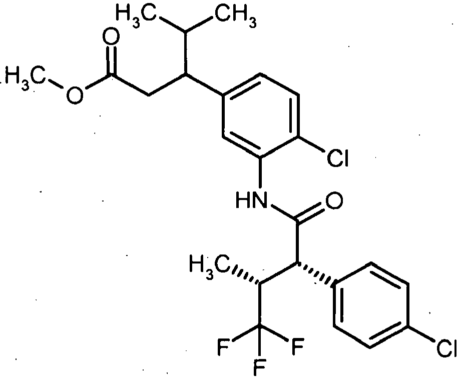

328 mg (1.23 mmol) (2*S*,3*R*)-2-(4-Chlorphenyl)-4,4,4-trifluor-3-methylbutansäure wurden in 17.5 ml Dichlormethan gelöst, mit 263 mg (1.97 mmol) 1-Chlor-*N*,*N*,2-trimethylprop-1-en-1-amin versetzt und 30 min bei Raumtemperatur gerührt. Anschließend wurde die Reaktionsmischung mit 299 µl (3.7 mmol) Pyridin und 315 mg (1.23 mmol) Methyl-3-(3-amino-4-chlorphenyl)-4-methyl-pentanoat (Enantiomer 1; Beispiel 17A) versetzt und über Nacht weiter gerührt. Das Reaktionsgemisch wurde danach im Vakuum eingeengt und das erhaltene Rohprodukt direkt mittels präparativer RP-HPLC gereinigt (Elutionsmittel Methanol/Wasser 80:20). Es wurden 237 mg der Zielverbindung erhalten (38% d. Th.).

LC-MS (Methode 5): Rₜ, = 1.43 min; m/z = 504/506 (M+H)⁺.

¹H-NMR (400 MHz, DMSO-*d₆*): δ [ppm] = 0.68 (d, 3H), 0.80 (d, 3H), 0.85 (d, 3H), 1.70-1.85 (m, 1H), 2.48-2.58 (m, 1H, teilweise verdeckt durch DMSO-Signal), 2.70-2.80 (m, 2H), 3.30-3.41 (m, 1H, teilweise verdeckt durch H₂O-Signal), 3.42 (s, 3H), 4.12 (d, 1H), 7.01 (dd, 1H), 7.31-7.37 (m, 2H), 7.43-7.50 (m, 4H), 9.83 (s, 1H).

[α]_{D}²⁰ = +111°, c = 0.25, Methanol.

### Beispiel 97A

### Methyl-3-(4-chlor-3-{[(2S,3R)-2-(4-chlorphenyl)-4,4,4-trifluor-3-methylbutanoyl]amino}phenyl)-4-methylpentanoat (Diastereomer 2)

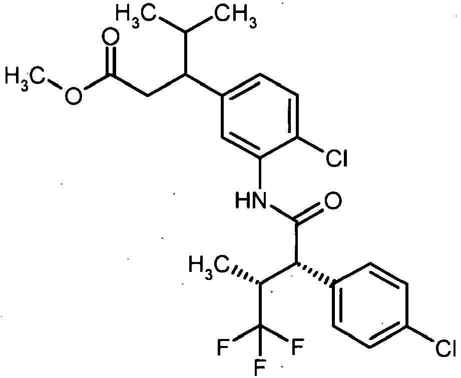

255 mg (0.96 mmol) (2*S*,3*R*)-2-(4-Chlorphenyl)-4,4,4-trifluor-3-methylbutansäure wurden in 14 ml Dichlormethan gelöst, mit 205 mg (1.53 mmol) 1-Chlor-*N*,*N*,2-trimethylprop-1-en-1-amin versetzt und 30 min bei Raumtemperatur gerührt. Anschließend wurde die Reaktionsmischung mit 232 µl (2.87 mmol) Pyridin und 245 mg (0.96 mmol) Methyl-3-(3-amino-4-chlorphenyl)-4-methylpentanoat (Enantiomer 2; Beispiel 18A) versetzt und über Nacht weiter gerührt. Das Reaktionsgemisch wurde danach im Vakuum eingeengt und das erhaltene Rohprodukt direkt mittels präparativer RP-HPLC gereinigt (Elutionsmittel Methanol/Wasser 80:20). Es wurden 228 mg der Zielverbindung erhalten (47% d. Th.).

LC-MS (Methode 5): Rₜ = 1.43 min; m/z = 504/506 (M+H)⁺.

¹H-NMR (400 MHz, DMSO-*d₆*): δ [ppm] = 0.67 (d, 3H), 0.80 (d, 3H), 0.85 (d, 3H), 1.71-1.82 (m, 1H), 2.47-2.58 (m, 1H, teilweise verdeckt durch DMSO-Signal), 2.70-2.80 (m, 2H), 3.29-3.41 (m, 1H, teilweise verdeckt durch H₂O-Signal), 3.43 (s, 3H), 4.12 (d, 1H), 7.01 (dd, 1H), 7.33 (d, 1H), 7.35 (d, 1H), 7.43-7.50 (m, 4H), 9.82 (s, 1H).

[α]_{D}²⁰ = +84.7°, c = 0.325, Methanol.

### Beispiel 98A

### tert. -Butyl-3-(4-chlor-3-{[(2S,3R)-2-(4-chlorphenyl)-4,4,4-trifluor-3-methylbutanoyl]amino}-phenyl)-3-cyclopropylpropanoat (Diastereomer 1)

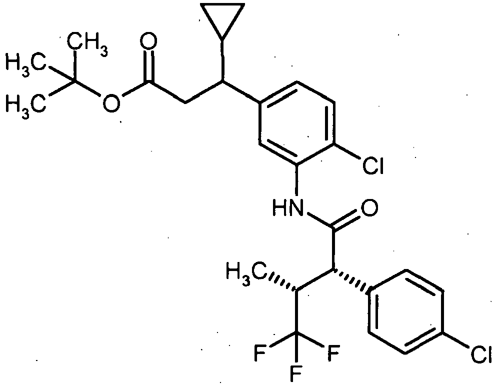

45 mg (0.17 mmol) (2*S*,3*R*)-2-(4-Chlorphenyl)-4,4,4-trifluor-3-methylbutansäure wurden in 1 ml Dichlormethan gelöst, mit 36 mg (0.27 mmol) 1-Chlor-*N*,*N*,2-trimethylprop-1-en-1-amin versetzt und 30 min bei Raumtemperatur gerührt. Anschließend wurde die Reaktionsmischung mit 41 µl (0.51 mmol) Pyridin und 50 mg (0.17 mmol) *tert*.-Butyl-3-(3-amino-4-chlorphenyl)-3-cyclopropyl-propanoat (Enantiomer 1; Beispiel 30A), gelöst in 1 ml Dichlormethan, versetzt und 1 h weiter gerührt. Das Reaktionsgemisch wurde danach im Vakuum eingeengt und das erhaltene Rohprodukt direkt über Kieselgel chromatographisch gereinigt (Laufmittel Cyclohexan/Essigsäureethylester 20:1). Es wurden 78 mg der Zielverbindung erhalten (85% d. Th.).

LC-MS (Methode 7): Rₜ = 1.52 min; m/z = 542/544 (M-H)⁻.

### Beispiel 99A

### tert. -Butyl-3-(4-chlor-3-{[(2S,3R)-2-(4-chlorphenyl)-4,4,4-trifluor-3-methylbutanoyl]amino}-phenyl)-3-cyclopropylpropanoat (Diastereomer 2)

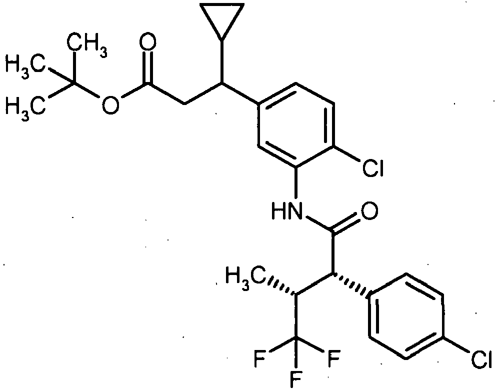

119 mg (0.45 mmol) (2*S*,3*R*)-2-(4-Chlorphenyl)-4,4,4-trifluor-3-methylbutansäure wurden in 2 ml Dichlormethan gelöst, mit 95 mg (0.71 mmol) 1-Chlor-*N*,*N*,2-trimethylprop-1-en-1-amin versetzt und 30 min bei Raumtemperatur gerührt. Anschließend wurde die Reaktionsmischung mit 108 µl (1.34 mmol) Pyridin und 132 mg (0.45 mmol) *tert.*-Butyl-3-(3-amino-4-chlorphenyl)-3-cyclopropyl-*p*ropanoat (Enantiomer 2; Beispiel 31A), gelöst in 2 ml Dichlormethan, versetzt und 1 h weiter gerührt. Das Reaktionsgemisch wurde danach im Vakuum eingeengt und das erhaltene Rohprodukt direkt über Kieselgel chromatographisch gereinigt (Laufmittel Cyclohexan/Essigsäureethylester 20:1). Es wurden 206 mg der Zielverbindung als farbloses Öl erhalten (85% d. Th.).

LC-MS (Methode 7): Rₜ = 1.53 min; m/z = 542/544 (M-H)⁻.

¹H-NMR (400 MHz, DMSO-*d*₆): δ [ppm] = 0.03-0.11 (m, 1H), 0.17-0.34 (m, 2H), 0.45-0.55 (m, 1H), 0.80 (d, 3H), 0.88-1.00 (m, 1H), 1.21 (s, 9H), 2.14-2.24 (m, 1H), 2.47-2.57 (m, 1H, verdeckt durch DMSO-Signal), 2.58-2.66 (m, 1H), 3.29-3.44 (m, 1H, teilweise verdeckt durch H₂O-Signal), 4.14 (d, 1H), 7.11 (dd, 1H), 7.37 (d, 1H), 7.40-7.51 (m, 5H), 9.82 (s, 1H).

Auf analoge Weise wurden die in der folgenden Tabelle aufgeführten Verbindungen hergestellt:

| **Beispiel** | **Name / Struktur / Edukte** | **Analytische Daten** |
|---|---|---|
| **100A** | *tert*.-Butyl-3-(4-chlor-3-{[(2*S*,3*R*)-2-(4-chlorphenyl)-4,4,4-trifluor-3-methylbutanoyl]amino}phenyl)-3-(1-methylcyclopropyl)propanoat | LC-MS (Methode 7): |
| | | Rₜ = 1.56 min; m/z = 556/558 (M-H)⁻. |
| | 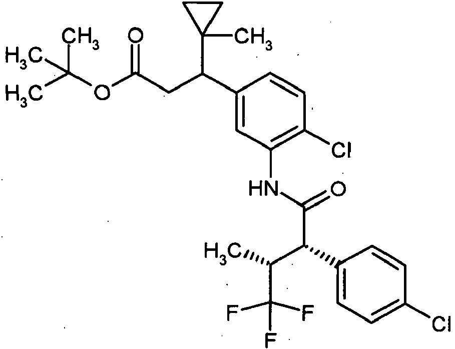 | |
| | aus *tert*.*-*Butyl-3-(3-amino-4-chlorphenyl)-3-(1-methylcyclopropyl)propanoat und (2*S*,3*R*)-2-(4-Chlorphenyl)-4,4,4-trifluor-3-methylbutansäure | |
| **101A** | *tert.* -Butyl-3-(4-chlor-3-{[(2*S*,3*R*)-2-(4-chlorphenyl)-4,4,4-trifluor-3-methylbutanoyl]amino}phenyl)-4-methoxy-4-methylpentanoat | LC-MS (Methode 5): |
| | | Rₜ = 1.48 min; m/z = 574/576 (M-H)⁻. |
| | 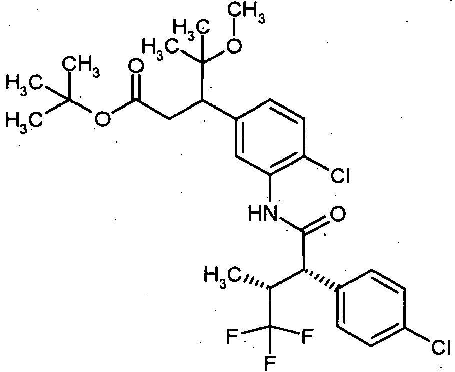 | |
| | aus *tert*.-Butyl-3-(3-amino-4-chlorphenyl)-4-methoxy-4-methylpentanoat und (2*S*,3*R*)-2-(4-Chlorphenyl)-4,4,4-trifluor-3-methylbutansäure | |
| **102A** | *tert.*-Butyl-3-(4-chlor-3-{[(2*S*,3*R*)-2-(4-chlorphenyl)-4,4,4-trifluor-3-methylbutanoyl]amino}phenyl)-3-(1-fluorcyclopropyl)propanoat | LC-MS (Methode 5): |
| | | Rₜ = 1.46 min; m/z = 560/562 (M-H)⁻ |
| | 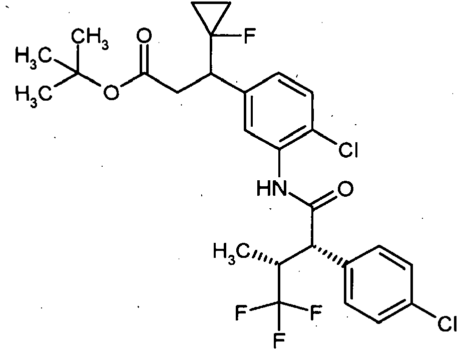 | |
| | aus *tert*.-Butyl-3-(3-amino-4-chlorphenyl)-3-(1-fluorcyclopropyl)propanoat und (2*S*,3*R*)-2-(4-Chlorphenyl)-4,4,4-trifluor-3-methylbutansäure | |
| **103A** | Methyl-3-(4-chlor-3-{[(2*S*,3*R*)-2-(4-chlorphenyl)-4,4,4-trifluor-3-methylbutanoyl]amino}phenyl)-3,4-dimethylpentanoat (*Diastereomer 1*) | LC-MS (Methode 7): |
| | | Rₜ = 1.48 min; m/z = 518/520 (M+H)⁺. |
| | 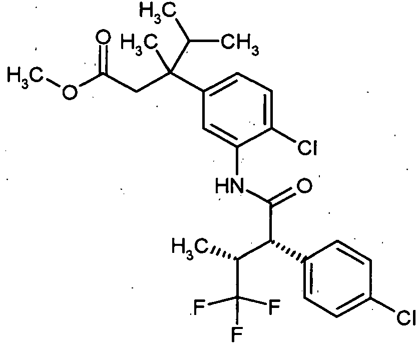 | ¹H-NMR (400 MHz, DMSO-*d₆*): δ [ppm] = 0.56 (d, 3H), 0.81 (dd, 6H), 1.29 (s, 3H), 1.82-1.93 (m, 1H), 2.58 (d, 1H), 2.77 (d, 1H), 3.30-3.44 (m, 1H), 3.33 (s, 3H), 4.12 (d, 1H), 7.12 (dd, 1H), 7.33 (d, 1H), 7.43-7.50 (m, 5H), 9.81 (s, 1H). |
| | aus Methyl-3-(3-amino-4-chlorphenyl)-3,4-dimethylpentanoat (Enantiomer 1, Beispiel 50A) und (+)-(2*S*,3*R*)-2-(4-Chlorphenyl)-4,4,4-trifluor-3-methylbutansäure | |
| **104A** | Methyl-3-(4-chlor-3-{[(2*S*,3*R*)-2-(4-chlorphenyl)-4,4,4-trifluor-3-methylbutanoyl]amino}phenyl)-3,4-dimethylpentanoat (*Diastereomer 2)* | LC-MS (Methode 5): |
| | | Rₜ = 1.48 min; m/z = 518/520 (M+H)⁺. |
| | 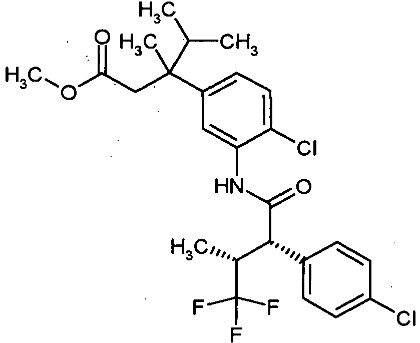 | ¹H-NMR (400 MHz, DMSO-*d₆*): δ [ppm] = 0.57 (d, 3H), 0.81 (dd, 6H), 1.29 (s, 3H), 1.80-1.92 (m, 1H), 2.58 (d, 1H), 2.77 (d, 1H), 3.29-3.46 (m, 1H), 3.35 (s, 3H), 4.12 (d, 1H), 7.12 (dd, 1H), 7.33 (d, 1H), 7.42-7.50 (m, 5H), 9.81 (s, 1H). |
| | aus Methyl-3-(3-amino-4-chlorphenyl)-3,4-dimethylpentanoat (Enantiomer 2, Beispiel 51A) und (+)-(2*S*,3*R*)-2-(4-Chlorphenyl)-4,4,4-trifluor-3-methylbutansäure | |
| **105A** | Methyl-3-(4-chlor-3-{[(2*S*,3*R*)-2-(4-chlorphenyl)-4,4,4-trifluor-3-methylbutanoyl]amino}phenyl)-3-cyclobutylbutanoat (*Diastereomer 1*) | LC-MS (Methode 5): |
| | | Rₜ = 1.51 min; m/z = 530/532 (M+H)⁺. |
| | 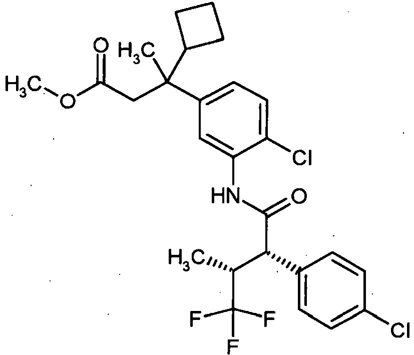 | ¹H-NMR (400 MHz, DMSO-*d₆*): δ [ppm] = 0.80 (d, 3H), 1.31 (s, 3H), 1.44-1.53 (m, 1H), 1.53-1.67 (m, 3H), 1.67-1.78 (m, 2H), 2.46 (d, 1H), 2.47-2.60 (m, 1H, teilweise verdeckt durch DMSO-Signal), 2.70 (d, 1H), 3.36-3.46 (m, 1H), 3.38 (s, 3H), 4.12 (d, 1H), 7.10 (dd, 1H), 7.34 (d, 1H), 7.42-7.51 (m, 5H), 9.81 (s, 1H). |
| | aus Methyl-3-(3-amino-4-chlorphenyl)-3-cyclobutylbutanoat (Enantiomer 1, Beispiel 52A) und (+)-(2*S*,3*R*)-2-(4-Chlorphenyl)-4,4,4-trifluor-3-methylbutansäure | |
| **106A** | Methyl-3-(4-chlor-3-{[(2*S*,3*R*)-2-(4-chlorphenyl)-4,4,4-trifluor-3-methylbutanoyl]amino}phenyl)-3-cyclobutylbutanoat (*Diastereomer 2*) | LC-MS (Methode 5): |
| | | Rₜ = 1.51 min; m/z = 530/532 (M+H)⁺. |
| | 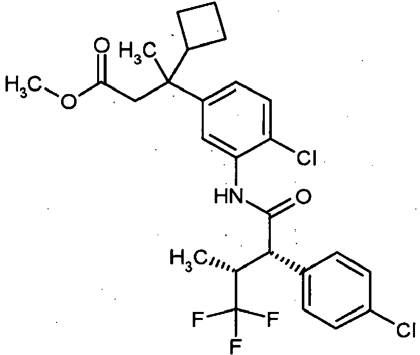 | ¹H-NMR (400 MHz, DMSO-*d₆*): δ [ppm] = 0.81 (d, 3H), 1.31 (s, 3H), 1.43-1.53 (m, 1H), 1.53-1.67 (m, 3H), 1.67-1.78 (m, 2H), 2.46 (d, 1H), 2.46-2.59 (m, 1H, teilweise verdeckt durch DMSO-Signal), 2.70 (d, 1H), 3.36-3.46 (m, 1H), 3.40 (s, 3H), 4.12 (d, 1H), 7.10 (dd, 1H), 7.34 (d, 1H), 7.43-7.50 (m, 5H), 9.81 (s, 1H). |
| | aus Methyl-3-(3-amino-4-chlorphenyl)-3-cyclobutylbutanoat (Enantiomer 2, Beispiel 53A) und (+)-(2*S*,3*R*)-2-(4-Chlorphenyl)-4,4,4-trifluor-3-methylbutansäure | |
| **107A** | Methyl-3-(4-chlor-3-{[(2*S*,3*R*)-2-(4-ethylphenyl)-4,4,4-trifluor-3-methylbutanoyl]amino}phenyl)-4-methylpentanoat (*Diastereomer 1*) | LC-MS (Methode 5): |
| | | Rₜ = 1.48 min; m/z = 498 (M+H)⁺. |
| | 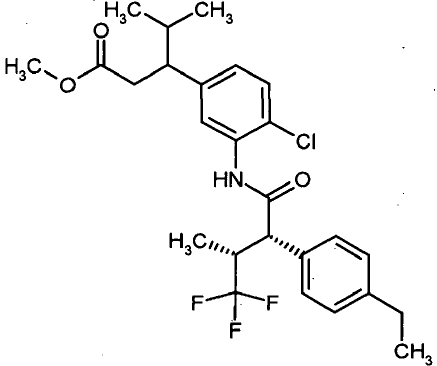 | ¹H-NMR (400 MHz, DMSO-*d₆*): δ [ppm] = 0.68 (d, 3H), 0.79 (d, 3H), 0.85 (d, 3H), 1.17 (t, 3H), 1.70-1.84 (m, 1H), 2.45-2.64 (m, 3H, teilweise verdeckt durch DMSO-Signal), 2.70-2.80 (m, 2H), 3.28-3.39 (m, 1H, teilweise verdeckt durch H₂O-Signal), 3.42 (s, 3H), 4.06 (d, 1H), 6.98 (dd, 1H), 7.21 (d, 2H), 7.30-7.39 (m, 4H), 9.73 (s, 1H). |
| | aus Methyl-3-(3-amino-4-chlorphenyl)-4-methyl-pentanoat (Enantiomer 1, Beispiel 17A) und (*2S*,3*R*)-2-(4-Ethylphenyl)-4,4,4-trifluor-3-methylbutansäure | |
| **108A** | Methyl-3-(4-chlor-3-{[(2*S*,3*R*)-2-(4-chlor-3-fluor-phenyl)-4,4,4-trifluor-3-methylbutanoyl]amino}-phenyl)-4-methylpentanoat (*Diastereomer 1*) | LC-MS (Methode 5): |
| | | Rₜ = 1.43 min; m/z = 522/524 (M+H)⁺. |
| | 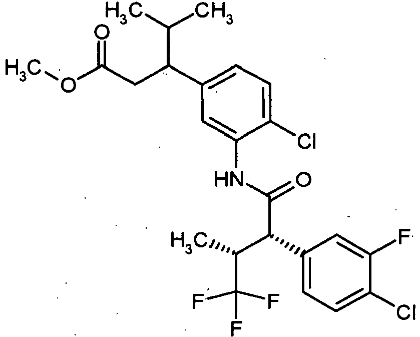 | ¹H-NMR (400 MHz, DMSO-*d₆*): δ [ppm] = 0.68 (d, 2.77H, Hauptdiastereomer), 0.84 (t, 6H), 1.25 (d, 0.23H, Nebendiastereomer), 1.71-1.84 (m, 1H), 2.46-2.60 (m, 1H, teilweise verdeckt durch DMSO-Signal), 2.70-2.81 (m, 2H), 3.36-3.49 (m, 1H), 3.43 (s, 3H), 4.15 (d, 1H), 7.02 (dd, 1H), 7.29-7.38 (m, 3H), 7.50 (dd, 1H), 7.63 (t, 1H), 9.87 (s, 0.925H, Hauptdiastereomer), 10.01 (s, 0.075H, Nebendiastereomer) (85% de). |
| | aus Methyl-3-(3-amino-4-chlorphenyl)-4-methyl-pentanoat (Enantiomer 1, Beispiel 17A) und (2*S*,3*R*)-2-(4-Chlor-3-fluorphenyl)-4,4,4-trifluor-3-methylbutansäure | |
| **109A** | Methyl-3-(4-chlor-3-{[(2*S*,3*R*)-4,4,4-trifluor-2-(4-isopropylphenyl)-3-methylbutanoyl]amino}-phenyl)-4-methylpentanoat (*Diastereomer 1*) | LC-MS (Methode 5): |
| | | Rₜ = 1.54 min; m/z = 512/514 (M+H)⁺. |
| | 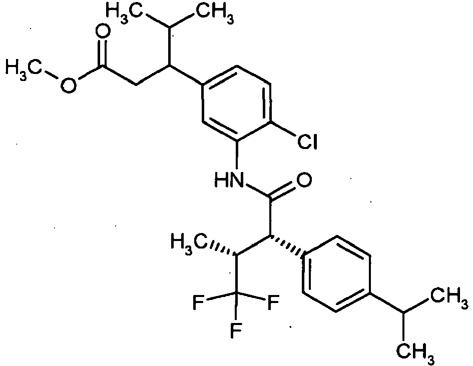 | ¹H-NMR (400 MHz, DMSO-*d₆*): δ [ppm] = 0.68 (d, 3H), 0.79 (d, 3H), 0.85 (d, 3H), 1.19 (d, 6H), 1.71-1.83 (m, 1H), 2.45-2.58 (m, 1H, verdeckt durch DMSO-Signal), 2.70-2.80 (m, 2H), 2.81-2.93 (m, 1H), 3.28-3.39 (m, 1H, teilweise verdeckt durch H₂O-Signal), 3.42 (s, 3H), 4.07 (d, 1H), 6.98 (dd, 1H), 7.24 (d, 2H), 7.31-7.41 (m, 4H), 9.73 (s, 1H). |
| | aus Methyl-3-(3-amino-4-chlorphenyl)-4-methylpentanoat (Enantiomer 1, Beispiel 17A) und (2*S*,3*R*)-4,4,4-Trifluor-2-(4-isopropylphenyl)-3-methylbutansäure | |
| **110A** | Methyl-3-(4-chlor-3-{[(2S,3R)-4,4,4-trifluor-2-(4-fluorphenyl)-3-methylbutanoyl]amino}phenyl)-4-methylpentanoat (*Diastereomer 1*) | LC-MS (Methode 5): |
| | | Rₜ = 1.36 min; m/z = 488/490 (M+H)⁺. |
| | 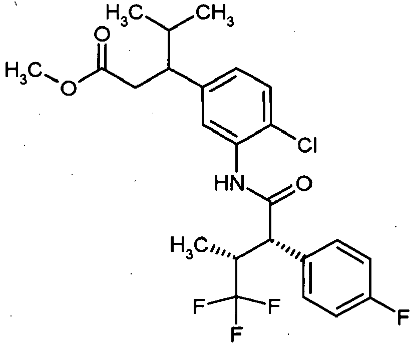 | ¹H-NMR (400 MHz, DMSO-*d₆*): δ [ppm] = 0.68 (d, 2.77H, Hauptdiastereomer), 0.80 (d, 3H), 0.85 (d, 3H), 1.25 (d, 0.23H, Nebendiastereomer), 1.71-1.83 (m, 1H), 2.45 (m, 1H, verdeckt durch DMSO-Signal), 2.70-2.81 (m, 2H), 3.28-3.40 (m, 1H, teilweise verdeckt durch H₂O-Signal), 3.42 (s, 3H), 4.11 (d, 1H), 7.00 (dd, 1H), 7.22 (t, 2H), 7.31-7.37 (m, 2H), 7.44-7.54 (m, 2H), 9.80 (s, 0.925H, Hauptdiastereomer), 9.93 (s, 0.075H, Nebendiastereomer) (85% de). |
| | aus Methyl-3-(3-amino-4-chlorphenyl)-4-methyl-pentanoat (Enantiomer 1, Beispiel 17A) und (2*S*,3*R*)-4,4,4-Trifluor-2-(4-fluorphenyl)-3-methylbutansäure | |
| **111A** | Methyl-3-[4-chlor-3-({4,4,4-trifluor-3-methyl-2-[4-(2,2,2-trifluorethyl)phenyl]butanoyl}amino)-phenyl]-4-methylpentanoat (*Diastereomerengemisch*) | LC-MS (Methode 5): |
| | | Rₜ = 1.40 min; m/z = 552/554 (M+H)⁺. |
| | 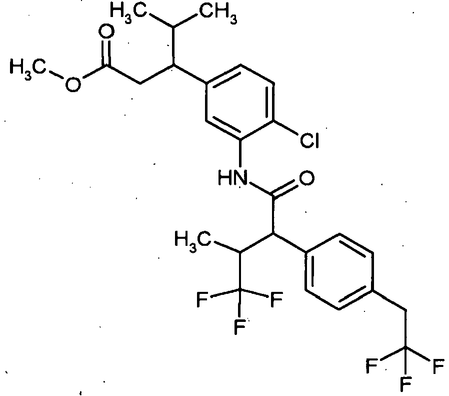 | |
| | aus Methyl-3-(3-amino-4-chlorphenyl)-4-methyl-pentanoat (Enantiomer 1, Beispiel 17A) und 4,4,4-Trifluor-3-methyl-2-[4-(2,2,2-trifluorethyl)-phenyl]butansäure *(Diastereomerengemisch)* | |
| **112A** | Methyl-3-[4-chlor-3-({(2*S*,3*R*)-2-[4-(2,2-difluor-cyclopropyl)phenyl]-4,4,4-trifluor-3-methyl-butanoyl}amino)phenyl]-4-methylpentanoat (*Diastereomerengemisch*) | LC-MS (Methode 5): |
| | | Rₜ = 1.39 min; m/z = 546/548 (M+H)⁺. |
| | 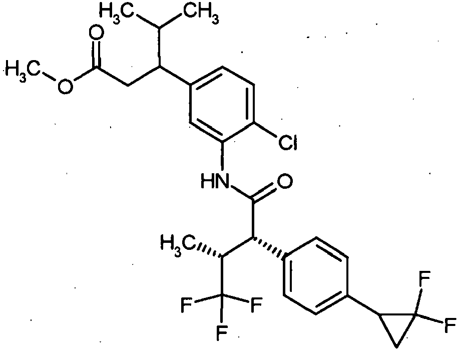 | |
| | aus Methyl-3-(3-amino-4-chlorphenyl)-4-methylpentanoat (Enantiomer 1, Beispiel 17A) und (2*S*,3*R*)-2-[4-(2,2-Difluorcyclopropyl)phenyl]-4,4,4-trifluor-3-methylbutansäure (*Diastereomerengemisch*) | |
| **113A** | Methyl-3-(3-{[(2*S*,3*R*)-2-(4-*tert*.-butylphenyl)-4,4,4-trifluor-3-methylbutanoyl]amino}-4-chlorphenyl)-4-methylpentanoat (*Diastereomer 1*) | LC-MS (Methode 5): |
| | | Rₜ = 1.52 min; m/z = 526/528 (M+H)⁺. |
| | 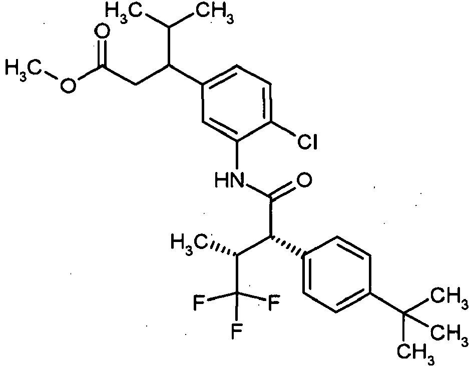 | ¹H-NMR (400 MHz, DMSO-*d₆*): δ [ppm] = 0.68 (d, 3H), 0.79 (d, 3H), 0.85 (d, 3H), 1.27 (s, 9H), 1.70-1.84 (m, 1H), 2.46-2.58 (m, 1H, teilweise verdeckt durch DMSO-Signal), 2.70-2.80 (m, 2H), 3.28-3.39 (m, 1H, teilweise verdeckt durch H₂O-Signal), 3.42 (s, 3H), 4.08 (d, 1H), 6.98 (dd, 1H), 7.31-7.43 (m, 6H), 9.72 (s, 0.96H, Hauptdiastereomer), 9.86 (s, 0.04H, Nebendiastereomer) (92% de). |
| | aus Methyl-3-(3-amino-4-chlorphenyl)-4-methylpentanoat (Enantiomer 1, Beispiel 17A) und (2*S*,3*R*)-2-(4-*tert*.-Butylphenyl)-4,4,4-trifluor-3-methylbutansäure | |
| **114A** | Methyl-3-(4-chlor-3-{[(2*S*,3*R*)-2-(4-chlor-3-methoxyphenyl)-4,4,4-trifluor-3-methylbutanoyl]-amino}phenyl)-4-methylpentanoat (*Diastereomer 1*) | LC-MS (Methode 4): |
| | | Rₜ = 2.91 min; m/z = 534/536 (M+H)⁺. |
| | 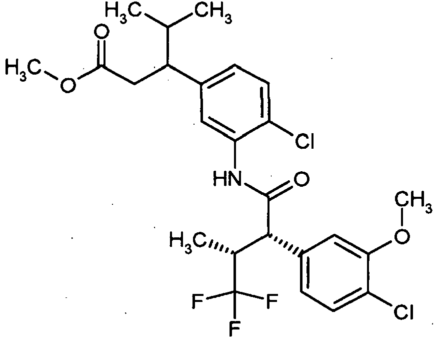 | |
| | aus Methyl-3-(3-amino-4-chlorphenyl)-4-methylpentanoat (Enantiomer 1, Beispiel 17A) und (2*S*,3*R*)-2-(4-Chlor-3-methoxyphenyl)-4,4,4-trifluor-3-methylbutansäure | |
| **115A** | Methyl-3-(4-chlor-3-{[(2*S*,3*R*)-4,4,4-trifluor-3-methyl-2-(4-methylphenyl)butanoyl]amino}-phenyl)-4-methylpentanoat (*Diastereomer 1*) | LC-MS (Methode 5): |
| | | Rₜ = 1.40 min; m/z = 484/486 (M+H)⁺. |
| | 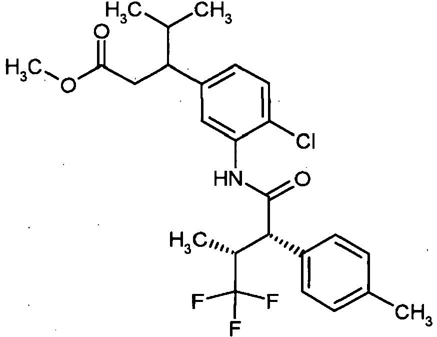 | ¹H-NMR (400 MHz, DMSO-*d₆*): δ [ppm] = 0.68 (d, 3H), 0.79 (d, 3H), 0.85 (d, 3H), 1.70-1.84 (m, 1H), 2.29 (s, 3H), 2.48-2.57 (m, 1H, verdeckt durch DMSO-Signal), 2.70-2.80 (m, 2H), 3.29-3.40 (m, 1H, teilweise verdeckt durch H₂O-Signal), 3.42 (s, 3H), 4.05 (d, 1H), 6.99 (dd, 1H), 7.18 (d, 2H), 7.29-7.38 (m, 4H), 9.73 (s, 0.94H, Hauptdiastereomer), 9.87 (s, 0.06H, Nebendiastereomer) (88% de). |
| | aus Methyl-3-(3-amino-4-chlorphenyl)-4-methyl-pentanoat (Enantiomer 1, Beispiel 17A) und (2*S*,3*R*)-4,4,4-Trifluor-3-methyl-2-(4-methyl-phenyl)butansäure | |
| **116A** | Methyl-3-(4-chlor-3-{[(2*S*,3*R*)-2-(4-chlor-3-methyl-phenyl)-4,4,4-trifluor-3-methylbutanoyl]amino}-phenyl)-4-methylpentanoat (*Diastereomer 1*) | LC-MS (Methode 5): |
| | | Rₜ = 1.51 min; m/z = 518/520 (M+H)⁺. |
| | 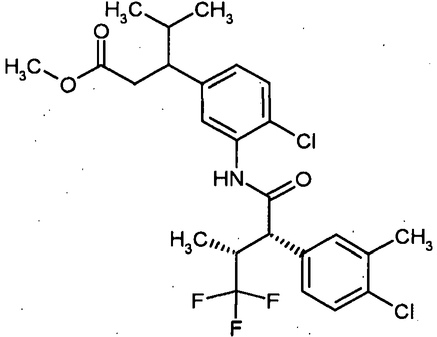 | ¹H-NMR (400 MHz, DMSO-*d₆*): δ [ppm] = 0.68 (d, 3H), 0.81 (d, 3H), 0.85 (d, 3H), 1.70-1.83 (m, 1H), 2.23 (s, 3H), 2.45-2.59 (m, 1H, teilweise verdeckt durch DMSO-Signal), 2.70-2.81 (m, 2H), 3.28-3.41 (m, 1H, teilweise verdeckt durch H₂O-Signal), 3.42 (s, 3H), 4.07 (d, 1H), 7.00 (dd, 1H), 7.27-7.45 (m, 5H), 9.81 (s, 0.94H, Hauptdiastereomer), 9.89 (s, 0.06H, Nebendiastereomer) (88% de). |
| | aus Methyl-3-(3-amino-4-chlorphenyl)-4-methylpentanoat (Enantiomer 1, Beispiel 17A) und (2*S*,3*R*)-2-(4-Chlor-3-methylphenyl)-4,4,4-trifluor-3-methylbutansäure | |
| **117A** | Methyl-3-(4-chlor-3-{[(4-chlorphenyl)(3,3-difluor-cyclopentyl)acetyl]amino}phenyl)-4-methylpentanoat (*Isomer 1*) | LC-MS (Methode 5): |
| | | Rₜ = 1.41 min; m/z = 512/514 (M+H)⁺. |
| | 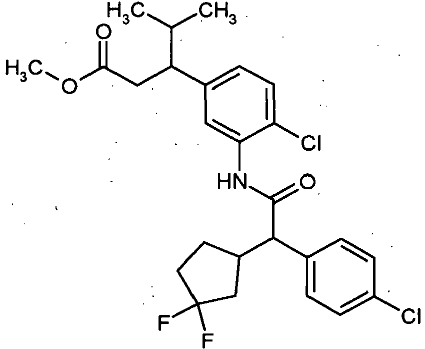 | ¹H-NMR (400 MHz, DMSO-*d₆*): δ [ppm] = 0.67 (d, 3H), 0.85 (d, 3H), 1.52-1.70 (m, 2H), 1.72-1.82 (m, 1H), 1.82-1.95 (m, 1H), 1.98-2.30 (m, 3H), 2.46-2.60 (m, 1H, teilweise ver-deckt durch DMSO-Signal), 2.70-2.80 (m, 2H), 2.80-2.93 (m, 1H), 3.43 (s, 3H), 3.78 (d, 1H), 7.02 (dd, 1H), 7.33 (d, 1H), 7.37 (d, 1H), 7.44 (q, 4H), 9.78 (s, 1H). |
| | aus Methyl-3-(3-amino-4-chlorphenyl)-4-methyl-pentanoat (Enantiomer 1, Beispiel 17A) und (4-Chlorphenyl)(3,3-difluorcyclopentyl)essigsäure (*Isomer 1*) | |
| **118A** | Methyl-3-(4-chlor-3-{[(4-chlorphenyl)(3,3-difluor-cyclopentyl)acetyl]amino}phenyl)-4-methylpentanoat (*Isomer 2*) | LC-MS (Methode 5): |
| | | Rₜ = 1.41 min; m/z = 512/514 (M+H)⁺. |
| | 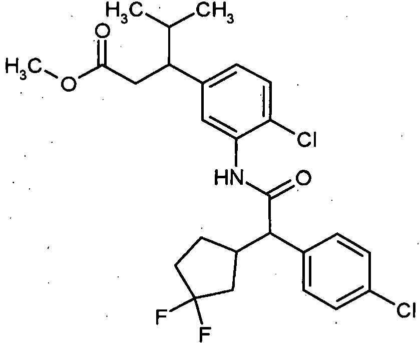 | ¹H-NMR (400 MHz, DMSO-*d₆*): δ [ppm] = 0.67 (d, 3H), 0.85 (d, 3H), 1.52-1.69 (m, 2H), 1.72-1.81 (m, 1H), 1.81-1.96 (m, 1H), 1.98-2.30 (m, 3H), 2.46-2.60 (m, 1H, teilweise verdeckt durch DMSO-Signal), 2.70-2.80 (m, 2H), 2.80-2.93 (m, 1H), 3.43 (s, 3H), 3.78 (d, 1H), 7.02 (dd, 1H), 7.34 (d, 1H), 7.36 (d, 1H), 7.44 (q, 4H), 9.78 (s, 1H). |
| | aus Methyl-3-(3-amino-4-chlorphenyl)-4-methylpentanoat (Enantiomer 1, Beispiel 17A) und (4-Chlorphenyl)(3,3-difluorcyclopentyl)essigsäure (*Isomer 2*) | |
| **119A** | Methyl-3-(4-chlor-3-{[(4-chlorphenyl)(3,3-difluor-cyclopentyl)acetyl]amino}phenyl)-4-methylpentanoat (*Isomer 3*) | LC-MS (Methode 5): |
| | | Rₜ = 1.42 min; m/z = 512/514 (M+H)⁺. |
| | 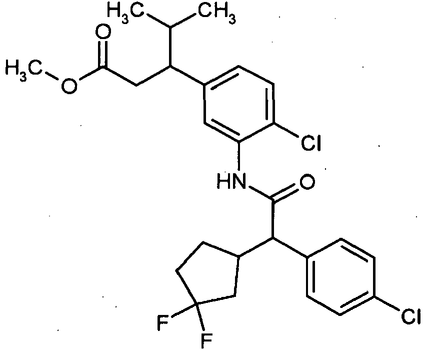 | ¹H-NMR (400 MHz, DMSO-*d₆*): δ [ppm] = 0.67 (d, 3H), 0.85 (d, 3H), 1.21-1.35 (m, 1H), 1.45-1.58 (m, 1H), 1.72-1.83 (m, 1H), 1.85-2.20 (m, 3H), 2.28-2.43 (m, 1H), 2.47-2.60 (m, 1H, teilweise verdeckt durch DMSO-Signal), 2.70-2.90 (m, 3H), 3.44 (s, 3H), 3.75 (d, 1H), 7.02 (dd, 1H), 7.33 (d, 1H), 7.37 (d, 1H), 7.44 (q, 4H), 9.74 (s, 1H). |
| | aus Methyl-3-(3-amino-4-chlorphenyl)-4-methylpentanoat (Enantiomer 1, Beispiel 17A) und (4-Chlorphenyl)(3,3-difluorcyclopentyl)essigsäure (*Isomer 3*) | |
| **120A** | Methyl-3-(4-chlor-3-{[(4-chlorphenyl)(3,3-difluor-cyclopentyl)acetyl]amino}phenyl)-4-methylpentanoat (*Isomer 4*) | LC-MS (Methode 5): |
| | | Rₜ = 1.42 min; m/z = 512/514 (M+H)⁺. |
| | 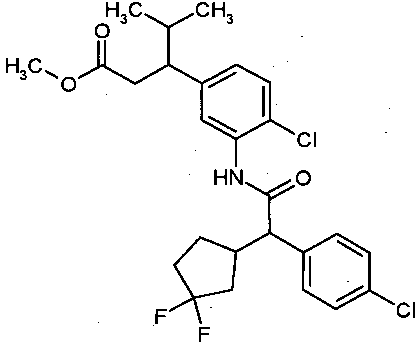 | ¹H-NMR (400 MHz, DMSO-*d₆*): 8 [ppm] = 0.68 (d, 3H), 0.86 (d, 3H), 1.21-1.35 (m, 1H), 1.45-1.58 (m, 1H), 1.71-1.83 (m, 1H), 1.85-2.20 (m, 3H), 2.29-2.44 (m, 1H), 2.46-2.61 (m, 1H, teilweise verdeckt durch DMSO-Signal), 2.70-2.90 (m, 3H), 3.43 (s, 3H), 3.75 (d, 1H), 7.02 (dd, 1H), 7.34 (d, 1H), 7.36 (d, 1H), 7.44 (q, 4H), 9.74 (s, 1H). |
| | aus Methyl-3-(3-amino-4-chlorphenyl)-4-methyl-pentanoat (Enantiomer 1, Beispiel 17A) und (4-Chlorphenyl)(3,3-difluorcyclopentyl)essigsäure (*Isomer 4*) | |

### Beispiel 121A

### Methyl-3-(4-chlor-3-{[(2S,3R)-2-(4-chlorphenyl)-4,4,4-trifluor-3-methylbutanoyl]amino}phenyl)-3-(2,2-difluorcyclopropyl)propanoat und Methyl-3-(4-chlor-3-{[(2S,3R)-2-(4-chlorphenyl)-4,4,4-trifluor-3-methylbutanoyl]amino}phenyl)-5,5-difluorhexanoat

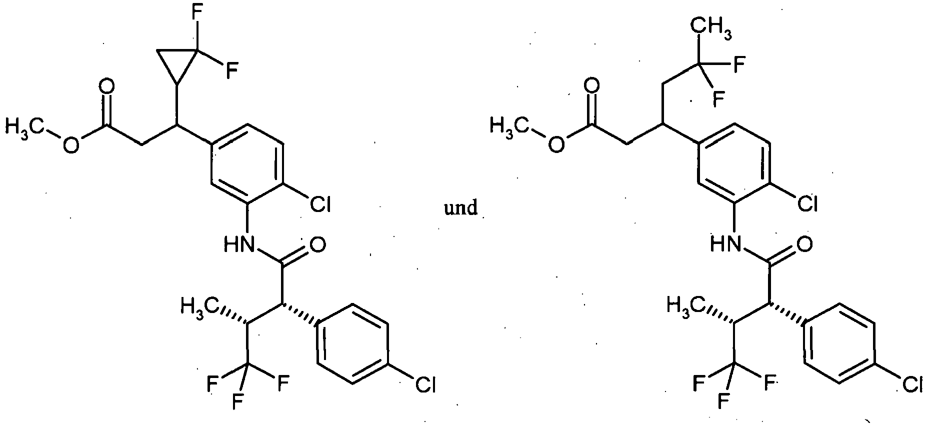

330 mg (1.24 mmol) (2*S*,3*R*)-2-(4-Chlorphenyl)-4,4,4-trifluor-3-methylbutansäure wurden in 10 ml Dichlormethan gelöst, mit 264 mg (1.98 mmol) 1-Chlor-*N*,*N*,2-trimethylprop-1-en-1-amin versetzt und 30 min bei Raumtemperatur gerührt. Anschließend wurde die Reaktionsmischung mit 300 µl (3.71 mmol) Pyridin und 360 mg des Gemisches bestehend aus Methyl-3-(3-amino-4-chlorphenyl)-3-(2,2-difluorcyclopropyl)propanoat und Methyl-3-(3-amino-4-chlorphenyl)-5,5-difluorhexanoat (Beispiel 59A), gelöst in 1 ml Dichlormethan, versetzt und 1 h weiter gerührt. Das Reaktionsgemisch wurde danach im Vakuum eingeengt und das erhaltene Rohprodukt direkt über Kieselgel chromatographisch gereinigt (Laufmittel Cyclohexan/Essigsäureethylester 20:1). Es wurden so 479 mg des Gemisches der beiden Zielverbindungen erhalten.

LC-MS (Methode 5): Rₜ = 1.33 min; m/z = 538/540/542 (M+H)⁺.

### Beispiele 122A - 125A

476 mg des Gemisches von Methyl-3-(4-chlor-3-{[(2*S*,3*R*)-2-(4-chlorphenyl)-4,4,4-trifluor-3-methylbutanoyl]amino}phenyl)-3-(2,2-difluorcyclopropyl)propanoat und Methyl-3-(4-chlor-3-{[(2*S*,3*R*)-2-(4-chlorphenyl)-4,4,4-trifluor-3-methylbutanoyl]amino}phenyl)-5,5-difluorhexanoat (Beispiel 121A) wurden mittels präparativer HPLC an chiraler Phase weiter aufgetrennt [Säule: Daicel Chiralpak AZ-H, 5 µm, 250 mm x 20 mm; Elutionsmittel: Isohexan/Isopropanol 95:5 (v/v); Fluss: 15 ml/min; UV-Detektion: 220 nm; Temperatur: 30°C]. Das zu Peak 2 und Peak 3 zunächst erhaltene Material wurde wieder vereinigt und dann durch erneute präparative HPLC an chiraler Phase aufgetrennt [Säule: Daicel Chiralpak AD-H, 5 µm, 250 mm x 20 mm; Elutionsmittel: Iso-hexan/Isopropanol 95:5 (v/v); Fluss: 15 ml/min; UV-Detektion: 220 nm; Temperatur: 30°C].

### Beispiel 122A

### Methyl-3-(4-chlor-3-{[(2S,3R)-2-(4-chlorphenyl)-4,4,4-trifluor-3-methylbutanoyl]amino}phenyl)-5,5-difluorhexanoat (Diastereomer 1)

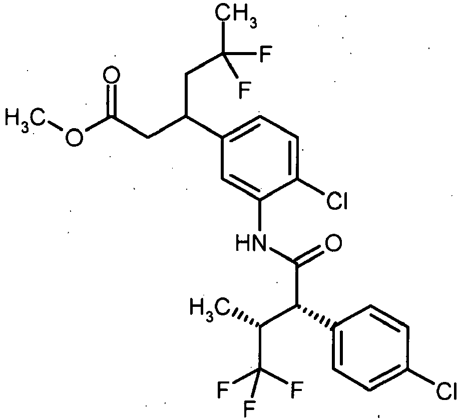

Ausbeute: 100 mg

Rₜ = 8.42 min; chemische Reinheit >99%, >99% de
[Säule: Daicel AZ-H, 5 µm, 250 mm x 4.6 mm; Elutionsmittel: Isohexan/(Isopropanol + 0.2% Trifluoressigsäure + 1% Wasser) 95:5 (v/v); Fluss: 1 ml/min; UV-Detektion: 220 nm; Temperatur: 30°C].

LC-MS (Methode 5): Rₜ = 1.33 min; m/z = 540/542 (M+H)⁺.

¹H-NMR (400 MHz, DMSO-*d*₆): δ [ppm] = 0.80 (d, 3H), 1.46 (t, 3H), 2.19-2.32 (m, 2H), 2.46-2.60 (m, 1H, teilweise verdeckt durch DMSO-Signal), 2.69-2.78 (m, 1H), 3.20-3.30 (m, 1H), 3.30-3.43 (m, 1H, verdeckt durch H₂O-Signal), 3.48 (s, 3H), 4.12 (d, 1H), 7.14 (dd, 1H), 7.37 (d, 1H), 7.42 (d, 1H), 7.43-7.50 (m, 4H), 9.84 (s, 1H).

### Beispiel 123A

### Methyl-3-(4-chlor-3-{[(2S,3R)-2-(4-chlorphenyl)-4,4,4-trifluor-3-methylbutanoyl]amino}phenyl)-5,5-difluorhexanoat (Diastereomer 2)

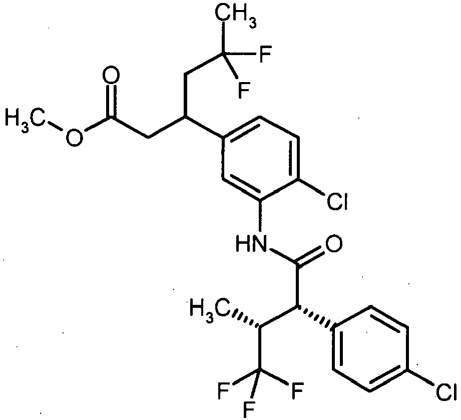

Ausbeute: 96 mg

Rₜ = 10.14 min; chemische Reinheit >94%, >99% de
[Säule: Daicel AZ-H, 5 µm, 250 mm x 4.6 mm; Elutionsmittel: Isohexan/(Isopropanol + 0.2% Trifluoressigsäure + 1% Wasser) 95:5 (v/v); Fluss: 1 ml/min; UV-Detektion: 220 nm; Temperatur: 30°C].

LC-MS (Methode 5): Rₜ = 1.33 min; m/z = 540/542 (M+H)⁺.

¹H-NMR (400 MHz, DMSO-*d*₆): δ [ppm] = 0.80 (d, 3H), 1.47 (t, 3H), 2.19-2.32 (m, 2H), 2.462.60 (m, 1H, teilweise verdeckt durch DMSO-Signal), 2.69-2.78 (m, 1H), 3.20-3.30 (m, 1H), 3.30-3.43 (m, 1H, verdeckt durch H₂O-Signal), 3.46 (s, 3H), 4.12 (d, 1H), 7.14 (dd, 1H), 7.37 (d, 1H), 7.41 (d, 1H), 7.43-7.50 (m, 4H), 9.84 (s, 1H).

### Beispiel 124A

### Methyl-3-(4-chlor-3-{[(2S,3R)-2-(4-chlorphenyl)-4,4,4-trifluor-3-methylbutanoyl]amino}phenyl)-3-(2,2-difluorcyclopropyl)propanoat (Isomer 1)

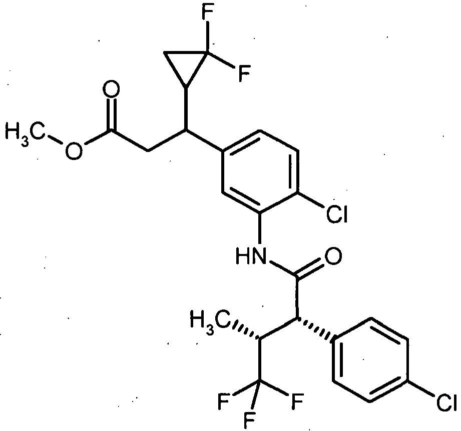

Ausbeute: 124 mg

Rₜ = 9.00 min; chemische Reinheit >96%
[Säule: Daicel AZ-H, 5 µm, 250 mm x 4.6 mm; Elutionsmittel: Isohexan/(Isopropanol + 0.2% Trifluoressigsäure + 1% Wasser) 95:5 (v/v); Fluss: 1 ml/min; UV-Detektion: 220 nm; Temperatur: 30°C].

LC-MS (Methode 5): Rₜ = 1.34 min; m/z = 538/540 (M+H)⁺.

¹H-NMR (400 MHz, DMSO-*d*₆): δ [ppm] = 0.80 (d, 3H), 1.06-1.18 (m, 1H), 1.38-1.51 (m, 1H), 2.01-2.16 (m, 1H), 2.64-2.82 (m, 3H), 3.28-3.54 (m, 1H, teilweise verdeckt durch H₂O-Signal), 3.50 (s, 3H), 4.12 (d, 1H), 7.22 (dd, 1H), 7.41 (d, 1H), 7.43-7.50 (m, 5H), 9.88 (s, 1H).

### Beispiel 125A

### Methyl-3-(4-chlor-3-{[(2S,3R)-2-(4-chlorphenyl)-4,4,4-trifluor-3-methylbutanoyl]amino}phenyl)-3-(2,2-difluorcyclopropyl)propanoat (Isomer 2)

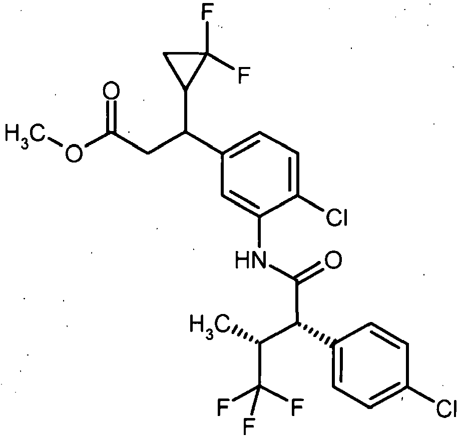

Ausbeute: 118 mg

Rₜ = 9.47 min; chemische Reinheit >99%
[Säule: Daicel AZ-H, 5 µm, 250 mm x 4.6 mm; Elutionsmittel: Isohexan/(Isopropanol + 0.2% Trifluoressigsäure + 1% Wasser) 95:5 (v/v); Fluss: 1 ml/min; UV-Detektion: 220 nm; Temperatur: 30°C].

LC-MS (Methode 5): Rₜ = 1.33 min; m/z = 538/540 (M+H)⁺.

¹H-NMR (400 MHz, DMSO-*d*₆): δ [ppm] = 0.80 (d, 3H), 1.06-1.18 (m, 1H), 1.38-1.52 (m, 1H), 2.01-2.15 (m, 1H), 2.63-2.83 (m, 3H), 3.28-3.58 (m, 1H, teilweise verdeckt durch H₂O-Signal), 3.49 (s, 3H), 4.12 (d, 1H), 7.21 (dd, 1H), 7.40 (d, 1H), 7.42-7.50 (m, 5H), 9.87 (s, 1H).

### Beispiel 126A

### tert. -Butyl-3-(4-chlor-3-{[(2S,3R)-2-(4-chlorphenyl)-4,4,4-trifluor-3-methylbutanoyl]amino}-phenyl)-3-(3,3-difluorcyclobutyl)propanoat (Diastereomerengemisch)

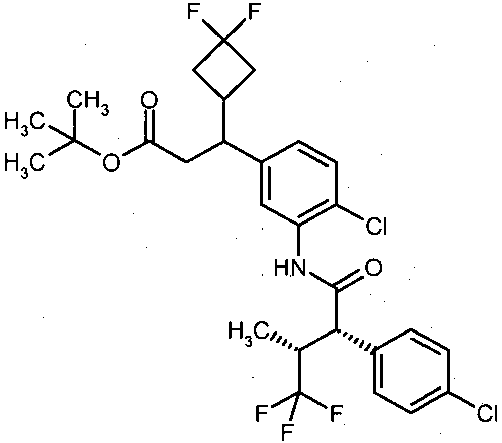

Eine Lösung von 76 mg (0.29 mmol) (2*S*,3*R*)-2-(4-Chlorphenyl)-4,4,4-trifluor-3-methylbutansäure, 45 mg (0.13 mmol) *tert*.-Butyl-3-(3-amino-4-chlorphenyl)-3-(3,3-difluorcyclobutyl)propanoat, 119 mg (0.31 mmol) *O*-(1*H*-7-Azabenzotriazol-1-yl)-*N*,*N*,*N'*,*N'*-tetramethyluronium-hexafluorophosphat (HATU) und 0.51 ml Pyridin wurden in 1 ml DMF über Nacht bei Raumtemperatur gerührt. Nach Beendigung der Reaktion wurde der Ansatz direkt ohne weitere Aufarbeitung mittels präparativer HPLC in seine Komponenten aufgetrennt. Es wurden 19 mg (25% d. Th.) der Titelverbindung als ein farbloses Öl erhalten.

LC-MS (Methode 5): Rₜ = 1.47 min; m/z = 592/594 (M-H)⁻.

Auf analoge Weise wurden die in der folgenden Tabelle aufgeführten Verbindungen hergestellt:

| **Beispiel** | **Name / Struktur / Edukte** | **Analytische Daten** |
|---|---|---|
| **127A** | *tert.* -Butyl-3-(4-chlor-3-{[(2*S*,3*R*)-2-(4-chlorphenyl)-4,4,4-trifluor-3-methylbutanoyl]amino}phenyl)-3-cyclobutylpropanoat (*Diastereomerengemisch*) | LC-MS (Methode 5): |
| | | Rₜ = 1.57 min; m/z = 556 (M-H)⁻. |
| | 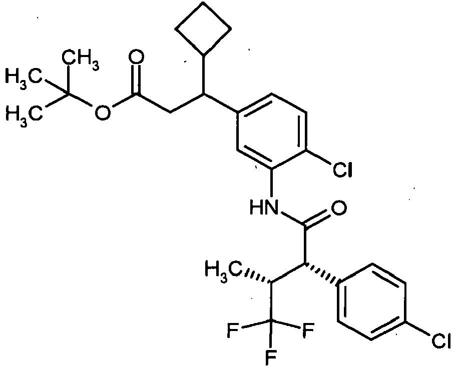 | ¹H-NMR (400 MHz, DMSO-*d₆*): beide Diastereomere: δ [ppm] = 0.80 (d, 3H), 1.15/1.18 (2s, zus. 9H), 1.46-1.62 (m, 2H), 1.62-1.76 (m, 3H), 1.96-2.06 (m, 1H), 2.16-2.28 (m, 1H), 2.32-2.48 (m, 2H), 2.76-2.87 (m, 1H), 3.35-3.45 (m, 1H), 4.13/4.14 (2d, zus. 1H), 7.00 (dt, 1H), 7.34 (d, 1H), 7.36-7.53 (m, 5H), 9.79/9.80 (2s, zus. 1H). |
| | aus (+/-)-*tert*.-Butyl-3-(3-amino-4-chlorphenyl)-3-cyclobutylpropanoat und (+)-(2*S*,3*R*)-2-(4-Chlorphenyl)-4,4,4-trifluor-3-methylbutansäure | |
| **128A** | *tert*.-Butyl-3-(4-chlor-3-{[(2*S*,3*R*)-2-(4-chlorphenyl)-4,4,4-trifluor-3-methylbutanoyl]amino}phenyl)-4-cyclopropylbutanoat (*Diastereomerengemisch*) | LC-MS (Methode 5): |
| | | Rₜ = 1.62 min; m/z = 556/558 (M-H)⁻. |
| | 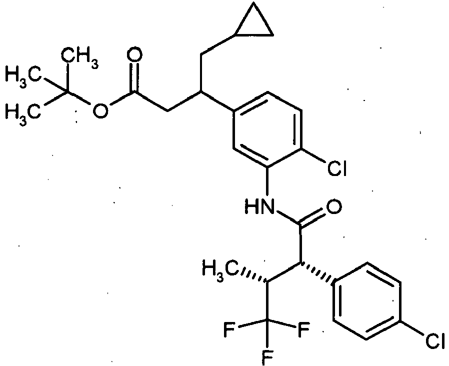 | |
| | aus *tert*.-Butyl-3-(3-amino-4-chlorphenyl)-4-cyclopropylbutanoat und (+)-(2*S*,3*R*)-2-(4-Chlorphenyl)-4,4,4-trifluor-3-methylbutansäure | |
| **129A** | Ethyl-3-(4-chlor-3-{[(2*S*,3*R*)-2-(4-chlorphenyl)-4,4,4-trifluor-3-methylbutanoyl]amino}phenyl)-3-cyclopropyl-2-methylpropanoat (*Diastereomerengemisch*) | LC-MS (Methode 7): |
| | | Rₜ = 1.49 min; m/z = 530/532 (M+H)⁺. |
| | 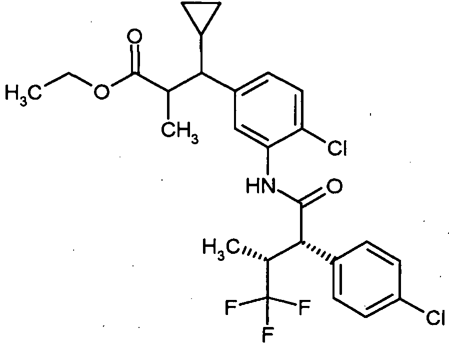 | |
| | aus Ethyl-3-(3-amino-4-chlorphenyl)-3-cyclopropyl-2-methylpropanoat (*Diastereomerengemisch*) und (+)-(2*S*,3*R*)-2-(4-Chlorphenyl)-4,4,4-trifluor-3-methylbutansäure | |
| **130A** | Methyl-3-(4-chlor-3-{[(2*S*,3*R*)-2-(4-chlorphenyl)-4,4,4-trifluor-3-methylbutanoyl]amino}phenyl)-3-cyclopropylbutanoat (*Diastereomerengemisch*) | LC-MS (Methode 5): |
| | | Rₜ = 1.41 min; m/z = 516/518 (M+H)⁺. |
| | 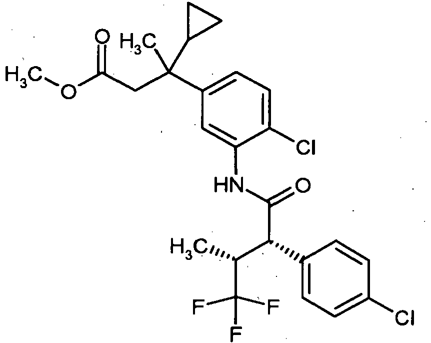 | |
| | aus Methyl-3-(3-amino-4-chlorphenyl)-3-cyclo-propylbutanoat (*Racemat*) und (+)-(2*S*,3*R*)-2-(4-Chlorphenyl)-4,4,4-trifluor-3-methylbutansäure | |

### Beispiel 131A

### 2-(1-Methylcyclopropyl)ethanol

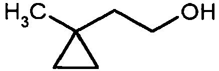

11.23 g (87.1 mmol) Zink-Kupfer-Paar wurden in 50 ml Diethylether aufgenommen und bei Raumtemperatur mit 6.76 ml (92.9 mmol) Chloriodmethan versetzt. Anschließend wurden 5.84 ml (58.1 mmol) 3-Methylbut-3-en-1-ol, gelöst in 10 ml Diethylether, hinzugetropft. Nach Beendigung der Zugabe wurde das Reaktionsgemisch auf 40°C erhitzt und über Nacht bei dieser Temperatur gerührt. Nach dem Abkühlen wurde der Ansatz über Kieselgur abgesaugt und das Kieselgur mehrmals mit Diethylether nachgewaschen. Die vereinigten Filtrate wurden mit gesättigter wässriger Natriumhydrogencarbonat-Lösung und mit Wasser gewaschen, über Magnesiumsulfat getrocknet und anschließend im Vakuum bis zur Trockene eingeengt. Der erhaltene Rückstand wurde durch Chromatographie an Silicagel gereinigt (Laufmittel Cyclohexan/Ethylacetat 10:1). Es wurden so 3.58 g (62% d. Th.) der Titelverbindung erhalten.

GC-MS (Methode 1): Rₜ = 1.23 min; m/z = 100 (M)⁺.

¹H-NMR (400 MHz, DMSO-*d*₆): δ [ppm] = 0.24-0.29 (m, 2H), 0.29-0.34 (m, 2H), 1.05 (s, 3H), 1.37 (t, 1H), 1.53 (t, 2H), 3.74-3.80 (m, 2H).

Analog zu Synthesebeispiel 1A wurde die folgende Verbindung erhalten:

| **Beispiel** | **Name / Struktur / Edukte** | **Analytische Daten** |
|---|---|---|
| **132A** | *tert*.-Butyl-(2*E*)-4-(1-methylcyclopropyl)-but-2-enoat | GC-MS (Methode 6): |
| | | Rₜ = 3.86 min; m/z = 214 (M+NH₄)⁺. |
| | 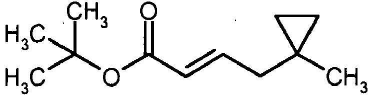 | ¹H-NMR (400 MHz, DMSO-*d*₆): δ [ppm] = 0.25-0.31 (m, 2H), 0.31-0.37 (m, 2H), 0.98 (s, 3H), 1.43 (s, 9H), 2.06-2.11 (m, 2H), 5.76-5.83 (m, 1H), 6.72-6.82 (m, 1H). |
| | aus *tert*.-Butyl(triphenyl-λ⁵-phosphanyliden)acetat und 2-(1-Methylcyclopropyl)ethanol | |

Analog zu Synthesebeispiel 4A / 5A wurde die folgende Verbindung erhalten:

| **Beispiel** | **Name / Struktur / Edukte** | **Analytische Daten** |
|---|---|---|
| **133A** | *tert*.-Butyl-(2*E*/*Z*)*-*3-(3-amino-4-chlorphenyl)-4-(1-methylcyclopropyl)but-2-enoat | LC-MS (Methode 5): |
| | | Rₜ = 1.42 min; m/z = 322 (M+H)⁺. |
| | 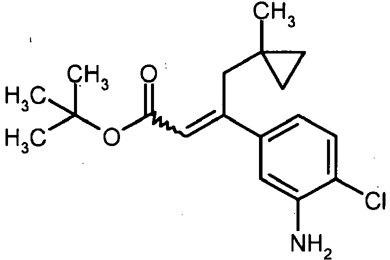 | ¹H-NMR (400 MHz, DMSO-*d*₆): δ [ppm] = 0.04-0.10 (m, 2H), 0.17-0.24 (m, 2H), 0.85 (s, 3H), 1.46 (s, 9H), 3.02 (s, 2H), 5.40 (br. s, 2H), 5.82 (s, 1H), 6.62 (dd, 1H), 6.88 (d, 1H), 7.17 (d, 1H). |
| | aus *tert*.-Butyl-(2*E*)-4-(1-methylcyclopropyl)-but-2-enoat und 5-Brom-2-chloranilin | |

### Beispiel 134A

### tert.-Butyl-3-(3-amino-4-chlorphenyl)-4-(1-methylcyclopropyl)butanoat

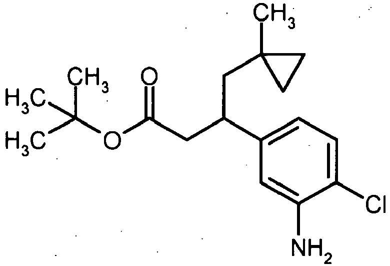

187 mg (0.58 mmol) *tert*.-Butyl-(2*E*/*Z*)-3-(3-amino-4-chlorphenyl)-4-(1-methylcyclopropyl)but-2-enoat wurden in 10 ml Ethylacetat gelöst und mit 11 mg (0.06 mmol) Platin(IV)oxid versetzt. Die Reaktionsmischung wurde bei RT über Nacht unter einer Wasserstoffatmosphäre bei Normaldruck gerührt. Nach erneuter Zugabe von 11 mg (0.06 mmol) Platin(IV)oxid wurde nochmals über Nacht bei RT unter Wasserstoffatmosphäre bei Normaldruck gerührt. Danach wurde das Reaktionsgemisch über Kieselgur abgesaugt und das Filtrat eingeengt. Es wurden 36 mg (19% d. Th.) der Zielverbindung erhalten.

LC-MS (Methode 5): Rₜ = 1.37 min; m/z = 324 (M+H)⁺.

¹H-NMR (400 MHz, DMSO-*d*₆): δ [ppm] = -0.10--0.03 (m, 1H), -0.03-0.04 (m, 1H), 0.13-0.25 (m, 2H), 0.95 (s, 3H), 1.27 (s, 9H), 1.40-1.52 (m, 2H), 2.24-2.33 (m, 1H), 2.47-2.58 (m, 1H, teilweise verdeckt durch DMSO-Signal), 2.95-3.05 (m, 1H), 5.19 (br. s, 2H), 6.41 (dd, 1H), 6.65 (d, 1H), 7.05 (d, 1H).

Analog zu Synthesebeispiel 99A wurde die folgende Verbindung hergestellt:

| **Beispiel** | **Name / Struktur / Edukte** | **Analytische Daten** |
|---|---|---|
| **135A** | *tert*.-Butyl-3-(4-chlor-3-{[(2*S*,3*R*)-2-(4-chlorphenyl)-4,4,4-trifluor-3-methylbutanoyl]amino}phenyl)-4-(1-methylcyclopropyl)butanoat (*Diastereomerengemisch*) | LC-MS (Methode 8): |
| | | Rₜ = 3.27 min; m/z = 570/571 (M-H)⁻. |
| | 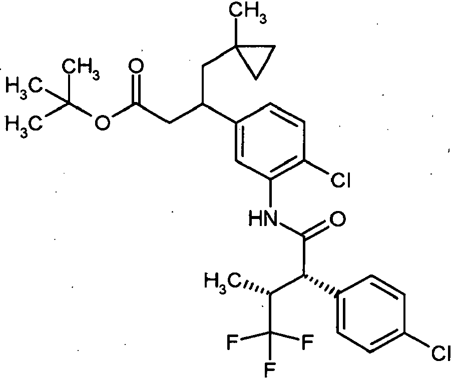 | ¹H-NMR (400 MHz, DMSO-*d*₆): δ [ppm] =- 0.14--0.07 (m, 1H), -0.07-0.02 (m, 1H), 0.12-0.19 (m, 1H), 0.19-0.25 (m, 1H), 0.80 (d, 3H), 0.93 (d, 3H), 1.19 (2s, 9H), 1.39-1.55 (m, 2H), 2.26-2.38 (m, 1H), 2.48-2.63 (m, 1H, teilweise verdeckt durch DMSO-Signal), 3.05-3.16 (m, 1H), 3.29-3.44 (m, 1H, teilweise verdeckt durch H₂O-Signal), 4.14 (dd, 1H), 7.06 (d, 1H), 7.34 (d, 1H), 7.39-7.51 (m, 5H), 9.80 (d, 1H). |
| | aus *tert*.-Butyl-3-(3-amino-4-chlorphenyl)-4-(1-methylcyclopropyl)butanoat und *(*2*S,*3*R)-*2-(4-Chlorphenyl)-4,4,4-trifluor-3-methylbutansäure | |

### Ausführungsbeispiele:

### Beispiel 1

### (+)-3-(4-Chlor-3-{[(2S,3R)-2-(4-chlorphenyl)-4,4,4-trifluor-3-methylbutanoyl]amino}phenyl)-4-methylpentansäure (Diastereomer 1)

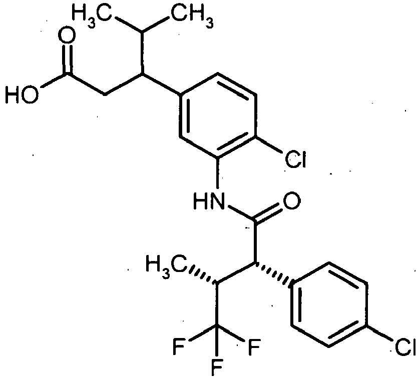

225 mg (0.45 mmol) Methyl-3-(4-chlor-3-{[(2*S*,3*R*)-2-(4-chlorphenyl)-4,4,4-trifluor-3-methyl-butanoyl]amino}phenyl)-4-methylpentanoat (Diastereomer 1; Beispiel 96A) wurden mit 4 ml konzentrierter Essigsäure und 2 ml konzentrierter Salzsäure versetzt. Die Reaktionsmischung wurde 2 h bei 100°C gerührt. Nach dem Abkühlen wurde das Reaktionsgemisch auf Eiswasser gegeben, und die entstandenen Kristalle wurden abgesaugt. Nach zweimaligem Waschen mit Wasser wurden die Kristalle im Hochvakuum-Trockenschrank bei 40°C über Nacht getrocknet. Es wurden so 193 mg (88% d. Th.) der Titelverbindung als weißer Feststoff erhalten.

LC-MS (Methode 7): Rₜ = 1.30 min; m/z = 490/492 (M+H)⁺.

¹H-NMR (400 MHz, DMSO-*d*₆): δ [ppm] = 0.68 (d, 3H), 0.80 (d, 3H), 0.84 (d, 3H), 1.70-1.80 (m, 1H), 2.36-2.48 (m, 1H), 2.61-2.70 (m, 1H), 2.70-2.80 (m, 1H), 3.29-3.43 (m, 1H, teilweise verdeckt durch H₂O-Signal), 4.13 (d, 1H), 7.00 (dd, 1H), 7.31-7.37 (m, 2H), 7.43-7.50 (m, 4H), 9.82 (s, 1H), 11.95 (br. s, 1H).

[α]_{D}²⁰ = +111°, c = 0.285, Methanol.

### Beispiel 2

### (+)-3-(4-Chlor-3-{[(2S,3R)-2-(4-chlorphenyl)-4,4,4-trifluor-3-methylbutanoyl]amino}phenyl)-4-methylpentansäure (Diastereomer 2)

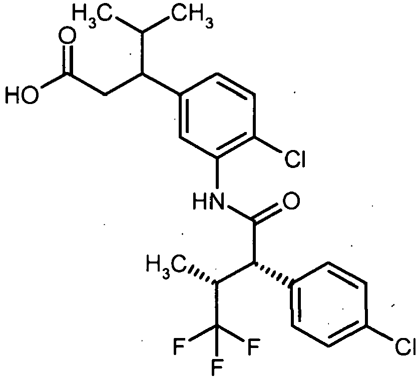

218 mg (0.43 mmol) Methyl-3-(4-chlor-3-{[(2*S*,3*R*)-2-(4-chlorphenyl)-4,4,4-trifluor-3-methyl-butanoyl]amino}phenyl)-4-methylpentanoat (Diastereomer 2; Beispiel 97A) wurden mit 4 ml konzentrierter Essigsäure und 2 ml konzentrierter Salzsäure versetzt. Die Reaktionsmischung wurde 2 h bei 100°C gerührt. Nach dem Abkühlen wurde das Reaktionsgemisch auf Eiswasser gegeben, und die entstandenen Kristalle wurden abgesaugt. Nach zweimaligem Waschen mit Wasser wurden die Kristalle im Hochvakuum-Trockenschrank bei 40°C über Nacht getrocknet. Es wurden so 188 mg (89% d. Th.) der Titelverbindung als weißer Feststoff erhalten.

LC-MS (Methode 7): Rₜ = 1.30 min; m/z = 490/492 (M+H)⁺.

¹H-NMR (400 MHz, DMSO-*d₆*): δ [ppm] = 0.67 (d, 3H), 0.80 (d, 3H), 0.84 (d, 3H), 1.69-1.80 (m, 1H), 2.39-2.48 (m, 1H), 2.62-2.70 (m, 1H), 2.71-2.79 (m, 1H), 3.29-3.44 (m, 1H, teilweise verdeckt durch H₂O-Signal), 4.13 (d, 1H), 7.00 (dd, 1H), 7.32-7.38 (m, 2H), 7.41-7.51 (m, 4H), 9.82 (s, 1H), 11.96 (br. s, 1H).

[α]_{D}²⁰ = +82°, c = 0.275, Methanol.

Auf analoge Weise wurden die in der folgenden Tabelle aufgeführten Verbindungen hergestellt:

| **Beispiel** | **Name / Struktur / Edukt** | **Analytische Daten** |
|---|---|---|
| **3** | 3-(4-Chlor-3-{[(2*S*;3*R*)-2-(4-chlorphenyl)-4,4,4-trifluor-3-methylbutanoyl]amino}phenyl)-3,4-dimethylpentansäure (*Diastereomer 1*) | LC-MS (Methode 5): |
| | | Rₜ = 1.30 min; m/z = 504/506 (M+H)⁺. |
| | 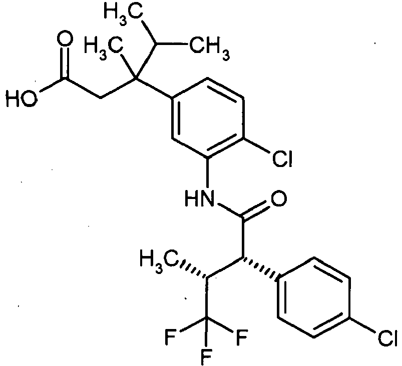 | ¹H-NMR (400 MHz, DMSO-*d₆*): δ [ppm] = 0.55 (d, 3H), 0.80 (d, 6H), 1.30 (s, 3H), 1.75-1.88 (m, 1H), 2.46-2.58 (d, 1H, verdeckt durch DMSO-Signal), 2.69 (d, 1H), 3.28-3.45 (m, 1H, teilweise verdeckt durch H₂O-Signal), 4.13 (d, 1H), 7.12 (dd, 1H), 7.33 (d, 1H), 7.43-7.51 (m, 5H), 9.81 (s, 1H), 11.75 (br. s, 1H). |
| | aus Methyl-3-(4-chlor-3-{[(2*S*,3*R*)-2-(4-chlorphenyl)-4,4,4-trifluor-3-methylbutanoyl]amino}-phenyl)-3,4-dimethylpentanoat (*Diastereomer 1*) | |
| | | [α]_{D}²⁰ = +95°, c = 0.285, Methanol. |
| **4** | 3-(4-Chlor-3-{[(2*S*,3*R*)-2-(4-chlorphenyl)-4,4,4-trifluor-3-methylbutanoyl]amino}phenyl)-3,4-dimethylpentansäure (*Diastereomer 2*) | LC-MS (Methode 5): |
| | | Rₜ = 1.30 min; m/z = 504/506 (M+H)⁺. |
| | 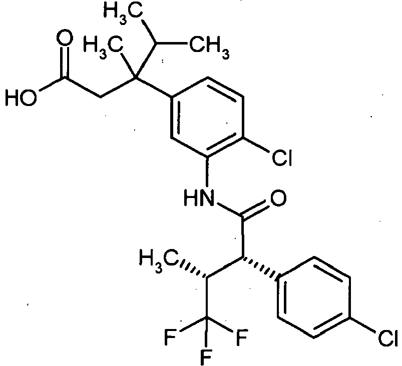 | ¹H-NMR (400 MHz, DMSO-*d₆*): δ [ppm] = 0.56 (d, 3H), 0.80 (d, 6H), 1.30 (s, 3H), 1.75-1.89 (m, 1H), 2.46-2.57 (d, 1H, verdeckt durch DMSO-Signal), 2.69 (d, 1H), 3.29-3.45 (m, 1H, teilweise verdeckt durch H₂O-Signal), 4.13 (d, 1H), 7.12 (dd, 1H), 7.32 (d, 1H), 7.42-7.48 (m, 4H), 7.49 (d, 1H), 9.81 (s, 1H), 11.75 (br. s, 1H). |
| | aus Methyl-3-(4-chlor-3-{[(2*S*,3*R*)-2-(4-chlorphenyl)-4,4,4-trifluor-3-methylbutanoyl]amino}-phenyl)-3,4-dimethylpentanoat (*Diastereomer 2*) | |
| | | [α]_{D}²⁰ = +105.7°, c = 0.305, Methanol. |
| **5** | 3-(4-Chlor-3-{[(2*S*,3*R*)-2-(4-chlorphenyl)-4,4,4-trifluor-3-methylbutanoyl]amino}phenyl)-3-cyclobutylbutansäure (*Diastereomer 1*) | LC-MS (Methode 5): |
| | | Rₜ = 1.32 min; m/z = 516/518 (M+H)⁺. |
| | 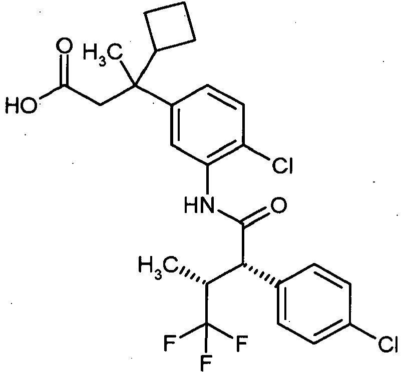 | ¹H-NMR (400 MHz, DMSO-*d₆*): δ [ppm] = 0.80 (d, 3H), 1.31 (s, 3H), 1.41-1.52 (m, 1H), 1.52-1.66 (m, 3H), 1.66-1.78 (m, 2H), 2.37 (d, 1H), 2.45-2.58 (m, 1H, verdeckt durch DMSO-Signal), 2.64 (d, 1H), 3.28-3.47 (m, 1H, teilweise verdeckt durch H₂O-Signal), 4.13 (d, 1H), 7.10 (dd, 1H), 7.33 (d, 1H), 7.40-7.52 (m, 5H), 9.81 (s, 1H), 11.83 (br. s, 1H). |
| | aus Methyl-3-(4-chlor-3-{[(2*S*,3*R*)-2-(4-chlorphenyl)-4,4,4-trifluor-3-methylbutanoyl]amino}-phenyl)-3-cyclobutylbutanoat (*Diastereomer 1*) | |
| | | [α]_{D}²⁰ = +105°, c = 0.250, Methanol. |
| **6** | 3-(4-Chlor-3-{[(2*S*,3*R*)-2-(4-chlorphenyl)-4,4,4-trifluor-3-methylbutanoyl]amino}phenyl)-3-cyclobutylbutansäure (*Diastereomer 2*) | LC-MS (Methode 5): |
| | | Rₜ = 1.32 min; m/z = 516/518 (M+H)⁺. |
| | 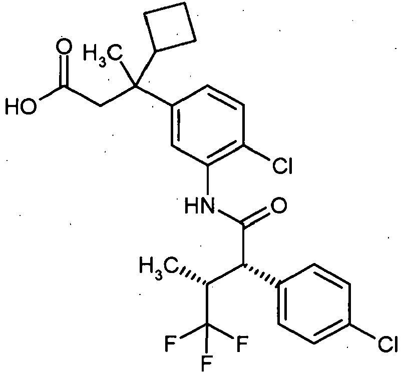 | ¹H-NMR (400 MHz, DMSO-*d₆*): δ [ppm] = 0.80 (d, 3H), 1.31 (s, 3H), 1.42-1.52 (m, 1H), 1.52-1.67 (m, 3H), 1.67-1.79 (m, 2H), 2.37 (d, 1H), 2.45-2.58 (m, 1H, verdeckt durch DMSO-Signal), 2.64 (d, 1H), 3.30-3.47 (m, 1H, teilweise verdeckt durch H₂O-Signal), 4.13 (d, 1H), 7.10 (dd, 1H), 7.33 (d, 1H), 7.41-7.52 (m, 5H), 9.81 (s, 1H), 11.84 (br. s, 1H). |
| | aus Methyl-3-(4-chlor-3-{[(2*S*,3*R*)-2-(4-chlorphenyl)-4,4,4-trifluor-3-methylbutanoyl]amino}-phenyl)-3-cyclobutylbutanoat (*Diastereomer 2*) | |
| | | [α]_{D}²⁰ = +100°, c = 0.30, Methanol. |
| **7** | 3-(4-Chlor-3-{[(2S,3R)-2-(4-chlorphenyl)-4,4,4-trifluor-3-methylbutanoyl]amino}phenyl)-3-cyclopropylbutansäure (*Diastereomerengemisch*) | LC-MS (Methode 4): |
| | | Rₜ = 1.54 min; m/z = 500/502 (M-H)⁻. |
| | 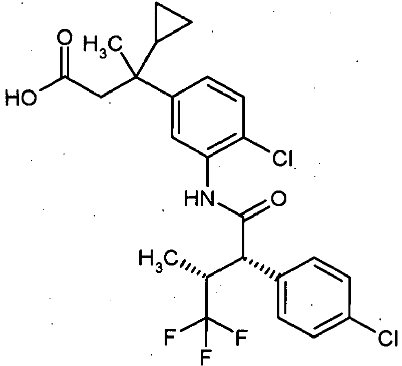 | ¹H-NMR (400 MHz, DMSO-*d₆*): δ [ppm] = 0.77-0.85 (m, 5H), 0.87-1.02 (m, 1H), 1.15-1.28 (m, 2H), 1.42 (s, 3H), 2.62-2.72 (m, 1H), 3.01 (d, 1H), 3.28-3.43 (m, 1H, teilweise verdeckt durch H₂O-Signal), 4.09-4.17 (m, 1H), 7.08 (dd, 1H), 7.38-7.53 (m, 6H), 9.92 (d, 1H). |
| | aus Methyl-3-(4-chlor-3-{[(2*S*,3*R*)-2-(4-chlorphenyl)-4,4,4-trifluor-3-methylbutanoyl]amino}-phenyl)-3-cyclopropylbutanoat (*Diastereomerengemisch*) | |
| **8** | 3-(4-Chlor-3-{[(2*S*,3*R*)-2-(4-ethylphenyl)-4,4,4-trifluor-3-methylbutanoyl]amino}phenyl)-4-methylpentansäure (*Diastereomer 1*) | LC-MS (Methode 5): |
| | | Rₜ = 1.32 min; m/z = 484 (M+H)⁺. |
| | 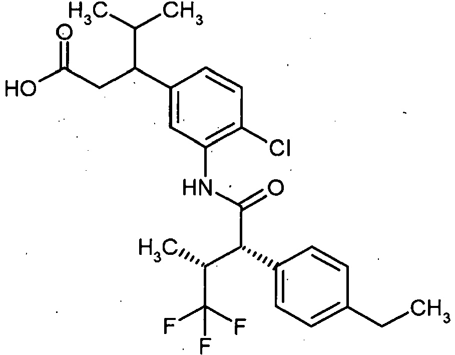 | ¹H-NMR (400 MHz, DMSO-*d*₆): δ [ppm] = 0.68 (d, 3H), 0.79 (d, 3H), 0.84 (d, 3H), 1.17 (t, 3H), 1.68-1.81 (m, 1H), 2.36-2.47 (m, 1H), 2.56-2.69 (m, 3H), 2.70-2.79 (m, 1H), 3.27-3.40 (m, 1H, teilweise verdeckt durch H₂O-Signal), 4.07 (d, 1H), 6.98 (dd, 1H), 7.20 (d, 2H), 7.30-7.41 (m, 4H), 9.73 (s, 1H), 11.95 (br. s, 1H). |
| | aus Methyl-3-(4-chlor-3-{[(2*S*,3*R*)-2-(4-ethyl-phenyl)-4,4,4-trifluor-3-methylbutanoyl]amino}-phenyl)-4-methylpentanoat (*Diastereomer 1*) | |
| **9** | 3-(4-Chlor-3-{[(2*S*,3*R*)-2-(4-chlor-3-fluorphenyl)-4,4,4-trifluor-3-methylbutanoyl]amino}phenyl)-4-methylpentansäure (*Diastereomer 1*) | LC-MS (Methode 5): |
| | | Rₜ = 1.28 min; m/z = 508/510 (M+H)⁺. |
| | 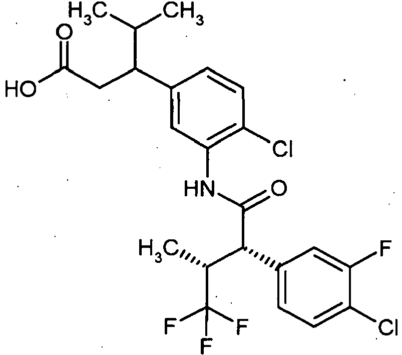 | ¹H-NMR (400 MHz, DMSO-*d₆*): δ [ppm] = 0.68 (d, 2.79H, Hauptdiastereomer), 0.84 (t, 6H), 1.25 (d, 0.21H, Nebendiastereomer), 1.69-1.81 (m, 1H), 2.39-2.48 (m, 1H), 2.61-2.70 (m, 1H), 2.70-2.81 (m, 1H), 3.37-3.48 (m, 1H), 4.15 (d, 1H), 7.01 (dd, 1H), 7.29-7.38 (m, 3H), 7.50 (dd, 1H), 7.62 (t, 1H), 9.87 (s, 0.93H, Hauptdiastereomer), 10.01 (s, 0.07H, Nebendiastereomer), 11.96 (br. s, 1H) (86% de). |
| | aus Methyl-3-(4-chlor-3-{[(2*S*,3*R*)-2-(4-chlor-3-fluorphenyl)-4,4,4-trifluor-3-methylbutanoyl]-amino}phenyl)-4-methylpentanoat (*Diastereomer 1*) | |
| **10** | 3-(4-Chlor-3-{[(2*S*,3*R*)-4,4,4-trifluor-2-(4-isopropylphenyl)-3-methylbutanoyl]amino}phenyl)-4-methylpentansäure *(Diastereomer 1)* | LC-MS (Methode 5): |
| | | Rₜ = 1.35 min; m/z = 498/500 (M+H)⁺. |
| | 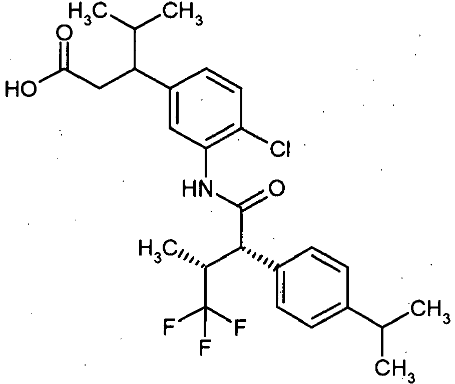 | ¹H-NMR (400 MHz, DMSO-d₆): δ [ppm] = 0.68 (d, 3H), 0.79 (d, 3H), 0.84 (d, 3H), 1.19 (d, 6H), 1.67-1.80 (m, 1H), 2.36-2.47 (m, 1H), 2.60-2.70 (m, 1H), 2.70-2.79 (m, 1H), 2.81-2.93 (m, 1H), 3.26-3.40 (m, 1H, verdeckt durch H₂O-Signal), 4.07 (d, 1H), 6.98 (dd, 1H), 7.20-7.28 (m, 2H), 7.30-7.43 (m, 4H), 9.73 (s, 1H), 11.95 (br. s, 1H). |
| | aus Methyl-3-(4-chlor-3-{[(2*S*,3*R*)-4,4,4-trifluor-2-(4-isopropylphenyl)-3-methylbutanoyl]amino}-phenyl)-4-methylpentanoat (*Diastereomer 1*) | |
| **11** | 3-(4-Chlor-3-{[(2*S*,3*R*)-4,4,4-trifluor-2-(4-fluorphenyl)-3-methylbutanoyl]amino}phenyl)-4-methylpentansäure (*Diastereomer 1*) | LC-MS (Methode 5): |
| | | Rₜ = 1.19 min; m/z = 474/476 (M+H)⁺. |
| | 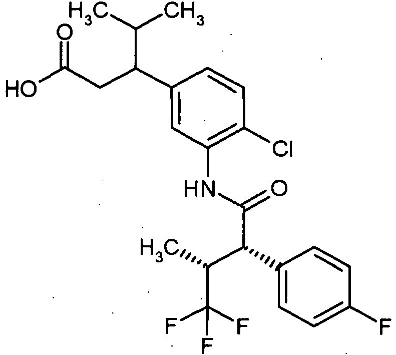 | ¹H-NMR (400 MHz, DMSO-*d₆*): δ [ppm] = 0.68 (d, 2.76H, Hauptdiastereomer), 0.80 (d, 3H); 0.84 (d, 3H), 1.25 (d, 0.24H, Nebendiastereomer), 1.68-1.80 (m, 1H), 2.36-2.47 (m, 1H), 2.60-2.70 (m, 1H), 2.70-2.80 (m, 1H), 3.29-3.44 (m, 1H, teilweise verdeckt durch H₂O-Signal), 4.12 (d, 1H), 7.00 (dd, 1H), 7.22 (t, 2H), 7.31-7.37 (m, 2H), 7.45-7.52 (m, 2H), 9.80 (s, 0.92H, Hauptdiastereomer), 9.94 (s, 0.08H, Nebendiastereomer), 11.96 (br. s, 1H) (84% de). |
| | aus Methyl-3-(4-chlor-3-{[(2*S*,3*R*)-4,4,4-trifluor-2-(4-fluorphenyl)-3-methylbutanoyl]amino}phenyl)-4-methylpentanoat (*Diastereomer 1*) | |
| **12** | 3-[4-Chlor-3-({4,4,4-trifluor-3-methyl-2-[4-(2,2,2-trifluorethyl)phenyl]butanoyl}amino)phenyl]-4-methylpentansäure (*Diastereomerengemisch*) | LC-MS (Methode 5): |
| | | Rₜ = 1.26 min; m/z = 538/540 (M+H)⁺. |
| | 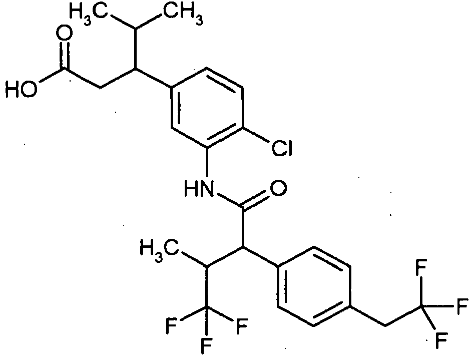 | ¹H-NMR (400 MHz, DMSO-*d₆*): δ [ppm] = 0.64-0.71 (m, 3H), 0.79 (d, 3H), 0.84 (d, 3H), 1.68-1.81 (m, 1H), 2.38-2.47 (m, 1H), 2.61-2.69 (m, 1H), 2.70-2.80 (m, 1H), 3.28-3.44 (m, 1H, teilweise verdeckt durch H₂O-Signal), 3.64 (q, 2H), 4.11 (d, 1H), 6.99 (d, 1H), 7.30-7.39 (m, 4H), 7.46 (d, 2H), 9.80 (s, 1H), 11.95 (br. s, 1H). |
| | aus Methyl-3-[4-chlor-3-({4,4,4-trifluor-3-methyl-2-[4-(2,2,2-trifluorethyl)phenyl]butanoyl}amino)-phenyl]-4-methylpentanoat (*Diastereomerengemisch*) | |
| **13** | 3-[4-Chlor-3-({(2*S*,3*R*)-2-[4-(2,2-difluorcyclo-propyl)phenyl]-4,4,4-trifluor-3-methylbutanoyl}-amino)phenyl]-4-methylpentansäure (*Diastereomerengemisch*) | LC-MS (Methode 5): |
| | | Rₜ = 1.26 min; m/z = 532/534 (M+H)⁺. |
| | 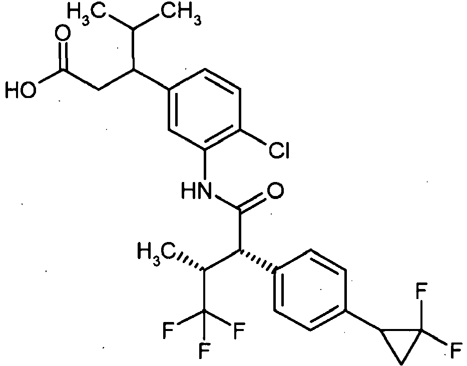 | ¹H-NMR (400 MHz, DMSO-*d₆*): δ [ppm] = 0.68 (d, 3H), 0.79 (d, 3H), 0.84 (d, 3H), 1.68-1.80 (m, 1H), 1.87-2.04 (m, 2H), 2.36-2.47 (m, 1H), 2.61-2.69 (m, 1H), 2.70-2.79 (m, 1H), 2.93-3.06 (m, 1H), 3.29-3.44 (m, 1H, teilweise verdeckt durch H₂O-Signal), 4.10 (d, 1H), 6.99 (dd, 1H), 7.27 (d, 2H), 7.33 (d, 1H), 7.37 (s, 1H), 7.42 (d, 2H), 9.77 (s, 1H), 11.95 (br. s, 1H). |
| | aus Methyl-3-[4-chlor-3-({(2*S*,3*R*)-2-[4-(2,2-difluorcyclopropyl)phenyl]-4,4,4-trifluor-3-methyl-butanoyl}amino)phenyl]-4-methylpentanoat (*Diastereomerengemisch*) | |
| **14** | 3-(3-{[(2*S*,3*R*)-2-(4-*tert*.-Butylphenyl)-4,4,4-trifluor-3-methylbutanoyl]amino}-4-chlorphenyl)-4-methylpentansäure (*Diastereomer 1*) | LC-MS (Methode 5): |
| | | Rₜ = 1.39 min; m/z = 512/514 (M+H)⁺. |
| | 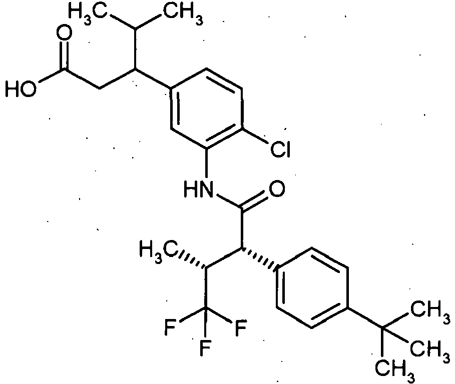 | ¹H-NMR (400 MHz, DMSO-*d₆*): δ [ppm] = 0.68 (d, 3H), 0.79 (d, 3H), 0.84 (d, 3H), 1.27 (s, 9H), 1.68-1.80 (m, 1H), 2.36-2.47 (m, 1H), 2.60-2.69 (m, 1H), 2.70-2.79 (m, 1H), 3.27-3.43 (m, 1H, teilweise verdeckt durch H₂O-Signal), 4.08 (d, 1H), 6.97 (dd, 1H), 7.30-7.44 (m, 6H), 9.73 (s, 0.96H, Hauptdiastereomer), 9.86 (s, 0.04H, Nebendiastereomer), 11.95 (br. s, 1H) (92% de). |
| | aus Methyl-3-(3-{[(2*S*,3*R*)-2-(4-*tert*.-butylphenyl)-4,4,4-trifluor-3-methylbutanoyl]amino}-4-chlor-phenyl)-4-methylpentanoat (*Diastereomer 1*) | |
| **15** | 3-(4-Chlor-3-{[(2*S*,3*R*)-2-(4-chlor-3-methoxyphenyl)-4,4,4-trifluor-3-methylbutanoyl]amino}-phenyl)-4-methylpentansäure (*Diastereomer 1*) | LC-MS (Methode 5): |
| | | Rₜ = 1.24 min; m/z = 520/522 (M+H)⁺. |
| | 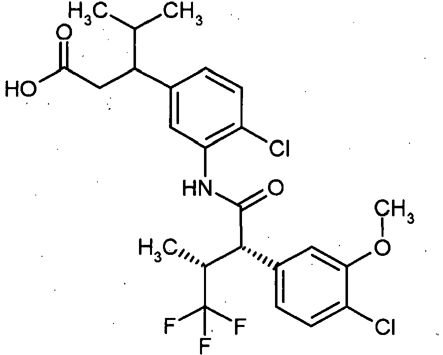 | ¹H-NMR (400 MHz, DMSO-*d₆*): δ [ppm] = 0.68 (d, 3H), 0.80-0.88 (m, 6H), 1.68-1.81 (m, 1H), 2.35-2.48 (m, 1H), 2.61-2.70 (m, 1H), 2.70-2.81 (m, 1H), 3.36-3.49 (m, 1H), 3.87 (s, 3H), 4.10 (d, 1H), 7.01 (t, 2H), 7.23 (d, 1H), 7.32-7.37 (m, 2H), 7.43 (d, 1H), 9.81 (s, 1H), 11.96 (br. s, 1H). |
| | aus Methyl-3-(4-chlor-3-{[(2*S*,3*R*)-2-(4-chlor-3-methoxyphenyl)-4,4,4-trifluor-3-methylbutanoyl]-amino}phenyl)-4-methylpentanoat (*Diastereomer 1*) | |
| **16** | 3-(4-Chlor-3-{[(2*S*,3*R*)-4,4,4-trifluor-3-methyl-2-(4-methylphenyl)butanoyl]amino}phenyl)-4-methylpentansäure (*Diastereomer 1*) | LC-MS (Methode 8): |
| | | Rₜ = 2.70 min; m/z = 470/472 (M+H)⁺. |
| | 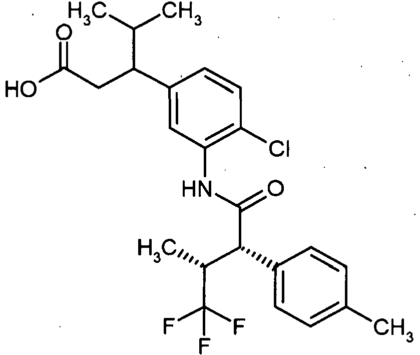 | ¹H-NMR (400 MHz, DMSO-*d*₆): δ [ppm] = 0.68 (d, 3H), 0.79 (d, 3H), 0.84 (d, 3H), 1.68-1.80 (m, 1H), 2.29 (s, 3H), 2.36-2.47 (m, 1H), 2.61-2.69 (m, 1H), 2.70-2.79 (m, 1H), 3.26-3.40 (m, 1H, teilweise verdeckt durch H₂O-Signal), 4.05 (d, 1H), 6.98 (dd, 1H), 7.17 (d, 2H), 7.29-7.39 (m, 4H), 9.73 (s, 0.96H, Hauptdiastereomer), 9.87 (s, 0.04H, Nebendiastereomer), 11.95 (br. s, 1H) (92% de). |
| | aus Methyl-3-(4-chlor-3-{[(2*S*,3*R*)-4,4,4-trifluor-3-methyl-2-(4-methylphenyl)butanoyl]amino}-phenyl)-4-methylpentanoat (*Diastereomer 1*) | |
| **17** | 3-(4-Chlor-3-{[(2*S*,3*R*)-2-(4-chlor-3-methylphenyl)-4,4,4-trifluor-3-methylbutanoyl]amino}phenyl)-4-methylpentansäure (*Diastereomer 1*) | LC-MS (Methode 5): |
| | | Rₜ = 1.33 min; m/z = 504/506 (M+H)⁺. |
| | 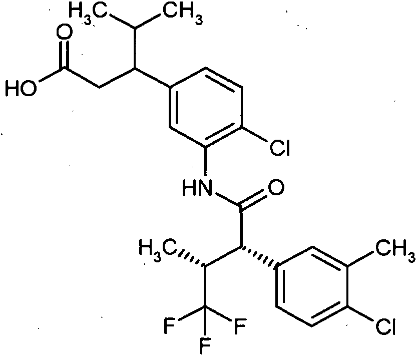 | ¹H-NMR (400 MHz, DMSO-*d₆*): δ [ppm] = 0.68 (d, 3H), 0.75-0.89 (m, 6H), 1.68-1.81 (m, 1H), 2.33 (s, 3H), 2.36-2.59 (m, 1H, teilweise verdeckt durch DMSO-Signal), 2.61-2.70 (m, 1H), 2.70-2.81 (m, 1H), 3.25-3.43 (m, 1H, teilweise verdeckt durch H₂O-Signal), 4.07 (d, 1H), 7.00 (d, 1H), 7.25-7.39 (m, 3H), 7.39-7.47 (m, 2H), 9.81 (s, 1H), 11.95 (br. s, 1H). |
| | aus Methyl-3-(4-chlor-3-{[(2*S*,3*R*)-2-(4-chlor-3-methylphenyl)-4,4,4-trifluor-3-methylbutanoyl]-amino}phenyl)-4-methylpentanoat (*Diastereomer 1*) | |
| **18** | 3-(4-Chlor-3-{[(4-chlorphenyl)(3,3-difluor-cyclopentyl)acetyl]amino}phenyl)-4-methyl-pentansäure (*Isomer 1*) | LC-MS (Methode 8): |
| | | Rₜ = 2.71 min; m/z = 498/500 (M+H)⁺. |
| | 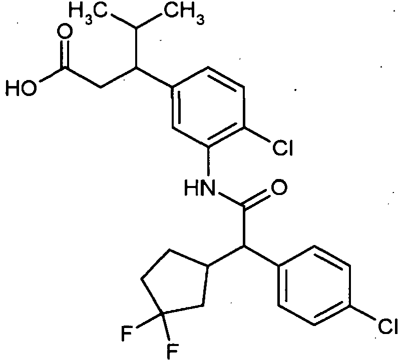 | ¹H-NMR (400 MHz, DMSO-*d₆*): δ [ppm] = 0.67 (d, 3H), 0.84 (d, 3H), 1.52-1.69 (m, 2H), 1.70-1.81 (m, 1H), 1.81-1.96 (m, 1H), 1.98-2.31 (m, 3H), 2.36-2.47 (m, 1H), 2.61-2.70 (m, 1H), 2.70-2.80 (m, 1H), 2.80-2.93 (m, 1H), 3.78 (d, 1H), 7.02 (dd, 1H), 7.31-7.39 (m, 2H), 7.44 (q, 4H), 9.78 (s, 1H), 11.95 (br. s, 1H). |
| | aus Methyl-3-(4-chlor-3-{[(4-chlorphenyl)-(3,3-difluorcyclopentyl)acetyl]amino}phenyl)-4-methylpentanoat (*Isomer 1*) | |
| **19** | 3-(4-Chlor-3-{[(4-chlorphenyl)(3,3-difluor-cyclopentyl)acetyl]amino}phenyl)-4-methyl-pentansäure (*Isomer 2*) | LC-MS (Methode 8): |
| | | Rₜ = 2.71 min; m/z = 498/500 (M+H)⁺. |
| | 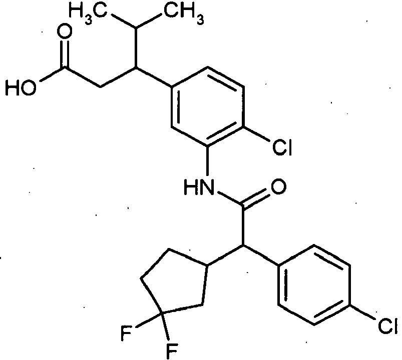 | ¹H-NMR (400 MHz, DMSO-*d₆*): δ [ppm] = 0.67 (d, 3H), 0.85 (d, 3H), 1.52-1.69 (m, 2H), 1.70-1.81 (m, 1H), 1.81-1.96 (m, 1H), 1.98-2.31 (m, 3H), 2.36-2.48 (m, 1H), 2.61-2.70 (m, 1H), 2.70-2.79 (m, 1H), 2.80-2.93 (m, 1H), 3.79 (d, 1H), 7.01 (dd, 1H), 7.32-7.39 (m, 2H), 7.43 (q, 4H), 9.77 (s, 1H), 11.95 (br. s, 1H). |
| | aus Methyl-3-(4-chlor-3-{[(4-chlorphenyl)-(3,3-difluorcyclopentyl)acetyl]amino}phenyl)-4-methylpentanoat (*Isomer 2*) | |
| **20** | 3-(4-Chlor-3-{[(4-chlorphenyl)(3,3-difluor-, cyclopentyl)acetyl]amino}phenyl)-4-methylpentansäure (*Isomer 3*) | LC-MS (Methode 8): |
| | | Rₜ = 2.71 min; m/z = 498/500 (M+H)⁺. |
| | 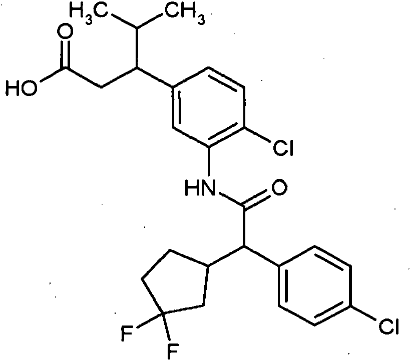 | ¹H-NMR (400 MHz, DMSO-*d₆*): δ [ppm] = 0.67 (d, 3H), 0.84 (d, 3H), 1.20-1.34 (m, 1H), 1.45-1.56 (m, 1H), 1.70-1.81 (m, 1H), 1.85-2.19 (m, 3H), 2.28-2.40 (m, 1H), 2.40-2.53 (m, 1H, teilweise verdeckt durch DMSO-Signal), 2.61-2.70 (m, 1H), 2.70-2.90 (m, 2H), 3.75 (d, 1H), 7.02 (dd, 1H), 7.34 (d, 1H), 7.37 (d, 1H), 7.44 (q, 4H), 9.74 (s, 1H), 11.95 (br. s, 1H). |
| | aus Methyl-3-(4-chlor-3-{[(4-chlorphenyl)-(3,3-difluorcyclopentyl)acetyl]amino}phenyl)-4-methylpentanoat (*Isomer 3*) | |
| **21** | 3-(4-Chlor-3-{[(4-chlorphenyl)(3,3-difluor-cyclopentyl)acetyl]amino}phenyl)-4-methyl-pentansäure (*Isomer 4*) | LC-MS (Methode 8): |
| | | Rₜ = 2.71 min; m/z = 498/500 (M+H)⁺. |
| | 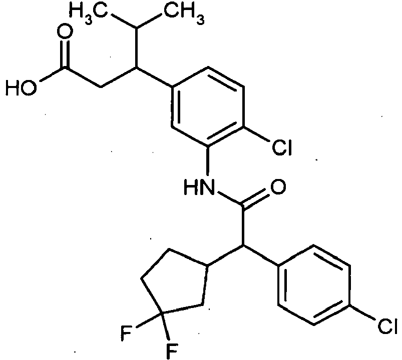 | ¹H-NMR (400 MHz, DMSO-*d₆*): δ [ppm] = 0.68 (d, 3H), 0.85 (d, 3H), 1.20-1.34 (m, 1H), 1.45-1.56 (m, 1H), 1.70-1.81 (m, 1H), 1.85-2.20 (m, 3H), 2.29-2.41 (m, 1H), 2.41-2.53 (m, 1H, teilweise verdeckt durch DMSO-Signal), 2.62-2.70 (m, 1H), 2.70-2.90 (m, 2H), 3.75 (d, 1H), 7.02 (dd, 1H), 7.32-7.39 (m, 2H), 7.44 (q, 4H), 9.73 (s, 1H), 11.95 (br. s, 1H). |
| | aus Methyl-3-(4-chlor-3-{[(4-chlorphenyl)-(3,3-difluorcyclopentyl)acety]amino}phenyl)-4-methylpentanoat (*Isomer 4*) | |

### Beispiel 22

### (+)-3-(4-Chlor-3-{[(2S,3R)-2-(4-chlorphenyl)-4,4,4-trifluor-3-methylbutanoyl]amino}phenyl)-3-cyclopropylpropansäure (Diastereomer 2)

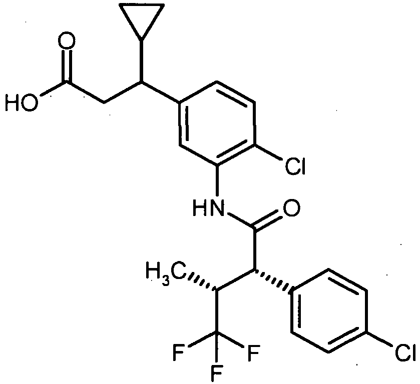

78 mg (0.14 mmol) *tert*.-Butyl-3-(4-chlor-3-{[(2*S*,3*R*)-2-(4-chlorphenyl)-4,4,4-trifluor-3-methyl-butanoyl]amino}phenyl)-3-cyclopropylpropanoat (Diastereomer 2; Beispiel 99A) wurden in 10 ml Dichlormethan gelöst und bei RT mit 0.33 ml (4.3 mmol) Trifluoressigsäure versetzt. Die Reaktionsmischung wurde 4 h bei RT gerührt und dann mit 10 ml Wasser verdünnt. Nach Trennung der Phasen wurde die wässrige Phase noch dreimal mit Dichlormethan extrahiert. Die vereinigten organischen Phasen wurden über Magnesiumsulfat getrocknet und im Vakuum eingeengt. Das so erhaltene Rohprodukt wurde durch präparative RP-HPLC gereinigt (Eluent Methanol/ Wasser 8:2 isokratisch). Es wurden 56 mg der Zielverbindung erhalten (81% d. Th.).

LC-MS (Methode 5): Rₜ = 1.20 min; m/z = 488/490 (M+H)⁺.

¹H-NMR (400 MHz, DMSO-*d₆*): δ [ppm] = 0.02-0.10 (m, 1H), 0.19-0.33 (m, 2H), 0.44-0.53 (m, 1H), 0.80 (d, 3H), 0.89-0.99 (m, 1H), 2.20-2.29 (m, 1H), 2.47-2.68 (m, 2H, teilweise verdeckt durch DMSO-Signal), 3.30-3.43 (m, 1H, teilweise verdeckt durch H₂O-Signal), 4.13 (d, 1H), 7.10 (dd, 1H), 7.36 (d, 1H), 7.42 (d, 1H), 7.43-7.50 (m, 4H), 9.84 (s, 1H), 12.04 (br. s, 1H).

[α]_{D}²⁰ = +98.8°, c = 0.325, Chloroform.

Auf analoge Weise wurden die in der folgenden Tabelle aufgeführten Verbindungen hergestellt:

| **Beispiel** | **Name / Struktur / Edukt** | **Analytische Daten** |
|---|---|---|
| **23** | (+)-3-(4-Chlor-3-{[(2*S*,3*R*)-2-(4-chlorphenyl)-4,4,4-trifluor-3-methylbutanoyl]amino}phenyl)-3-cyclopropylpropansäure (*Diastereomer 1*) | LC-MS (Methode 5): |
| | | Rₜ = 1.20 min; m/z = 488/490 (M+H)⁺. |
| | 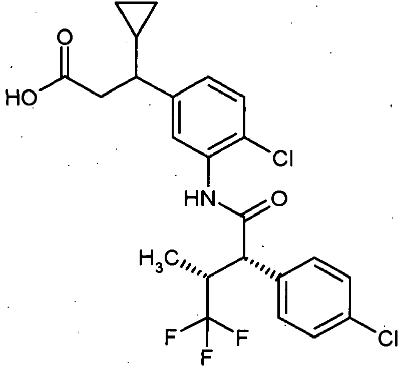 | ¹H-NMR (400 MHz, DMSO-*d₆*): δ [ppm] = 0.03-0.12 (m, 1H), 0.19-0.35 (m, 2H), 0.44-0.54 (m, 1H), 0.80 (d, 3H), 0.88-0.99 (m, 1H), 2.20-2.29 (m, 1H), 2.47-2.69 (m, 2H, teilweise verdeckt durch DMSO-Signal), 3.29-3.43 (m, 1H, teilweise verdeckt durch H₂O-Signal), 4.13 (d, 1H), 7.10 (dd, 1H), 7.36 (d, 1H), 7.41 (d, 1H), 7.43-7.50 (m, 4H), 9.84 (s, 1H), 12.03 (br. s, 1H). |
| | aus *tert*.-Butyl-3-(4-chlor-3-{[(2*S*,3*R*)-2-(4-chlor-phenyl)-4,4,4-trifluor-3-methylbutanoyl]amino}-phenyl)-3-cyclopropylpropanoat (*Diastereomer 1*) | |
| | | [α]_{D}²⁰= +57.3°, c = 0.355, Chloroform. |
| **24** | 3-(4-Chlor-3-{[(2*S*,3*R*)-2-(4-chlorphenyl)-4,4,4-trifluor-3-methylbutanoyl]amino}phenyl)-3-cyclobutylpropansäure *(Diastereomerengemisch)* | LC-MS (Methode 5): |
| | | Rₜ = 1.31 min; m/z = 502 (M+H)⁺. |
| | 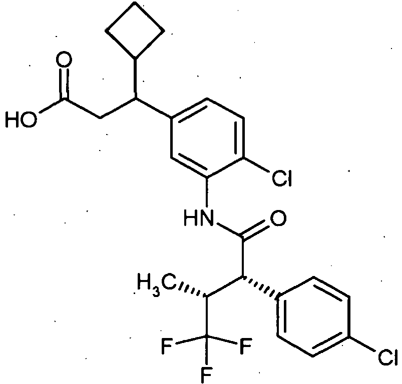 | ¹H-NMR (400 MHz, DMSO-*d₆*): δ [ppm] = 0.80 (d, 3H), 1.44-1.62 (m, 2H), 1.62-1.75 (m, 3H), 1.97-2.02 (m, 1H), 2.29 (dd, 1H), 2.33-2.42 (m, 1H), 2.46 (dd, 1H), 2.87 (td, 1H), 3.36-3.42 (m, 1H), 4.13 (d, 1H), 7.01 (dd, 1H), 7.33 (d, 1H), 7.37 (t, 1H), 7.43-7.51 (m, 4H), 9.81 (s, 1H), 11.99 (br. s, ca. 1H). |
| | aus *tert*.-Butyl-3-(4-chlor-3-{[(2*S*,3*R*)-2-(4-chlorphenyl)-4,4,4-trifluor-3-methylbutanoyl]amino}-phenyl)-3-cyclobutylpropanoat *(Diastereomerengemisch)* | |
| **25** | 3-(4-Chlor-3-{[(2*S*,3*R*)-2-(4-chlorphenyl)-4,4,4-trifluor-3-methylbutanoyl]amino}phenyl)-4-methoxy-4-methylpentansäure (*Diastereomerengemisch*) | LC-MS (Methode 7): |
| | | Rₜ = 1.26 min; m/z = 520/522 (M+H)⁺. |
| | 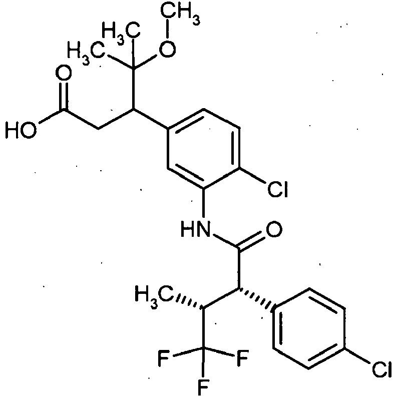 | ¹H-NMR (400 MHz, DMSO-*d₆*): δ [ppm] = 0.80 (d, 3H), 0.94 (d, 3H), 1.00 (d, 3H), 1.33-1.40 (m, 1H), 2.70-2.78 (m, 1H), 3.10 (s, 3H), 3.11-3.18 (m, 1H), 3.32-3.44 (m, 1H, teilweise verdeckt durch H₂O-Signal), 4.12 (d, 1H), 7.08 (dd, 1H), 7.33 (dd, 1H), 7.42 (d, 1H), 7.43-7.50 (m, 4H), 9.83 (d, 1H), 11.91 (br. s, ca. 1H). |
| | aus *tert*.-Butyl-3-(4-chlor-3-{[(2*S*,3*R*)-2-(4-chlor-phenyl)-4,4,4-trifluor-3-methylbutanoyl]amino}-phenyl)-4-methoxy-4-methylpentanoat (*Diastereomerengemisch*) | |
| **26** | 3-(4-Chlor-3-{[(2*S*,3*R*)-2-(4-chlorphenyl)-4,4,4-trifluor-3-methylbutanoyl]amino}phenyl)-3-(1-methylcyclopropyl)propansäure (*Diastereomerengemisch*) | LC-MS (Methode 5): |
| | | Rₜ = 1.27 min *(Diastereomer 1),* m/z = 502/504 (M+H)⁺; |
| | | Rₜ = 1.31 min *(Diastereomer 2),* m/z = 502/504 (M+H)⁺. |
| | 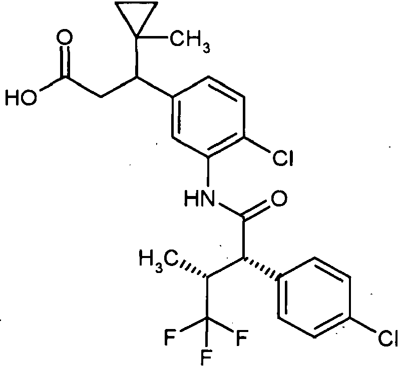 | ¹H-NMR (400 MHz, DMSO-*d₆*): δ [ppm] = 0.80 (d, 3H), 0.88-0.96 (m, 5H), 1.66-1.78 (m, 2H), 2.76-2.85 (m, 1H), 3.05-3.17 (m, 1H), 3.30-3.45 (m, 1H, teilweise verdeckt durch H₂O-Signal), 3.57-3.66 (m, 1H), 4:10-4.18 (m, 1H), 7.19 (dd, 1H), 7.40-7.51 (m, 6H), 9.92 (d, 1H). |
| | aus *tert*.-Butyl-3-(4-chlor-3-{[(2*S*,3*R*)-2-(4-chlor-phenyl)-4,4,4-trifluor-3-methylbutanoyl]amino}-phenyl)-3-(1-methylcyclopropyl)propanoat (*Diastereomerengemisch*) | |
| **27** | 3-(4-Chlor-3-{[(2*S*,3*R*)-2-(4-chlorphenyl)-4,4,4-trifluor-3-methylbutanoyl]amino}phenyl)-3-(1-fluorcyclopropyl)propansäure (*Diastereomerengemisch*) | LC-MS (Methode 5): |
| | | Rₜ = 1.18 min; m/z = 506/508 (M+H)⁺. |
| | 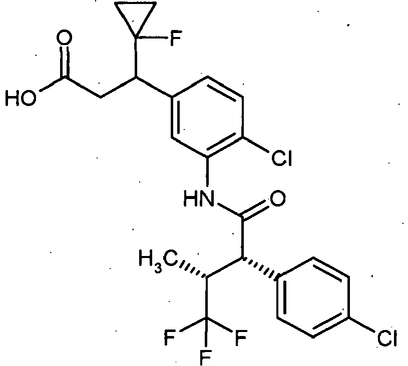 | ¹H-NMR (400 MHz, DMSO-*d₆*): δ [ppm] = 0.65-0.90 (m, 2H), 0.80 (d, 3H), 0.90-1.08 (m, 1H), 1.11-1.31 (m, 1H), 1.56-1.73 (m, 1H), 2,69-2.89 (m, 2H), 3.30-3.44 (m, 1H, teilweise verdeckt durch H₂O-Signal), 4.13 (d, 1H), 7.14 (dd, 1H), 7.39 (d, 1H), 7.42-7.52 (m, 5H), 9.87 (s, 1H), 11.85-12.70 (br. s, 1H). |
| | aus *tert*.-Butyl-3-(4-chlor-3-{[(2*S*,3*R*)-2-(4-chlor-phenyl)-4,4,4-trifluor-3-methylbutanoyl]amino}-phenyl)-3-(1-fluorcyclopropyl)propanoat (*Diastereomerengemisch*) | |
| **28** | 3-(4-Chlor-3-{[(2*S*,3*R*)-2-(4-chlorphenyl)-4,4,4-trifluor-3-methylbutanoyl]amino}phenyl)-3-(3,3-difluorcyclobutyl)propansäure (*Diastereomerengemisch*) | LC-MS (Methode 5): |
| | | Rₜ = 1.24 min; m/z = 538/540 (M+H)⁺. |
| | 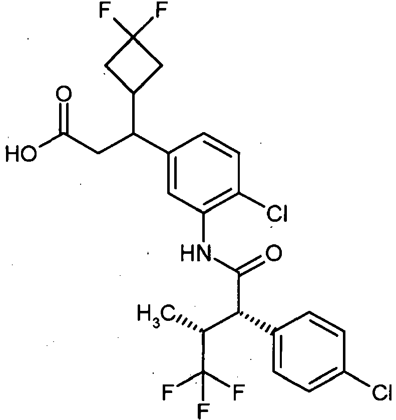 | ¹H-NMR (400 MHz, DMSO-*d₆)*: δ [ppm] = 0.80 (d, 2.29H), 1.21-1.31 (m, 1.71H), 2.02-2.17 (m, 1H), 2.18-2.39 (m, 3H), 2.40-2.75 (m, 2H, teilweise verdeckt durch DMSO-Signal), 2.91-3.03 (m, 1H), 3.17-3.44 (m, 1H, teilweise verdeckt durch H₂O-Signal), 4.13 (d, 1H), 7.05-7.16 (m, 1H), 7.33-7.53 (m, 6H), 9.85 (s, 0.7H), 9.98 (s, 0.3H), 11.96-12.18 (br. s, 1H). |
| | aus *tert*.-Butyl-3-(4-chlor-3-{[(2*S*,3*R*)-2-(4-chlor-phenyl)-4,4,4-trifluor-3-methylbutanoyl]amino}-phenyl)-3-(3,3-difluorcyclobutyl)propanoat (*Diastereomerengemisch*) | |
| **29** | 3-(4-Chlor-3-{[(2*S*,3*R*)-2-(4-chlorphenyl)-4,4,4-trifluor-3-methylbutanoyl]amino}phenyl)-4-cyclopropylbutansäure (*Diastereomerengemisch*) | LC-MS (Methode 5): |
| | | Rₜ = 1.29 min; m/z = 502/504 (M+H)⁺. |
| | 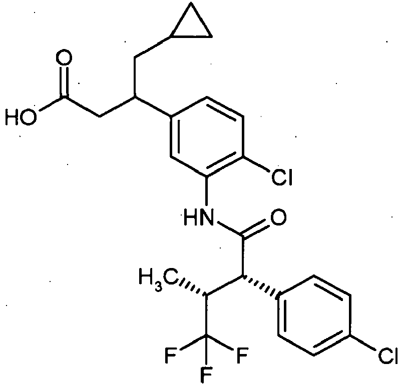 | |
| | aus *tert*.-Butyl-3-(4-chlor-3-{[(2*S*,3*R*)-2-(4-chlor-phenyl)-4,4,4-trifluor-3-methylbutanoyl]amino}-phenyl)-4-cyclopropylbutanoat (*Diastereomerengemisch*) | |

### Beispiel 30

### 3-(4-Chlor-3-{[(3R)-2-(4-chlorphenyl)-4,4,4-trifluor-3-methylbutanoyl]amino}phenyl)-3-cyclo-propyl-2-methylpropansäure (Diastereomerengemisch)

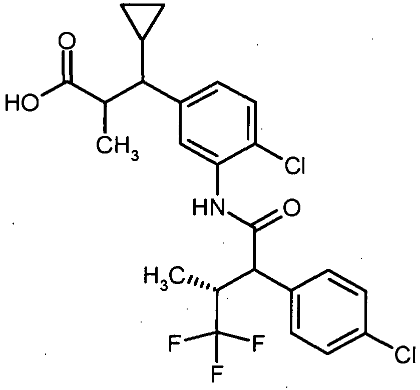

250 mg (0.47 mmol) Ethyl-3-(4-chlor-3-{[(2*S*,3*R*)-2-(4-chlorphenyl)-4,4,4-trifluor-3-methyl-butanoyl]amino}phenyl)-3-cyclopropyl-2-methylpropanoat (Diastereomerengemisch; Beispiel 129A) wurden in einer Mischung aus 1.0 ml Methanol, 0.5 ml THF und 0.5 ml Wasser gelöst und bei 0°C mit 40 mg (0.94 mmol) Lithiumhydroxid-Monohydrat versetzt. Die Mischung wurde zunächst 1 h bei 0°C und anschließend über Nacht bei RT gerührt. Danach wurde die Reaktionslösung nochmals mit 40 mg (0.94 mmol) Lithiumhydroxid-Monohydrat versetzt und auf 50°C erwärmt. Nach weiterem Rühren bei dieser Temperatur über Nacht wurde dem Reaktionsgemisch noch 1 ml Methanol zudosiert und die Mischung weitere 12 h bei 60°C gerührt. Danach wurde die Lösung mit Wasser verdünnt und mit 1 N Salzsäure sauer gestellt (pH ca. 2). Die wässrige Phase wurde dreimal mit Ethylacetat extrahiert. Die vereinigten organischen Phasen wurden über Magnesiumsulfat getrocknet und im Vakuum eingeengt. Es wurden 204 mg (86% d. Th.) der Titelverbindung als Diastereomerengemisch erhalten.

LC-MS (Methode 7): Rₜ = 1.26 min, m/z = 502/504 (M+H)⁺ *(Diastereomer 1);* Rₜ = 1.27 min, m/z = 502/504 (M+H)⁺ *(Diastereomer 2);* Rₜ = 1.28 min, m/z = 502/504 (M+H)⁺ *(Diastereomer 3*); Rₜ = 1.30 min, m/z = 502/504 (M+H)⁺ *(Diastereomer 4).*

¹H-NMR (400 MHz, DMSO-*d*₆): δ [ppm] = -0.20--0.05 (m, 0.85H), 0.13-0.36 (m, 2H), 0.47-0.65 (m, 0.85H), 0.68-0.75 (m, 0.3H), 0.80 (d, 2.63H), 0.93-1.09 (m, 1H), 1.17 (d, 1.5H), 1.21-1.29 (m, 1.87H), 1.84-2.08 (m, 1H), 2.61-2.77 (m, 1H), 3.16-3.27 (m, 0.5H), 3.28-3.43 (m, 0.5H, teilweise verdeckt durch H₂O-Signal), 4.09-4.17 (m, 1H), 6.70-6.78 (m, 0.16H), 7.02-7.13 (m, 1H), 7.30-7.53 (m, 5.84H), 9.80-10.01 (m, 1H), 11.79-12.35 (br. m, 1H).

### Beispiel 31

### 3-(4-Chlor-3-{[(3R)-2-(4-chlorphenyl)-4,4,4-trifluor-3-methylbutanoyl]amino}phenyl)-3-(2,2-di-fluorcyclopropyl)propansäure (Diastereomerengemisch 1)

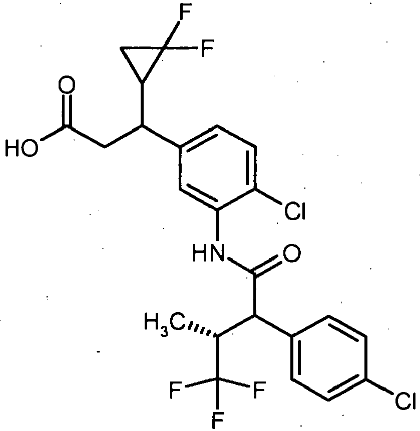

114 mg (0.21 mmol) Methyl-3-(4-chlor-3-{[(2*S*,3*R*)-2-(4-chlorphenyl)-4,4,4-trifluor-3-methyl-butanoyl]amino}phenyl)-3-(2,2-difluorcyclopropyl)propanoat (Isomer 1; Beispiel 124A) wurden in einer Mischung aus 2 ml Dioxan und 1 ml Wasser gelöst und mit 27 mg (0.64 mmol) Lithiumhydroxid-Monohydrat versetzt. Die Mischung wurde über Nacht bei RT gerührt. Danach wurde die Lösung mit Wasser verdünnt und mit 1 N Salzsäure sauer gestellt (pH ca. 2). Der ausgefallene Feststoff wurde abgesaugt und über Nacht im Hochvakuum getrocknet. Es wurden 89 mg (80% d. Th.) der Titelverbindung als Diastereomerengemisch in Form eines weißen Feststoffs erhalten.

LC-MS (Methode 5): Rₜ = 1.26 min; m/z = 524/526 (M+H)⁺.

¹H-NMR (400 MHz, DMSO-*d₆*): δ [ppm] = 0.80 (d, 1.63H), 1.04-1.19 (m, 1H), 1.26 (d, 1.37H), 1.36-1.50 (m, 1H), 1.97-2.14 (m, 1H), 2.46-2.82 (m, 3H, teilweise verdeckt durch DMSO-Signal), 3.15-3.43 (m, 1H, teilweise verdeckt durch H₂O-Signal), 4.07-4.17 (m, 1H), 7.17-7.26 (m, 1H), 7.36-7.53 (m, 6H), 9.87 (s, 0.55H), 10.01 (s, 0.45H), 12.16 (br. s, 1H).

### Beispiel 32

### 3-(4-Chlor-3-{[(3R)-2-(4-chlorphenyl)-4,4,4-trifluor-3-methylbutanoyl]amino}phenyl)-3-(2,2-di-fluorcyclopropyl)propansäure (Diastereomerengemisch 2)

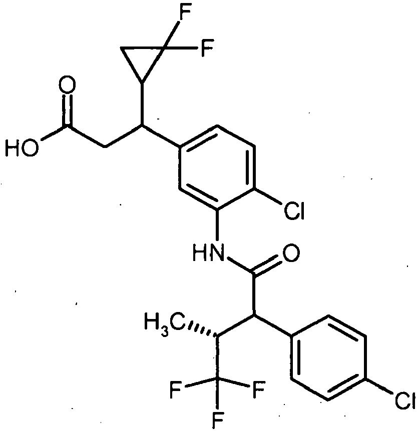

115 mg (0.21 mmol) Methyl-3-(4-chlor-3-{[(2*S*,3*R*)-2-(4-chlorphenyl)-4,4,4-trifluor-3-methyl-butanoyl]amino}phenyl)-3-(2,2-difluorcyclopropyl)propanoat (Isomer 2; Beispiel 125A) wurden in einer Mischung aus 2 ml Dioxan und 1 ml Wasser gelöst und mit 27 mg (0.64 mmol) Lithium-hydroxid-Monohydrat versetzt. Die Mischung wurde über Nacht bei RT gerührt. Danach wurde die Lösung mit Wasser verdünnt und mit 1 N Salzsäure sauer gestellt (pH ca. 2). Die wässrige Phase wurde dreimal mit Dichlormethan extrahiert. Die vereinigten organischen Phasen wurden über Magnesiumsulfat getrocknet und im Vakuum eingeengt. Es wurden 101 mg (90% d. Th.) der Titelver-bindung als Diastereomerengemisch in Form eines farblosen Öls erhalten.

LC-MS (Methode 5): Rₜ = 1.26 min; m/z = 524/526 (M+H)⁺.

¹H-NMR (400 MHz, DMSO-*d₆*): δ [ppm] = 0.80 (d, 1.68H), 1.05-1.18 (m, 1H), 1.26 (d, 1.32H), 1.35-1.50 (m, 1H), 1.96-2.12 (m, 1H), 2.44-2.82 (m, 3H, teilweise verdeckt durch DMSO-Signal), 3.15-3.42 (m, 1H, teilweise verdeckt durch H₂O-Signal), 4.08-4.16 (m, 1H), 7.17-7.25 (m, 1H), 7.37-7.52 (m, 6H), 9.87 (s, 0.56H), 10.01 (s, 0.44H), 12.16 (br. s, 1H).

### Beispiel 33 und Beispiel 34

### (+)-3-(4-Chlor-3-{[(2S,3R)-2-(4-chlorphenyl)-4,4,4-trifluor-3-methylbutanoyl]amino}phenyl)-3-cyclobutylpropansäure (Diastereomer 1 und 2)

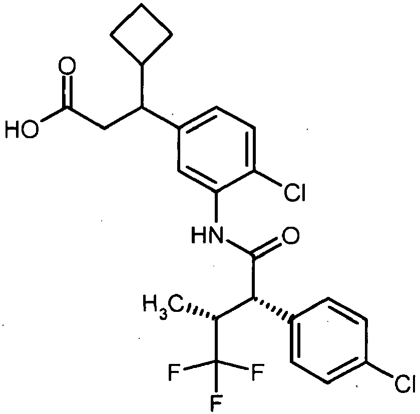

Das oben erhaltene Diastereomerengemisch der 3-(4-Chlor-3-{[(2*S*,3*R*)-2-(4-chlorphenyl)-4,4,4-trifluor-3-methylbutanoyl]amino}phenyl)-3-cyclobutylpropansäure (Beispiel 24) wurde durch präparative HPLC an chiraler Phase weiter aufgetrennt [Säule: Daicel Chiralpak AD-H, 5 µm, 250 mm x 20 mm; Injektionsvolumen: 0.40 ml; Eluent: 90% Isohexan / 10% Isopropanol; Fluss: 15 ml/min; Detektion: 220 nm; Temperatur: 25°C]. Ausgehend von 63 mg Diastereomerengemisch wurden 29 mg von Diastereomer 1 *(Beispiel 33)* und 32 mg von Diastereomer 2 *(Beispiel 34)* erhalten.

### Beispiel 33 (Diastereomer 1):

LC-MS (Methode 5): Rₜ = 1.31 min; m/z = 502 (M+H)⁺.

¹H-NMR (400 MHz, DMSO-*d₆*): δ [ppm] = 0.80 (d, 3H), 1.45-1.62 (m, 2H), 1.62-1.79 (m, 3H), 1.97-2.03 (m, 1H), 2.24-2.39 (m, 2H), 2.42-2.47 (m, 1H), 2.87 (td, 1H), 3.35-3.40 (m, 1H), 4.13 (d, 1H), 7.01 (dd, 1H), 7.23-7.39 (m, 2H), 7.42-7.54 (m, 4H), 9.81 (s, 1H), 11.98 (br. s, 1H).

[α]_{D}²⁰ = +69°, c = 0.260, Chloroform.

### Beispiel 34 (Diastereomer 2):

LC-MS (Methode 5): Rₜ = 1.31 min; m/z = 502 (M+H)⁺.

¹H-NMR (400 MHz, DMSO-*d*₆): δ [ppm] = 0.80 (d, 3H), 1.45-1.63 (m, 2H), 1.63-1.76 (m, 3H), 1.98-2.04 (m, 1H), 2.22-2.42 (m, 2H), 2.44-2.48 (m, 1H), 2.87 (td, 1H), 4.13 (d, 1H), 7.02 (dd, 1H), 7.33 (d, 1H), 7.37 (d, 1H), 7.42-7.51 (m, 4H), 9.81 (s, 1H), 12.00 (br. s, 1H).

[α]_{D}²⁰ = +53°, c = 0.250, Chloroform.

### Beispiel 35 und Beispiel 36

### 3-(4-Chlor-3-{[(2S,3R)-2-(4-chlorphenyl)-4,4,4-trifluor-3-methylbutanoyl]amino}phenyl)-4-cyclo-propylbutansäure (Diastereomer 1 und 2)

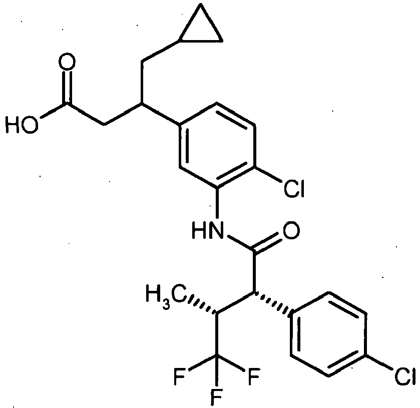

55 mg (0.11 mmol) des Diastereomerengemisches von 3-(4-Chlor-3-{[(2*S*,3*R*)-2-(4-chlorphenyl)-4,4,4-trifluor-3-methylbutanoyl]amino}phenyl)-4-cyclopropylbutansäure (Beispiel 29) wurden mittels präparativer HPLC an chiraler Phase weiter aufgetrennt [Säule: Daicel Chiralpak AD-H, 5 µm, 250 mm x 20 mm; Elutionsmittel: Isohexan/Ethanol 90:10 (v/v); Fluss: 15 ml/min; UV-Detektion: 220 nm; Temperatur: 30°C]:

### Beispiel 35 (Diastereomer 1):

Ausbeute: 28 mg

Rₜ = 7.47 min; chemische Reinheit >99%; >99% de
[Säule: Chiralpak AD-H, 5 µm, 250 mm x 4.6 mm; Elutionsmittel: Isohexan/(Ethanol + 0.2% Trifluoressigsäure + 1% Wasser) 90:10 (v/v); Fluss: 1 ml/min; UV-Detektion: 220 nm; Temperatur: 30°C].

LC-MS (Methode 5): Rₜ = 1.26 min; m/z = 502/504 (M+H)⁺.

¹H-NMR (400 MHz, DMSO-*d₆*): δ [ppm] = -0.14--0.06 (m, 1H), -0.06--0.03 (m, 1H), 0.22-0.37-(m, 2H), 0.39-0.50 (m, 1H), 0.80 (d, 3H), 1.27-1.36 (m, 1H), 1.45-1.56.(m, 1H), 2.39-2.47 (m, 1H), 2.57-2.66 (m, 1H), 2.99-3.09 (m, 1H), 3.28-3.43 (m, 1H, teilweise verdeckt durch H₂O-Signal), 4.13 (d, 1H), 7.07 (dd, 1H), 7.35 (d, 1H), 7.41 (d, 1H), 7.43-7.50 (m, 1H), 9.82 (s, 1H), 12.02 (br. s, 1H).

[α]_{D}²⁰ = +41°, c = 0.260, Chloroform.

### Beispiel 36 (Diastereomer 2):

Ausbeute: 25 mg

Rₜ = 8.75 min; chemische Reinheit >99%; >98.7% de
[Säule: Chiralpak AD-H, 5 µm, 250 mm x 4.6 mm; Elutionsmittel: Isohexan/(Ethanol + 0.2% Trifluoressigsäure + 1% Wasser) 90:10 (v/v); Fluss: 1 ml/min; UV-Detektion: 220 nm; Temperatur: 30°C].

LC-MS (Methode 5): Rₜ = 1.26 min; m/z = 502/504 (M+H)⁺.

¹H-NMR (400 MHz, DMSO-*d₆*): δ [ppm] = -0.14--0.07 (m, 1H), -0.06--0.02 (m, 1H), 0.22-0.36 (m, 2H), 0.38-0.49 (m, 1H), 0.80 (d, 3H), 1.27-1.36 (m, 1H), 1.46-1.55 (m, 1H), 2.39-2.47 (m, 1H), 2.58-2.66 (m, 1H), 2.99-3.09 (m, 1H), 3.28-3.43 (m, 1H, teilweise verdeckt durch H₂O-Signal), 4.13 (d, 1H), 7.07 (dd, 1H), 7.35 (d, 1H), 7.42 (d, 1H), 7.43-7.50 (m, 4H), 9.82 (s, 1H), 12.02 (br. s, 1H).

Analog zu Beispiel 22 wurde die folgende Verbindung hergestellt:

| **Beispiel** | **Name / Struktur / Edukt** | **Analytische Daten** |
|---|---|---|
| **37** | 3-(4-Chlor-3-{[(2*S*,3*R*)-2-(4-chlorphenyl)-4,4,4-trifluor-3-methylbutanoyl]amino}phenyl)-4-(1-methylcyclopropyl)butansäure (*Diastereomerengemisch*) | LC-MS (Methode 7): |
| | | Rₜ = 1.34 min; m/z = 516/518 (M+H)⁺. |
| | 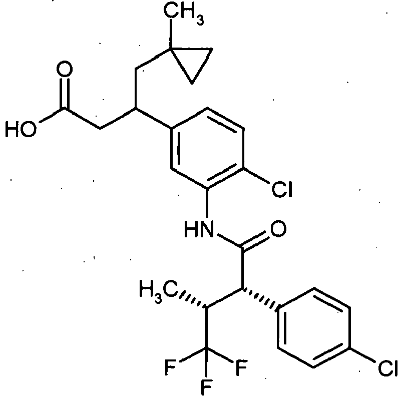 | ¹H-NMR (400 MHz, DMSO-*d*₆): δ [ppm] = -0.16--0.09 (m, 1 H), -0.09--0.02 (m, 1H), 0.11-0.18 (m, 1H), 0.18-0.25 (m, 1H), 0.80 (d, 3H), 0.92 (d, 3H), 1.47-1.55 (m, 2H), 2.31-2.42 (m, 1H), 2.57-2.65 (m, 1H), 3.05-3.20 (m, 1H), 3.28-3.43 (m, 1H, teilweise verdeckt durch H₂O-Signal), 4.12 (d, 1H), 7.01-7.13 (m, 1H), 7.33 (d, 1H), 7.39-7.51 (m, 5H), 9.81 (d, 1H), 12.03 (br. s, 1H). |
| | aus *tert*.-Butyl-3-(4-chlor-3-{[(2*S*,3*R*)-2-(4-chlor-phenyl)-4,4,4-trifluor-3-methylbutanoyl]amino}-phenyl)-4-(1-methylcyclopropyl)butanoat (*Diastereomerengemisch*) | |

### B. Bewertung der pharmakologischen Wirksamkeit

Die pharmakologische Wirkung der erfindungsgemäßen Verbindungen kann in folgenden Assays gezeigt werden:

### B-1. Stimulation der rekombinanten löslichen Guanylatcyclase (sGC) in vitro

Die Untersuchungen zur Stimulation der rekombinanten löslichen Guanylatcyclase (sGC) durch die erfindungsgemäßen Verbindungen mit und ohne Natriumnitroprussid sowie mit und ohne den Hämabhängigen sGC-Inhibitor 1*H*-1,2,4-Oxadiazolo[4,3-a]chinoxalin-1-on (ODQ) werden nach der in folgender Literaturstelle im Detail beschriebenen Methode durchgeführt: M. Hoenicka, E. M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als x-fache Stimulation der Basalaktivität angegeben. Das Ergebnis für Beispiel 22 ist in Tabelle 1 gezeigt:

**Tabelle 1: Stimulation (x-fach) der rekombinanten löslichen Guanylatcyclase (sGC) in vitro durch Beispiel 22**

| **Konzentration Beispiel 22 [µM]** | **Häm-haltige sGC** | | | **Häm-freie sGC Basal (n=5)** |
|---|---|---|---|---|
| | **Basal (n=5)** | **+ 0.01 µM DEA/NO** | **+ 10 µM ODQ** | |
| 0 | 1.0 ± 0.0 | 3.6 ± 1.0 | 5.1 ± 1.5 | 1.0 ± 0.0 |
| 0.01 | 1.6 ± 0.3 | 4.4 ± 1.3 | 5.7 ± 1.6 | 1.2 ± 0.1 |
| 0.1 | 1.6 ± 0.5 | 3.4 ± 0.9 | 6.1 ± 1.7 | 1.6 ± 0.5 |
| 1.0 | 2.4 ± 1.0 | 4.4 ± 1.4 | 8.4 ± 2.2 | 4.9 ± 1.5 |
| 10 | 4.9 ± 1.2 | 7.8 ± 2.5 | 18.3 ± 5.4 | 14.2 ± 2.0 |

| | | | | |
|---|---|---|---|---|
| [DEA/NO = *2-(N,N-*Diethylamino)diazenolat-2-oxid; ODQ = 1*H*-1,2,4-Oxadiazolo[4,3-a]chinoxalin-1-on]. | | | | |

Aus der Tabelle 1 ist ersichtlich, dass eine Stimulation sowohl des Häm-haltigen als auch des Hämfreien Enzyms erreicht wird. Weiterhin zeigt die Kombination von Beispiel 22 mit 2-(*N,N-*Diethyl-amino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch 1*H*-1,2,4-Oxadiazolo[4,3-a]chinoxalin-1-on (ODQ), einen Häm-abhängigen Inhibitor der löslichen Guanylatcyclase, nicht blockiert, sondern sogar gesteigert. Die Ergebnisse in Tabelle 1 belegen somit den Wirkmechanismus der erfindungsgemäßen Verbindungen als Aktivatoren der löslichen Guanylatcyclase.

### B-2. Wirkung an rekombinanter Guanylatcyclase-Reporterzelllinie

Die zelluläre Wirkung der erfindungsgemäßen Verbindungen wird an einer rekombinanten Guanylat-cyclase-Reporterzelllinie, wie in F. Wunder et al., Anal. Biochem. 339, 104-112 (2005) beschrieben, bestimmt.

Repräsentative Ergebnisse zu den erfindungsgemäßen Verbindungen sind in Tabelle 2 aufgeführt:

**Tabelle 2: sGC-aktivierende Wirkung in der CHO-Reporterzelle in vitro**

| **Beispiel Nr.** | **MEC [nM]** |
|---|---|
| 1 | 3 |
| 2 | 6.5 |
| 3 | 0.3 |
| 4 | 3 |
| 5 | 0.3 |
| 6 | 1 |
| 7 | 300 |
| 8 | 1 |
| 9 | 1 |
| 10 | 0.3 |
| 11 | 3 |
| 12 | 1 |
| 13 | 0.3 |
| 14 | 0.3 |
| 15 | 3 |
| 16 | 3 |
| 17 | 1 |
| 18 | 300 |
| 19 | 30 |
| 20 | 1000 |
| 21 | 10 |
| 22 | 1.8 |
| 23 | 3 |
| 25 | 10 |
| 26 | 10 |
| 27 | 3 |
| 28 | 30 |
| 30 | 10 |
| 31 | 3 |
| 32 | 3 |
| 33 | 1 |
| 34 | 10 |
| 35 | 0.3 |
| 36 | 3 |

| | |
|---|---|
| (MEC = minimale effektive Konzentration). | |

### B-3. Stimulation der sGC-Enzymaktivität

Lösliche Guanylatcyclase (sGC) setzt unter Stimulation GTP zu cGMP und Pyrophosphat (PPi) um. PPi wird mit Hilfe des nachfolgend beschriebenen Tests nachgewiesen. Das im Test entstehende Signal nimmt mit fortschreitender Umsetzung zu und dient als Maß für die sGC-Enzymaktivität unter der gegebenen Stimulation.

Zur Durchführung des Tests werden 29 µl Enzymlösung [0-10 nM lösliche Guanylatcyclase (hergestellt nach Hönicka et al., J. Mol. Med. 77, 14-23 (1999)) in 50 mM TEA, 2 mM MgCl₂, 0.1% BSA (Fraktion V), 0.005% Brij^{®}, pH 7.5] in eine Mikroplatte vorgelegt und 1 µl der zu testenden Substanz (als seriell verdünnte Lösung in DMSO) hinzugegeben. Der Ansatz wird 10 min bei Raumtemperatur inkubiert. Anschließend werden 20 µl Detektionsmix [1.2 nM Firefly-Luciferase (*Photinus pyralis*-Luciferase, Fa. Promega), 29 µM Dehydro-Luziferin (hergestellt nach Bitler & McElroy, Arch. Biochem. Biophys. 72, 358 (1957)), 122 µM Luziferin (Fa. Promega), 153 µM ATP (Fa. Sigma) und 0.4 mM DTT (Fa. Sigma) in 50 mM TEA, 2 mM MgCl₂, 0.1% BSA (Fraktion V), 0.005% Brij^{®}, pH 7.5] zugegeben. Die Enzymreaktion wird durch Zugabe von 20 µl Substratlösung [1.25 mM Guanosin-5'-triphosphat (Fa. Sigma) in 50 mM TEA, 2 mM MgCl₂, 0.1% BSA (Fraktion V), 0.005% Brij^{®}, pH 7.5] gestartet und kontinuierlich luminometrisch vermessen. Das Maß der Stimulation durch die zu testende Substanz kann relativ zum Signal der nicht stimulierten Reaktion bestimmt werden.

Durch Zugabe von 25 µM 1*H*-1,2,4-Oxadiazolo[4,3-a]chinoxalin-1-on (ODQ) zur Enzymlösung und anschließende 30-minütige Inkubation wird die Aktivierung der Häm-freien Guanylatcyclase untersucht und mit der Stimulation des nativen Enzyms verglichen.

Repräsentative Ergebnisse zu den erfindungsgemäßen Verbindungen sind in Tabelle 3 aufgeführt:

**Tabelle 3: Aktivierende Wirkung am sGC-Enzym in vitro**

| **Beispiel Nr.** | **MEC [nM]** | **EC₅₀ [nM]** |
|---|---|---|
| 1 | 1 | 22 |
| 2 | 4 | 89 |
| 3 | 1 | 37 |
| 4 | 2.4 | 110 |
| 5 | 0.3 | 5.2 |
| 6 | 1.1 | 56 |
| 10 | 0.5 | 10 |
| 12 | 1.1 | 17 |
| 13 | 0.5 | 14 |
| 14 | 0.5 | 8.4 |
| 22 | 2.4 | 68 |
| 25 | 5.1 | 220 |
| 27 | 1.7 | 68 |
| 30 | 17 | 640 |
| 33 | 0.4 | 11 |
| 35 | 1 | 11 |

| | | |
|---|---|---|
| (MEC = minimale effektive Konzentration; EC₅₀ = Konzentration bei 50% der maximalen Wirksamkeit). | | |

### B-4. Gefäßrelaxierende Wirkung in vitro

Kaninchen werden durch intravenöse Injektion von Thiopental-Natrium narkotisiert bzw. getötet (ca. 50 mg/kg) und entblutet. Die *Arteria Saphena* wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 4 aufgeführt:

**Tabelle 4: Gefäßrelaxierende Wirkung in vitro**

| **Beispiel Nr.** | **IC₅₀ [nM]** |
|---|---|
| 3 | 801 |
| 10 | 131 |
| 14 | 269 |
| 16 | 767 |
| 22 | 137 |

### B-5. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (2) Empfänger, die über einen Multiplexer mit einem (3) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer AG, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden, instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versüchsnummer trägt.

Im Standardablauf werden über je 10 Sekunden Dauer gemessen: (*1*) systolischer Blutdruck (SBP), (2) diastolischer Blutdruck (DBP), (*3*) arterieller Mitteldruck (MAP) und (4) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefühlt.

## Patentansprüche

1. Verbindung der Formel (I) 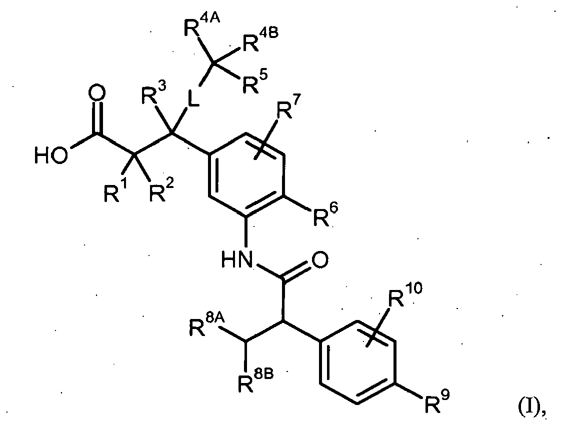 in welcher
R¹, R² und R³ unabhängig voneinander für Wasserstoff oder Methyl stehen,
L für eine Bindung oder für -CH₂- steht,
R^{4A} und R^{4B} unabhängig voneinander für Methyl, Trifluormethyl oder Ethyl stehen
oder
R^{4A} und R^{4B} miteinander verknüpft sind und gemeinsam mit dem Kohlenstoffatom, an das sie gebunden sind, einen Cyclopropyl- oder Cyclobutyl-Ring bilden, der bis zu zweifach mit Fluor substituiert sein kann,
R⁵ für Wasserstoff, Fluor, Methyl oder Methoxy steht,
R⁶ für Wasserstoff, Fluor, Chlor, Brom, Cyano, Methyl, Trifluormethyl, Ethyl, Methoxy oder Trifluormethoxy steht,
R⁷ für Wasserstoff, Fluor, Chlor oder Methyl steht,
R^{8A} für Methyl oder Ethyl steht,
R^{8B} für Trifluormethyl steht,
oder
R^{8A} und R^{8B} miteinander verknüpft sind und gemeinsam mit dem Kohlenstoffatom, an das sie gebunden sind, einen optional difluor-substituierten Cyclopentyl-Ring der Formel 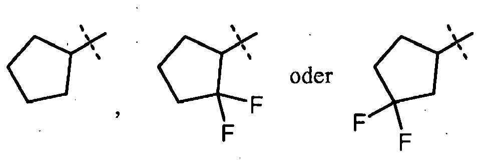 bilden,
R⁹ für Fluor, Chlor, Brom, Cyano, (C₁-C₄)-Alkyl, (C₂-C₄)-Alkenyl, Cyclopropyl oder Cyclobutyl steht, wobei
(C₁-C₄)-Alkyl und (C₂-C₄)-Alkenyl bis zu dreifach mit Fluor
und
Cyclopropyl und Cyclobutyl bis zu zweifach mit Fluor substituiert sein können,
und
R¹⁰ für Wasserstoff, Fluor, Chlor, Methyl, Trifluormethyl, Ethyl oder Methoxy steht,
sowie ihre Salze, Solvate und Solvate der Salze.

2. Verbindung der Formel (I) nach Anspruch 1, in welcher
R¹ für Wasserstoff oder Methyl steht,
R² für Wasserstoff steht,
R³ für Wasserstoff oder Methyl steht,
L für eine Bindung oder für -CH₂- steht,
R^{4A} und R^{4B} beide für Methyl stehen oder miteinander verknüpft sind und gemeinsam mit dem Kohlenstoffatom, an das sie gebunden sind, einen Cyclopropyl- oder Cyclobutyl-Ring bilden, der bis zu zweifach mit Fluor substituiert sein kann,
R⁵ für Wasserstoff, Fluor, Methyl oder Methoxy steht,
R⁶ für Fluor, Chlor, Methyl oder Ethyl steht,
R⁷ für Wasserstoff oder Fluor steht,
R^{8A} für Methyl steht,
R^{8B} für Trifluormethyl steht,
oder
R^{8A} und R^{8B} miteinander verknüpft sind und gemeinsam mit dem Kohlenstoffatom, an das sie gebunden sind, einen difluor-substituierten Cyclopentyl-Ring der Formel 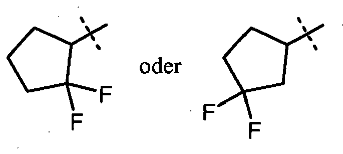 bilden,
R⁹ für Fluor, Chlor, (C₁-C₄)-Alkyl, (C₂-C₃)-Alkenyl, Cyclopropyl oder Cyclobutyl steht, wobei
(C₁-C₄)-Alkyl und (C₂-C₃)-Alkenyl bis zu dreifach mit Fluor
und
Cyclopropyl und Cyclobutyl bis zu zweifach mit Fluor substituiert sein können,
und
R¹⁰ für Wasserstoff, Fluor, Chlor, Methyl oder Methoxy steht,
sowie ihre Salze, Solvate und Solvate der Salze.

3. Verbindung der Formel (I) nach Anspruch 1 oder 2, in welcher
R¹ und R² beide für Wasserstoff stehen,
R³ für Wasserstoff oder Methyl steht,
L für eine Bindung oder für -CH₂- steht,
R^{4A} und R^{4A} beide für Methyl stehen oder miteinander verknüpft sind und gemeinsam mit dem Kohlenstoffatom, an das sie gebunden sind, einen Cyclopropyl- oder Cyclobutyl-Ring bilden, der bis zu zweifach mit Fluor substituiert sein kann,
R⁵ für Wasserstoff, Fluor oder Methyl steht,
R⁶ für Chlor steht,
R⁷ für Wasserstoff steht,
R^{8A} für Methyl steht,
R^{8B} für Trifluormethyl steht,
R⁹ für Fluor, Chlor, Methyl, Trifluormethyl, Ethyl, 2,2,2-Trifluorethyl, Isopropyl, *tert.-*Butyl, Cyclopropyl oder 2,2-Difluorcyclopropyl steht,
und
R¹⁰ für Wasserstoff, Fluor, Methyl oder Methoxy steht,
sowie ihre Salze, Solvate und Solvate der Salze.

4. Verbindung nach Anspruch 1 entsprechend der folgenden Formel 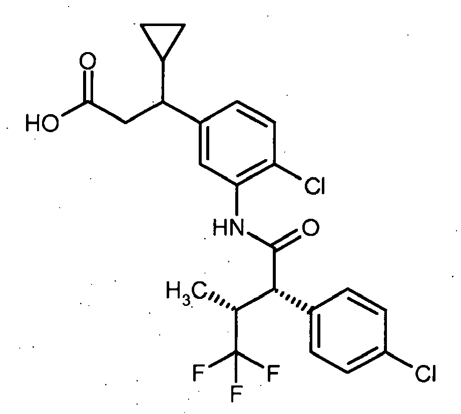 sowie dessen Salze, Solvate und Solvate der Salze.

5. Verfahren zur Herstellung einer Verbindung der Formel (I), wie in den Ansprüchen 1 bis 4 definiert, **dadurch gekennzeichnet, dass** man eine Carbonsäure der Formel (II) 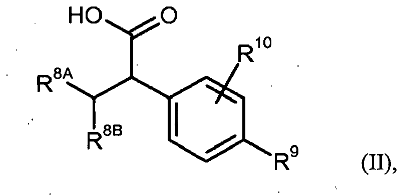 in welcher R^{8A}, R^{8B}, R⁹ und R¹⁰ die in den Ansprüchen 1 bis 4 angegebenen Bedeutungen haben,
in einem inerten Lösungsmittel mit Hilfe eines Kondensationsmittels oder über die Zwischenstufe des entsprechenden Carbonsäurechlorids in Gegenwart einer Base mit einem Amin der Formel (III) 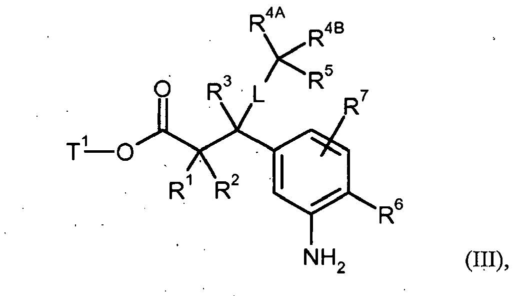 in welcher L, R¹, R², R³, R^{4A}, R^{4B}, R⁵, R⁶ und R⁷ die in den Ansprüchen 1 bis 4 angegebenen Bedeutungen haben
und
T¹ für (C₁-C₄)-Alkyl oder Benzyl steht,
zu einem Carbonsäureamid der Formel (IV) 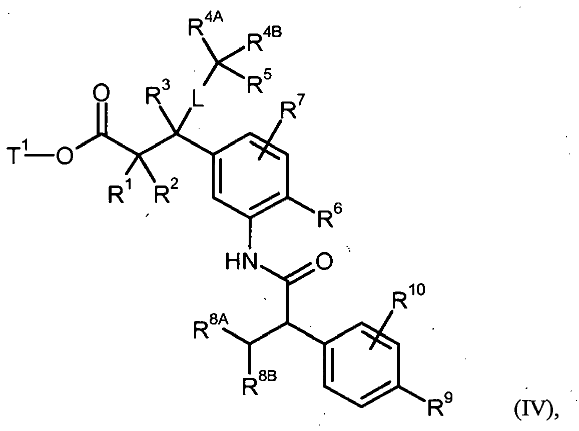 in welcher L, R¹, R², R³, R^{4A}, R^{4B}, R⁵, R⁶, R⁷, R^{8A}, R^{8B}, R⁹, R¹⁰ und T¹ die oben angegebenen Bedeutungen haben,
kuppelt und anschließend den Ester-Rest T¹ durch basische oder saure Solvolyse oder im Fall, dass T¹ für Benzyl steht, auch durch Hydrogenolyse unter Erhalt der Carbonsäure der Formel (I) abspaltet
und gegebenenfalls die Verbindungen der Formel (I) nach dem Fachmann bekannten Methoden in ihre Enantiomere und/oder Diastereomere trennt und/oder mit den entsprechenden (i) Lösungsmitteln und/oder (ii) Basen zu ihren Solvaten, Salzen und/oder Solvaten der Salze umsetzt.

6. Verbindung, wie in einem der Ansprüche 1 bis 4 definiert, zur Behandlung und/oder Prävention von Krankheiten.

7. Verwendung einer Verbindung, wie in einem der Ansprüche 1 bis 4 definiert, zur Herstellung eines Arzneimittels zur Behandlung und/oder Prävention von Herzinsuffizienz, Angina pectoris, Hypertonie, pulmonaler Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

8. Arzneimittel enthaltend eine Verbindung, wie in einem der Ansprüche 1 bis 4 definiert, in Kombination mit einem oder mehreren inerten, nicht-toxischen, pharmazeutisch geeigneten Hilfsstoffen.

9. Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

10. Arzneimittel nach Anspruch 8 oder 9 zur Behandlung und/oder Prävention von Herzinsuffizienz, Angina pectoris, Hypertonie, pulmonaler Hypertonie, thromboembolischen Erkrankungen, Ischämien, Gefäßerkrankungen, Mikrozirkulationsstörungen, Niereninsuffizienz, fibrotischen Erkrankungen und Arteriosklerose.

## Claims

1. Compound of the formula (I) 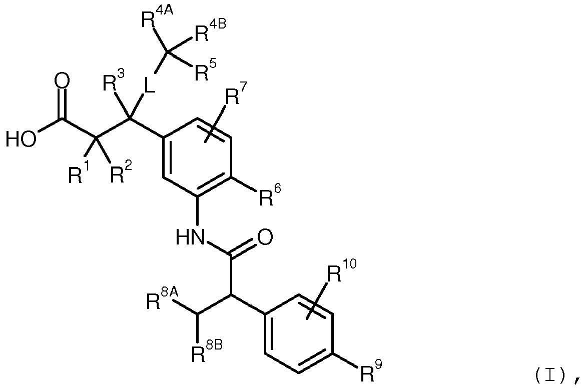 in which
R¹, R² and R³ independently of one another represent hydrogen or methyl,
L represents a bond or represents -CH₂-,
R^{4A} and R^{4B} independently of one another represent methyl, trifluoromethyl or ethyl
or
R^{4A} and R^{4B} are attached to one another and together with the carbon atom to which they are attached form a cyclopropyl or cyclobutyl ring which may be substituted up to two times by fluorine,
R⁵ represents hydrogen, fluorine, methyl or methoxy,
R⁶ represents hydrogen, fluorine, chlorine, bromine, cyano, methyl, trifluoromethyl, ethyl, methoxy or trifluoromethoxy,
R⁷ represents hydrogen, fluorine, chlorine or methyl,
R^{8A} represents methyl or ethyl,
R^{8B} represents trifluoromethyl,
or
R^{8A} and R^{8B} are attached to one another and together with the carbon atom to which they are attached form an optionally difluoro-substituted cyclopentyl ring of the formula 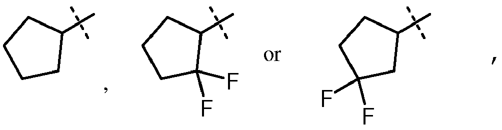
R⁹ represents fluorine, chlorine, bromine, cyano, (C₁-C₄)-alkyl, (C₂-C₄)-alkenyl, cyclopropyl or cyclobutyl, where (C₁-C₄)-alkyl and (C₂-C₄)-alkenyl may be substituted up to three times by fluorine and cyclopropyl and cyclobutyl may be substituted up to two times by fluorine,
and
R¹⁰ represents hydrogen, fluorine, chlorine, methyl, trifluoromethyl, ethyl or methoxy,
and salts, solvates and solvates of the salts thereof.

2. Compound of the formula (I) according to Claim 1 in which
R¹ represents hydrogen or methyl,
R² represents hydrogen,
R³ represents hydrogen or methyl,
L represents a bond or represents -CH₂-,
R^{4A} and R^{4B} both represent methyl or are attached to one another and together with the carbon atom to which they are attached form a cyclopropyl or cyclobutyl ring which may be substituted up to two times by fluorine,
R⁵ represents hydrogen, fluorine, methyl or methoxy,
R⁶ represents fluorine, chlorine, methyl or ethyl,
R⁷ represents hydrogen or fluorine,
R^{8A} represents methyl,
R^{8B} represents trifluoromethyl,
or
R^{8A} and R^{8B} are attached to one another and together with the carbon atom to which they are attached form a difluoro-substituted cyclopentyl ring of the formula 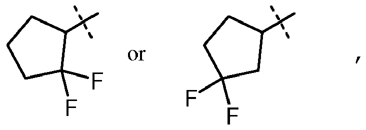
R⁹ represents fluorine, chlorine, (C₁-C₄)-alkyl, (C₂-C₃)-alkenyl, cyclopropyl or cyclobutyl, where (C₁-C₄)-alkyl and (C₂-C₃)-alkenyl may be substituted up to three times by fluorine and cyclopropyl and cyclobutyl may be substituted up to two times by fluorine,
and
R¹⁰ represents hydrogen, fluorine, chlorine, methyl or methoxy,
and salts, solvates and solvates of the salts thereof.

3. Compound of the formula (I) according to Claim 1 or 2 in which
R¹ and R² both represent hydrogen,
R³ represents hydrogen or methyl,
L represents a bond or represents -CH₂-,
R^{4A} and R^{4B} both represent methyl or are attached to one another and together with the carbon atom to which they are attached form a cyclopropyl or cyclobutyl ring which may be substituted up to two times by fluorine,
R⁵ represents hydrogen, fluorine or methyl,
R⁶ represents chlorine,
R⁷ represents hydrogen,
R^{8A} represents methyl,
R^{8B} represents trifluoromethyl,
R⁹ represents fluorine, chlorine, methyl, trifluoromethyl, ethyl, 2,2,2-trifluoroethyl, isopropyl, *tert*-butyl, cyclopropyl or 2,2-difluorocyclopropyl,
and
R¹⁰ represents hydrogen, fluorine, methyl or methoxy,
and salts, solvates and solvates of the salts thereof.

4. Compound according to Claim 1 corresponding to the following formula 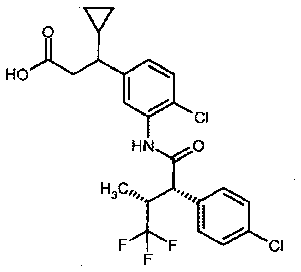 and salts, solvates and solvates of the salts thereof.

5. Process for preparing a compound of the formula (I) as defined in any of Claims 1 to 4, **characterized in that** a carboxylic acid of the formula (II) 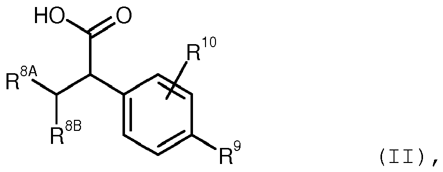 in which R^{8A}, R^{8B}, R⁹ and R¹⁰ have the meanings given in any of Claims 1 to 4,
is coupled in an inert solvent with the aid of a condensing agent or via the intermediate of the corresponding carbonyl chloride in the presence of a base with an amine of the formula (III) 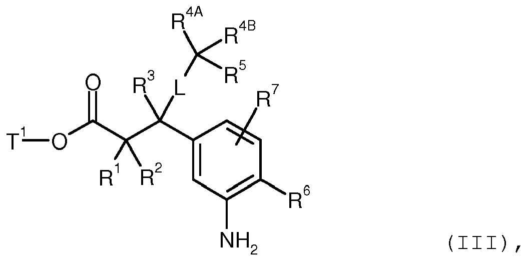 in which L, R¹, R², R³, R^{4A}, R^{4B}, R⁵, R⁶ and R⁷ have the meanings given in Claims 1 to 4
and
T¹ represents (C₁-C₄)-alkyl or benzyl, to give a carboxamide of the formula (IV) 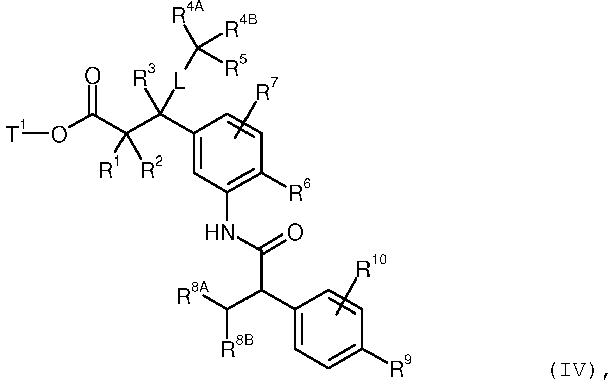 in which L, R¹, R², R³, R^{4A}, R^{4B}, R⁵, R⁶, R⁷, R^{8A}, R^{8B}, R⁹, R¹⁰ and T¹ have the meanings given above,
and the ester radical T¹ is then removed by basic or acidic solvolysis or, in the case that T¹ represents benzyl, also by hydrogenolysis to give the carboxylic acid of the formula (I) and the compounds of the formula (I) are optionally separated by methods known to the person skilled in the art into their enantiomers and/or diastereomers and/or reacted with the appropriate (i) solvents and/or (ii) bases to give their solvates, salts and/or solvates of the salts.

6. Compound as defined in any of Claims 1 to 4 for the treatment and/or prevention of diseases.

7. Use of a compound as defined in any of Claims 1 to 4 for preparing a medicament for the treatment and/or prevention of heart failure, angina pectoris, hypertension, pulmonary hypertension, thromboembolic disorders, ischaemias, vascular disorders, microcirculation impairments, renal insufficiency, fibrotic disorders and arteriosclerosis.

8. Medicament comprising a compound as defined in any of Claims 1 to 4 in combination with one or inert, non-toxic, pharmaceutically suitable excipients.

9. Medicament comprising a compound as defined in any of Claims 1 to 4 in combination with one or more further active compounds selected from the group consisting of organic nitrates, NO donors, cGMP-PDE inhibitors, stimulators of guanylate cyclase, agents having antithrombotic activity, agents lowering blood pressure, and agents altering lipid metabolisms.

10. Medicament according to Claim 8 or 9 for the treatment and/or prevention of heart failure, angina pectoris, hypertension, pulmonary hypertension, thromboembolic disorders, ischaemias, vascular disorders, microcirculation impairments, renal insufficiency, fibrotic disorders and arteriosclerosis

## Revendications

1. Composé de formule (I) 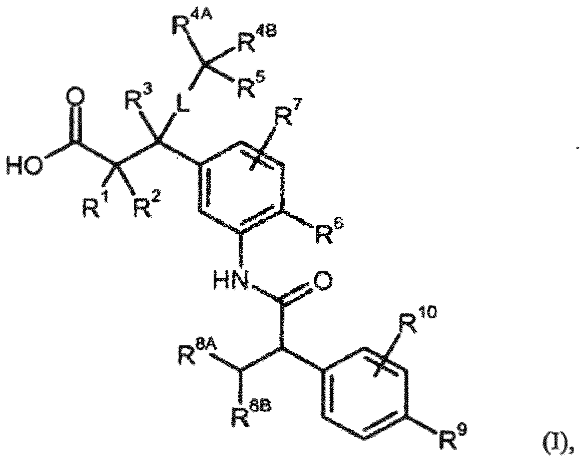 dans laquelle
R¹, R² et R³ représentent indépendamment les uns des autres hydrogène ou méthyle,
L représente une liaison ou -CH₂-,
R^{4A} et R^{4B} représentent indépendamment l'un de l'autre méthyle, trifluorométhyle ou éthyle,
ou
R^{4A} et R^{4B} sont reliés l'un avec l'autre et forment ensemble avec l'atome de carbone auquel ils sont reliés un cycle cyclopropyle ou cyclobutyle, qui peut être substitué jusqu'à deux fois avec fluor,
R⁵ représente hydrogène, fluor, méthyle ou méthoxy,
R⁶ représente hydrogène, fluor, chlore, brome, cyano, méthyle, trifluorométhyle, éthyle, méthoxy ou trifluorométhoxy,
R⁷ représente hydrogène, fluor, chlore ou méthyle,
R^{8A} représente méthyle ou éthyle,
R^{8B} représente trifluorométhyle,
ou
R^{8A} et R^{8B} sont reliés l'un avec l'autre et forment ensemble avec l'atome de carbone auquel ils sont reliés un cycle cyclopentyle éventuellement à substitution difluor de formule 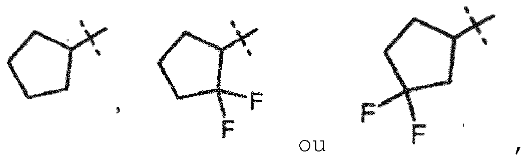
R⁹ représente fluor, chlore, brome, cyano, alkyle en (C₁-C₄), alcényle en (C₂-C₄), cyclopropyle ou cyclobutyle,
alkyle en (C₁-C₄) et alcényle en (C₂-C₄) pouvant être substitués jusqu'à trois fois avec fluor
et
cyclopropyle et cyclobutyle pouvant être substitués jusqu'à deux fois avec fluor,
et
R¹⁰ représente hydrogène, fluor, chlore, méthyle, trifluorométhyle, éthyle ou méthoxy,
ainsi que leurs sels, solvates et solvates des sels.

2. Composé de formule (I) selon la revendication 1,
dans lequel
R¹ représente hydrogène ou méthyle,
R² représente hydrogène,
R³ représente hydrogène ou méthyle,
L représente une liaison ou -CH₂-,
R^{4A} et R^{4B} représentent tous les deux méthyle ou sont reliés l'un avec l'autre et forment ensemble avec l'atome de carbone auquel ils sont reliés un cycle cyclopropyle ou cyclobutyle, qui peut être substitué jusqu'à deux fois avec fluor,
R⁵ représente hydrogène, fluor, méthyle ou méthoxy,
R⁶ représente fluor, chlore, méthyle ou éthyle,
R⁷ représente hydrogène ou fluor,
R^{8A} représente méthyle,
R^{8B} représente trifluorométhyle,
ou
R^{8A} et R^{8B} sont reliés l'un avec l'autre et forment ensemble avec l'atome de carbone auquel ils sont reliés un cycle cyclopentyle éventuellement à substitution difluor de formule 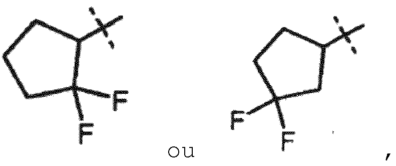
R⁹ représente fluor, chlore, alkyle en (C₁-C₄), alcényle en (C₂-C₃), cyclopropyle ou cyclobutyle,
alkyle en (C₁-C₄) et alcényle en (C₂-C₃) pouvant être substitués jusqu'à trois fois avec fluor
et
cyclopropyle et cyclobutyle pouvant être substitués jusqu'à deux fois avec fluor,
et
R¹⁰ représente hydrogène, fluor, chlore, méthyle ou méthoxy,
ainsi que leurs sels, solvates et solvates des sels.

3. Composé de formule (I) selon la revendication 1 ou 2, dans lequel
R¹ et R² représentent tous les deux hydrogène,
R³ représente hydrogène ou méthyle,
L représente une liaison ou -CH₂-,
R^{4A} et R^{4B} représentent tous les deux méthyle ou sont reliés l'un avec l'autre et forment ensemble avec l'atome de carbone auquel ils sont reliés un cycle cyclopropyle ou cyclobutyle, qui peut être substitué jusqu'à deux fois avec fluor,
R⁵ représente hydrogène, fluor ou méthyle,
R⁶ représente chlore,
R⁷ représente hydrogène,
R^{8A} représente méthyle,
R^{8B} représente trifluorométhyle,
R⁹ représente fluor, chlore, méthyle, trifluorométhyle, éthyle, 2,2,2-trifluoroéthyle, isopropyle, tert.-butyle, cyclopropyle ou 2,2-difluorocyclopropyle,
et
R¹⁰ représente hydrogène, fluor, méthyle ou méthoxy,
ainsi que leurs sels, solvates et solvates des sels.

4. Composé selon la revendication 1, correspondant à la formule suivante : 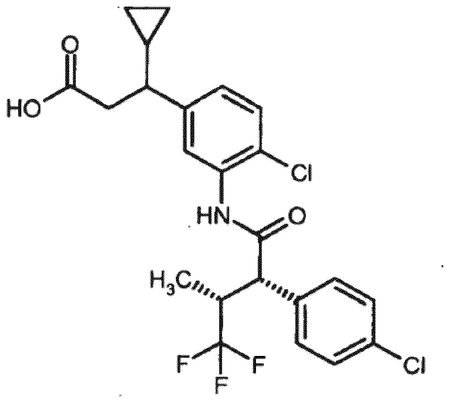 ainsi que ses sels, solvates et solvates des sels.

5. Procédé de fabrication d'un composé de formule (I), tel que défini dans les revendications 1 à 4, **caractérisé en ce qu'**un acide carboxylique de formule (II) 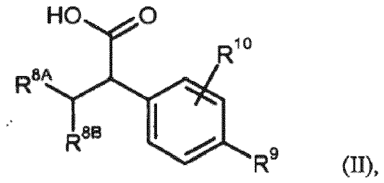 dans laquelle R^{8A}, R^{8B}, R⁹ et R¹⁰ ont les significations indiquées dans les revendications 1 à 4,
est couplé dans un solvant inerte à l'aide d'un agent de condensation ou par l'intermédiaire du chlorure d'acide carboxylique correspondant en présence d'une base avec une amine de formule (III) 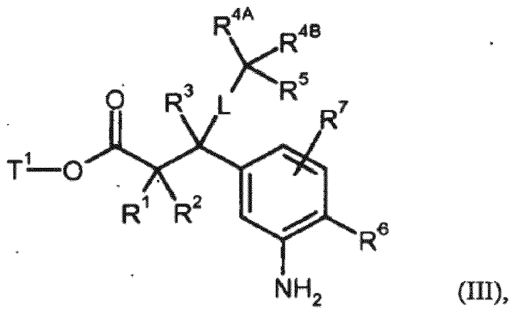 dans laquelle L, R¹, R², R³, R^{4A}, R^{4B}, R⁵, R⁶ et R⁷ ont les significations indiquées dans les revendications 1 à 4,
et
T¹ représente alkyle en (C₁-C₄) ou benzyle,
pour former un amide d'acide carboxylique de formule (IV) 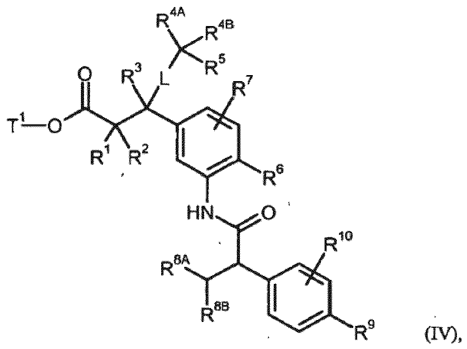 dans laquelle L, R¹, R², R³, R^{4A}, R^{4B}, R⁵, R⁶, R⁷, R^{8A}, R^{8B}, R⁹, R¹⁰ et T¹ ont les significations indiquées précédemment,
puis le radical ester T¹ est clivé par solvolyse basique ou acide ou, lorsque T¹ représente benzyle, également par hydrogénolyse pour obtenir l'acide carboxylique de formule (I),
et les composés de formule (I) sont éventuellement séparés par des méthodes connues de l'homme du métier en leurs énantiomères et/ou diastéréomères et/ou transformés avec (i) les solvants et/ou (ii) les bases appropriés en leurs solvates, sels et/ou solvates des sels.

6. Composé, tel que défini dans l'une quelconque des revendications 1 à 4, pour le traitement et/ou la prévention de maladies.

7. Utilisation d'un composé, tel que défini dans l'une quelconque des revendications 1 à 4, pour la fabrication d'un médicament pour le traitement et/ou la prévention de l'insuffisance cardiaque, de l'angine de poitrine, de l'hypertension, de l'hypertension pulmonaire, des maladies thromboemboliques, des ischémies, des maladies vasculaires, des troubles de la microcirculation, de l'insuffisance rénale, des maladies fibrotiques et de l'artériosclérose.

8. Médicament contenant un composé, tel que défini dans l'une quelconque des revendications 1 à 4, en combinaison avec un ou plusieurs adjuvants inertes, non toxiques, pharmaceutiquement appropriés.

9. Médicament contenant un composé, tel que défini dans l'une quelconque des revendications 1 à 4, en combinaison avec un ou plusieurs autres agents actifs choisis dans le groupe constitué par les nitrates organiques, les donneurs de NO, les inhibiteurs de cGMP-PDE, les stimulateurs de guanylate cyclase, les agents antithrombotiques, les agents abaissant la tension artérielle, ainsi que les agents modifiant le métabolisme des lipides.

10. Médicament selon la revendication 8 ou 9, pour le traitement et/ou la prévention de l'insuffisance cardiaque, de l'angine de poitrine, de l'hypertension, de l'hypertension pulmonaire, des maladies thromboemboliques, des ischémies, des maladies vasculaires, des troubles de la microcirculation, de l'insuffisance rénale, des maladies fibrotiques et de l'artériosclérose.
